(19) Europäisches Patentamt
European Patent Office
Office européen des brevets

(11) EP 4 421 068 A1

(12) # EUROPEAN PATENT APPLICATION
published in accordance with Art. 153(4) EPC

(43) Date of publication:
**28.08.2024 Bulletin 2024/35**

(21) Application number: **22882982.6**

(22) Date of filing: **21.10.2022**

(51) International Patent Classification (IPC):
***C07D 237/22*** *(2006.01)* ***C07D 237/34*** *(2006.01)*
***C07D 253/07*** *(2006.01)* ***C07D 401/12*** *(2006.01)*
***C07D 401/14*** *(2006.01)* ***C07D 403/12*** *(2006.01)*
***C07D 405/12*** *(2006.01)* ***C07D 471/04*** *(2006.01)*
***C07D 487/04*** *(2006.01)* ***C07D 491/048*** *(2006.01)*
***A61K 31/501*** *(2006.01)* ***A61K 31/502*** *(2006.01)*
***A61K 31/5025*** *(2006.01)* ***A61K 31/53*** *(2006.01)*
***A61P 29/00*** *(2006.01)* ***A61P 19/02*** *(2006.01)*
***A61P 17/00*** *(2006.01)*

(52) Cooperative Patent Classification (CPC):
**A61K 31/495; A61K 31/501; A61K 31/502; A61K 31/5025; A61K 31/53; A61P 1/00; A61P 1/16; A61P 3/10; A61P 9/10; A61P 11/00; A61P 11/06; A61P 13/12; A61P 17/00; A61P 17/06; A61P 19/02;** (Cont.)

(86) International application number:
**PCT/CN2022/126702**

(87) International publication number:
**WO 2023/066377 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.10.2021 CN 202111233736**
**18.03.2022 CN 202210273508**

(71) Applicant: **Zhejiang Aixplorer Biotech Co., Ltd.**
**Jiaxing, Zhejiang 314031 (CN)**

(72) Inventors:
- **XU, David Daqiang**
  **Jiaxing, Zhejiang 314031 (CN)**
- **ZHANG, Suoming**
  **Jiaxing, Zhejiang 314031 (CN)**
- **DANG, Kuifeng**
  **Jiaxing, Zhejiang 314031 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **NITROGEN-CONTAINING COMPOUND, PREPARATION METHOD THEREFOR AND APPLICATION THEREOF**

(57) A compound represented by formula (I), a solvate thereof, a pharmaceutically acceptable salt thereof, or a solvate of the pharmaceutically acceptable salt thereof, a preparation method therefor and an application thereof. The nitrogen-containing compound has good inhibitory activity against NLRP3.

EP 4 421 068 A1

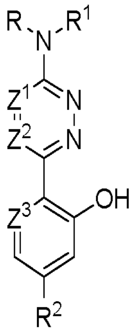

I

(52) Cooperative Patent Classification (CPC): (Cont.)
**A61P 19/06; A61P 21/00; A61P 25/16; A61P 25/28; A61P 29/00; A61P 35/00; C07D 237/22; C07D 237/26; C07D 237/34; C07D 253/07; C07D 401/04; C07D 401/12; C07D 401/14; C07D 403/12; C07D 405/12; C07D 471/04; C07D 487/04; C07D 491/048**

## Description

The present application claims the priority of Chinese patent application No. 202111233736.X filed on October 22, 2021 and Chinese patent application No. 2022102735083 filed on March 18, 2022. The Chinese patent applications are incorporated herein by reference in its entirety.

TECHNICAL FIELD

The present invention belongs to the technical field of organic synthesis and specifically relates to a group of NLRP3 inhibitors and their uses.

BACKGROUND

Inflammatory response is a common physiological and pathological activity in the body, and inflammasomes play an important regulatory role in this response. Nucleotide-binding oligomerization domain-like receptor protein 3 (NOD-like receptor protein 3, NLRP3) is one of the key regulatory proteins in inflammasomes. NLRP3 inflammasomes are a multiprotein complex consisting of the NLRP3 protein itself, cysteinyl asparagin-1, and an apoptosis- associated speck-like protein containing CARD (ASC), which recognizes a wide range of pathogenic microorganisms and stress-related endogenous signaling molecules.

Some studies have found that activation of NLRP3 and their associated molecular regulatory signaling pathways are closely associated with the development and progression of a variety of diseases. For example, abnormal activation of NLRP3 inflammasome has been associated with the development of various inflammatory diseases such as Muckle-Wells syndrome (MWS), familial cold autoinflammatory syndrome, neonatal-onset multisystemic inflammatory diseases, Alzheimer's disease, Parkinson's disease, nonalcoholic fatty liver disease, atherosclerosis, asthma, nephropathy, enterocolitis, neoplasia, gout, neurodegenerative diseases, diabetes, and obesity. Therefore, the diseases related to the activation of NLRP3 and its related molecular regulatory signaling pathway have received more and more attention, and is a hot spot for drug research and development.

Current drugs for the treatment of NLRP3-related diseases include the recombinant IL-1 receptor antagonist anakinra, the IL-1β-neutralizing antibody canakinumab, and the soluble IL-1 receptor-trapping agent rilonacept, all of which are biologics. Some small molecule inhibitors of NLRP3 have been reported in recent years, e.g., glibenclamide, parthenolide, and 3,4-methylenedioxy-beta-nitrostyrene. Patent documents WO2021193897, WO2020234715, WO2018015445, WO2018215818 disclose a series of NLRP3 inhibitors. However, the above drugs or drug candidates are still limited by their low specificity or poor activity or high safety risks. Therefore, there is a need to develop a new generation of small molecule NLRP3 inhibitors with high specificity and activity for the treatment of autoimmune diseases caused by the aberrant activation of NLRP3.

CONTENTS OF THE PRESENT INVENTION

The present invention addresses the relatively simple and limited structures of the existing NLRP3 inhibitors, and aims to provide a group of nitrogen-containing compounds, their preparation methods and applications. These compounds have good inhibitory activity against NLRP3.

The present invention provides a group of compounds represented by General Formula I, the solvates thereof, the pharmaceutically acceptable salts thereof or the solvates of the pharmaceutically acceptable salts thereof:

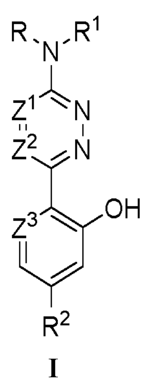

**I**

Among them, $Z^1$, $Z^2$ and $Z^3$ are independently N or $CR^Z$;

Each $R^Z$ is independently H, halogen, or $C_1$-$C_6$ alkyl;

Or, $Z^1$ and $Z^2$ are $CR^Z$, and the $R^Z$ on $Z^1$ and $Z^2$ together form a ring Y with the carbon connected to it. The ring Y is a $C_5$~$C_6$ cycloalkenyl, a 5-6-membered heterocycloalkenyl, a benzene, a 5-6-membered heteroaromatic ring, a $C_5$~$C_6$ cycloalkenyl substituted by one or more $R^{Z-1}$, a 5- to 6-membered heterocycloalkenyl substituted by one or more $R^{Z-2}$, a one or more $R^{Z-3}$-substituted benzene ring, or a one or more $R^{Z-4}$-substituted 5- to 6-membered heteroaromatic ring;

Among them, the numbers of $R^{Z-1}$, $R^{Z-2}$, $R^{Z-3}$ and $R^{Z-4}$ are denoted as a, b, c and d, respectively;

$R^{Z-1}$, $R^{Z-2}$, $R^{Z-3}$ and $R^{Z-4}$ are independent $C_1$~$C_6$ alkyls, $C_1$~$C_6$ alkoxy or halogens;

R is H;

R1 is $C_1$~$C_6$ alkyl, $C_3$~$C_6$ cycloalkyl, 3-10-membered heterocycloalkyl, $C_1$~$C_6$ alkyl group substituted by one or more $R^{1-1}$, $C_3$~$C_6$ cycloalkyl group substituted by one or more $R^{1-2}$ or 3~10-memberedheterocycloalkyl group substituted by one or more $R^{1-3}$;

$R^{1-1,}$ $R^{1-2}$ and $R^{1-3}$ are independently $-NH_2$, -OH, $C_1$~$C_6$ alkyl, $C_3$~$C_6$ cycloalkyl, 3-6-memberedheterocycloalkyl, $-(S=O)_2-C_1$~$C_6$ alkyl, $C_1$~$C_6$ alkyl group substituted by one or more $R^a$, $C_3$~$C_6$ cycloaklyl group substituted by one or more $R^b$, or 3~6-memberedmembered heterocycloalkyl group substituted by one or more $R^g$;

$R^a$, $R^b$, and $R^g$ are independently -OH, -COOH, $-(C=O)NH_2$, $-(C=O)NHR^c$, $C_3$~$C_6$ cycloalkyl, or $C_3$~$C_6$ cycloalkyl group substituted by one or more $R^d$s

$R^c$ and R $^d$are independently -OH, $C_1$~$C_6$ alkyl, $C_1$~$C_6$ alkyl group substituted by one or more $R^e$, $C_3$~$C_6$ cycloalkyl group, or $C_3$~$C_6$ cycloalkyl group substituted by one or more $R^f$s

$R^e$ and $R^f$ are independently -OH or $C_1$~$C_6$ alkyls;

$R^2$ is a halogen, $C_1$~$C_6$ alkyl group or $C_1$~$C_6$ alkyl group substituted by one or more halogens;

In the 5-6-membered heterocycloalkenyl group, 5-6-membered heteroaromatic ring, and 3~10-membered heterocycloalkyl group and 3~6-membered heterocycloalkyl group, the heteroatom is independently one or more of N, O and S, and the number of heteroatoms is 1, 2 or 3 independently;

The compound shown in general formula I satisfies at least one of the following conditions:

(1) At least one of $Z^1$, $Z^2$ and $Z^3$ is N;

(2) $Z^1$ and $Z^2$ are $CR^Z$s, and the $R^Z$s on $Z^1$ and $Z^2$ form a ring Y together with the carbon connected to them.

In one embodiment, the numbers of $R^{Z-1,}$ $R^{Z-2,}$ $R^{Z-3}$ and $R^{Z-4}$ are denoted a, b, c and d, respectively, where a, b, c and d may be independently 1, 2 or 3.

In an embodiment, in $R^Z$, the $C_1$-$C_6$ alkyl group may be a $C_1$-$C_3$ alkyl group, such as a methyl group.

In an embodiment, in $R^Z$, the halogen may be F, Cl, Br or I, such as F or Cl.

In an embodiment, each $R^Z$ can be independent of H, F, Cl, or $-CH_3$.

In an embodiment, in the ring Y, the $C_5$~$C_6$ cycloalkyl ring can be independent of cyclopentyl, and at this moment, the structure in formula I

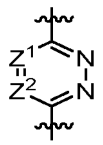

can be

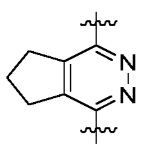

or

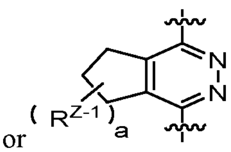

.

In an embodiment, in the ring Y, the 5~6-membered heterocyclic ring can be independently a dihydrofuran ring, and at this time, the structure in formula I

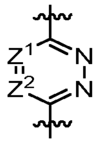

can be

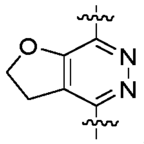, 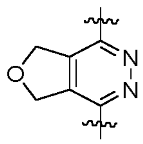

or

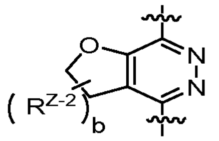.

In an embodiment, in the ring Y, the 5~6-membered heterocyclic ring can be independently a dihydrofuran ring, and at this time, the structure in formula I

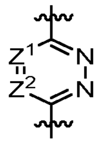

can be

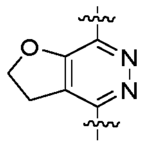, 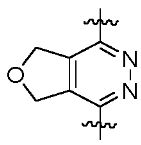

or

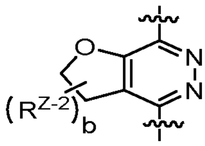 or 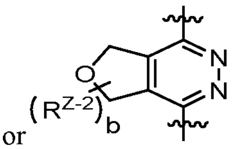.

In an embodiment, in the ring Y, the 5~6-membered heteroaromatic ring can be independently furan ring, pyridine ring or pyrrole ring, and at this moment, the structure in formula I

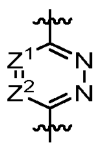

can be

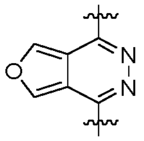,

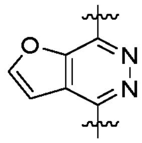, 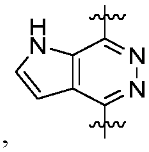, 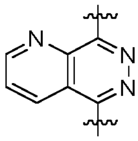, 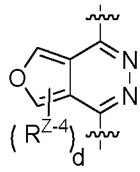, 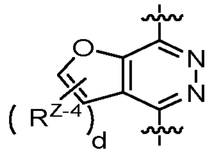, 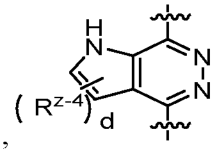, 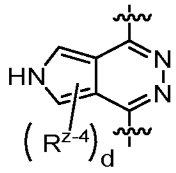,

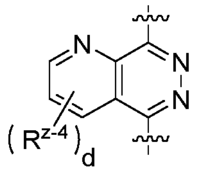

In an embodiment

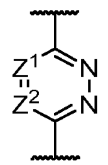

may be

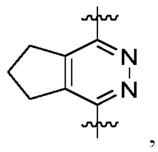, 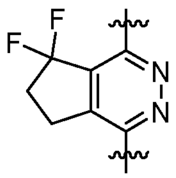, 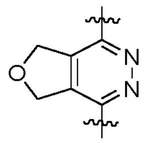, 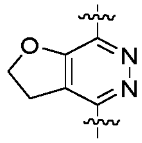,

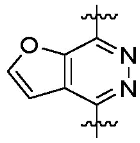, 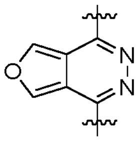, 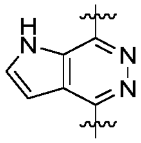, 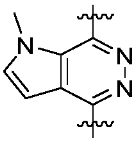, 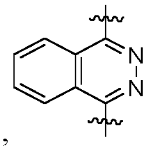, 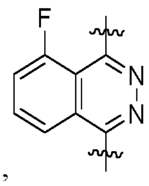, 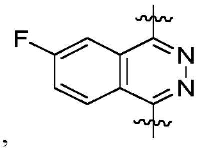,

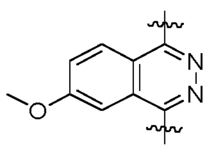, 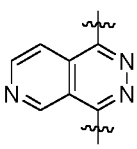, 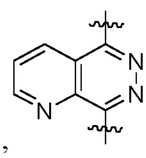.

In an embodiment, in $R^{Z-1,}$ $R^{Z-2,}$ $R^{Z-3}$ and $R^{Z-4}$ the $C_1$~$C_6$ alkyl group can be an alkyl group of $C_1$-$C_3$ independently, such as a methyl group.

In an embodiment, in $R^{Z-1,}$ $R^{Z-2,}$ $R^{Z-3}$ and $R^{Z-4}$, the alkoxy group of described $C_1$~$C_6$ can be independently $C_{1\sim}C_3$ alkoxy group, such as methoxy group.

In an embodiment, in $R^{Z-1,}$ $R^{Z-2,}$ $R^{Z-3}$ and $R^{Z-4}$, the halogen may be F, Cl, Br or I independently, such as F.

In one embodiment, $R^{Z-1}$ is F.

In one embodiment, $R^{Z-3}$ is either methoxy or fluorine.

In one embodiment, $R^{Z-4}$ is methyl.

In an embodiment, the carbon atom connected to N in Formula I in $R^1$ is chiral, and the configuration of the carbon atom is preferably the R configuration; when $R^1$ is a $C_1$~$C_6$ alkyl group substituted by one or more $R^{1-1}$s, a $C_3$~$C_6$ cycloalkyl group substituted by one or more $R^{1-2}$ or a 3-10-membered heterocycloalkyl group substituted by one or more $R^{1-3}$s, the compound shown in Formula I can be

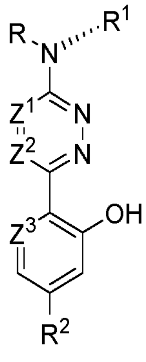

I

.

In one embodiment, in $R^1$, the $C_1$~$C_6$ alkyl group can be independently $C_1$~$C_3$ alkyl group, such as methyl group.

In one embodiment, in $R^1$, the $C_3$~$C_6$ cycloalkyl group can be independently cyclopropyl, cyclobutyl, cyclopentyl or cyclohexyl, preferably cyclobutyl or cyclohexyl.

In one embodiment, in $R^1$, the 3-10-membered heterocycloalkyl group can be a single ring or a bicyclic 3-10-membered hetero-alkyl group independently; when the 3~10-memberedhetero-alkyl group is a bicyclic hetero-alkyl group, it can be a 6-membered heterocyclic and 5-membered hetero-alkyl group, such as piperidine ring and tetrahydropyrrole ring, preferably as

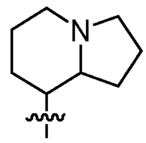

;

when the described 3-10-membered heterocyclic alkyl group is a monocyclic hetero-alkyl group, it can be 3~6-membered hetero-alkyl group, preferably 5-6-membered hetero-alkyl group, such as piperidinyl group, more for example

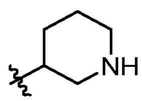

.

In a certain embodiment, in $R^{1-1}$, $R^{1-2}$ and $R^{1-3}$, the $C_1$~$C_6$ alkyl group can be methyl, ethyl, n-propyl, isopropyl, tert-butyl or isobutyl, independently.

In a certain embodiment, in $R^{1-1}$, $R^{1-2}$ and $R^{1-3}$, the $C_3$~$C_6$ cycloalkyl group can be independently cyclopropyl or cyclobutyl.

In a certain embodiment, in $R^{1-1}$, $R^{1-2}$ and $R^{1-3}$, the 3~6-membered heterocycloalkyl group can be independently a 5-6-membered heterocycloalkyl group, such as tetrahydropyrrole, more for example

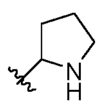

.

In a certain embodiment, in $R^a$ and $R^b$, the $C_3$~$C_6$ cycloalkyl group can be independently cyclopropyl.

In a certain embodiment, in $R^c$ and $R^d$, the $C_1$~$C_6$ alkyl group can be independently $C_1$~$C_3$ alkyl group, such as isopropyl group.

In a certain embodiment, in $R^c$ and $R^d$, the $C_3$~$C_6$ cycloaklyl group can be independently cyclobutyl.

In a certain embodiment, in $R^e$ and $R^f$, the $C_1$~$C_6$ alkyl group can be independently $C_1$~$C_3$ alkyl group, such as methyl group.

In an embodiment, $R^1$ may be any of the following structures:

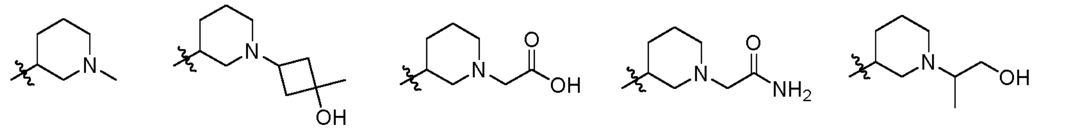

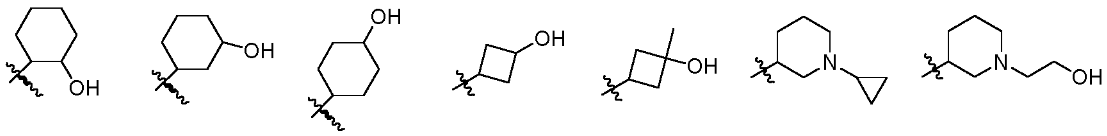

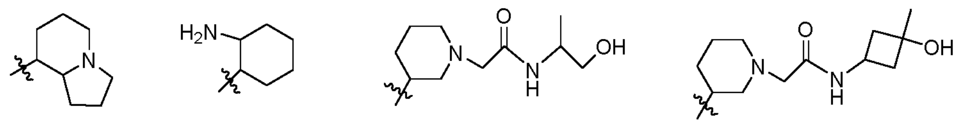

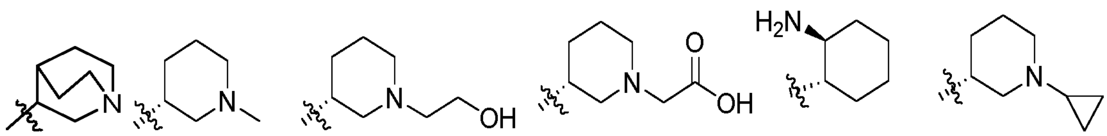

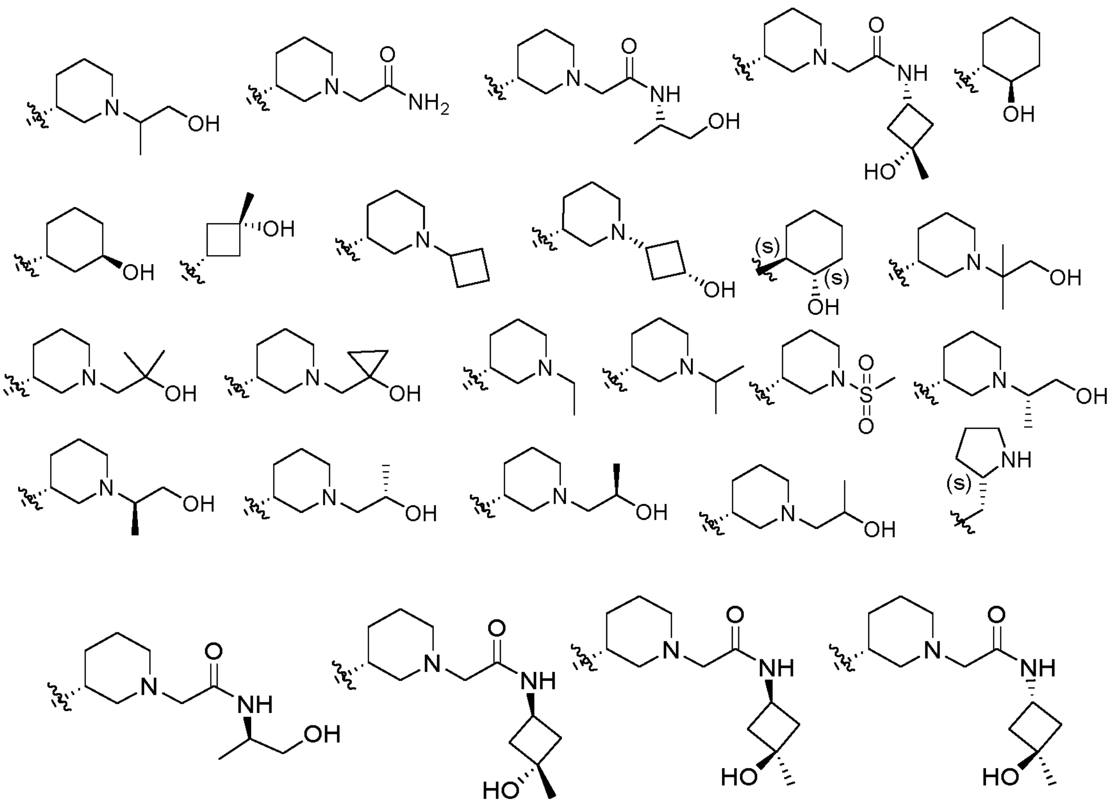

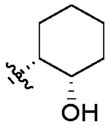 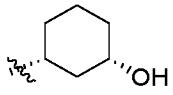 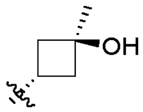 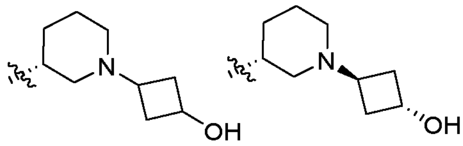

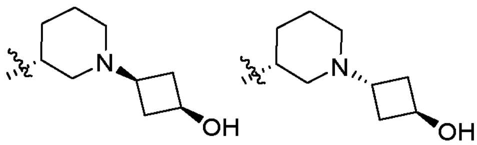 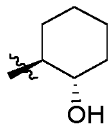 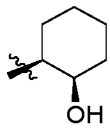 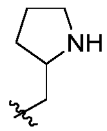 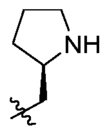 .

In one embodiment, in $R^2$, the halogen may be F, Cl, Br or I independently, e.g. For Cl.

In a certain embodiment, in $R^2$, the $C_1$~$C_6$ alkyl group can be independently $C_1$~$C_3$ alkyl group, such as methyl group.

In one embodiment, $R^2$ may be $-CH_3$, Cl, or $-CF_3$.

In an embodiment, $Z^1$ is N.

In one embodiment, $Z^2$ and $Z^3$ are independent $CR^Z$s.

In one embodiment, each $R^Z$is independently of H or $C_1$-$C_6$ alkyl groups.

In a certain embodiment, when Z'and $Z^2$ are $CR^Z$, and the $R^Z$ on $Z^1$ and $Z^2$ and the carbon connected to them together form a ring Y, the ring Y is a benzene ring, a 5-6-membered heteroaromatic ring, a benzene ring substituted by one or more $R^{Z-3}$ or a 5-6-membered heteroaromatic ring substituted by one or more $R^{Z-4}$.

In a certain embodiment, when $Z^1$and $Z^2$ are $CR^Z$, and the $R^Z$ on $Z^1$ and $Z^2$ and the carbon connected to them together form a ring Y, the ring Y is a benzene ring, a 5-6-membered heteroaromatic ring, a benzene ring substituted by one or more $R^{Z-3}$ or a 5-6-membered heteroaromatic ring substituted by one or more $R^{Z-4}$, and the $R^{Z-3}$ and $R^{Z-4}$ are halogens independently.

In an embodiment, R'is a C3-C6 cycloalkyl group, a 3-10-membered heterocycloalkyl group, a C3-C6 cycloalkyl group substituted by one or more $R^{1-2}$ groups, or a 3-10-membered heterocycloalkyl group substituted by one or more $R^{1-3}$ groups.

In an embodiment, $R^{1-2}$ and $R^{1-3}$ are independently -OH, $C_1$~$C_6$ alkyl, $C_3$~$C_6$ cycloalkyl or $C_1$~$C_6$ alkyl group substituted by one or more $R^a$s.

In one embodiment, $R^a$ is -OH independently.

In an embodiment, the compound shown in General Formula I,

$Z^1$ is N;

$Z^2$ and $Z^3$ are independently for $CR^Z$;

Each $R^Z$ is independently of H or $C_1$-$C_6$ alkyl group;

Alternatively, $Z^1$ and $Z^2$ are $CR^Z$, and the $R^Z$ on $Z^1$ and $Z^2$ forms a ring Y together with the carbon connected to it, and the ring Y is a benzene ring, a 5-6-membered heteroaromatic ring, a benzene ring substituted by one or more $R^{Z-3}$ or a 5-6-membered heteroaromatic ring replaced by one or more $R^{Z-4}$s;

$R^{Z-3}$ and $R^{Z-4}$ are halogens independently;

R is H;

$R^1$ is $C_3$~$C_6$ cycloalkyl, 3-10-membered heterocycloalkyl, C3-C6 cycloalkyl group substituted by one or more $R^{1-2}$ or 3-10-membered heterocycloalkyl group substituted by one or more $R^{1-3}$;

$R^{1-2}$ and $R^{1-3}$ are independently -OH, $C_1$~$C_6$ alkyl, $C_3$~$C_6$ cycloalkyl or $C_1$~$C_6$ alkyl group substituted by one or more $R^a$;

$R^a$ is -OH independently.

In an embodiment, at least one of $Z^1$, $Z^2$ and $Z^3$ is N.

In an embodiment, $Z^1$ is N.

In an embodiment, $Z^2$ is N.

In one embodiment, Z3 is N.

In an embodiment, the compound shown in general formula I is the compound shown in general formula I-1

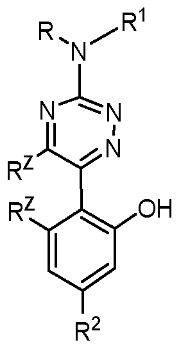

I-1

.

In an embodiment, the compound shown in general formula I is a compound as shown in General formula I-1

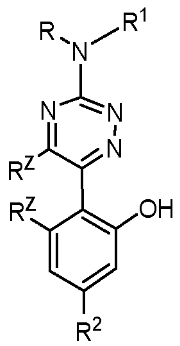

I-1

;

Wherein, $R^1$ is a $C_3$~$C_6$ cycloalkyl group substituted by one or more $R^{1-2}$s, $R^{1-2}$ is $-NH_2$ or -OH independently, and preferably, the $R^{1-2}$ is located at the ortho position of the linker group in R1

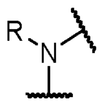

R is H;
Each $R^Z$ is independently of H or $C_1$-$C_6$ alkyl group;
$R^2$ is a $C_1$~$C_6$ alkyl group substituted by one or more halogens.

In an embodiment, the compound shown in general formula I is a compound as shown in formula I-1

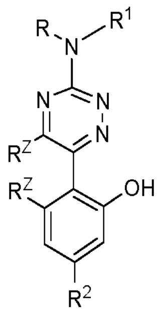

I-1

;

wherein $R^1$ is a cyclohexyl group substituted by one or more $R^{1-2}$s, $R^{1-2}$ is $-NH_2$ or -OH independently, and preferably,

the $R^{1-2}$ is located

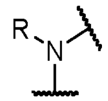

in the ortho position of the linker group in $R^1$
R is H;
Each $R^Z$ is independently of H or $C_1$-$C_6$ alkyl group;
$R^2$ is a $C_1$~$C_6$ alkyl group substituted by one or more halogens.

In an embodiment, the compound shown in General formula I is the compound shown in General formula I-2

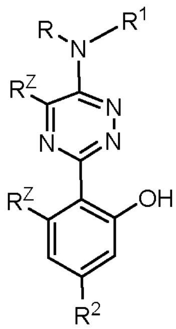

**I-2**

.

In an embodiment, the compound shown in General formula I is the compound shown in General formula I-3

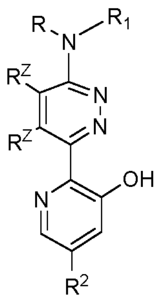

**I-3**

.

In an embodiment, the compound shown in General formula I is the compound shown in General formula I-4

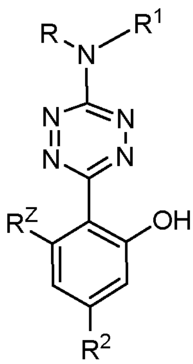

**I-4**

.

In an embodiment, the compound shown in General formula I is the compound shown in General formula I-5

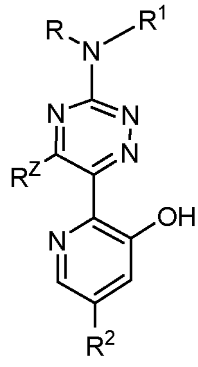

**I-5**

In one embodiment, $Z^1$ and $Z^2$ are $CR^Z$, and $R^Z$ on $Z^1$ and $Z^2$ together form a ring Y with the carbon connected to them.

In an embodiment, the compound shown in General formula I is the compound shown in General formula **I-6**

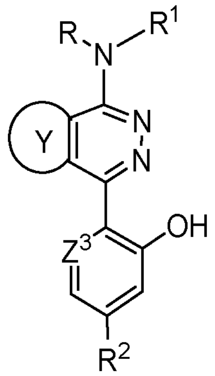

**I-6**

In an embodiment, the compound shown in General formula I is any of the following:

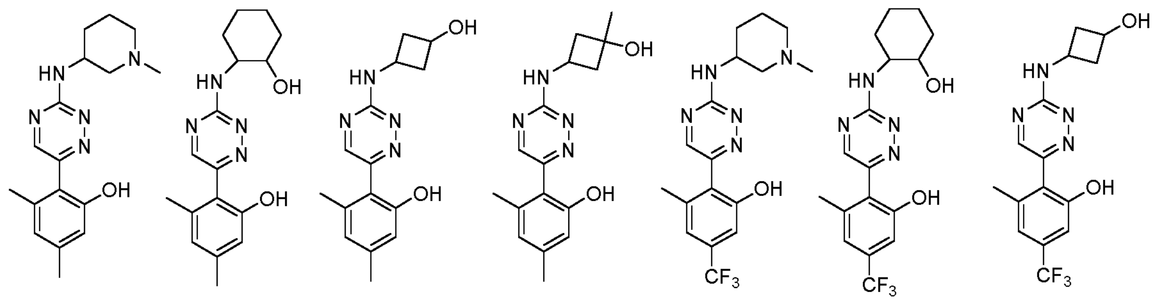

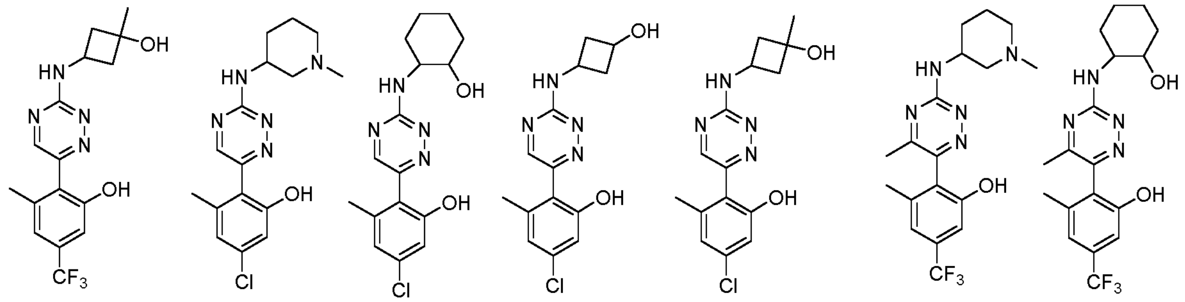

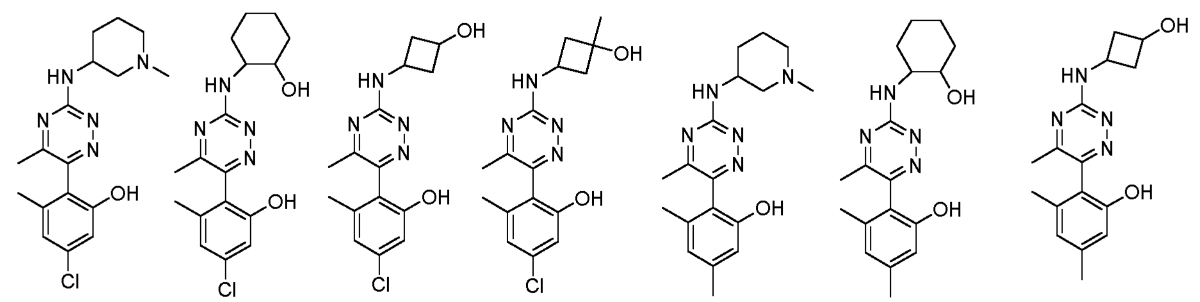

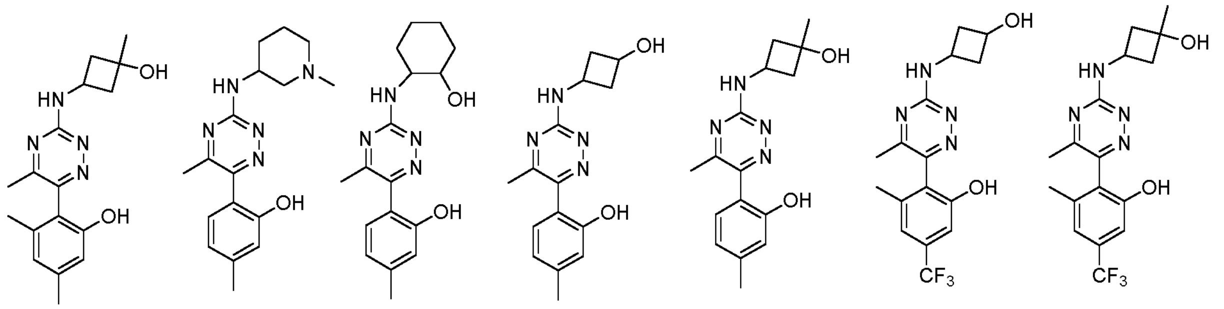

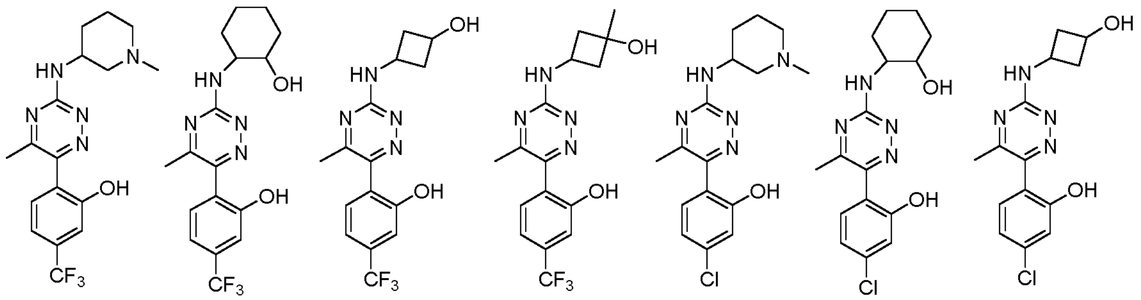

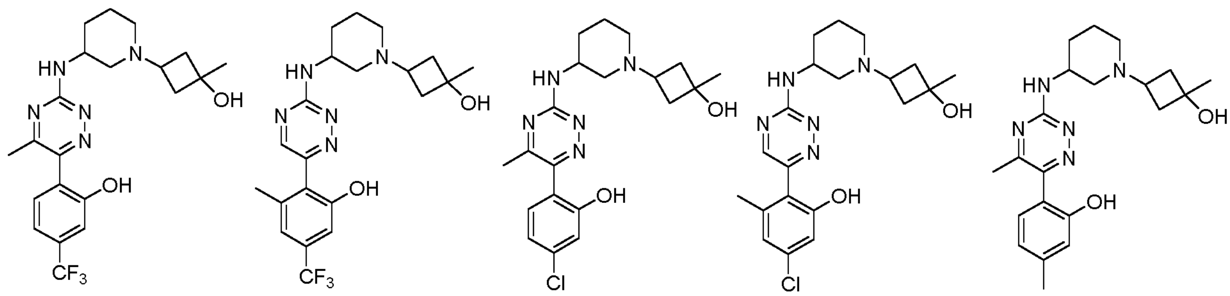

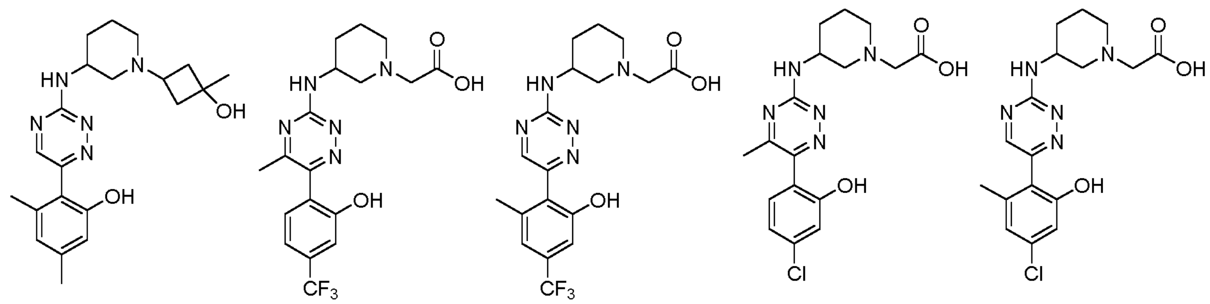

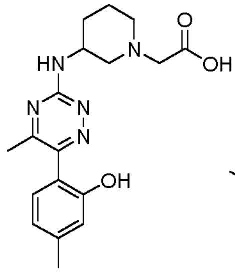
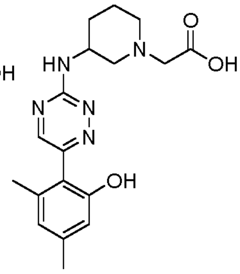
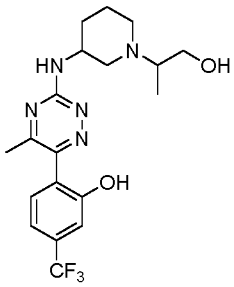
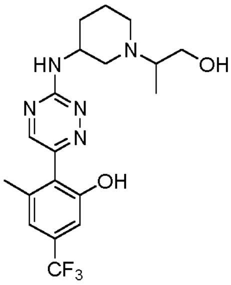
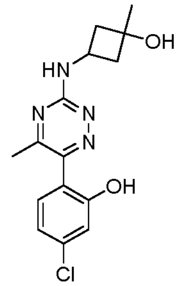

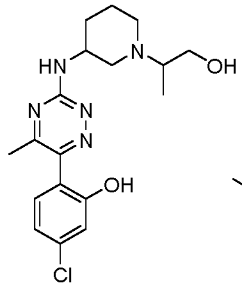
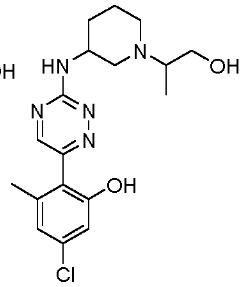
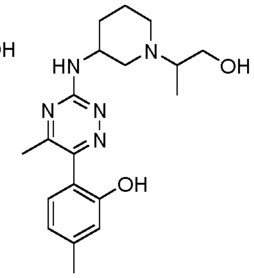
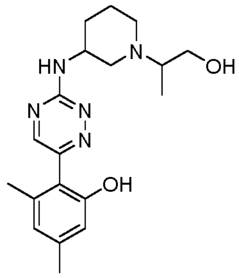
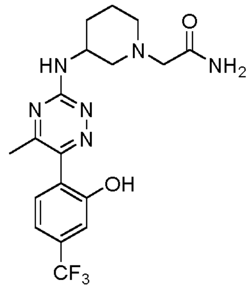

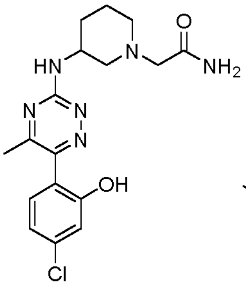
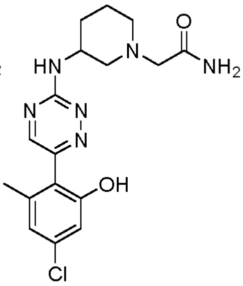
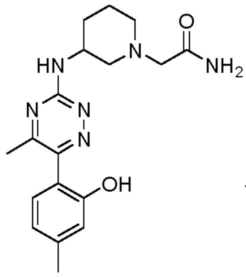
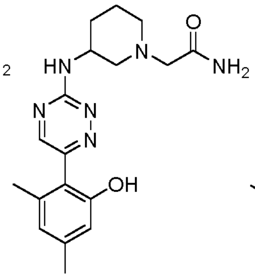
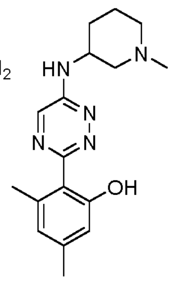

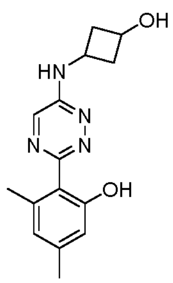
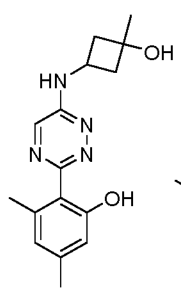
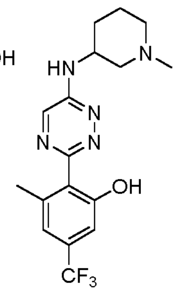
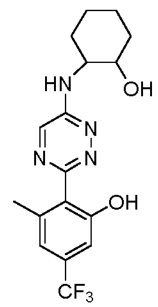
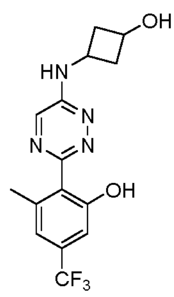
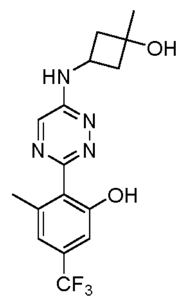

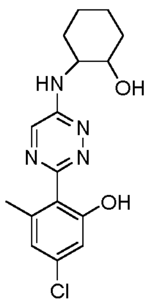
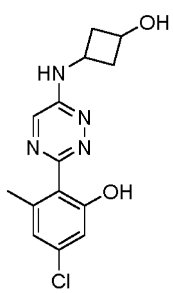
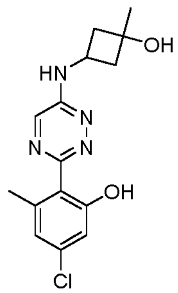
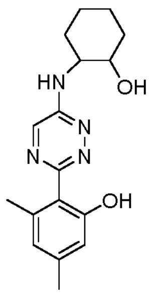
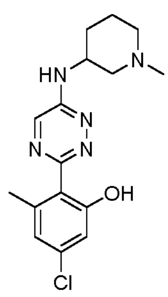
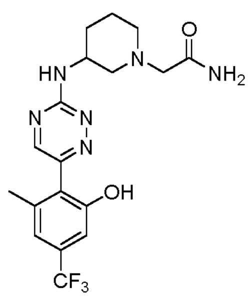

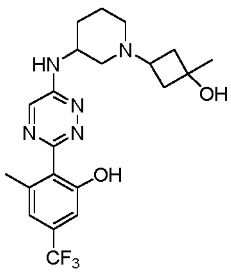
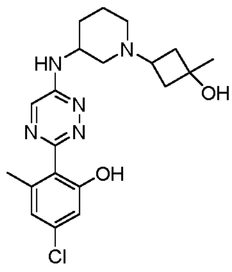
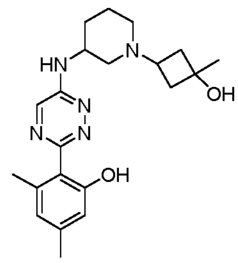
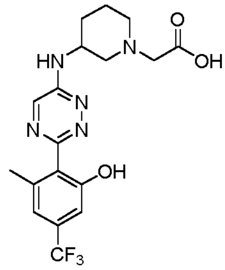
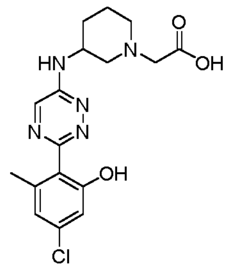

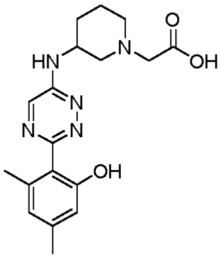
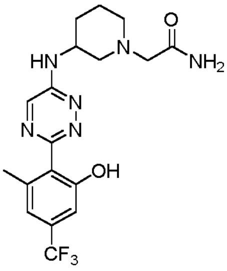
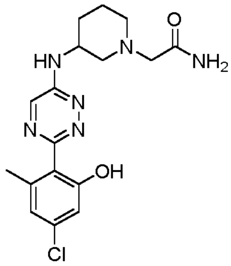
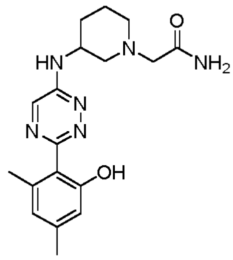
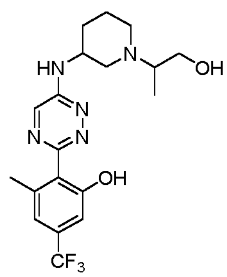

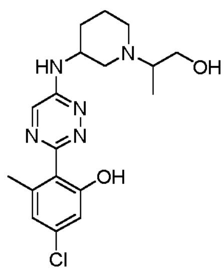
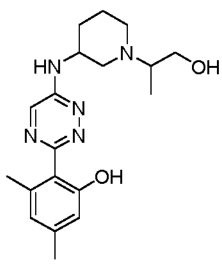
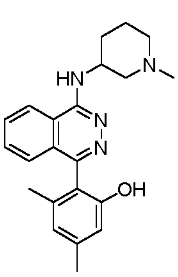
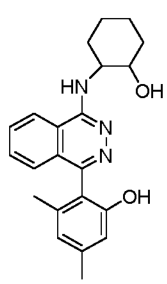
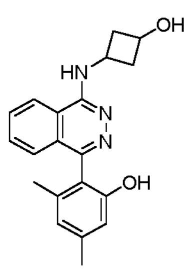

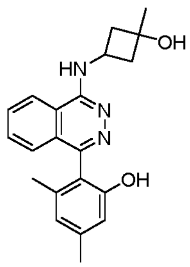
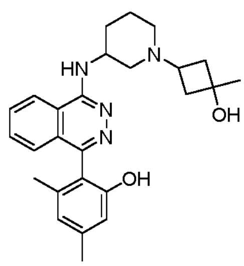
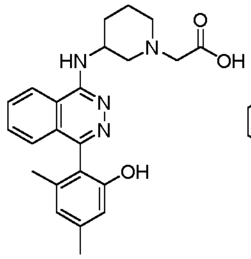
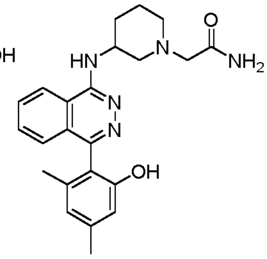
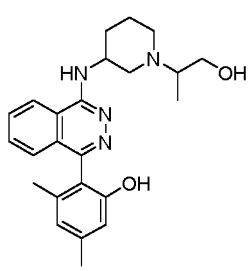

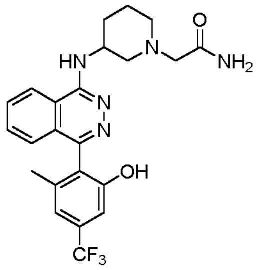
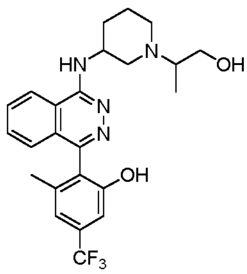
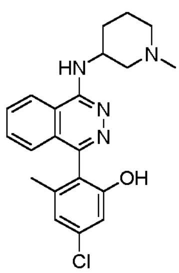
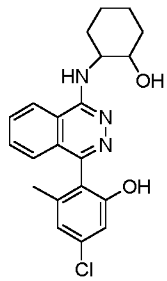
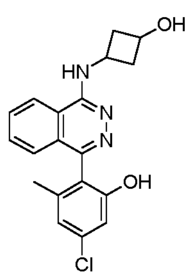

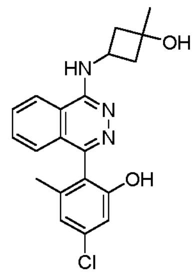
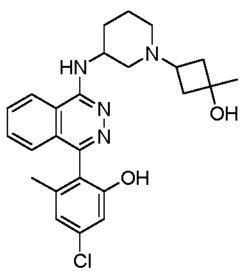
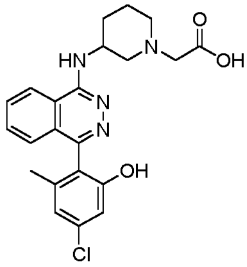
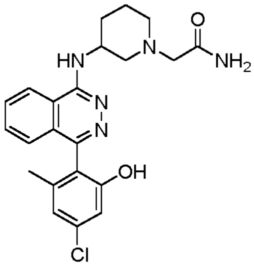
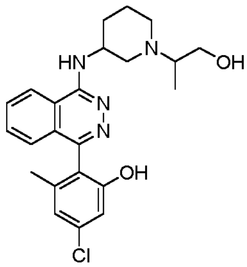

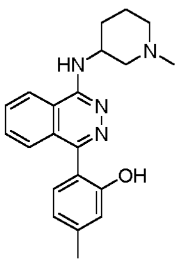
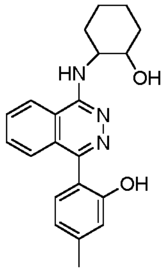
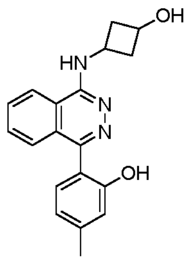
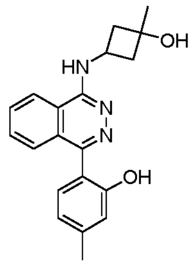
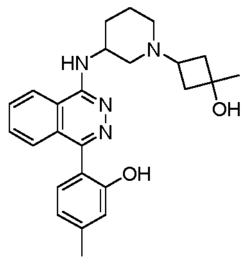

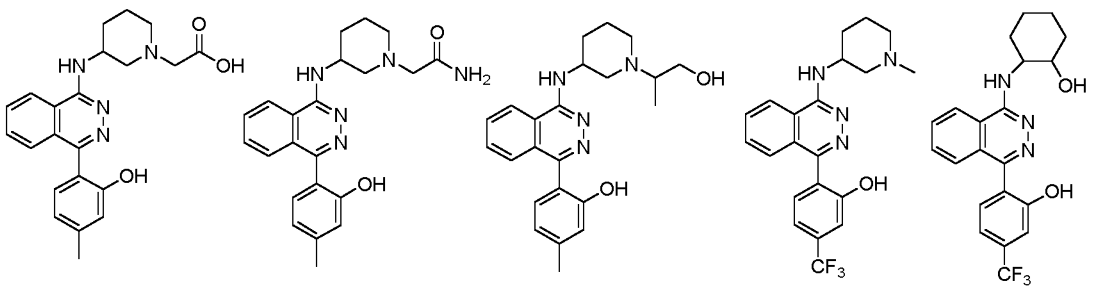

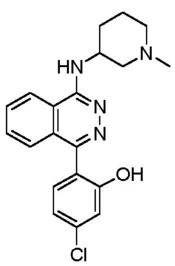
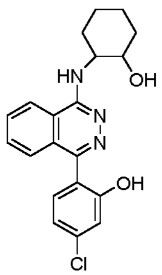
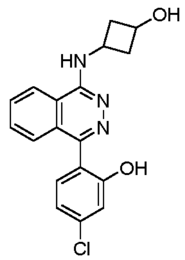
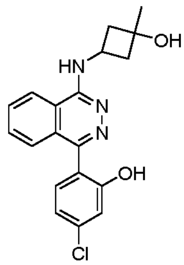
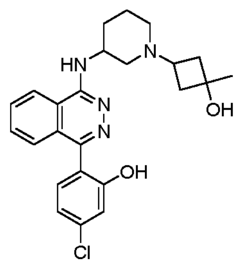
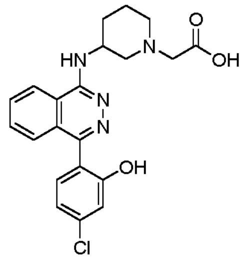

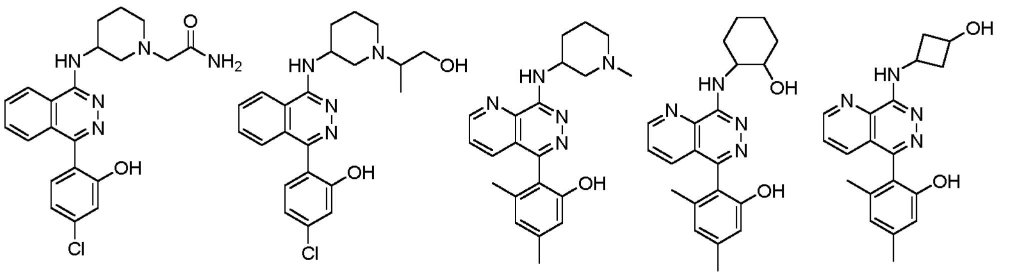

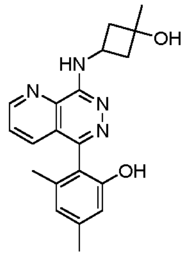
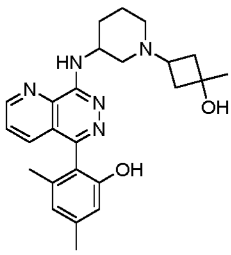
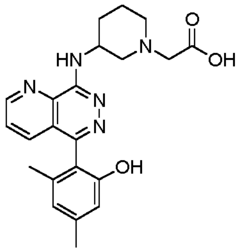
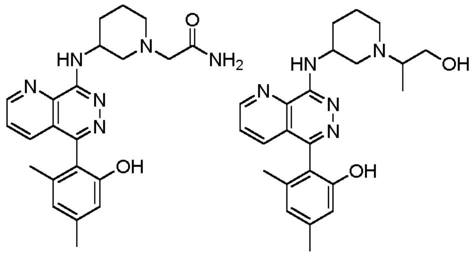

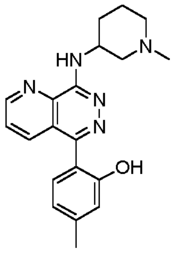
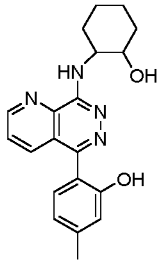
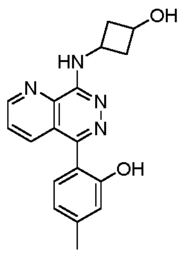
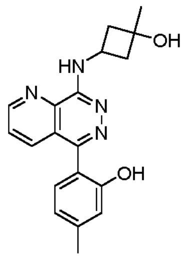
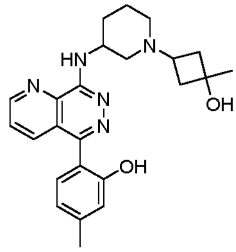
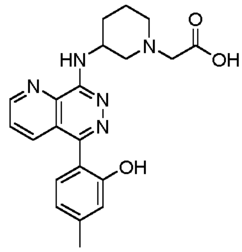

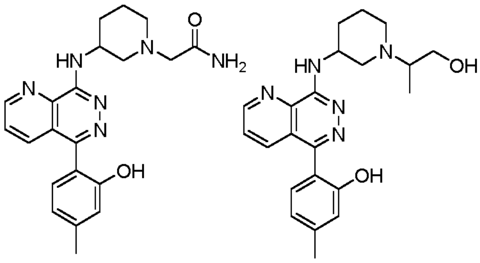
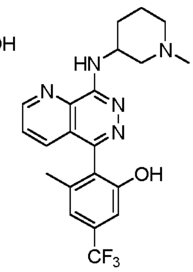
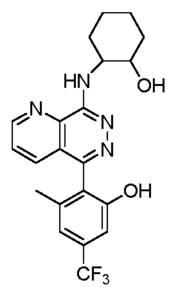
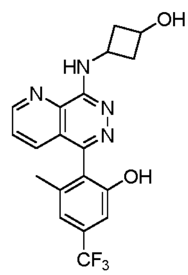
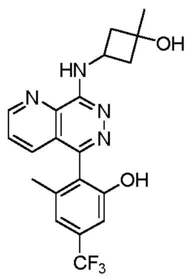

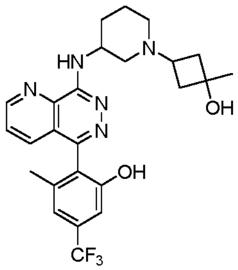
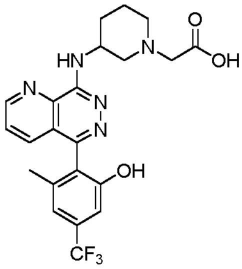
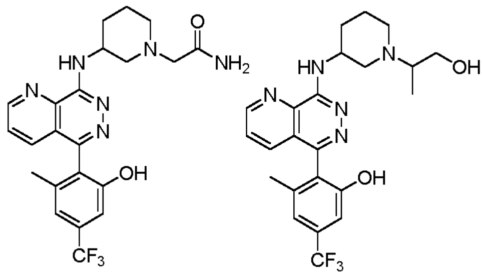
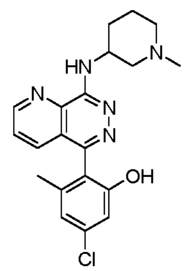

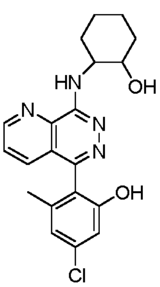
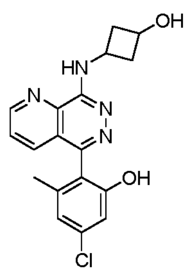
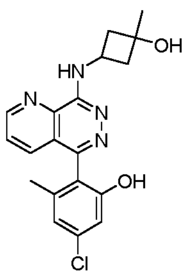
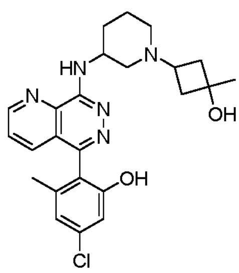
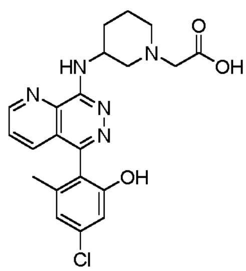

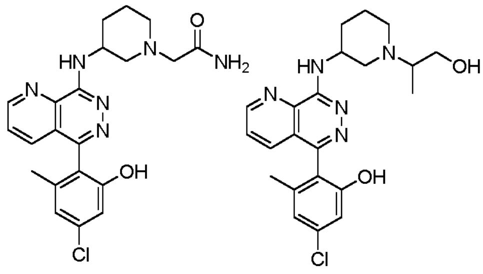
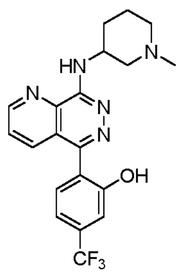
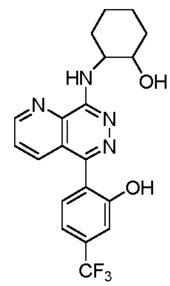
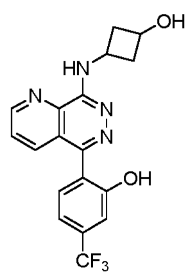

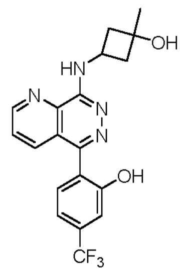
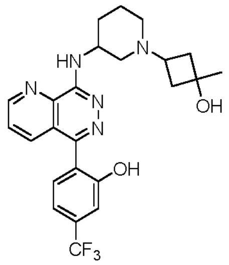
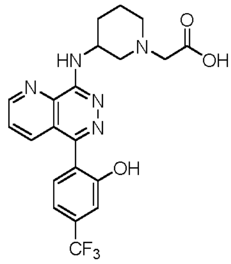
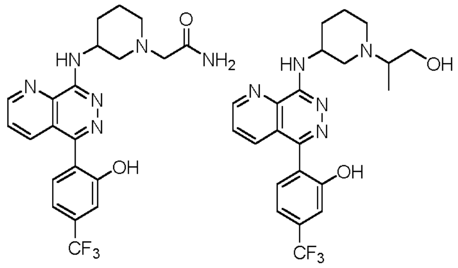

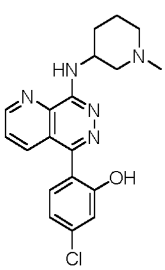
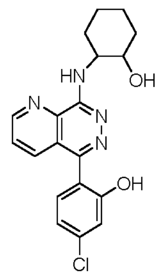
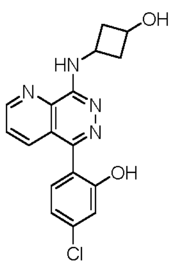
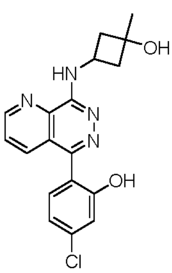
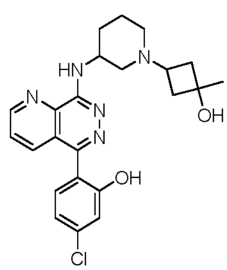
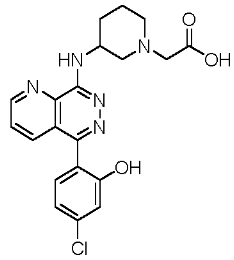

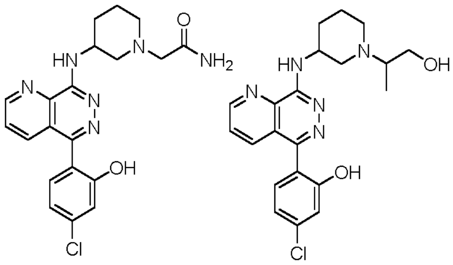
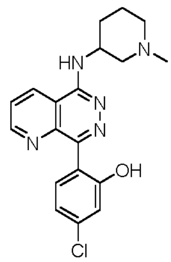
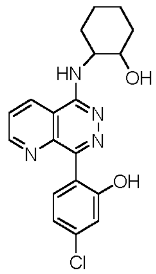
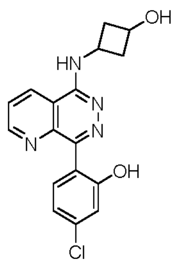
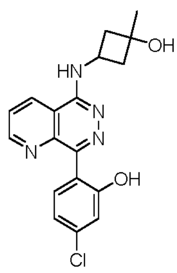

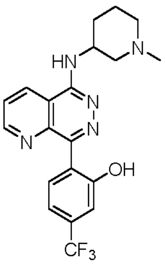
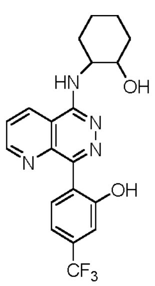
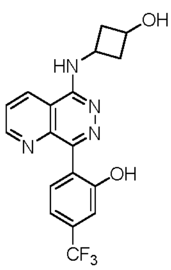
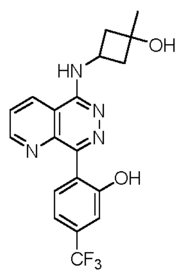
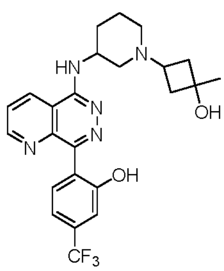
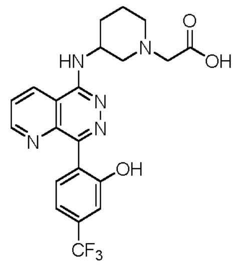

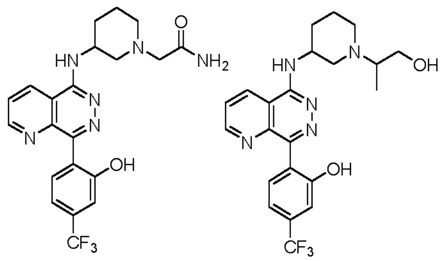
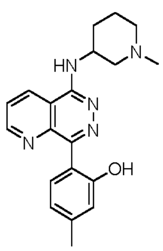
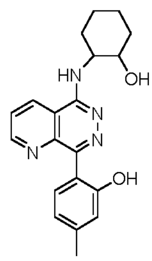
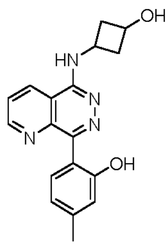
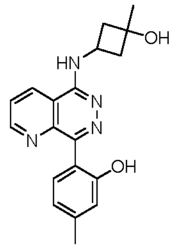

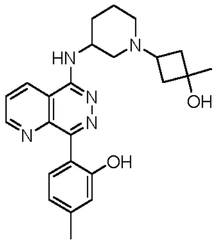
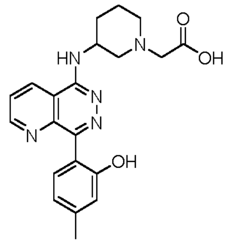
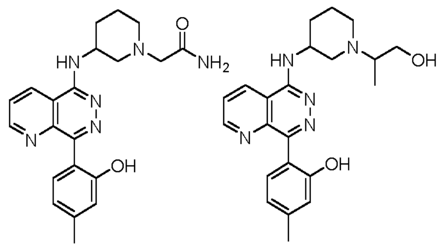
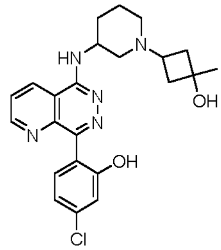

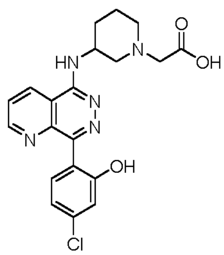
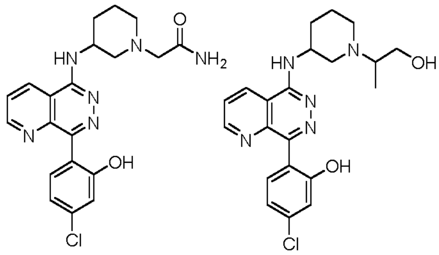
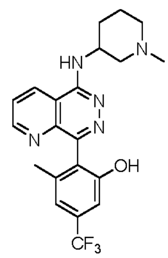
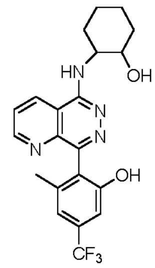
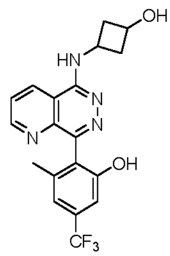

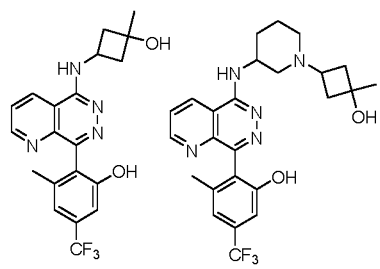
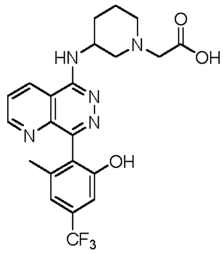
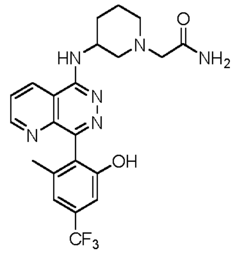
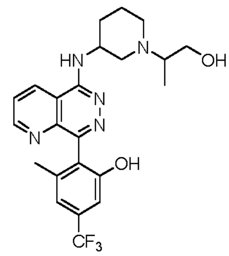

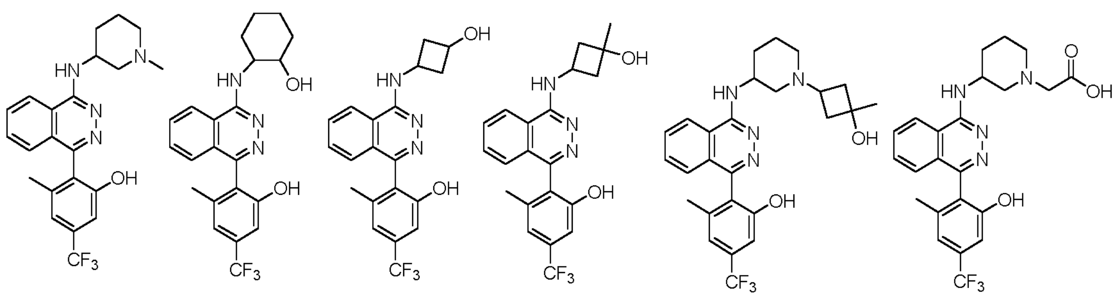

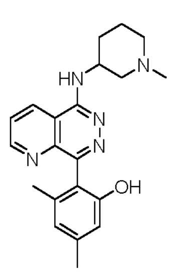
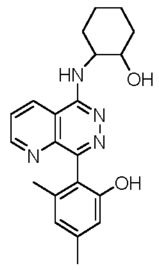
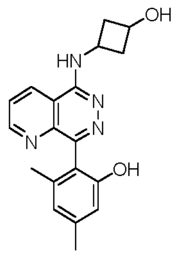
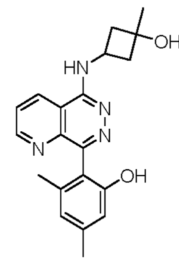
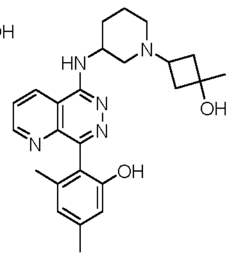
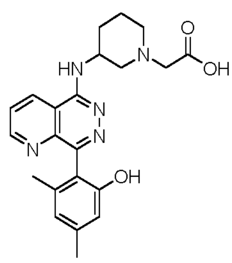

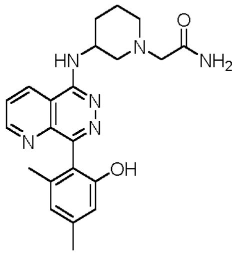
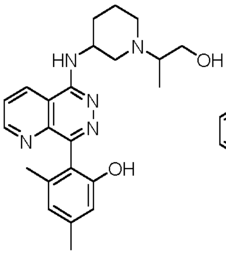
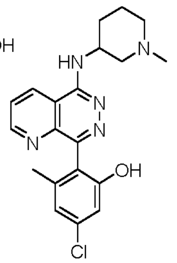
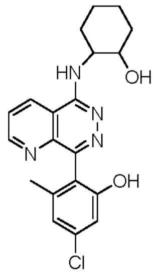
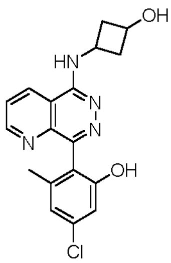

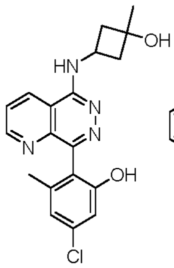
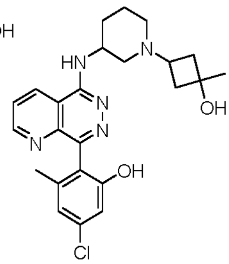
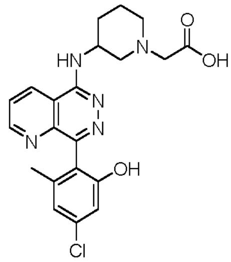
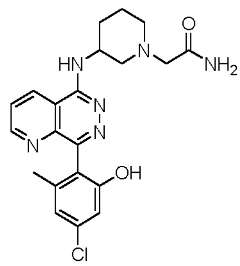
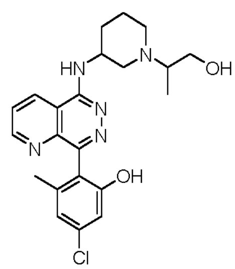

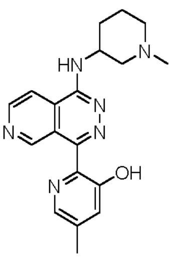
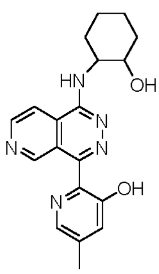
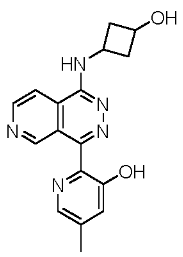
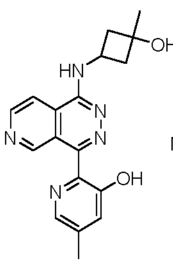
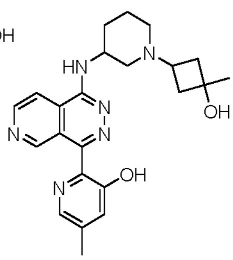
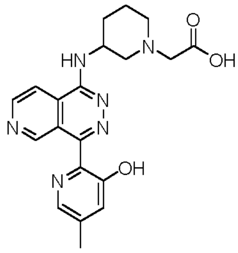

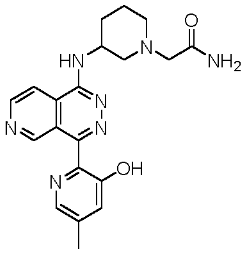
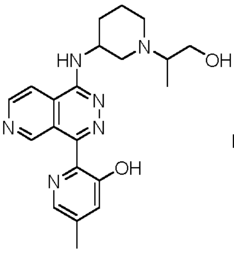
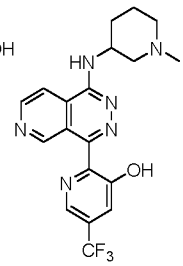
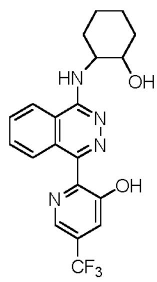
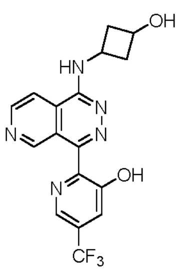

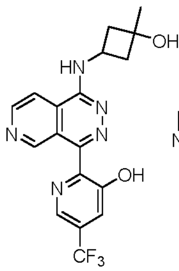
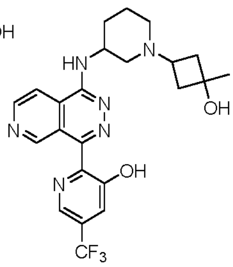
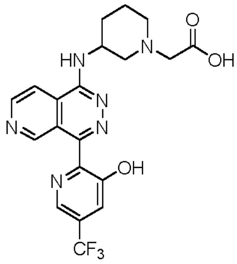
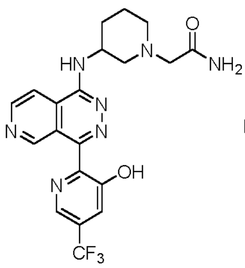
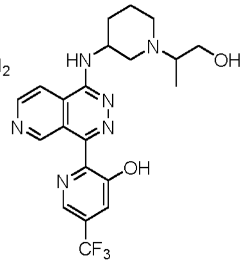

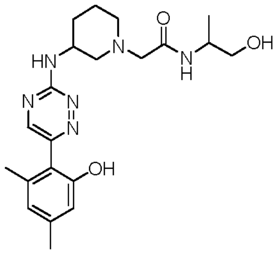
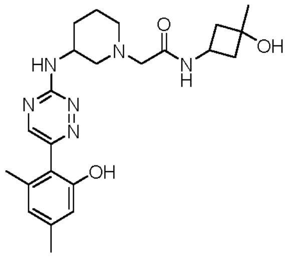
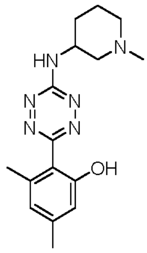
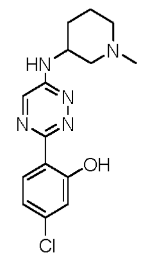

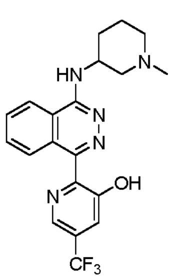
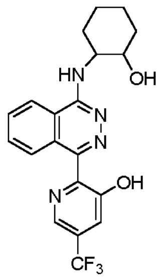
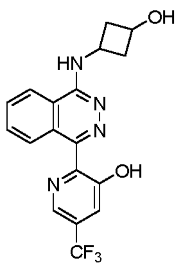
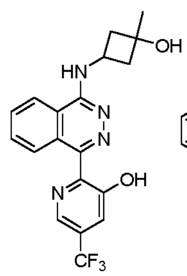
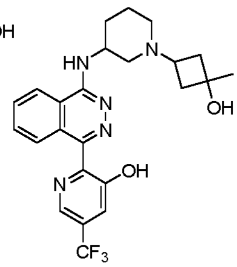
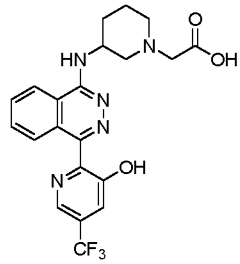

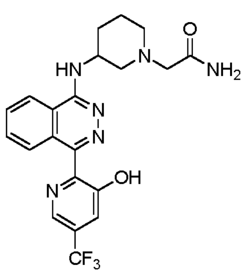
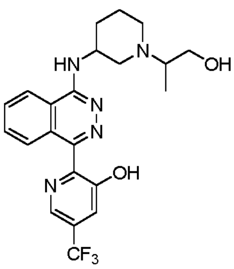
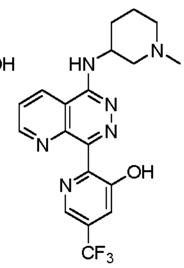
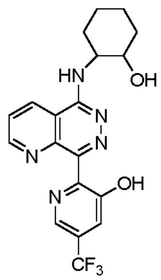
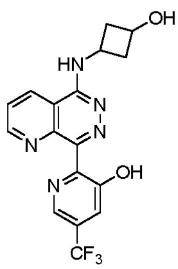

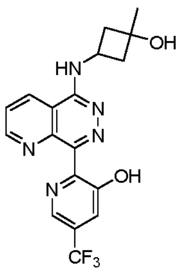
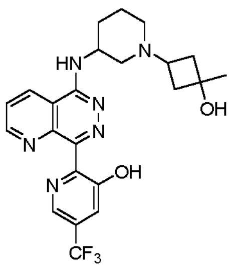
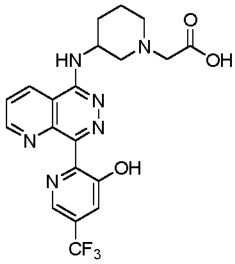
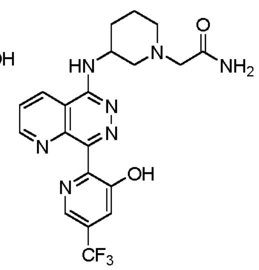
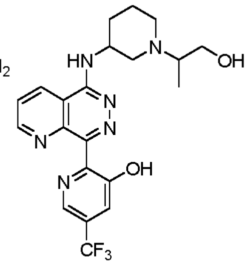

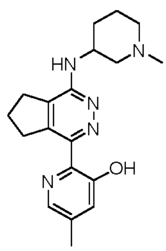
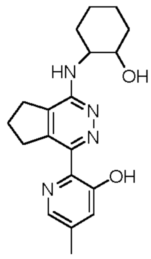
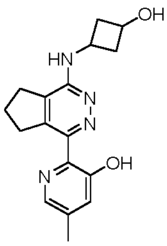
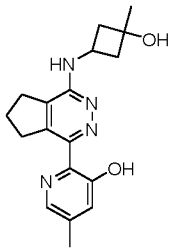
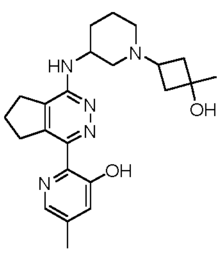
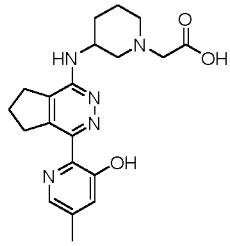
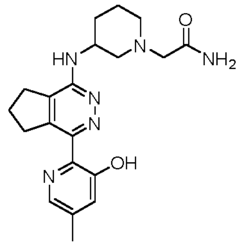
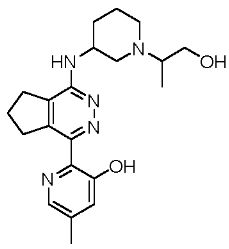
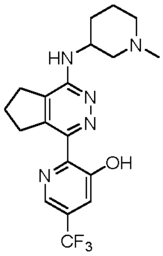
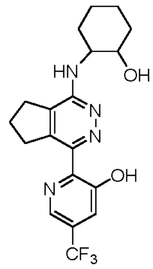
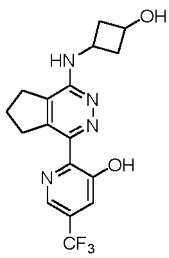
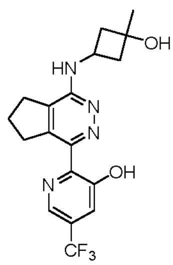
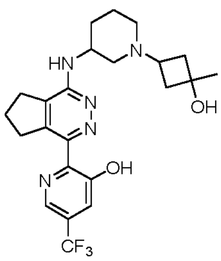
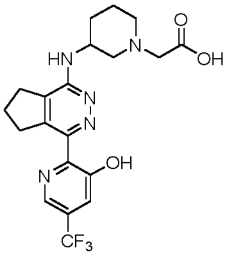
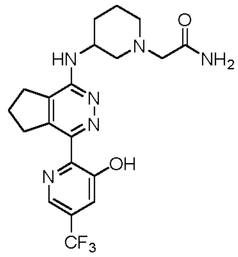
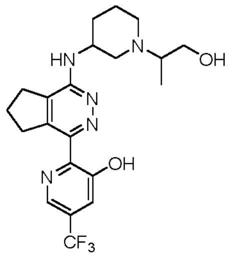
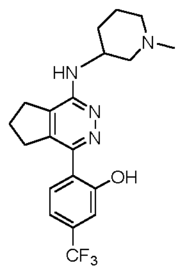
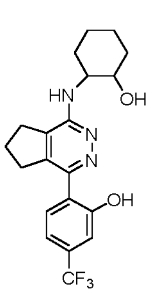
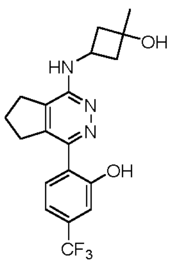
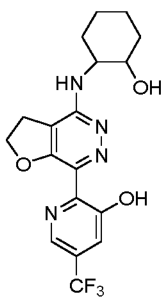
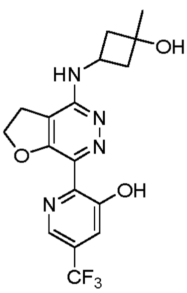
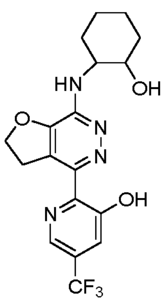
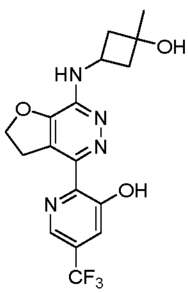
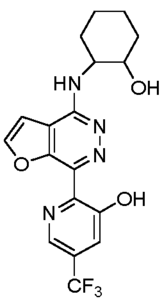
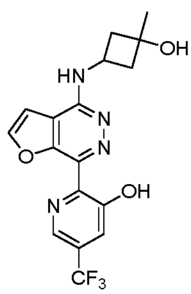

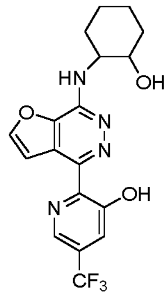 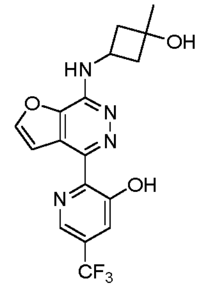 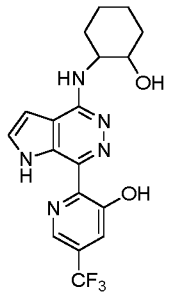 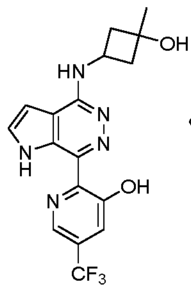 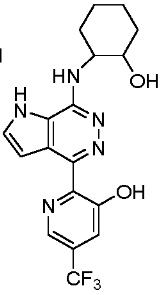 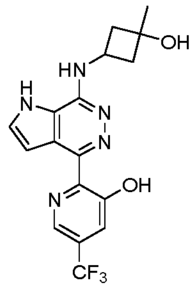

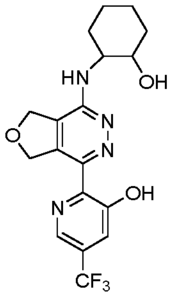 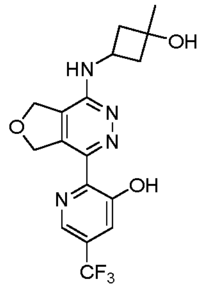 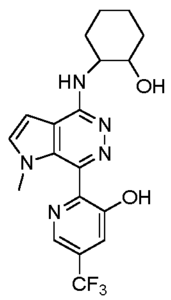 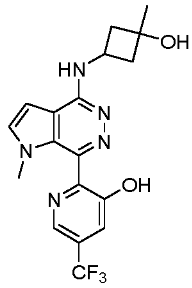 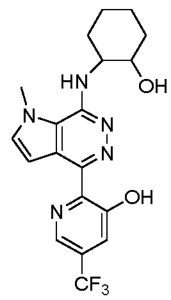 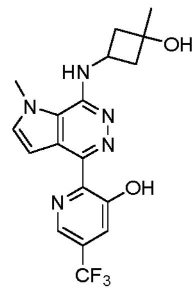

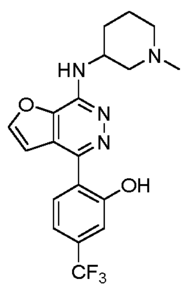 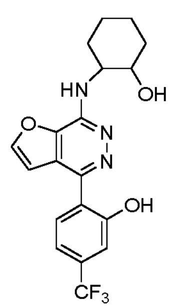 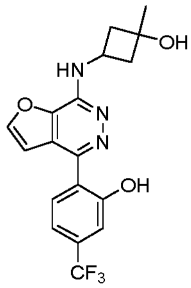 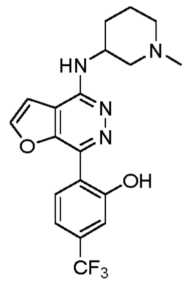 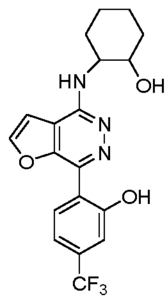 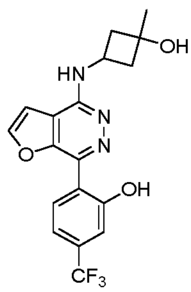

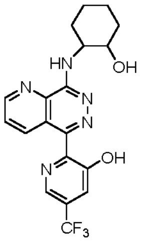 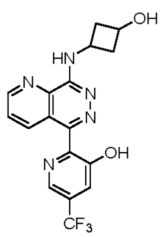 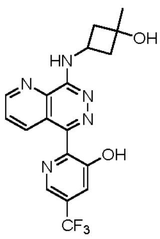 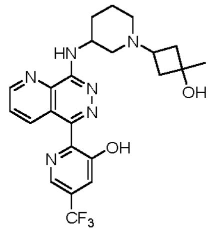 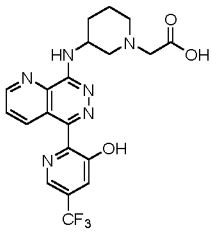 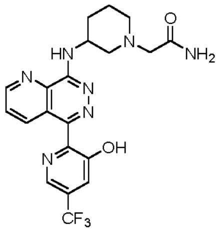

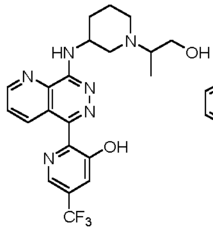 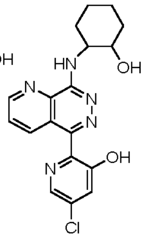 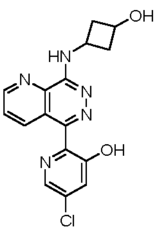 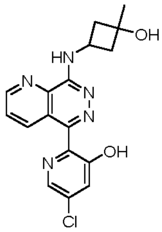 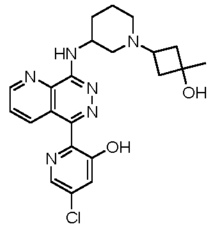 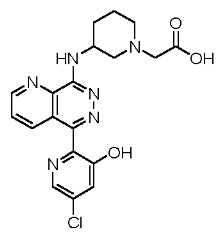

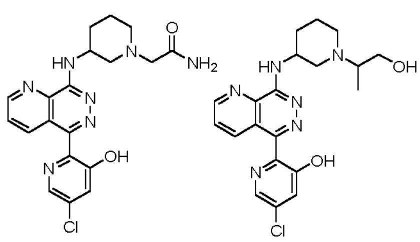
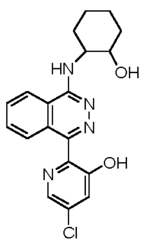
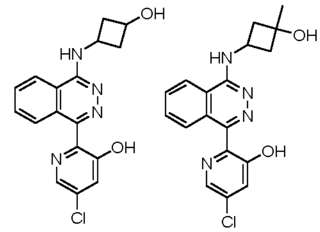
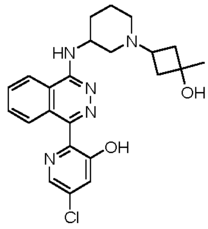

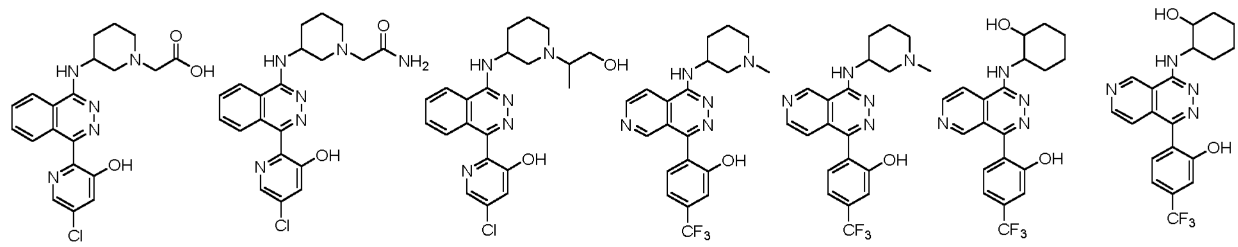

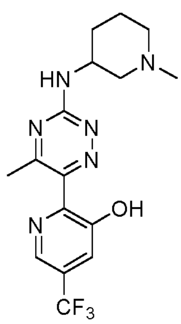
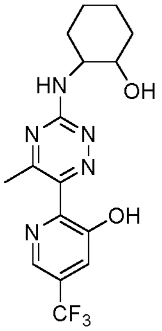
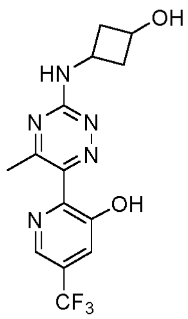
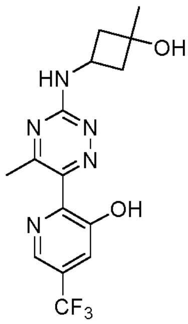
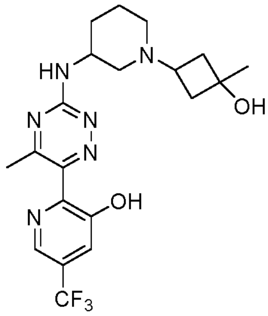

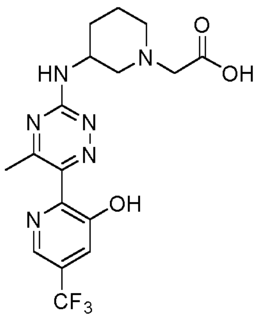
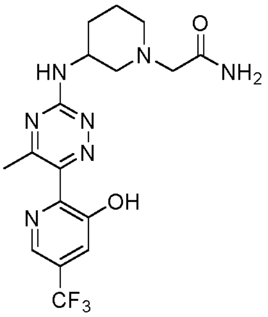
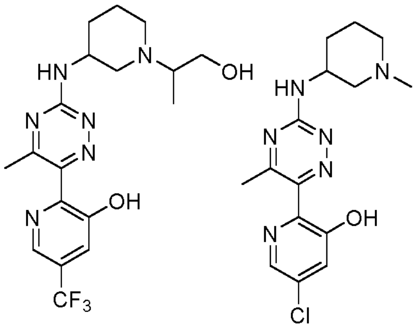
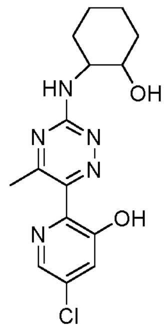

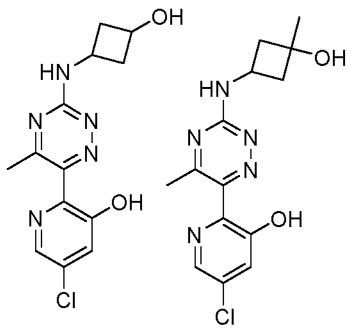
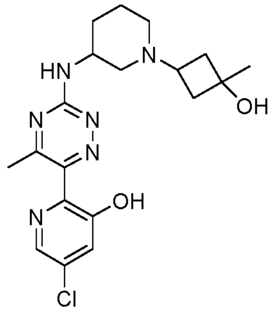
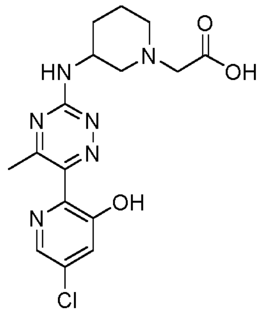
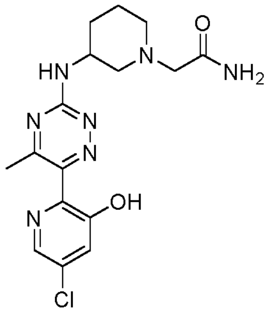

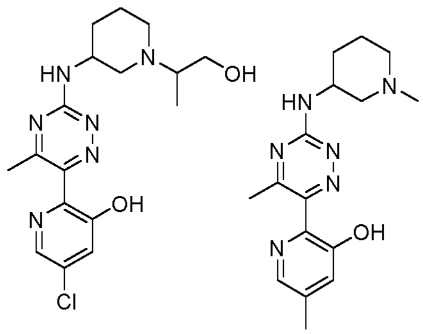 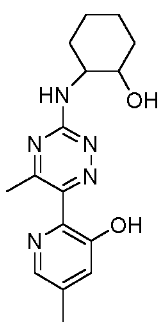 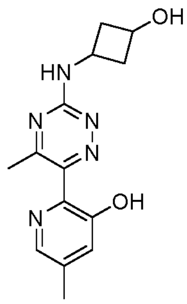 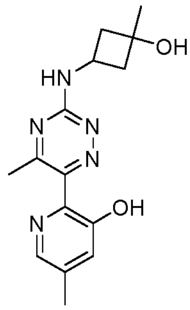

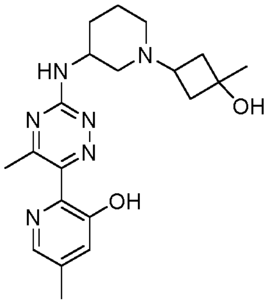 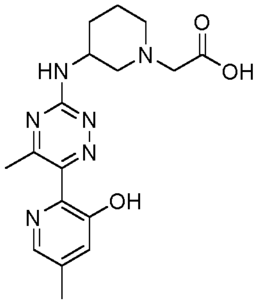 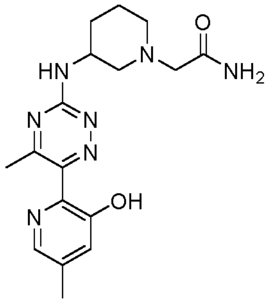 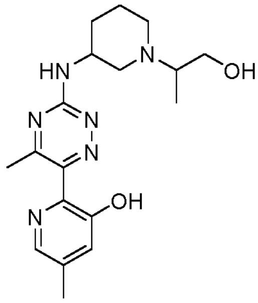

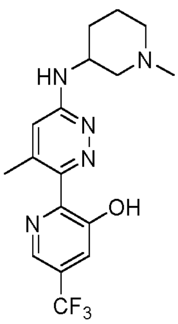 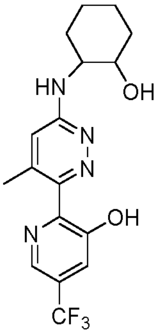 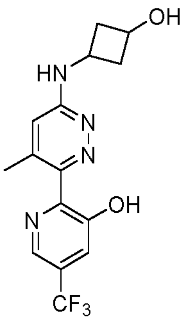 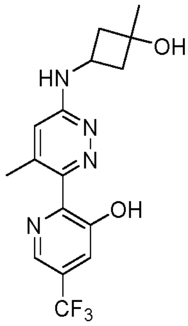 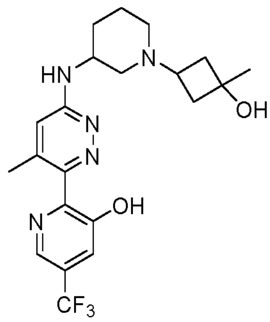

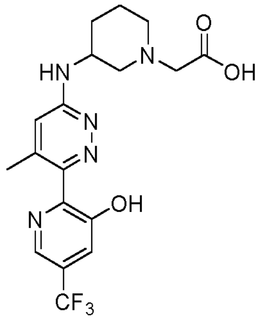 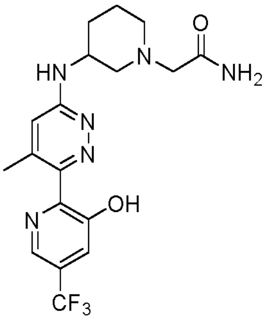 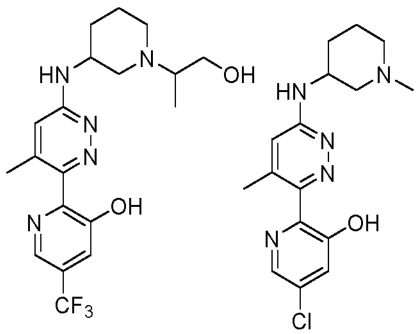 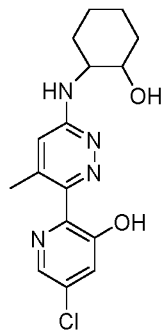

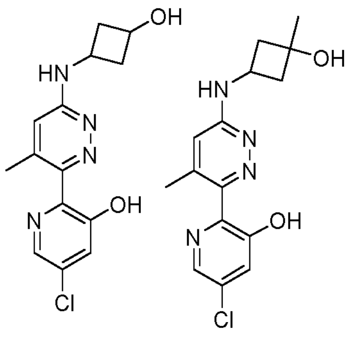
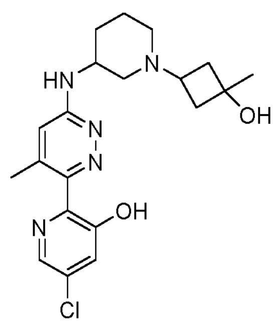
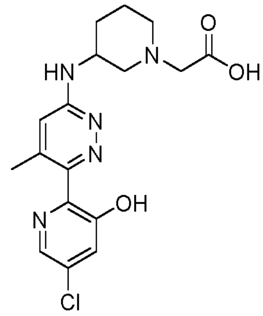
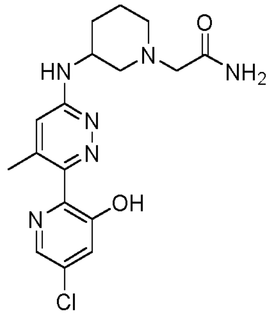
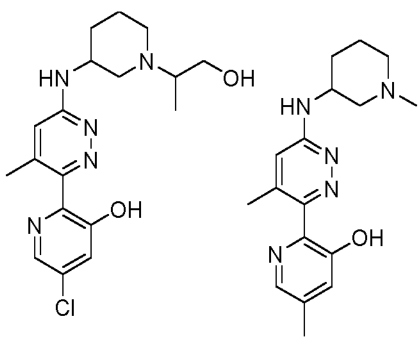
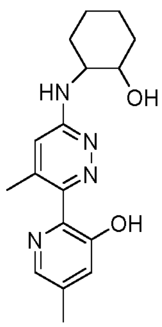
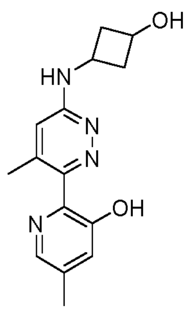
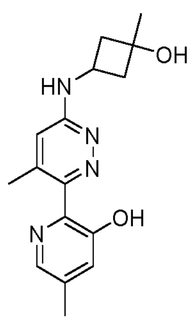
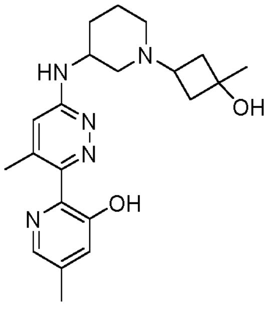
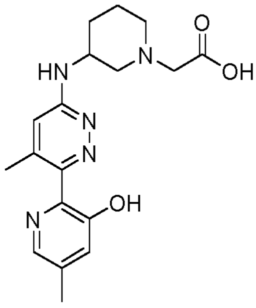
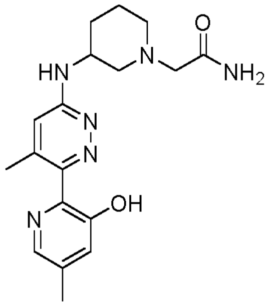
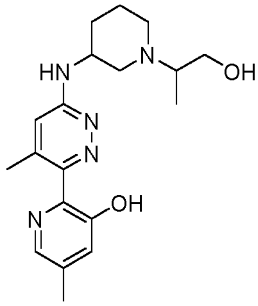
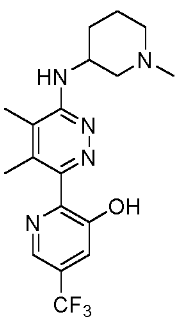
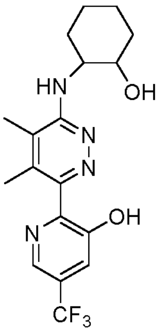
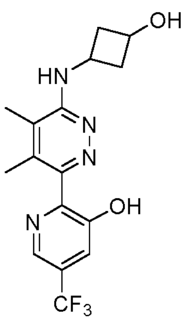
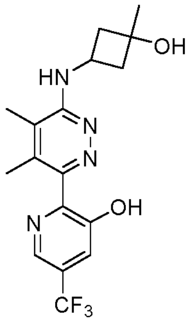
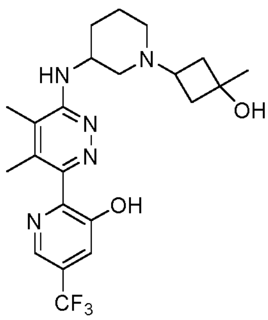

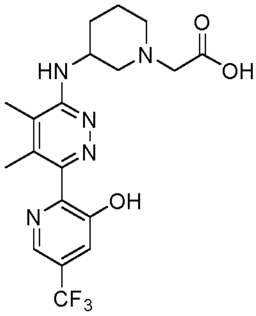
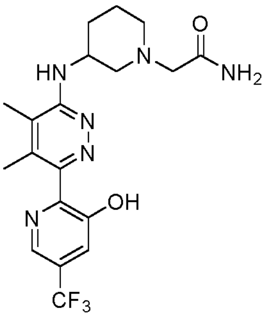
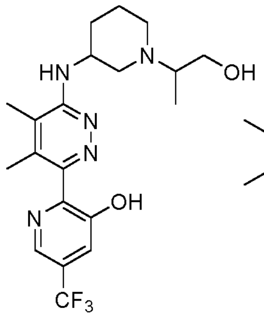
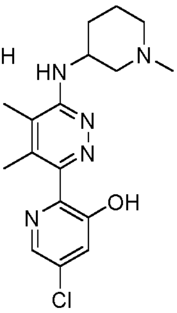
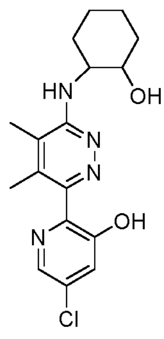

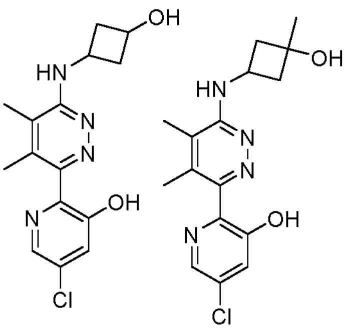
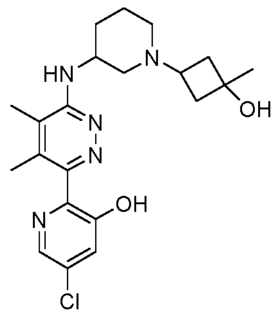
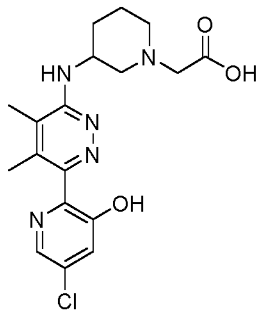
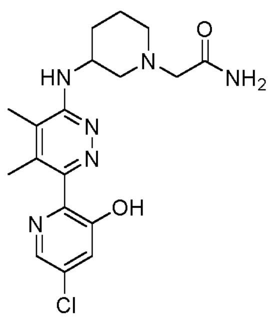

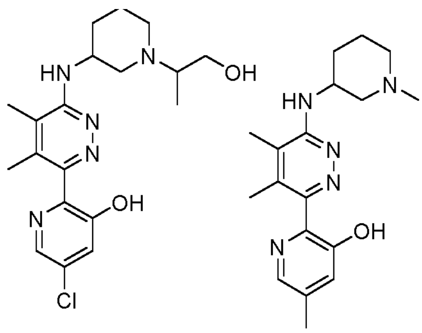
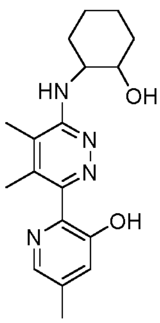
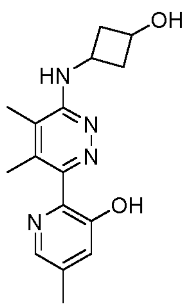
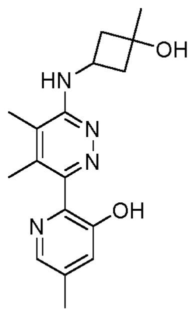

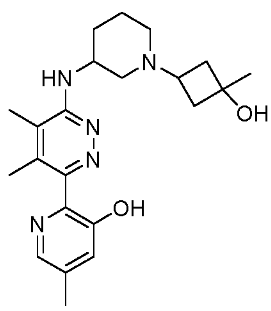
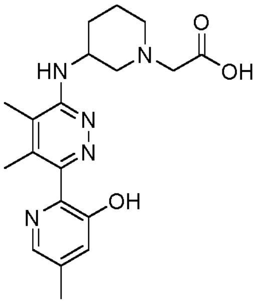
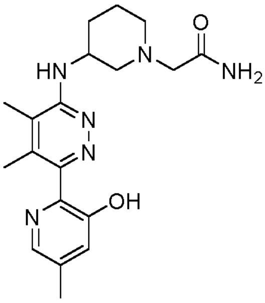
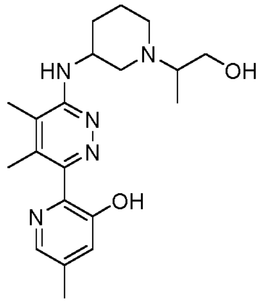

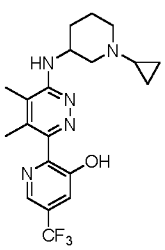
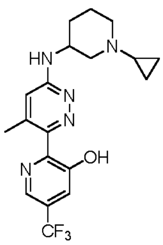
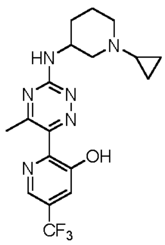
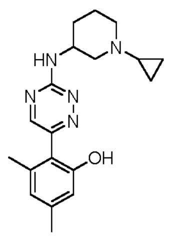
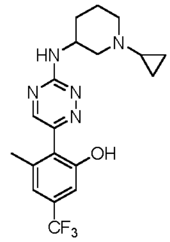
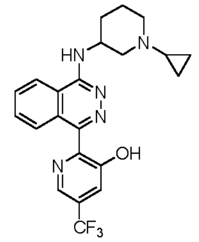

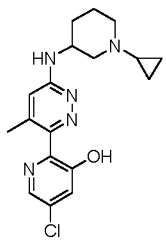 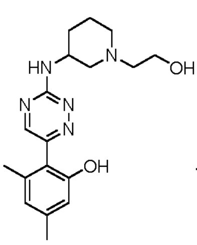 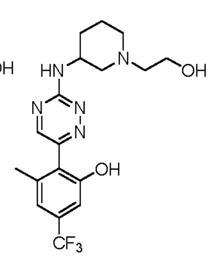 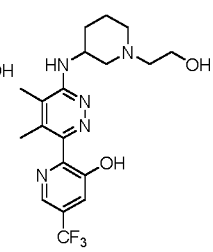 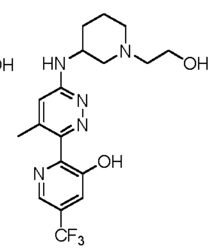 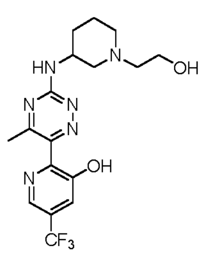

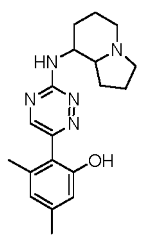 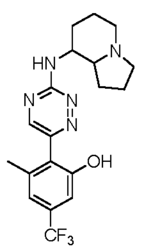 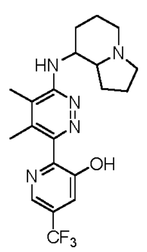 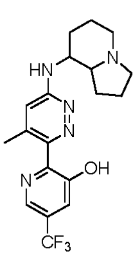 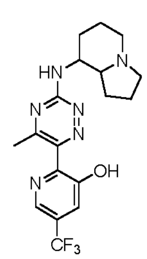 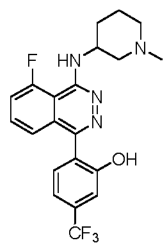 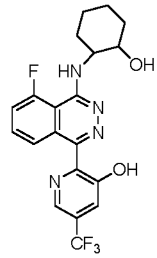

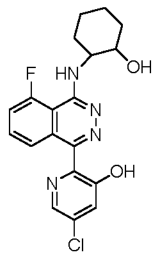 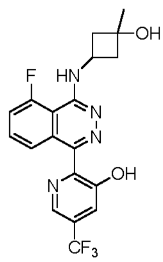 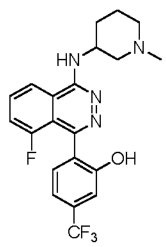 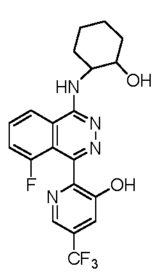 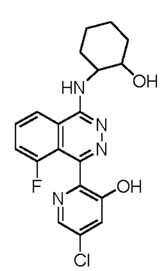 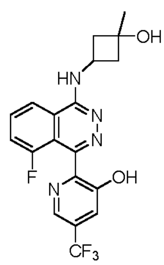 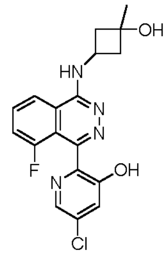 .

In an embodiment, the compound shown in General formula I is any of the following:

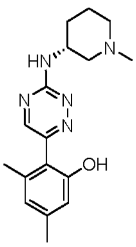 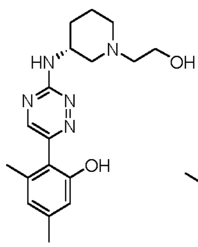 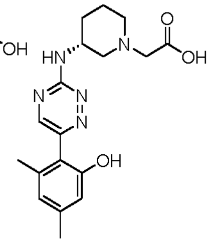 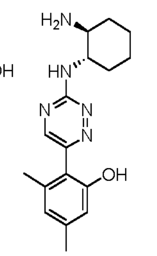 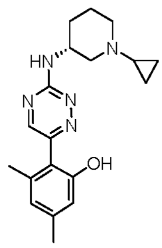

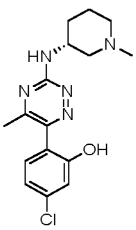 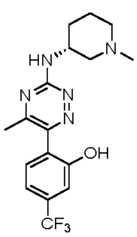

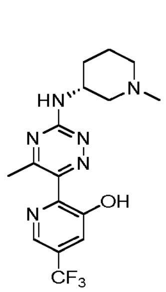 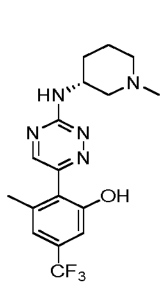 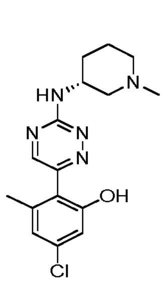 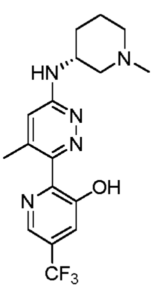 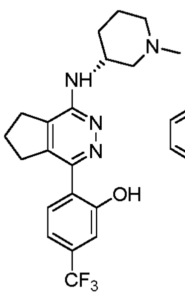 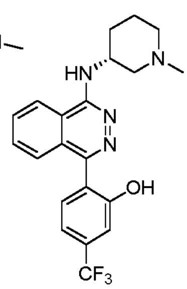 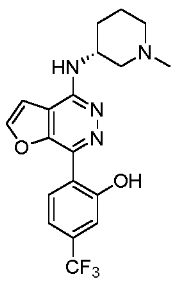

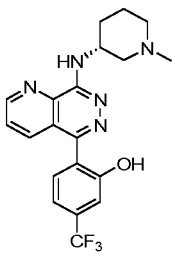 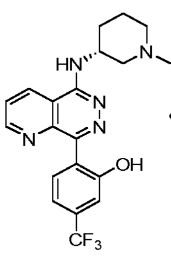 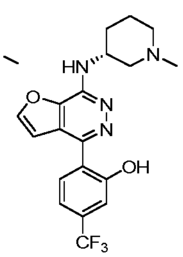 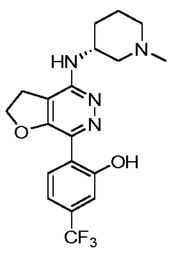 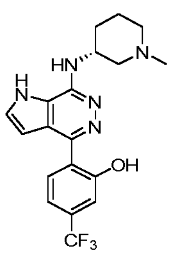 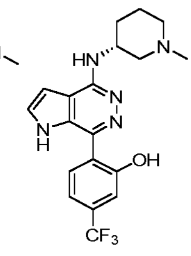 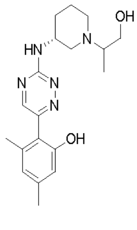

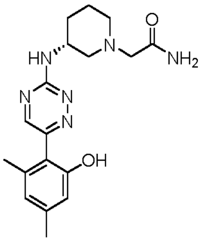 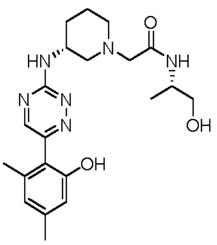 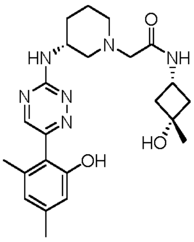 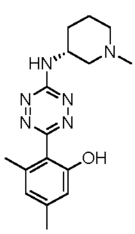 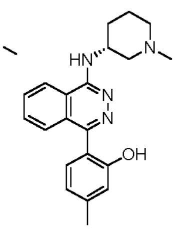 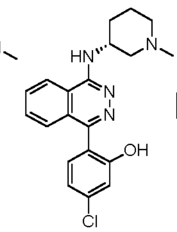 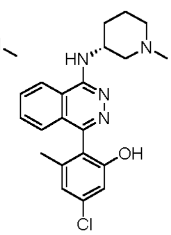

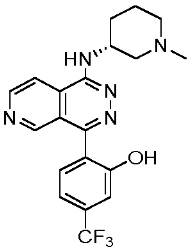 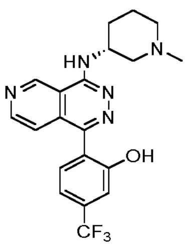 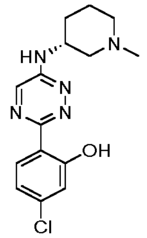 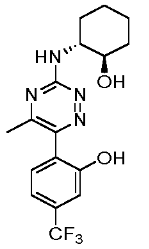 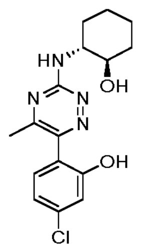 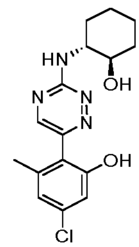 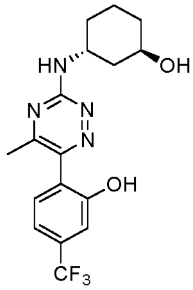

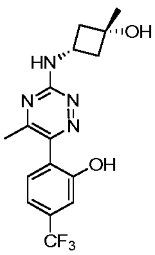 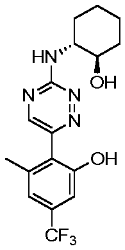 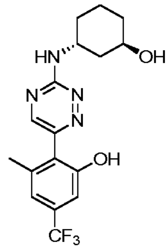 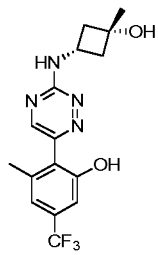

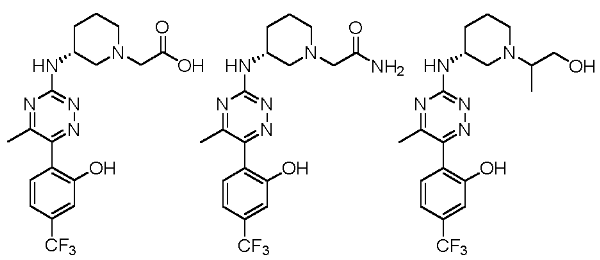

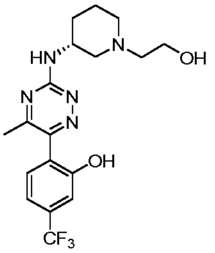

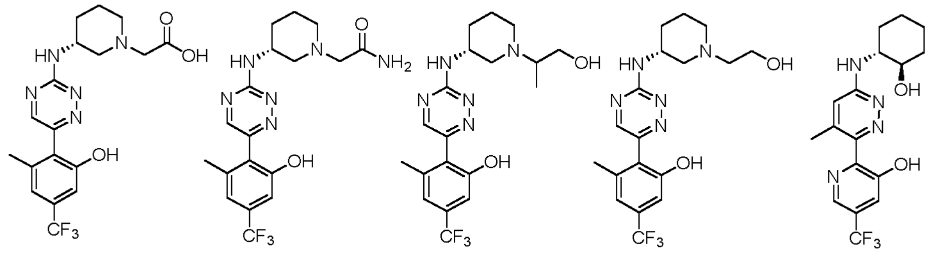

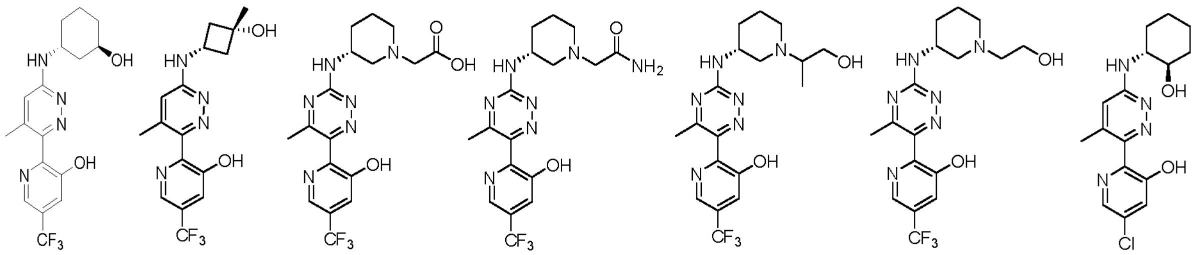

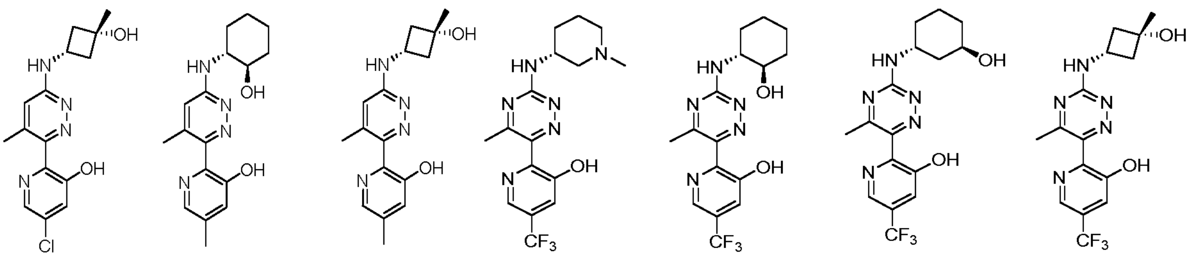

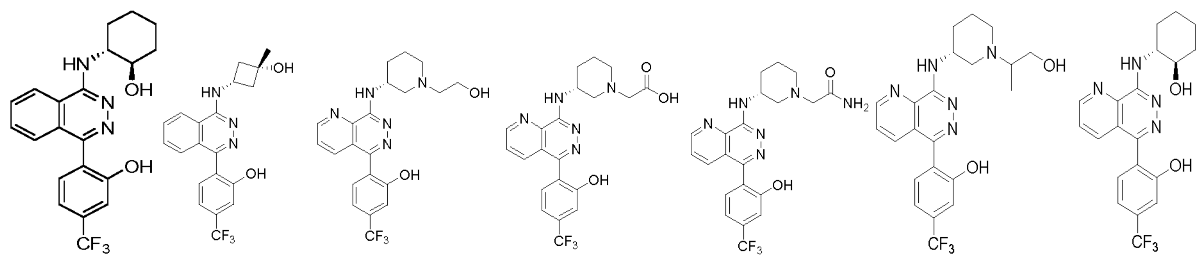

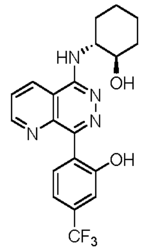 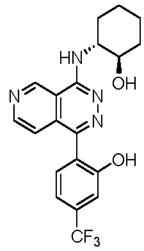 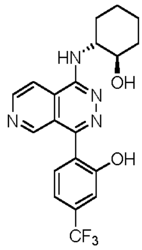 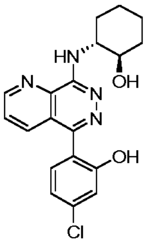 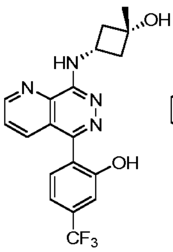 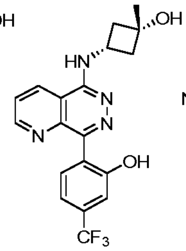 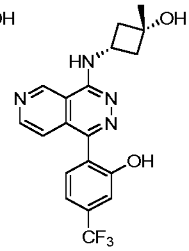

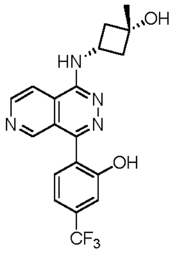 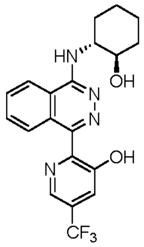 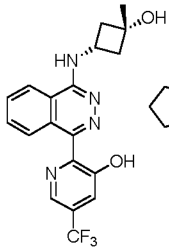 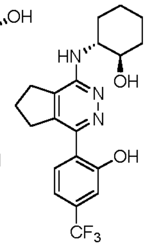 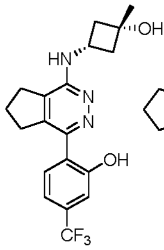 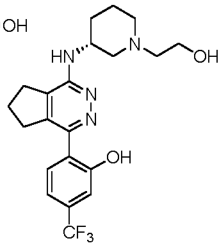 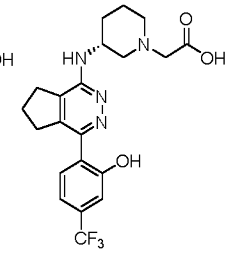

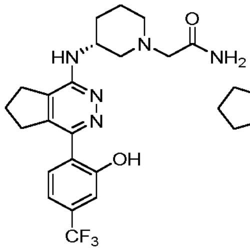 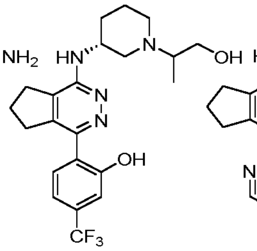 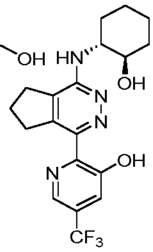 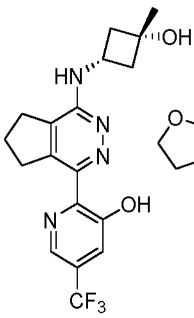 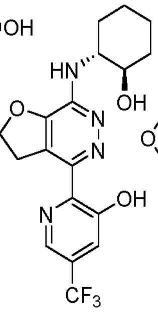 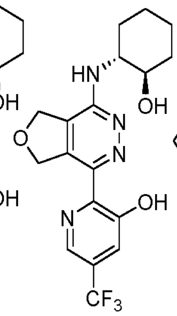 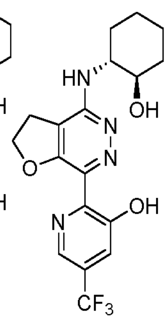

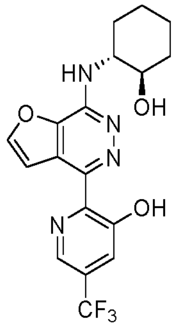 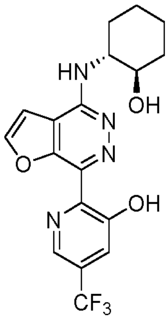 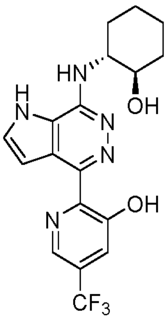 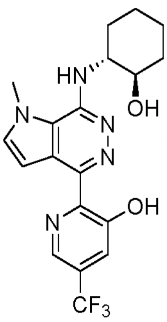 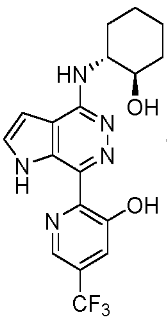 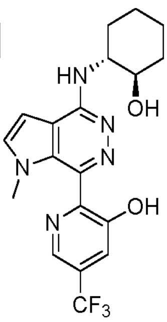 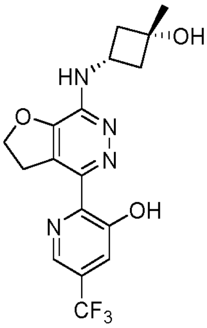

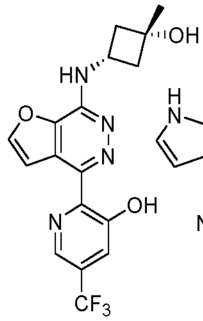 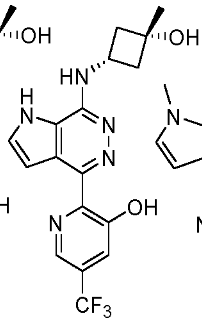 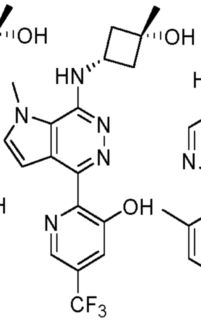 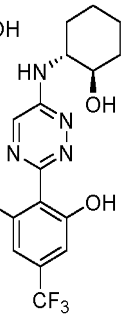 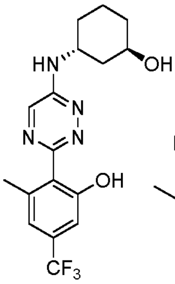 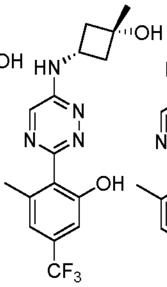 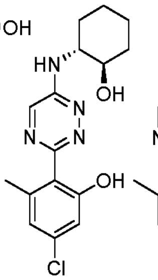 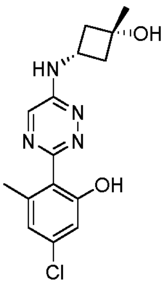

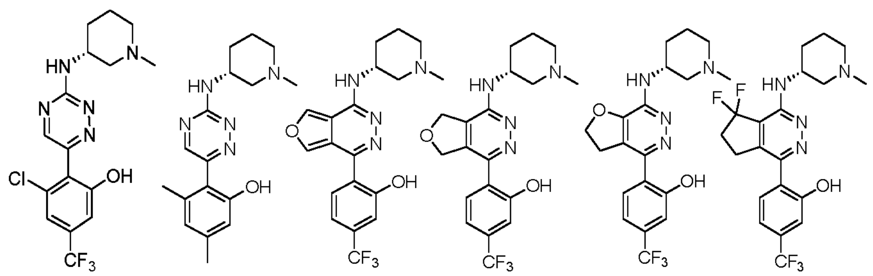

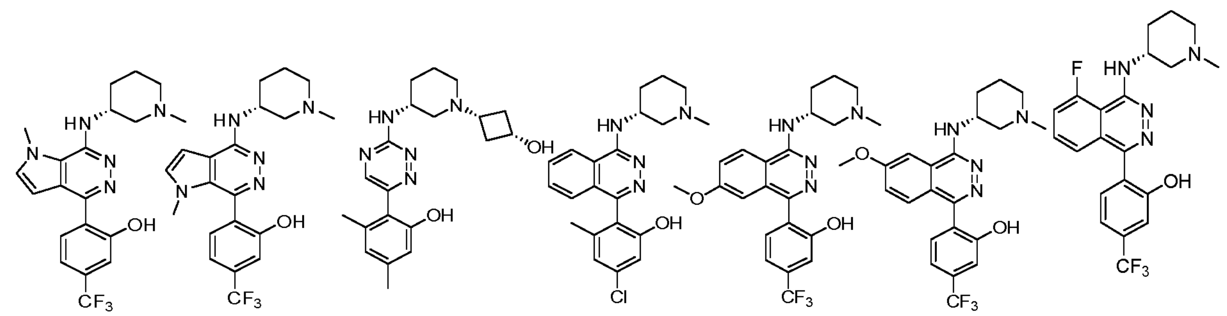

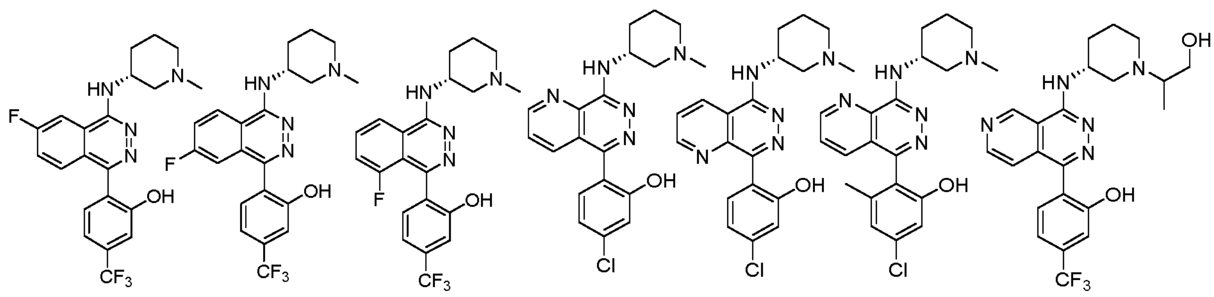

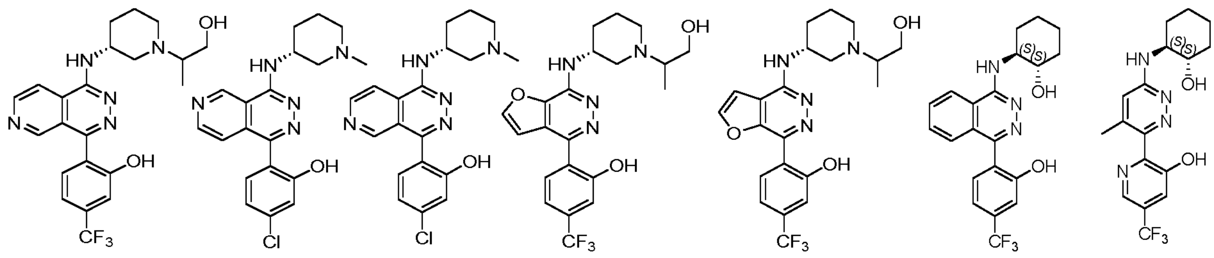

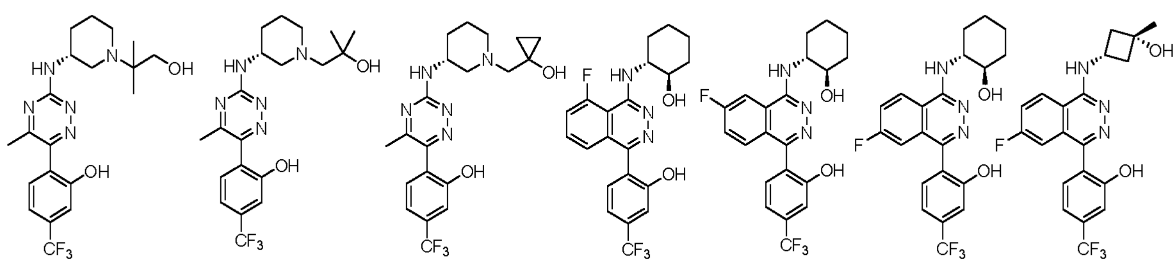

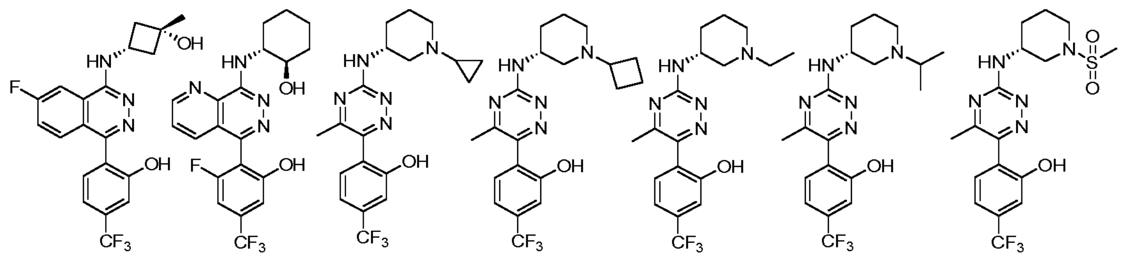

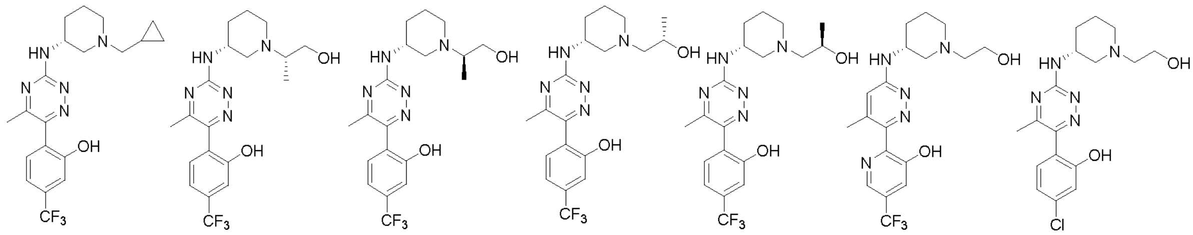

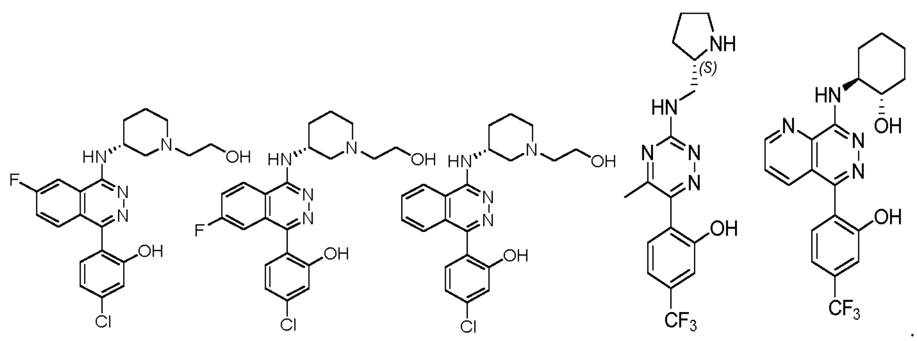

.

The present invention also provides a method for preparing a compound as shown in general formula I, which may be any of the following methods:

Method 1:

It consists of the following steps: in a solvent, under the action of an acid, compound 1 undergoes a reaction as shown below;

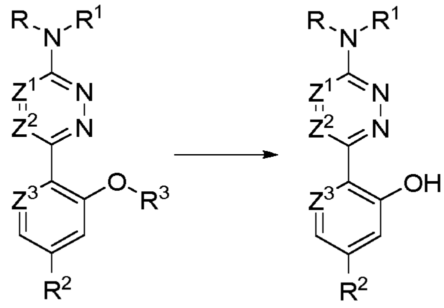

1 I ;

$R^3$ is a conventional hydroxyl protecting group in the art, such as $C_1$~$C_6$ alkyl group or Ci~Ce alkoxy substituted $C_1$~$C_6$ alkyl;

Described $C_1$~$C_6$ alkyl group can be $-CH_3$; Described $C_1$~$C_6$ alkoxy substituted $C_1$~$C_6$ alkyl group can be $-CH_2OCH_2CH_3$;

Described solvent can be a commonly used solvent for such reaction in the art, such as one or more of ester solvent, alcohol solvent and halogenated hydrocarbon solvent; described ester solvent can be ethyl acetate; described

alcohol solvent can be methanol; described halogenated hydrocarbon solvent can be dichloromethane;
The acid may be an acid commonly used in such reactions in the art, such as an inorganic acid and/or a Lewis acid; the inorganic acid may be HCl; the Lewis acid may be boron tribromide;
Preferably, when $R^3$ is $C_1$~$C_6$ alkyl group, the solvent is a halogenated hydrocarbon solvent, and the acid is a Lewis acid;
Preferably, when $R^3$ is $C_1$~$C_6$ alkoxy substituted $C_1$~$C_6$ alkyl group, the solvent is an ester solvent and/or an alcohol solvent, and the acid is an inorganic acid;
Described method 1 may further include the following steps: in a solvent, under the action of a base, compound 2 and compound 3 react as in the following to obtain compound I;

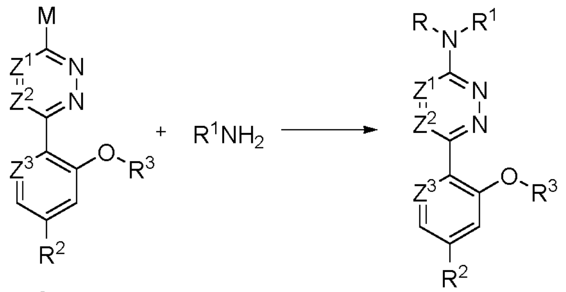

2 3 1 ;

M is a halogen, such as F, Cl, Br or I, preferably Cl, and $R^3$ is a conventional hydroxyl protecting group in the art, such as $-CH_3$;
The solvent can be a commonly used solvent for such a reaction in the art, such as one or more of nitrogen-containing compound the solvent, ether solvents or alcohol solvents, and more for example, one or more of N-methylpyrrolidone, n-butanol and dioxanes;
The base may be a commonly used base for such reactions in the art, such as diisopropyl ethylamine;

Method 2:

It consists of the following steps: in a so1vent, under the action of a base, compound 9 and react with compound 3 to generate I as shown below;

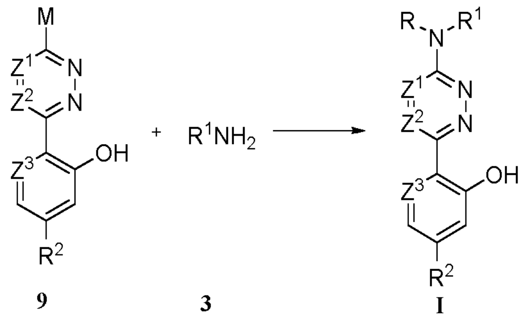

9 3 I ;

Among them, the definition of each of the above substituents is as mentioned above, except for M, M is a halogen, such as F, Cl, Br or I, preferably Cl;
The solvent can be a commonly used solvent for such reactions in the art, such as N-methylpyrrolidone;
The base may be a commonly used base for such reactions in the art, such as diisopropyl ethylamine;

Method 3:

It consists of the following steps: in a solvent, under the action of a palladium catalyst and a base, compound 4 and compound 5 undergo the reaction as shown below;

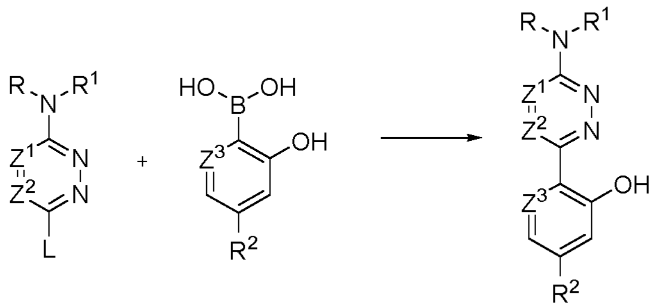

4 5 I ;

Among them, except for L, the definitions of the above substituents are as mentioned above: L is a halogen, such as F, Cl, Br or I, preferably Cl;

Described solvent can be a commonly used solvent for such reaction in this art, such as ether solvent and/or water; Preferably, the solvent is 1,4-dioxane and $H_2O$;

The palladium catalyst can be a palladium catalyst commonly used for such reactions in this art, such as $Pd(PPh_3)_4$;

The base can be a base commonly used in such reactions in the art, such as carbonate of alkali metal, preferably cesium carbonate.

The present invention also provides a group of compounds as shown in formula 1, 2, 3, 4, 5 or 9

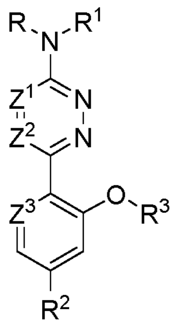

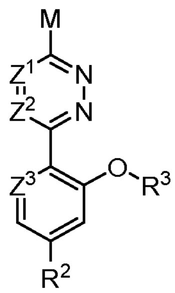

$R^1NH_2$

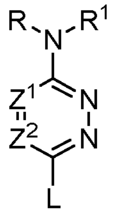

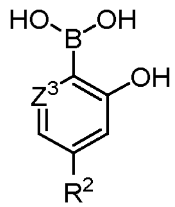

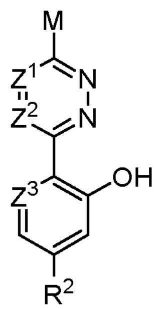

1 2 3 4 5 9 ;

In each of the above formula, the definition of each substituent is as mentioned above.

In a particular scenario, the described

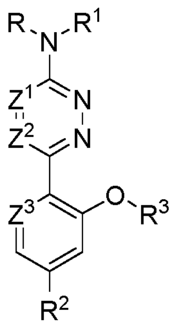

1

may be

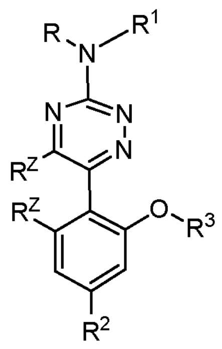

**6**

or may be

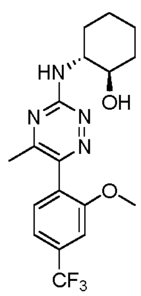

or

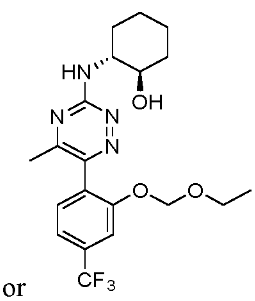

.

In a particular scenario, the described

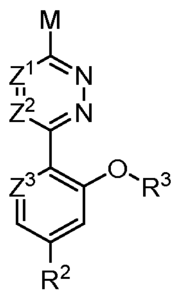

**2**

may be

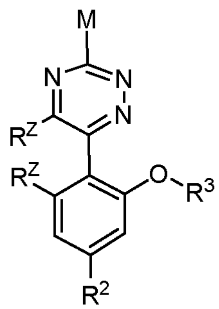

**7**

or may be

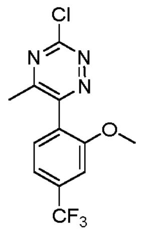

or

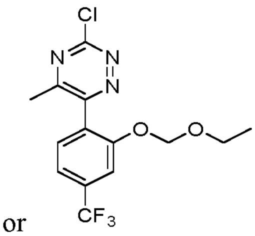

.

In one scenario, the described

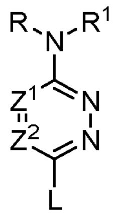

**4**

can be

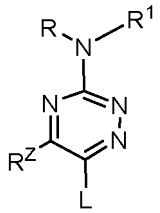

**8**

.

In a scenario, the described

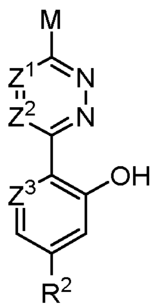

**9**

can be

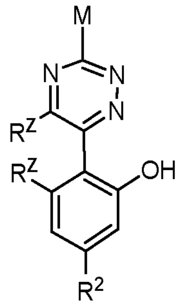

10

or can be

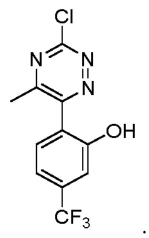

The invention also provides a pharmaceutical composition comprising substance X and pharmaceutically acceptable excipients;

Described substance X is a compound as shown in General formula I, a solvate thereof, a pharmaceutically acceptable salt thereof or a solvate of the salt.

The invention also provides an application of a substance X or the above-mentioned pharmaceutical composition in the preparation of an NLRP3 inhibitor or a drug for the prevention and/or treatment of NLRP3-related diseases;

Described substance X is a compound as shown in General formula I, a solvate thereof, a pharmaceutically acceptable salt thereof or a solvate of the salt.

In a certain scenario, the NLRP3-related disease refers to the disease that responds to NLRP3 inhibition. The described disease is selected from cryopyrin-associated periodic syndrome (CAPS), Muckle-Wells syndrome (MWS), familial cold autoinflammatory syndrome (FCAS), neonatal-onset multisystemic inflammatory diseases (NOMID), multiple sclerosis (MS), amyotrophic lateral sclerosis, rheumatoid arthritis, psoriasis, Alzheimer's disease, Parkinson's disease, Non-alcoholic fatty liver disease, atherosclerosis, asthma, COPD, pulmonary idiopathic fibrosis, chronic kidney disease, inflammatory bowel diseases, tumors, type 1 diabetes, type 2 diabetes, and gout.

In one scenario, the disease associated with NLRP3 is inflammatory bowel disease.

The invention also provides an application of a substance X or the above-mentioned pharmaceutical composition in the preparation of drugs for the prevention and/or treatment of inflammatory bowel disease;

Described substance X is a compound as shown in General formula I, a solvatethereof, a pharmaceutically acceptable salt thereof or a solvent of the salt.

Unless otherwise specified, the terms used in the present invention have the following meanings:

The term "pharmaceutically acceptable" refers to being relatively non-toxic, safe, and suitable for use by patients.

The term "pharmaceutically acceptable salt" refers to the salt obtained by the reaction of a compound with a pharmaceutically acceptable acid or base. When the compound contains relatively acidic functional groups, base adduct salts can be obtained by reacting the compound with sufficient amounts of a pharmaceutically acceptable base in a suitable inert solvent. Pharmaceutically acceptable salts include, but are not limited to: sodium salts, potassium salts, calcium salts, aluminum salts, magnesium salts, bismuth salts, ammonium salts, etc. When a compound contains a relatively basic functional group, acid adduct salts can be obtained by reacting the compound with a sufficient amount of a pharmaceutically acceptable acid in a suitable inert solvent. Pharmaceutically acceptable acid adduct salts include, but are not limited to: hydrochloride, sulfate, mesylate, etc. See Handbook of Pharmaceutical Salts: Properties, Selection, and Use (P. Heinrich Stahl, Camille G. Wermuth, 2011, 2nd Revised Edition).

The term "solvate" refers to a substance formed by the combination of a chemical compound with a solvent (including but not limited to: water, methanol, ethanol, etc.). Solvates are divided into stoichiometric solvates and non-stoichiometric solvates. Solvates include, but are not limited to: monohydrates.

The term "solvates of pharmaceutically acceptable salts" refers to substances formed by the combination of a compound with a pharmaceutically acceptable acid or base, solvent (including, but not limited to: water, methanol,

ethanol, etc.). Among them, the amount of solvent can be stoichiometric or non-stoichiometric either. Solvates of pharmaceutically acceptable salts include, but are not limited to: monohydrochloride monohydrate.

The

" 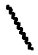 "

in the structural fragment refers to the structural fragment through which it is connected to the rest of the molecule. For example,

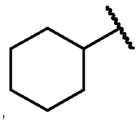
,

it refers to cyclohexyl.

The "-" at the end of the group refers to the fact that the group is attached to the rest of the molecule at this site. For example, -COOH refers to the carboxyl group.

The term "one or more" refers to one, two or three, preferably one or two.

The term "halogen" refers to fluorine, chlorine, bromine or iodine.

The term "alkyl" refers to a linear or branched, saturated monovalent hydrocarbon group with a specified number of carbon atoms (e.g., $C_1$~$C_6$). Alkyl groups include, but are not limited to: methyl, ethyl, n-propyl, isopropyl, n-butyl, isobutyl, sec-butyl, tert-butyl, n-amyl, n-hexyl, etc.

The term "alkoxy" refers to the group $R^X$-O-, and $R^X$ is defined in the same way as the term "alkyl". Alkoxy groups include, but are not limited to: methoxy, ethoxy, n-propoxy, isopropoxy, etc.

The term "cycloalkenyl ring" refers to a cyclic, unsaturated monovalent carbon ring with a specified number of carbon atoms (e.g., $C_5$~$C_6$), which has one or more (e.g., 1, 2, or 3) carbon-carbon sp2 double bonds, which are single rings, are not aromatic, and which meet either of the following conditions: 1. are connected to the rest of the molecule by two or more single bonds, and 2) share two atoms and one bond with the rest of the molecule.

The term "heterocycloalkenyl ring" refers to a cyclic, unsaturated monovalent ring with a specified number of rings (e.g., 5~6 membered), a specified number of heteroatoms (e.g., 1, 2, or 3), a specified heteroatom species (one or more of N, O, and S), which has one or more (e.g., 1, 2, or 3) carbon-carbon sp2 double bonds, which are single rings and are not aromatic. And it satisfies any of the following conditions: 1. It is connected to the rest of the molecule by two or more single bonds, and 2. It shares two atoms and one bond with the rest of the molecule.

The term "heteroaromatic ring" has a specified number of ring atoms (e.g., 5~10 -membered), a specified number of heteroatoms (e.g., 1, 2 or 3), a specified heteroatom species (one or more of N, O and S), a cyclical and unsaturated monovalent group, which is a single ring and has aromatic properties, and it satisfies any of the following conditions: 1. It is connected to the rest of the molecule by two or more single bonds, and 2. It shares two atoms and one bond with the rest of the molecule.

The term "cycloalkyl" refers to a cyclical, saturated monovalent hydrocarbon group with a specified number of carbon atoms (e.g., $C_3$~$C_6$). Cycloalkyl groups include, but are not limited to:

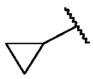 , , 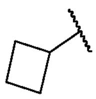 ,

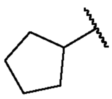 , 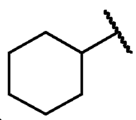 ,

The term "heterocycloalkyl" refers to a cyclic and saturated monovalent group with a specified number of rings (e.g., 3-10-membered-membered), a specified number of heteroatoms (e.g., 1, 2 or 3), a specified heteroatom species (one or more of N, O and S), which are single or double rings, preferably single rings. Heterocycloalkyl groups are attached to the rest of the molecule by carbon atoms or heteroatoms. Hetero-alkyl groups include, but are not limited to:

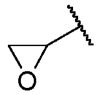 , , 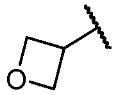 , 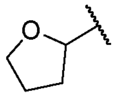 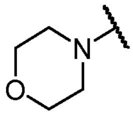 , 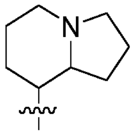

The term "pharmaceutically acceptable excipients" refers to all substances contained in pharmaceutical formulations except the active pharmaceutical ingredient, and is generally divided into two categories: excipients and additives. For details, please refer to the Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

The term "treatment" refers to the elimination of the cause or the relief of symptoms.

The term "prevention" refers to reducing the risk of developing disease.

In addition, it should be noted that, unless otherwise expressly stated, the manner in which the present invention is described "...Independently" should be broadly understood to mean that the individual groups described are independent of each other and can be independently of the same or different specific groups. In more detail, the way it is described is "...Independently" can mean that the specific selections expressed by the same symbols do not affect each other in different groups, or it can mean that the specific seletions expressed by the same symbols do not affect each other in the same group.

Positive progressive effect, the compounds shown in General Formula I of the present invention have good activity in NLRP3 inhibition, can be developed as drugs for the treatment and/or prevention of NLRP3-related diseases, and they have been demonstrated that their inhibitory activity against pyroptosis of human THP-1 cells, $IC_{50}$, can reach the level of $\mu$M or even nM. Further, the compounds of the present invention have no inhibitory effect on hERG pathway, have few side effects, and have good pharmacokinetic properties, show a good inhibitory effect of inflammasomes in vivo, and are well tolerated.

DETAILED DESCRIPTION OF THE PREFERRED EMBODIMENT

Some embodiments are further disclosed in detail in the following exhibits, which are not in any way intended to limit the scope of the claims.

**Embodiment 1**

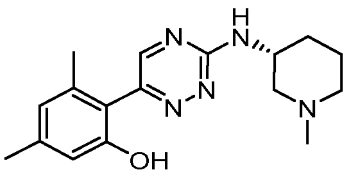

(R)-3,5-Dimethyl-2-(3-((1-methylpiperidin-3-yl)atnino)-1,2,4-triazin-6-yl)phenol

The specific reaction Formula is as follows:

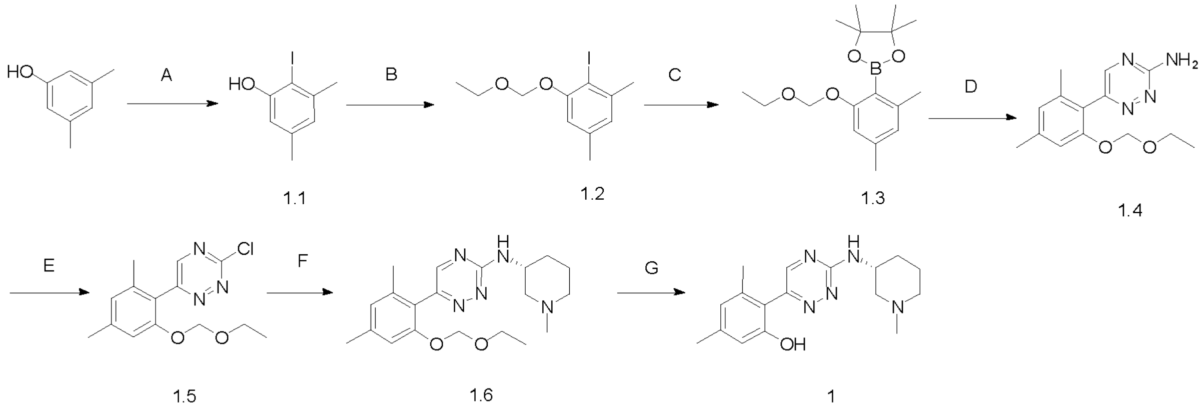

1.1 1.2 1.3 1.4

1.5 1.6 1

Step A: 2-iodo-3,5-dimethylphenol (compound 1.1)

Dissolve 3,5-dimethylphenol (10.0g, 81.86mmol) into 400mL toluene, cool to 0 °C, add sodium hydride (6.55g, 163.71mmol) in batches, stir for 50 minutes at 0 °C and under the protection of nitrogen, raise to room temperature and continue the stirring for 15 minutes, cool to 0 °C again, add iodine (20.78g, 81.86mmol) in batches, and stir for 1 hour. 0.5 M aqueous solution of hydrochloric acid was added to the reaction solution to quench the reaction, extracted with ethyl acetate (400mL×2), combined the organic phases, washed with brine, dried with anhydrous sodium sulfate, filtered, concentrated under reduced pressure, and purified by column chromatography to obtain a product of 1.1 (15g, yield: 74%).

Step B: 1-(ethoxymethoxy)-2-iodo-3,5-xylene (compound 1.2)

Compound 1.1 (5.0 g, 20.16 mmol) was dissolved into 60 mL of N'N-dimethylformamide (DMF), then cesium carbonate (6.57 g, 20.16 mmol) and chloromethyl ether (2.48 g, 26.20 mmol) were added, and the reaction was stirred for 16 hours at room temperature. The reaction was quenched with water, extracted with ethyl acetate (200mL×2), combined the organic phases, washed with brine, dried with anhydrous sodium sulfate, filtered, concentrated under reduced pressure, and purified by column chromatography to obtain a product of 1.2 (5.0 g, yield: 81%).

Step C: 2-(2-(ethoxymethoxy)-4,6-dimethylphenyl)-4,4,5,5-tetramethyl-1,3,2-dioxaborane (compound 1.3)

Compound 1.2 (3.65g, 11.92mmol) was added to 60mL of 1,4-dioxane, followed by 4,4,5,5-tetramethyl-1,3,2-dioxaborane (3.05g, 23.85mmol), triethylamine (3.6g, 35.77mmol), $Pd(OAc)_2$(267 mg, 1.19 mmol) and CyJohnphos (835 mg, 2.38 mmol), and let the reaction stir overnight at 95 °C and under nitrogen protection. The reaction solution was filtered, quenched with water, extracted with ethyl acetate (60mL×2), combined the organic phases, washed with brine solution, dried with anhydrous sodium sulfate, filtered, concentrated under reduced pressure, and purified by column chromatography to obtain a product of 1.3 (1.74g, yield: 48%). LCMS ESI(+)m/z: 307.2 (M+1).

Step D: 6-(2-(ethoxymethoxy)-4,6-dimethylphenyl)-1,2,4-triazin-3-amine (compound 1.4)

The compounds 6-bromo-1,2,4-triazin-3-amine (1.00g, 5.71mmol) and compound 1.3 (1.75g, 5.71mmol) were dissolved in 30mL of dioxane and 5mL of water, and cesium carbonate (5.57g, 17.14mmol) and $PdCl_2$(dppf) (466mg, 0.57mmol) were added , the reaction solution was stirred at 100 °C and under nitrogen protection for 16 h. 50mL of water was added to the reaction solution, extracted with ethyl acetate (80mL×2), combined the organic phases, washed with brine, dried with anhydrous sodium sulfate, filtered, concentrated under reduced pressure, and purified by column chromatography to obtain a product of 1.4 (390mg, yield: 28%). LCMS ESI(+)m/z: 275.2 (M+1).

Step E: 3-chloro-6-(2-(ethoxymethoxy)-4,6-dimethylphenyl)-1,2,4-triazine (compound 1.5)

The compound 1.4 (390mg, 1.42mmol) was dissolved in 30mL of acetonitrile, and then , copper chloride (287mg, 2.13mmol) and tert-butyl nitrite (220mg, 2.13mmol) were added at 0 °C under the protection of nitrogen, and the reaction solution was stirred at 60°C for 1.5 hours. The reaction solution was concentrated under reduced pressure, and the product was purified by column chromatography to obtain 1.5 (158mg, yield: 38%). LCMS ESI(+)m/z: 294.1 (M+1).

Step F: (R)-6-(2-(ethoxymethoxy)-4,6-dimethylphenyl)-N-(1-methylpiperidin-3-yl)-1,2,4-triazin-3-amine (compound 1.6)

Compound 1.5 (44mg, 0.15mmol) was dissolved in 2mL n-butanol, (R)-1-methylpiperidine-3-atnine (43mg, 0.37mmol) and diisopropylethylamine (77mg, 0.60mmol) were added, and the reaction solution was stirred at 180 °C with microwave for 2 hours. The reaction solution was concentrated under reduced pressure, and the product was purified by column chromatography to obtain 1.6 (30 mg, yield: 54%). LCMS ESI(+)m/z: 372.2 (M+1).

Step G: (R)-3,5-dimethyl-2-(3-((1-methylpiperidin-3-yl)amino)-1,2,4-triazin-6-yl)phenol (compound 1)

Dissolve compound 1.6 (30 mg, 0.08 mmol) in 8 mL of hydrochloric acid/ethyl acetate (2 M (mol/L)) and stir for 1 hour at room temperature. The reaction solution was concentrated under reduced pressure and purified by reversed-phase prep-HPLCHPLC to obtain compound 1 (20 mg, yield: 80%). LCMS ESI(+)m/z: 314.2 <M+1).1H NMR (400 MHz, DMSO-J6) δ 10.89 (s, 1H), 8.28 (s, 1H), 7.94-7.64 (m, 1H), 6.67 (s, 1H), 6.62 (s, 1H), 4.39-4.37 (m, 1H), 3.62 (d, *J* = 12.6 Hz, 1H), 3.39 (d, *J* = 9.6 Hz, 1H), 3.09 - 2.81 (m, 2H), 2.80 (s, 3H), 2.26 (s, 3H), 2.10 (s, 4H), 1.90 (dd, *J*= 41.2, 8.6 Hz, 2H), 1.69 - 1.50 (m, 1H).

Embodiment 2

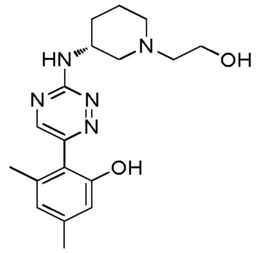

(R)-2-(3-((1-(2-hydroxyethyl)piperidin-3-yl)amino)-1,2,4-triazin-6-yl)-3,5-dimethylphenol

The specific reaction Formula is as follows:

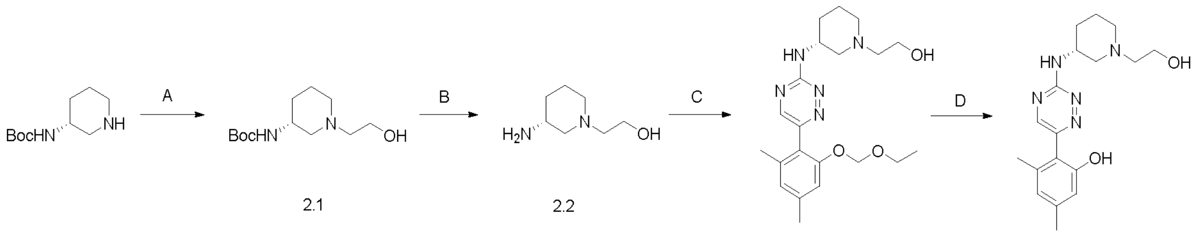


2.1 2.2 2.3 2

Step A: (R)-tert-butyl(1-(2-hydroxyethyl)piperidin-3-yl)carbatnate (compound 2.1)

Dissolve (R)-tert-butylpiperidin-3-ylcarbamate (1g, 5mmol) in 10mL of acetonitrile solution, add bromoethanol (812mg, 6.5mmol), sodium carbonate (800mg, 7.5mmol), and stir at 60°C for 6 hours. The reaction solution was filtered, the solvent was spun dry, and the compound was purified by column chromatography to obtain compound 2.1 (1.1g, yield: 90%). LCMS ESI(+)m/z: 245.2(M+1).

Step B: (R)-2-(3-aminopiperidin-1-yl)ethanol (compound 3.2)

Dissolve compound 2.1 (1.1g, 4.5 mmol) in 10 mL of dichloromethane solution, add 4 mL of trifluoroacetic acid, and stir for 2 hours at room temperature. Spin dry the solvent to obtain crude compound 2.2 (2g, yield: 100%). LCMS ESI(+)m/z: 145.2(M+1).

Step C: (R)-2-(3-((6-(2-(ethoxymethoxy)-4,6-dimethylphenyl)-1,2,4-triazin-3-yl)amino)piperidin-1-yl)ethanol (compound 2.3)

Compound 2.2 (110mg, 0.43mmol) was dissolved in 5mL of n-butanol solution, and 3-chloro-6-(2-(ethoxymethoxy)-4,6-dimethylphenyl)-1,2,4-triazine (50mg, 0.17mmol) and diisopropylethylamine (111mg, 0.8mmol) were added and stirred at 160°C for 3 hours. Compound 2.3 (50 mg, yield: 80%) was obtained by spinning the solvent and column chromatography. LCMS ESI(+)m/z: 402.2(M+1).

Step D: (R)-2-(3-((1-(2-hydroxyethyl)piperidin-3-yl)amino)-1,2,4-triazin-6-yl)-3,5-dimethylphenol (compound 2)

Compound 2.3 (50 mg, 0.13 mmol) was dissolved in 5 mL of ethyl acetate solution, 5 mL of ethyl acetate hydrochloride was added, and stirred for 2 h at room temperature. Compound 2 (25 mg, yield: 58%) was purified by spin-drying solvent and reversed-phase preparation. LCMS ESI(+)m/z: 344.2(M+1). $^1$H NMR (400 MHz, DMSO) $\delta$ 10.57 (s, 1H), 8.28 (s, 1H), 6.68 (s, 1H), 6.62 (s, 1H), 4.50 (s, 1H), 3.85 (s, 2H), 3.70 (s, 2H), 3.54 (s, 2H), 3.23 (s, 2H), 2.93 (d, *J*= 9.3 Hz, 2H), 2.26 (s, 3H), 2.10 (s, 3H), 1.96 (d, *J*= 12.4 Hz, 2H), 1.63 (d,J= 11.8 Hz, 1H).

## Embodiment 3

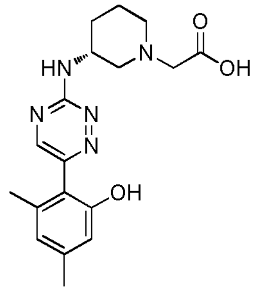

(R)-2-(3-((6-(2-hydroxy-4,6-dimethylphenyl)-1,2,4-triazin-3-yl)amino)piperidin-1-yl)acetic acid

The specific reaction Formula is as follows:

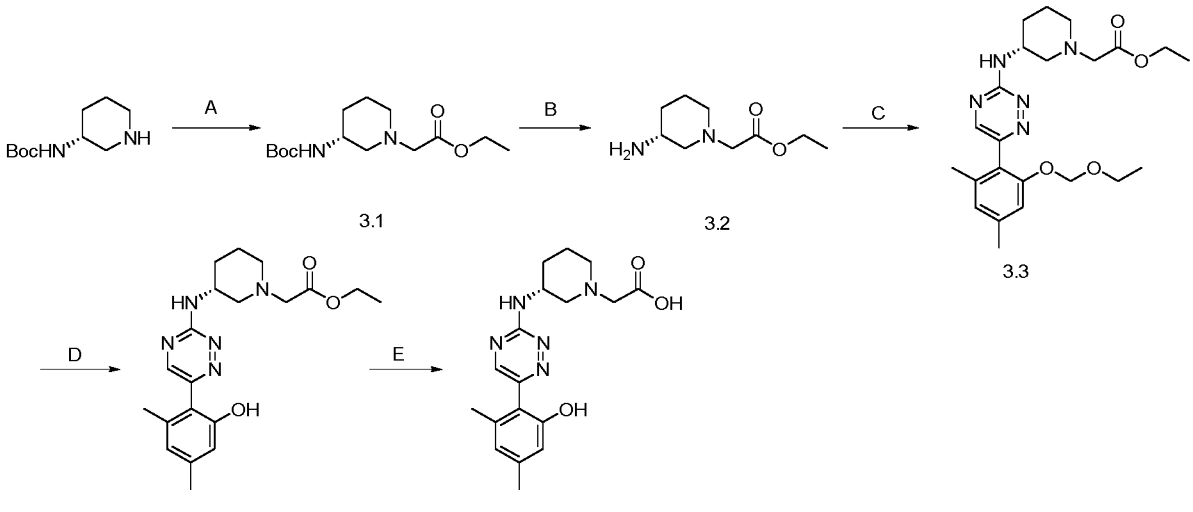


3.1 3.2 3.3 3.4 3

Step A: (R)-ethyl2-(3-((tert-butoxycarbonyl)amino)piperidin-1-yl)acetate (compound 3.1)

(R)-tert-butylpiperidine-3-ylcarbamate (500mg, 2.5mmol) was dissolved in 10mL of acetonitrile solution, ethyl bromoacetate (406mg, 3.25mmol) and sodium carbonate (400mg, 3.75mmol) were added, and stirred at 60°C for 6 hours. The reaction solution was filtered, the solvent was spun dry, and the compound was purified by column chromatography to obtain compound 3.1 (670mg, yield: 93%). LCMS ESI(+)m/z: 287.2(M+1).

Step B: (R)-2-(3-aminopiperidin-1-yl)ethyl acetate (compound 3.2)

Dissolve compound 3.1 (670 mg, 2.34 mmol) in 10 mL of dichloromethane solution, add 4 mL of trifluoroacetic acid, and stir for 2 hours at room temperature. Spin dry the solvent to obtain crude compound 3.2 (1.5g, yield: 100%). LCMS ESI(+)m/z: 187.1(M+1).

Step C: (R)-2-(3-((6-(2-(ethoxymethoxy)-4,6-dimethylphenyl)-1,2,4-triazin-3-yl)amino)piperidin-1-yl)ethyl acetate (compound 3.3)

Compound 3.2 (60mg, 0.20mmol) was dissolved in 5mL of n-butanol solution, and 3-chloro-6-(2-(ethoxymethoxy)-4,6-dimethylphenyl)-1,2,4-triazine (68mg, 0.23mmol) and diisopropylethylamine (133mg, 1.02mmol) were added and stirred at 160°C for 3 hours under microwave. The solvent was spun dry and the column chromatography was purified to obtain compound 3.3 (60 mg, yield: 66%). LCMS ESI(+)m/z: 444.3(M+1).

Step D: (R)-ethyl2-(3-((6-(2-hydroxy-4,6-dimethylphenyl)-1,2,4-triazin-3-yl)amino)piperidin-1-yl)acetate (compound 3.4)

Compound 3.3 (60 mg, 0.14 mmol) was dissolved in 5 mL of ethyl acetate solution, 5 mL of ethyl acetate hydrochloride solution was added, and stirred for 2 h at room temperature. The solvent was spun dry and the column chromatography was purified to obtain compound 3.4 (100 mg, yield: 58%). LCMS ESI(+)m/z: 386.2(M+1).

Step E: (R)-2-(3-((6-(2-hydroxy-4,6-dimethylphenyl)-1,2,4-triazin-3-yl)amino)piperidine-1-yl)acetic acid (compound 3)

Compound 3.4 (100 mg, 0.26 mmol) was dissolved in 5 mL of ethanol and 1 mL of aqueous solution, sodium hydroxide (52 mg, 1.3 mmol) was added, and stirred for 1 h at room temperature. Compound 3 (20 mg, yield: 22%) was obtained by spinning the solvent and reversed-phase preparation. LCMS ESI(+)m/z: 358.2(M+1). $^1$H NMR (400 MHz, DMSO) δ 9.44 (s, 1H), 8.21 (s, 1H), 7.56 (s, 1H), 6.60 (s, 2H), 4.06 (s, 1H), 3.21 (s, 2H), 3.08 (d, *J* = 8.9 Hz, 1H), 2.83 (d, *J* = 10.7 Hz, 1H), 2.39 (t, *J* = 10.1 Hz, 2H), 2.23 (s, 3H), 2.06 (s, 3H), 1.88 (d, *J* = 8.7 Hz, 1H), 1.74 (dd, *J* = 9.3, 4.2 Hz, 1H), 1.60 (d, *J* = 10.6 Hz, 1H), 1.42 (d, *J* = 9.5 Hz, 1H).

**Embodiment 4**

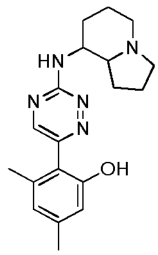

(R)-3,5-dimethyl-2-(9-((1,2,3,5,6,7,8,8a-octahydroindozine-9-yl)amino)-1,2,4-triazin-6-yl)phenol

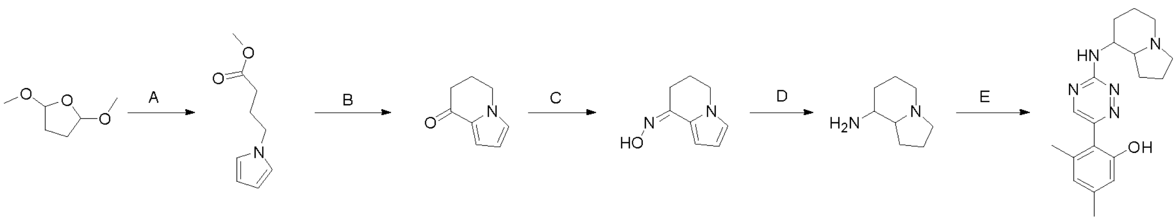


4.1 4.2 4.3 4.4 4

Step A: Methyl 4-(pyrrole-1-yl)butyrate (compound 4.1)

2,5-dimethoxytetrahydrofuran (5.1g, 38.7mmol), methyl 4-aminobutyrate hydrochloride (6.0g, 38.7mmol) and sodium acetate (3.21g, 38.7mmol) were added to the mixture of 60mL of water and 39mL of acetic acid, and the reaction was heated to 80°C for 2 hours. The reaction solution was extracted with 100mL of dichloromethane, washed with water, dried with anhydrous sodium sulfate, filtered, concentrated under reduced pressure, and purified by column chromatography to obtain a product of 4.1 (4.52g, yield: 70%). LCMS ESI(+)m/z: 168.1 (M+1).

Step B: 6,7-Dihydro-8(5H)-indolazinone (compound 4.2)

Compound 4.1 (4.52g, 27.1mmol) was dissolved in 180mL of methylene chloride, cooled to about 0 °C under nitrogen, phosphorus tribromide (7.8g, 28.7mmol) was slowly added dropwise to the reaction solution, and the mixture was stirred in an ice water bath for 30 minutes. The reaction was quenched by addition of the solution to 70mL of ice water, sodium bicarbonate saturated solution was added to adjust PH to neutral, stratified, washed organic phase with water, dried the organic phase with anhydrous sodium sulfate, filtered, filtrate concentrated under reduced pressure,

and purified by column chromatography to obtain a product of 4.2 (1.64g, yield: 44.9%). LCMS ESI(+)m/z: 136.1 (M+1).

Step C: 1-Azabicyclo[4.3.0]azelaic acid-6,8-diene-5-oxime (compound 4.3)

Compound 4.2 (1.24g, 9.18mmol), hydroxylamine hydrochloride (1.27g, 1.27mmol) and sodium acetate (2.48g, 30.2mmol) were added to a mixture of 14mL of water and 14mL of ethanol, and the reaction was heated to 80 °C for 2 hours. The reaction solution was concentrated to dry under reduced pressure, dissolved with 50mL ethyl acetate, washed with water, combined the organic phases, dried with anhydrous sodium sulfate, filtered, and the filtrate was concentrated until the white solid product was 4.3 (1.1g, yield: 79.7%). LCMS ESI(+)m/z: 151.1 (M+1).

Step D: 1,2,3,5,6,7,8,8a-Octahydroindolezine-8-atnmonia (compound 4.4)

Compound 4.3 (300mg, 2.2mmol) and platinum dioxide (40mg, 0.17mmol) were added to 50mL of methanol, hydrogenated at 0.1Mpa pressure, and the reaction was completed by stirring for 2 hours at room temperature. The reaction solution was filtered, and the filtrate was concentrated to dry compound 4.4 (250 mg, yield: 81.1%)LCMS ESI(+)m/z: 141.1(M+1).

Step E: (R)-3,5-dimethyl-2-(9-((1,2,3,5,6,7,8,8a-octahydroindolazine-9-yl)amino)-1,2,4-triazin-6-yl)phenol (compound 4)

Compounds 4.4 (50 mg, 0.357 mmmol), 3-chloro-6-(2-(ethoxymethoxy)-4, 6-dimethylphenyl)-1,2,4-triazine (40 mg, 0.136 mmol) and N,N-diisopropylethylamine (184.6 mg, 1.4 mmol) were added to 3mL of dioxane, and the reaction was heated to 140 °C under microwave for 1 hour. The reaction solution was extracted with 20mL of dichloromethane, washed with water, combined the organic phases, dried with anhydrous sodium sulfate, filtered, concentrated under reduced pressure, and purified compound 4 (10mg, yield: 21.7%) was prepared with reversed-phase. LCMS ESI(+)m/z: 340.2 (M+1). $^1$H NMR (400 MHz, DMSO-d6) δ 9.44 (d, *J* = 11.8 Hz, 1H), 8.22 (s, 1H), 8.17 (d, *J* = 4.4 Hz, 1H), 6.60 (s, 2H), 3.10 - 2.96 (m, 4H), 2.23 (s, 3H), 2.06 (s, 3H), 1.96 (dd, *J*= 29.5, 9.3 Hz, 4H), 1.70 - 1.46 (m, 6H).

Embodiment 5

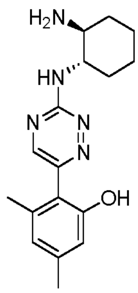

2-(3-((1S,2S)-2-aminocyclohexyl)amino)-1,2,4-triazin-6-yl)-3,5-dimethylphenol

The specific reaction Formula is as follows:

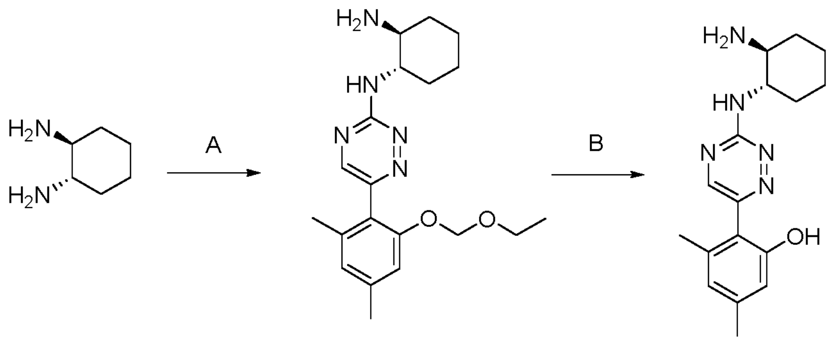


5.1 5

Step A: (1S,2S)-N1-(6-(2-(ethoxymethoxy)-4,6-dimethylphenyl)-1,2,4-triazin-3-yl)cyclohexane-1,2-diamine (compound 5.1)

(1S,2S)-cyclohexane-1,2-diamine (22.8 mg, 0.2 mmol), 3-chloro-6-(2-ethoxymethoxy)-4,6-dimethylphenyl)-1,2,4-triazine (29.3 mg, 0.1 mmol) and N,N-diisopropylethylamine (38.7 mg, 0.3 mmol) were dissolved in 3mL of n-butanol and placed in the microwave at 160 °C for 2 hours. The reaction solution is concentrated to obtain a crude product of compound 5.1, which is directly used in the next step.

Step B: 2-(3-((1S,2S)-2-aminocyclohexyl)amino)-1,2,4-triazin-6-yl)-3,5-dimethylphenol (compound 5)

Dissolve compound 5.1 crude in 5 mL of ethyl acetate solution (2M) of hydrogen chloride and stir for 0.5 hours at room temperature. The reaction solution was concentrated under reduced pressure and purified by reversed-phase prep-HPLCto obtain compound 5 (25mg, yield: 64.6%). LCMS ESI(+)m/z: 314.2 (M+1).$^1$H NMR (400 MHz, DMSO) δ 9.76 (s, 1H), 8.42 (d, J = 17.9 Hz, 1H), 8.31 - 8.05 (m, 3H), 6.68 (s, 1H), 6.61 (s, 1H), 4.08 (s, 1H), 3.38 - 2.99 (m, 1H), 2.24 (s, 3H), 2.16 - 2.06 (m, 4H), 2.02 (d, J = 11.7 Hz, 1H), 1.74 (d, J = 6.8 Hz, 2H), 1.58 - 1.35 (m, 2H), 1.34 - 1.20 (m, 2H).

## Embodiment 6

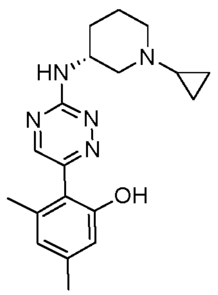

(R)-2-(3-((1-cyclopropylpiperidin-3-yl)amino)-1,2,4-triazin-6-yl)-3,5-dimethylphenol

The specific reaction Formula is as follows:

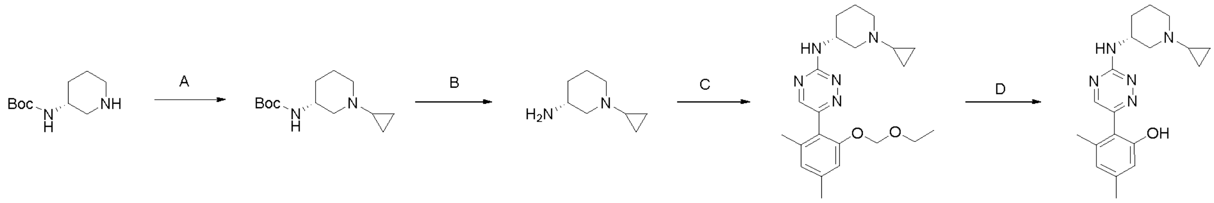


6.1 6.2 6.3 6

Step A: Tert-butyl(R)-(1-cyclopropylpiperidin-3-yl)carbamate (compound 6.1)

Tert-butyl(R)-piperidin-3-ylcarbamate (300mg, 1.5mmol) and (1-ethoxycyclopropoxy)trimethylsilane (522mg, 3mmol) were dissolved in 8mL of tetrahydrofuran and 8mL of methanol, and then sodium cyanoborohydride (141mg, 2.25mmol) was added sequentially at room temperature with nitrogen protection, Glacial acetic acid (450 mg, 7.5 mmol) and 4A active molecular sieve (0.5 g), the reaction solution was stirred at 60 °C for 7 h, using TLC to monitor the reaction to complete. The reaction solution is filtered, and the filtrate is concentrated under reduced pressure. The residue was dissolved with ethyl acetate, washed with 1M sodium hydroxide (4mL) and saturated saline, dried with anhydrous sodium sulfate, filtered, and concentrated under reduced pressure to obtain a crude product of 6.1 (489mg, yield: 100%).

Step B: (R)-1-cyclopropylpiperidin-3-atnine (compound 6.2)

Dissolve compound 6.1 (489 mg, 2 mmol) in 4 mL of hydrochloric acid/ethyl acetate (2 M) and stir for 1 h at room temperature. Use TLC to monitor the reaction to completion. The reaction solution was concentrated under reduced pressure to obtain a crude product of 6.2 (369mg, yield: 100%).

Step C: (R)-N-(1-cyclopropylpiperidin-3-yl)-6-(2-(ethoxymethoxy)-4,6-dimethylphenyl)-1,2,4-triazin-3-amine (compound 6.3)

The compound 6.2 (50 mg, 0.28 mmol) was dissolved in 2mL of n-butanol, and 3-chloro-6-(2-(ethoxymethoxy)-4,6-dimethylphenyl)-1,2,4-triazine (40 mg, 0.14 mmol) and diisopropylethylamine (90 mg, 0.70 mmol) were added, and the reaction solution was stirred at 150 °C under microwave for 1 hour. Use LCMS to monitor the reaction to completion. The reaction solution was concentrated under reduced pressure to obtain a crude product of 6.3 (60mg, yield: 100%). LCMS ESI(+)m/z: 398.1 (M+1).

Step D: (R)-2-(3-((1-cyclopropylpiperidin-3-yl)amino)-1,2,4-triazin-6-yl)-3,5-dimethylphenol (compound 6).

Dissolve compound 6.3 (60 mg, 0.15 mmol) in 3 mL of hydrogen chloride/ethyl acetate (2 M) and stir for 1 h at room temperature. Use LCMS to monitor the reaction to completion. The reaction solution was concentrated under reduced pressure and purified by reversed-phase prep-HPLCto obtain compound 6 (20 mg, yield: 39%). LCMS ESI(+)m/z: 340.1 (M+1). $^1$H NMR(400 MHz, DMSO-d6) $\delta$ 10.45 (s, 1H), 8.33 (d, *J* = 16.9 Hz, 1H), 8.14 (d, *J* = 92.0 Hz, 2H), 6.62 (d, J= 10.1 Hz, 3H), 4.64 - 4.10 (m, 2H), 3.04 (dd, *J* = 22.3, 11.0 Hz, 2H), 2.89 (s, 1H), 2.23 (s, 4H), 2.07 (s, SH), 1.88 (d, J= 29.6 Hz, 3H), 1.58 (s, 1H), 1.24 (s, 1H), 1.12 (d, J= 10.6 Hz, 2H), 0.81 (t, J= 8.4 Hz, 2H).

## Embodiment 7

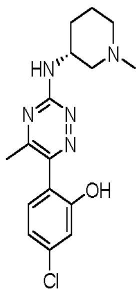

(R)-5-Chloro-2-(5-methyl-3-((1-methylpiperidin-3-yl)amino)-1,2,4-triazin-6-yl)phenol

The specific reaction Formula is as follows:

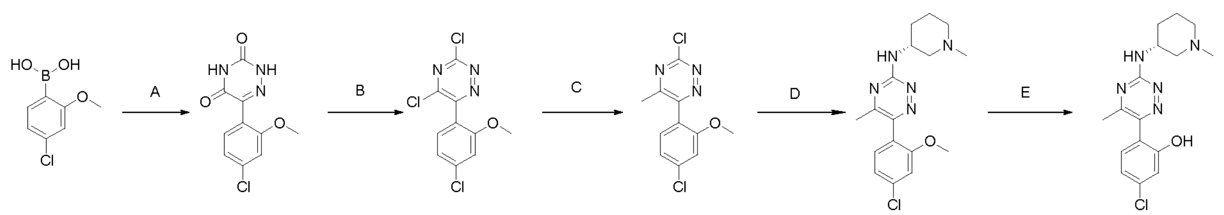

7.1 7.2 7.3 7.4 7

Step A: 6-(4-chloro-2-methoxyphenyl)-1,2,4-triazin-3,5(2H,4H)-dione (compound 7.1)

The compounds (4-chloro-2-methoxyphenyl)boric acid (1.00g, 5.36mmol) and 6-bromo-1,2,4-triazin-3,5(2H,4H)-dione (1.24g, 6.44mmol) were dissolved in 25mL of dioxane and 3mL of water, and potassium carbonate (2.22g, 16.1mmol) and $PdCl_2$(dppf) (390mg, 0.536mmol) were added, the reaction solution was stirred at 100 °C under nitrogen for 16 h. 50mL of water was added to the reaction solution, the pH was adjusted to 4 with citric acid solution, extracted with ethyl acetate (80mL×2), the organic phase was combined, washed with brine, dried with anhydrous sodium sulfate, filtered, concentrated under reduced pressure, and purified by column chromatography to obtain a product of 7.1 (1.27g, yield: 94%). LCMS ESI(+)m/z: 254 (M+1).

Step B: 3,5-Dichloro-6-(4-chloro-2-methoxyphenyl)-1,2,4-triazine (compound 7.2)

Compound 7.1 (1.27g, 5.01mmol) was dissolved in 20mL of phosphorus oxychloride, 2mL of diisopropylethylamine was added, and the reaction solution was stirred at 100 °C under nitrogen for 10 hours. The reaction solution was concentrated under reduced pressure, the residue was added dropwise to 100mL of water, the pH was adjusted to 8 with sodium bicarbonate solution, extracted with ethyl acetate (100mL×3), the organic phase was combined, washed with brine, dried with anhydrous sodium sulfate, filtered, concentrated under reduced pressure, and the product was purified by column chromatography to obtain a product of 7.2 (780mg, yield: 54%). LCMS ESI(+)m/z: 290 (M+1).

Step C: 3-Chloro-6-(4-chloro-2-methoxyphenyl)-5-methyl-1,2,4-triazine (compound 7.3)

Compound 7.2 (280mg, 0.964mmol) was dissolved in 10mL of anhydrous tetrahydrofuran, cooled to -60 °C, under the protection of nitrogen, magnesium methyl bromide (1.93mL, 1.93mmol) was added dropwise, the reaction solution was stirred at -60 °C for 0.5 hours, and then gradually raised to -20 °C, and the stirring continued for 1 hour. Ammonium chloride solution was added to the reaction solution, extracted with ethyl acetate (100mL×2), combined the organic phases, washed with brine, dried with anhydrous sodium sulfate, filtered, concentrated under reduced pressure, and purified by column chromatography to obtain a product of 7.3 (35mg, yield: 14%). LCMS ESI(+)m/z: 270 (M+1).

Step D: (R)-6-(4-chloro-2-methoxyphenyl)-5-methyl-N-(1-methylpiperidin-3-yl)-1,2,4-triazin-3-amine (compound 7.4)

The compounds 7.3 (35mg, 0.13mmol) and (R)-1-methylpiperidine-3-atnine hydrochloride (31mg, 0.168mmol) were dissolved in 3mL of n-butanol, diisopropylethylamine (66.8mg, 0.518mmol) was added, and the reaction solution was microwaved at 150 °C under nitrogen for 2 hours. The reaction solution was concentrated under reduced pressure, and the residue was purified by column chromatography to obtain a product of 7.4 (60mg, yield: 100%). LCMS ESI(+)m/z: 348.2 (M+1).

Step E: (R)-5-chloro-2-(5-methyl-3-((1-methylpiperidin-3-yl)amino)-1,2,4-triazin-6-yl)phenol (compound 7)

Dissolve compound 7.4 (60 mg, 0.172 mmol) in 8 mL of dichloromethane, add boron tribromide (173 mg, 0.690 mmol) dropwise at 0 °C and nitrogen protection, stirring at 0 °C for 1 h. 5mL of water was added dropwise to quench the reaction, the pH was adjusted to 8 with sodium bicarbonate solution, extracted with ethyl acetate (40mL×2), the organic phase was combined, washed with brine, dried with anhydrous sodium sulfate, filtered, concentrated under reduced pressure, and purified with reversed-phase prep-HPLCto obtain compound 7 (15mg, yield: 26%). LCMS ESI(+)m/z: 334.1 (M+1).$^{1H\ NMR\ (400\ MHz,\ DMSO-}$*d6*) δ 10.65-10.61 (m, 2H), 8.13-8.10 (m, 1H), 7.27 (d, J= 8.1 Hz, 1H), 7.09 (d, J= 2.0 Hz, 1H), 6.99 (dd, J= 8.2, 2.0 Hz, 1H), 4.35-4.30 (m, 1H), 3.69 - 3.48 (m, 1H), 3.36-3.30 (m, 1H), 3.04 - 2.73 (m, 5H), 2.24 (d, *J*= 5.3 Hz, 3H), 2.04 (d, *J* = 11.2 Hz, 1H), 1.98 - 1.73 (m, 2H), 1.59 - 1.42 (m, 1H).

**Embodiment 8**

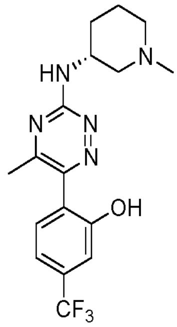

(R)-2-(5-methyl-3-((1-methylpiperidin-3-yl)amino)-1,2,4-triazin-6-yl)-5-(trifluoromethyl)phenol

The specific reaction Formula is as follows:

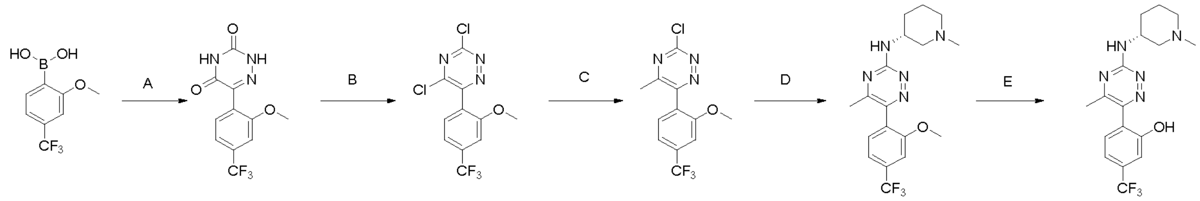


8.1 8.2 8.3 8.4 8

Step A: 6-(2-methoxy-4-(trifluoromethyl)phenyl)-1,2,4-triazin-3,5(2H,4H)-dione (compound 8.1)

(2-Methoxy-4-(trifluoromethyl)phenyl)boric acid (900mg, 4.09mmol) and 6-bromo-1,2,4-triazin-3,5(2H,4H)-dione (943mg, 4.91mmol) were dissolved in 10mL of dioxane and 1mL of water, and potassium carbonate (1.69g, 12.28mmol) and $PdCl_2$(dppf) (297mg, 0.40mmol) were added, the reaction mixture was microwaved at 100 °C under nitrogen for 2 hours. 50mL of water was added to the reaction solution, the pH was adjusted to 4 with citric acid solution, extracted with ethyl acetate (80mL×2), the organic phase was combined, washed with brine, dried with anhydrous sodium sulfate, filtered, concentrated under reduced pressure, and purified by column chromatography to obtain a product of 8.1 (1.08g, yield: 91%). LCMS ESI(+)m/z: 288.1 (M+1).

Step B: 3,5-Dichloro-6-(2-methoxy-4-(trifluoromethyl)phenyl)-1,2,4-triazine (compound 8.2)

Compound 8.1 (800mg, 2.79mmol) was dissolved in 10mL of phosphorus oxychloride, 1mL of diisopropylethylamine was added, and the reaction solution was stirred at 100°C under nitrogen for 7 hours. The reaction solution was concentrated under reduced pressure, the residue was added dropwise to 100mL of water, the pH was adjusted to 8 with sodium bicarbonate solution, extracted with ethyl acetate (40mL×2), the organic phase was combined, washed with brine, dried with anhydrous sodium sulfate, filtered, concentrated under reduced pressure, and purified by column chromatography to obtain a product of 8.2 (640mg, yield: 71%). LCMS ESI(+)m/z: 324 (M+1).

Step C: 3-chloro-6-(2-methoxy-4-(trifluoromethyl)phenyl)-5-methyl-1,2,4-triazine (compound 8.3)

Compound 8.2 (100 mg, 0.31 mmol) was dissolved in 6mL of anhydrous tetrahydrofuran, cooled to -60 °C, under the protection of nitrogen, magnesium methyl bromide (0.46 mL, 0.46 mmol) was added dropwise, and the reaction solution was stirred at -60 °C for 0.5 hours, and then gradually increased to -20 °C, and continued to stir at -20 °C for 1 hour. Ammonium chloride solution was added to the reaction solution, extracted with ethyl acetate (40mL×2), combined the organic phases, washed with brine, dried with anhydrous sodium sulfate, filtered, concentrated under reduced pressure, and purified by column chromatography to obtain a product of 8.3 (15mg, yield: 16%). LCMS ESI(+)m/z: 304 (M+1).

Step D: (R)-6-(2-methoxy-4-(trifluoromethyl)phenyl)-5-methyl-N-(1-methylpiperidin-3-yl)-1,2,4-triazin-3-amine (compound 8.4)

The compounds 8.3 (15mg, 0.05mmol) and (R)-1-methylpiperidine-3-amine hydrochloride (10mg, 0.05mmol) were dissolved in 3mL of n-butanol, diisopropylethylamine (32mg, 0.247mmol) was added, and the reaction solution was microwaved at 180 °C under nitrogen for 2 hours. The reaction solution was concentrated under reduced pressure, and the residue was purified by column chromatography to obtain a product of 8.4 (30mg, yield: 100%). LCMS ESI(+)m/z: 382.2 (M+1).

Step E: (R)-2-(5-methyl-3-((1-methylpiperidin-3-yl)amino)-1,2,4-triazin-6-yl)-5-(trifluoromethyl)phenol (compound 8).

Dissolve compound 8.4 (30 mg, 0.08 mmol) in 8 mL of methylene chloride, add boron tribromide (59 mg, 0.23 mmol) dropwise at 0 °C under nitrogen, stirring at 0 °C for 1h. 5mL of water was added dropwise to quench the reaction, the pH was adjusted to 8 with sodium bicarbonate solution, extracted with ethyl acetate (40mL×2), the organic phase was combined, washed with brine, dried with anhydrous sodium sulfate, filtered, concentrated under reduced pressure, and purified with reversed-phase prep-HPLCto obtain compound 8 (7mg, yield: 31%). LCMS ESI(+)m/z: 368.2 (M+1).$^1$H NMR (400 MHz, Methanol-d4) δ 8.50 (s, 1H), 7.46 (d, *J* = 7.8 Hz, 1H), 7.26 (d, *J* = 8.8 Hz, 1H), 7.20 (s, 1H), 4.34-4.30 (m, 1H), 3.50 ( d, *J* = 14.1 Hz, 1H), 3.23 (d, *J* = 12.0 Hz, 1H), 2.91 - 2.75 (m, 2H), 2.74 (s, 3H), 2.32 (s, 3H), 2.18 - 2.00 (m, 2H), 1.97 - 1.79 (m, 1H), 1.72-1.66 (m, 1H).

**Embodiment 9**

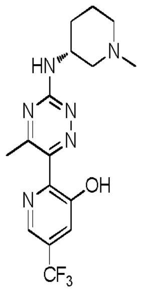

(R)-2-(5-methyl-3-((1-methylpiperidin-3-yl)amino)-1,2,4-triazin-6-yl)-5-(trifluoromethyl)pyridin-3-ol

The specific reaction Formula is as follows:

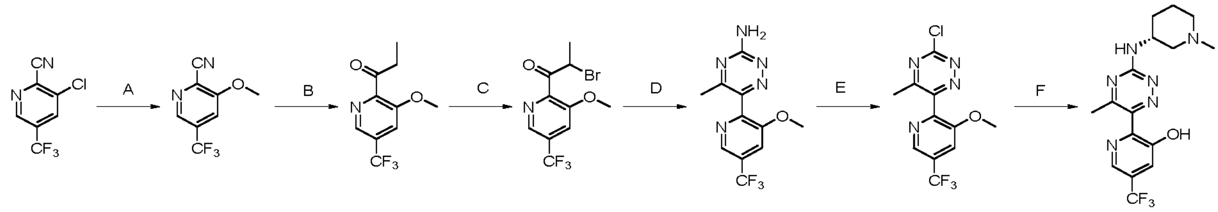


9.1 9.2 9.3 9.4 9.5 9

Step A: 3-Methoxy-5-(trifluoromethyl)picolinonitrile(compound 9.1)

3-chloro-S-(trifluoromethyl)picolinonitrile (3.0g, 14.56mmol) was dissolved in 30mL of methanol, sodium methoxide (1.18g, 21.84mmol) was added, and the reaction solution was stirred at 70°C for 16 hours. The reaction solution was concentrated under reduced pressure, 50mL of water was added to the residue, the solids were precipitated by pulping, the crude product was filtered and valuum dried to obtain compound 9.1 (2.52 g, yield: 86%). LCMS ESI(+)m/z: 203 (M+1).

Step B: 1-(3-methoxy-5-(trifluoromethyl)pyridin-2-yl)-1-one (compound 9.2)

Compound 9.1 (1.58 g, 7.82 mmol) was dissolved in 20 mLTHF, magnesium ethyl bromide (11.7 mL, 11.71 mmol) was added under nitrogen in an ice water bath, and the reaction was stirred for 2.5 hours at room temperature. 50mL of saturated ammonium chloride was added to the reaction solution, extracted with ethyl acetate (50mL×2), combined the organic phases, washed with brine, dried with anhydrous sodium sulfate, filtered, concentrated under reduced pressure, and purified by column chromatography to obtain a product of 9.2 (730mg, yield: 40%). LCMS ESI(+)m/z: 234.1 (M+1).

Step C: 2-bromo-1-(3-methoxy-5-(trifluoromethyl)pyridin-2-yl)propane-1-one (compound 9.3).

Compound 9.2 (570 mg, 2.45 mmol) was dissolved with 20 mL of acetonitrile, NBS (522 mg, 2.94 mmol) and p-toluenesulfonic acid (93 mg, 0.49 mmol) were added, and the reaction solution was stirred at 60°C for 4 hours. 50mL of water was added to the reaction solution, extracted with ethyl acetate (80mL×2), combined the organic phases, washed with brine, dried with anhydrous sodium sulfate, filtered, concentrated under reduced pressure, and purified by column chromatography to obtain a product of 9.3 (625mg, yield: 82%). LCMS ESI(+)m/z: 312, 314 (M+1).

Step D: 6-(3-methoxy-5-(trifluoromethyl)pyridin-2-yl)-5-methyl-1,2,4-triazin-3-amine (compound 9.4)

Compound 9.3 (280 mg, 0.87 mmol) was dissolved in 10 mLDMF, aminoguanidine hydrochloride (114 mg, 1.04 mmol), triethylamine (264 mg, 2.61 mmol) and sodium iodide (130 mg, 0.87 mmol) were added, and the reaction solution was stirred at room temperature for 16 hours. The reaction solution was concentrated under reduced pressure, 50mL of water was added to the residue, extracted with ethyl acetate (80mL×2), combined the organic phases, washed with brine, dried with anhydrous sodium sulfate, filtered, concentrated under reduced pressure, and purified with reversed-phase prep-HPLCto obtain a product of 9.4 (70mg, yield: 28%). LCMS ESI(+)m/z: 286.1 (M+1).

Step E: 3-chloro-6-(3-methoxy-5-(trifluoromethyl)pyridin-2-yl)-5-methyl-1,2,4-triazine (compound 9.5)

Compound 9.4 (70 mg, 0.25 mmol) was dissolved in 4 mLTHF, anhydrous copper chloride (50 mg, 0.37 mmol) and tert-butyl nitrite (38 mg, 0.37 mmol) were added, and the reaction solution was stirred at 60°C for 1 hour. The reaction solution was concentrated under reduced pressure, 50mL of water was added to the residue, the pH was adjusted to 7-8 with saturated sodium bicarbonate, extracted with dichloromethane (80mL×2), the organic phase was combined, washed with brine, dried with anhydrous sodium sulfate, filtered, concentrated under reduced pressure, and purified by column chromatography to obtain a product of 9.5 (13mg, yield: 17%). LCMS ESI(+)m/z: 305 (M+1).

Step F: (R)-2-(5-methyl-3-((1-methylpiperidin-3-yl)amino)-1,2,4-triazin-6-yl)-5-(trifluoromethyl)pyridin-3-ol (compound 9).

Compound 9.5 (13 mg, 0.04 mmol) was dissolved in 3 mL of NMP, (R)-1-methylpiperidine-3-atnine (16 mg, 0.09 mmol) and diisopropylethylamine (31 mg, 0.24 mmol) were added, and the reaction solution was stirred at 160°C for 3 hours. 50mL of water was added to the reaction solution, extracted with ethyl acetate (80mL×2), combined the organic phases, washed with brine, dried with anhydrous sodium sulfate, filtered, concentrated under reduced pressure, and purified with reversed-phase prep-HPLC to obtain compound 9 (2.4mg, yield: 16%). LCMS ESI(+)m/z: 369.2 (M+1). $^1$H NMR (400 MHz, MeOD) $\delta$ 8.46 (d, *J* = 16.5 Hz, 2H), 7.59 (d, *J* = 1.3 Hz, 1H), 4.26 (s, 1H), 3.52 (d, *J* = 10.4 Hz, 1H), 3.22 (d, *J* = 12.1 Hz, 1H), 2.93 - 2.78 (m, 2H), 2.74 (s, 3H), 2.65 (s, 3H), 2.16 - 2.01 (m, 2H), 1.85 (ddd,*J* = 21.3, 10.6, 3.8 Hz, 1H), 1.75 - 1.62 (m, 1H).

**Embodiment 10**

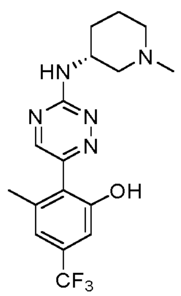

(R)-3-methyl-2-(3-((1-methylpiperidin-3-yl)amino)-1,2,4-triazin-6-yl)-5-(trifluoromethyl)phenol

The specific reaction Formula is as follows:

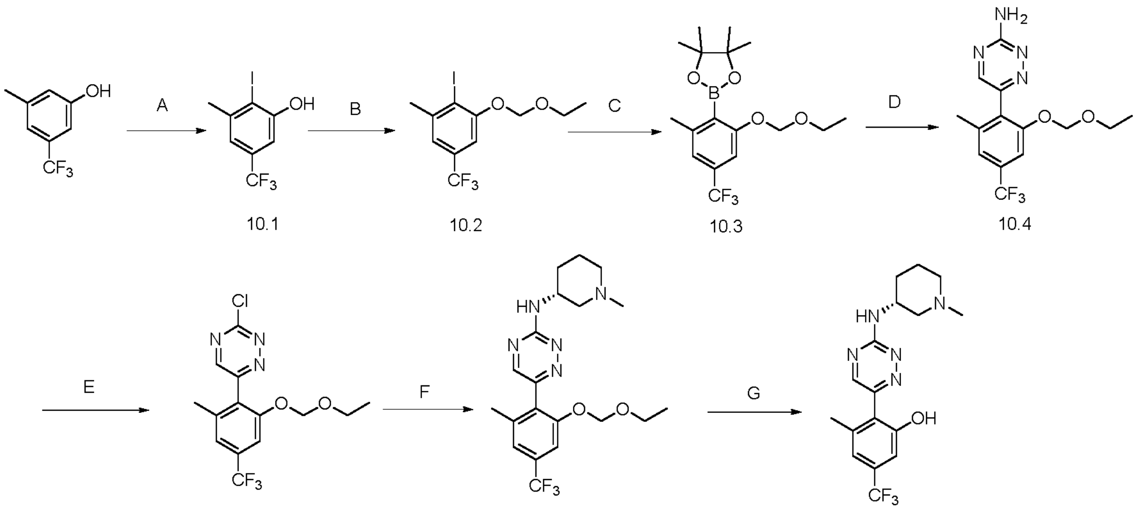

10.1 10.2 10.3 10.4
10.5 10.6 10

Step A: 2-Iodo-3-methyl-5-(trifluoromethyl)phenol (compound 10.1)

3-methyl-5-(trifluoromethyl)phenol (500mg, 2.84mmol) was dissolved in 7mL of toluene, sodium hydride (227mg, 5.68mmol) was added with an ice-water bath cooling and stirred for half an hour, then iodine (720mg, 2.84mmol) was added, stirred with an ice-water bath cooling for 3 hours, and then the reaction was quenched with an aqueous solution of hydrochloric acid, and extracted with ethyl acetate (30mL×2). The organic phase was combined, washed with brine, dried with anhydrous sodium sulfate, filtered, concentrated under reduced pressure, and reversed-phase prep-HPLC and purified to obtain a product of 10.1 (457mg, yield: 53.2%). LCMS ESI(+)m/z: 303.1 (M+1).

Step B: 1-(ethoxymethoxy)-2-iodo-3-methyl-5-(trifluoromethyl)benzene (compound 52.2)

Compound 10.1 (457mg, 1.51mmol) was dissolved in 4mL of DMF, cesium carbonate (493mg, 1.51mmol) and (chloromethoxy)ethane (179mg, 1.89mmol) were added with an ice-water bath cooling and stirred for 3 hours, and then the reaction was quenched with water solution and extracted with ethyl acetate (30mL×2). The organic phase was combined, washed with brine, dried with anhydrous sodium sulfate, filtered, concentrated under reduced pressure, and purified by reversed-phase prep-HPLC to obtain a product of 10.2 (160mg, yield: 29.4%). LCMS ESI(+)m/z: 361.1 (M+1).

Step C: 2-(2-(ethoxymethoxy)-6-methyl-4-(trifluoromethyl)phenyl)-4,4,5,5-tetramethyl-1,3,2-dioxaborolane (compound 10.3)

Compound 10.2 (160 mg, 0.444 mmol) was added to 3 mL of 1,4-dioxane, followed by pinaol biborate (284 mg, 2.22 mmol), triethylamine (328 mg, 3.24 mmol), CyJohnphos (Chinese full name 2-(dicyclohexylphosphine) biphenyl) (39 mg, 0.111 mmol) and Pd(OAc)$_2$ (15mg, 0.066mmol), let the reaction stir at 95 °C for 18 hours, quench the reaction with water, filter the reaction solution, and extract with ethyl acetate (60mL×3).The organic phase was combined, washed

with brine, dried with anhydrous sodium sulfate, filtered, concentrated under reduced pressure, and purified by column chromatography to obtain a product of 10.3 (50mg, yield: 31.3%). LCMS ESI(+)m/z: 361.1 (M+1).

Step D: 6-(2-(ethoxymethoxy)-6-methyl-4-(trifluoromethyl)phenyl)-1,2,4-triazin-3-amine (compound 10.4)

The compounds 10.3 (50 mg, 0.139 mmol) and 6-bromo-1,2,4-triazin-3-amine (25 mg, 0.139 mmol) were dissolved in 2 mL of dioxane and 0.2 mL of water, and cesium carbonate (91 mg, 0.278 mmol) and $Pd(PPh_3)_4$ (32 mg, 0.028 mmol) were added , the reaction solution was stirred at 110 °C under nitrogen for 2 hours in a microwave environment. 10mL of water was added to the reaction solution, extracted with ethyl acetate (20mL×2), combined the organic phases, washed with brine, dried with anhydrous sodium sulfate, filtered, concentrated under reduced pressure, and purified by column chromatography to obtain a product of 10.4 (31mg). LCMS ESI(+)m/z: 329.1 (M+1).

Step E: 3-chloro-6-(2-(ethoxymethoxy)-6-methyl-4-(trifluoromethyl)phenyl)-1,2,4-triazine (compound 10.5)

Compound 10.4 (31mg, 0.095mmol) was dissolved in 2mL of acetonitrile, and then copper chloride (19mg, 0.143mmol) and tert-butyl nitrite (15mg, 0.143mmol) were added under nitrogen at 0 °C, and the reaction solution was stirred at 60°C for 1.5 hours. The reaction solution was concentrated under reduced pressure and purified by column chromatography to obtain a product of 10.5 (15mg, yield: 45.3%). LCMS ESI(+)m/z: 348.1 (M+1).

Step F: (R)-6-(2-(ethoxymethoxy)-6-methyl-4-(trifluoromethyl)phenyl)-N-(1-methylpiperidin-3-yl)-1,2,4-triazin-3-amine (compound 10.6)

Compound 10.5 (15mg, 0.043mmol) was dissolved in 2mL n-butanol, (R)-1-methylpiperidine-3-amine (10mg, 0.052mmol) and diisopropylethylamine (27mg, 0.215mmol) were added, and the reaction solution was stirred at 150°C under microwave for 2 hours. The reaction solution was concentrated under reduced pressure to obtain a crude product of 10.6 (15mg). LCMS ESI(+)m/z: 425.1 (M+1).

Step G: (R)-3-methyl-2-(3-((1-methylpiperidin-3-yl)amino)-1,2,4-triazin-6-yl)-5-(trifluoromethyl)phenol (compound 10)

Dissolve compound 10.6 (20 mg, 0.08 mmol) in 8 mL of hydrogen chloride/ethyl acetate (2 M) solution and stir for 1 hour at room temperature. The reaction solution was concentrated under reduced pressure and purified with reversed-phase prep-HPLC to obtain compound 10 (3 mg). LCMS ESI(+)m/z: 367.1 (M+1). $^1$H NMR (400 MHz, DMSO) $\delta$ 10.31 (s, 1H), 8.30 (s, 1H), 7.15 (d, J = 1.7 Hz, 1H), 7.08 (d, J = 1.8 Hz, 1H), 3.99 (s, 1H), 2.93 (s, 1H), 2.68 (d, J = 12.1 Hz, 1H), 2.20 (d, J = 16.1 Hz, 6H), 1.92 (dd, J = 23. 5, 11.8 Hz,3H), 1.78 - 1.67 (m, 1H), 1.55 (d, J = 12.5 Hz, 1H), 1.40 - 1.30 (m, 1H).

**Embodiment 11**

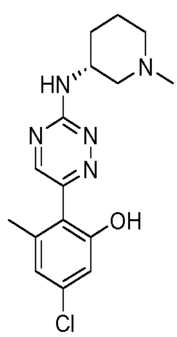

(R)-5-chloro-3-methyl-2-(3-((1-methylpiperidin-3-yl)amino)-1,2,4-triazin-6-yl)phenol

The specific reaction Formula is as follows:

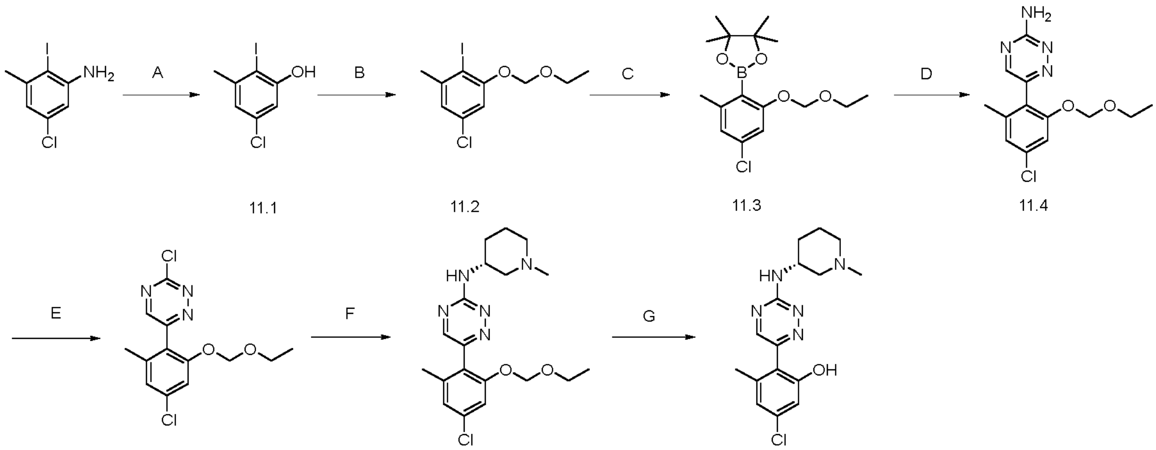


11.1 11.2 11.3 11.4 11.5 11.6 11

Step A: 5-chloro-2-iodo-3-methylphenol (compound 11.1)

5-chloro-2-iodo-3-methylaniline (1 g, 3.74 mmol) is dissolved in 4.5 mL of HCl (1 M), and then sodium nitrite aqueous solution (310 mg, 4.49 mmol) is slowly added dropwise with an ice water bath cooling. After stirring at 0°C for 15 minutes, concentrated sulfuric acid (1.8 mL) was added to the reaction solution and heated and refluxed for 1 hour. Use TLC to monitor the reaction to completion. The reaction was quenched by adding an aqueous solution to the reaction solution, extracted with ethyl acetate (150mL×2), combined the organic phases, washed with brine, dried with anhydrous sodium sulfate, filtered, and concentrated under reduced pressure. The resulting residue was purified by column chromatography to obtain a product of 11.1 (578 mg, yield: 57%).

Step B: 5-chloro-1-(ethoxymethoxy)-2-iodo-3-toluene (compound 11.2)

11.1 (578 mg, 2.15 mmol) and (chloromethoxy)ethane (407 mg, 4.3 mmol) were dissolved in 40 mL of DMF, and then at room temperature, cesium carbonate (1.4 g, 4.3 mmol) was added, and the reaction solution was stirred overnight at room temperature. Use TLC to monitor the reaction to completion. An aqueous solution was added to the reaction solution and quenched, extracted with ethyl acetate (100mL×2), the organic phases were combined, and washed with brine, dried with anhydrous sodium sulfate, filtered, and concentrated under reduced pressure. The resulting residue was purified by column chromatography to obtain a product of 11.2 (416 mg, yield: 59%).

Step C: 2-(4-chloro-2-(ethoxymethoxy)-6-methylphenyl)-4,4,5,5-tetramethyl-1,3,2-dioxybenzaldehyde (compound 11.3)

Compound 11.2 (416 mg, 1.27 mmol) is added to 5 mL of 1,4-dioxane, followed by 4,4,5,5-tetramethyl-1,3,2-dioxaborolane (488 mg, 3.81 mmol), triethylamine (530 μL, 3.81 mmol), $Pd(OAc)_2$(57 mg, 0.254 mmol) and CyJohnphos (2-(dicyclohexylphosphine)biphenyl) (134 mg, 0.381 mmol) and let the reaction stir overnight at 100 °C and under the protection of argon. Use TLC to monitor the reaction to completion. The reaction solution was concentrated under reduced pressure, and the product was purified by column chromatography to obtain 11.3 (217mg, yield: 53%).

Step D: 6-(4-chloro-2-(ethoxymethoxy)-6-methylphenyl)-1,2,4-triazin-3-amine (compound 11.4)

The compounds 11.3 (217 mg, 0.67 mmol) and 6-bromo-1,2,4-triazin-3-amine (98 mg, 0.56 mmol) were dissolved in 6 mL of dioxane and 1 mL of water, cesium carbonate (547 mg, 1.68 mmol) and PdCl2(dppf) (129 mg, 0.112 mmol) were added, and the reaction solution was stirred at 100 °C and under argon for 16 hours. Use LCMS to monitor the reaction to completion. 50mL of water was added to the reaction solution, extracted with ethyl acetate (80mL×2), combined the organic phases, washed with brine, dried with anhydrous sodium sulfate, filtered, concentrated under reduced pressure, and purified by column chromatography to obtain a product of 11.4 (84mg, yield: 43%). LCMS ESI(+)m/z: 295.1 (M+1).

Step E: 3-chloro-6-(4-chloro-2-(ethoxymethoxy)-6-methylphenyl)-1,2,4-triazine (compound 11.5)

Compound 11.4 (84 mg, 0.285 mmol) was dissolved in 5 mL of acetonitrile, and then copper chloride (58 mg, 0.428 mmol) and tert-butyl nitrite (44 mg, 0.428 mmol) were added under nitrogen with an ice water bath cooling. The

reaction solution is then stirred at 60 °C for 2 h. Use LCMS to monitor the reaction to completion. The reaction solution was concentrated under reduced pressure, and the product was purified by column chromatography to obtain 11.5 (8 mg, yield: 9%). LCMS ESI(+)m/z: 315.1 (M+1).

Step F: (R)-6-(4-chloro-2-(ethoxymethoxy)-6-methylphenyl)-N-(1-methylpiperidin-3-yl)-1,2,4-triazin-3-amine (compound 11.6)

Compound 11.5 (8 mg, 0.0256 mmol) was dissolved in 2 mL of n-butanol, (R)-1-methylpiperidine-3-amine (5 mg, 0.0282 mmol) and diisopropylethylamine (16 mg, 0.128 mmol) were added, and the reaction solution was stirred at 150 °C under microwave for 1 hour. Use LCMS to monitor the reaction to completion. The reaction solution was concentrated under reduced pressure to obtain a crude product of 11.6 (25mg). LCMS ESI(+)m/z: 392.1 (M+1).

Step G: (R)-5-chloro-3-methyl-2-(3-((1-methylpiperidin-3-yl)amino)-1,2,4-triazin-6-yl)phenol (compound 11).

Dissolve compound 11.6 (25 mg, 0.064 mmol) in 3 mL of 2 M hydrochloric acid/ethyl acetate and stir for 1 h at room temperature. Use LCMS to monitor the reaction to completion. The reaction solution was concentrated under reduced pressure and purified by reversed-phase prep-HPLC to obtain compound 11 (6.5 mg, yield: 76%). LCMS ESI(+)m/z: 334.1 (M+1). $^1$H NMR (400 MHz, MeOD) $\delta$ 6.83 (s, 1H), 6.76 (d, *J* = 1.8 Hz, 1H), 5.45 - 5.32 (m, 1H), 4.29 (s, 1H), 4.11 (s, 1H), 3.79 (d, *J*= 10.0 Hz, 1H), 3.69 (d, J= 8.1 Hz, 1H), 3.53 (d, *J* = 11.9 Hz, 1H), 2.99 (d, *J* = 11.9 Hz, 1H), 2.94 (t, *J* = 6.3 Hz, 3H), 2.30 (s, 3H) , 2.22 (d, *J* = 12.9 Hz, 1H), 2.11 (d, *J* = 15.0 Hz, 1H), 2.02 (d, *J* = 6.0 Hz, 1H), 1.66 (d, *J* = 12.3 Hz, 1H).

**Embodiment 12**

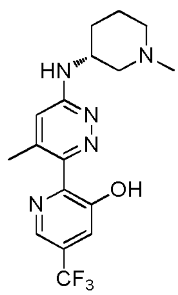

(R)-2-(4-methyl-6-((1-methylpiperidin-3 -yl)amino)pyridazin-3-yl)-5 -(trifluoromethyl)pyridin-3 -ol

The specific reaction Formula is as follows:

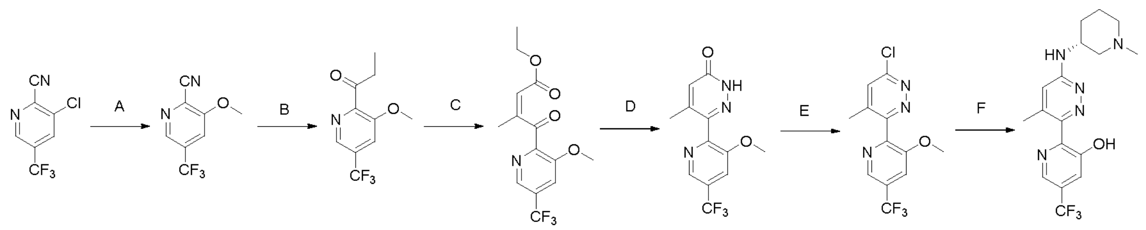


12.1 12.2 12.3 12.4 12.5 12

Step A: 3-methoxy-5-(trifluoromethyl)picolinonitrile (compound 12.1)

3-chloro-S-(trifluoromethyl)picolinonitrile (3.0 g, 14.56mmol) was dissolved in 30mL of methanol, sodium methoxide (1.18 g, 21.84mmol) was added, and the reaction solution was stirred at 70°C for 16 hours. The reaction solution was concentrated under reduced pressure, 50mL of water was added to the residue, the solids were precipitated by beating, the crude product was filtered, and the compound was 12.1 (2.52 g, yield: 86%) was obtained by vacuum drying. LCMS ESI(+)m/z: 203 (M+1).

Step B: 1-(3-methoxy-5-(trifluoromethyl)pyridin-2-yl)propyl-1-one (compound 12.2)

Compound 12.1 (1.58 g, 7.82 mmol) was dissolved in 20 mLTHF, magnesium ethyl bromide (11.7 mL, 11.71 mmol) was added under nitrogen with an ice water bath cooling, and the reaction was then stirred for 2.5 hours at room temperature. 50mL of saturated ammonium chloride was added to the reaction solution, extracted with ethyl acetate (50mL×2), combined the organic phases, washed with brine, dried with anhydrous sodium sulfate, filtered, concentrated under reduced pressure, and purified by column chromatography to obtain a product of 12.2 (730mg, yield: 40%). LCMS ESI(+)m/z: 234.1 (M+1).

Step C: 2-hydroxy-4-(3-methoxy-5-(trifluoromethyl)pyridin-2-yl)-3-methyl-4-oxobutyrate ethyl ester (compound 12.3).

Compound 12.2 (200 mg, 0.86 mmol) was dissolved with 10 mL of THF, LDA(0.65 mL, 1.29 mmol) was added dropwise at -78°C, stirred at -78°C for 30 minutes, ethyl 2-oxoacetate (132 mg, 1.29 mmol) was added, and the reaction solution was stirred at -78°C under nitrogen for 1 hour. 50mL of saturated ammonium chloride was added to the reaction solution, extracted with ethyl acetate (80mL×2), combined the organic phases, washed with brine, dried with anhydrous sodium sulfate, filtered, and concentrated under reduced pressure to obtain a crude product of 12.3 (306mg). LCMS ESI(+)m/z: 318.1 (M+1).

Step D: 6-(3-methoxy-5-(trifluoromethyl)pyridin-2-yl)-5-methylpyridazin-3(2H)-one (compound 12.4)

Compound 12.3 (306 mg, 0.97 mmol) was dissolved in 10 mL of absolute ethanol, hydrazine hydrate (485 mg, 9.70 mmol) was added, and the reaction solution was stirred at 90°C for 3 hours. The reaction solution was concentrated under reduced pressure to obtain a crude compound of 12.4 (282mg), which was directly used in the next reaction. LCMS ESI(+)m/z: 286.1 (M+1).

Step E: 6-chloro-3-(3-methoxy-5-(trifluoromethyl)pyridin-2-yl)-4-methylpyridazine (compound 12.5)

Compound 12.4 (282 mg, 0.99 mmol) was dissolved in 5 mL of phosphorus oxychloride and the reaction solution was stirred at 60 °C for 3.5 hours. The reaction solution was concentrated under reduced pressure, 50mL of water was added to the residue, the pH was adjusted to 7-8 with saturated sodium bicarbonate, extracted with dichloromethane (80mL×2), the organic phase was combined, washed with brine, dried with anhydrous sodium sulfate, filtered, concentrated under reduced pressure, and the product was purified by column chromatography to obtain 12.5 (110mg, yield: 37%). LCMS ESI(+)m/z: 304 (M+1).

Step F: (R)-2-(4-methyl-6-((1-methylpiperidin-3-yl)atnino)pyridazin-3-yl)-5-(trifluoromethyl)pyridin-3-ol (compound 12).

Compound 12.5 (110 mg, 0.36 mmol) was dissolved in 3 mL of NMP, (R)-1-methylpiperidin-3-amine (101 mg, 0.55 mmol) and diisopropylethylamine (233 mg, 1.80 mmol) were added, and the reaction solution was stirred at 150°C with microwave for 3 hours. 50mL of water was added to the reaction solution, extracted with ethyl acetate (80mL×2), combined the organic phases, washed with brine, dried with anhydrous sodium sulfate, filtered, concentrated under reduced pressure, and purified with reversed-phase prep-HPLCto obtain compound 12 (10.4mg, yield: 8%). LCMS ESI(+)m/z: 368.2 (M+1). $^1$H NMR (400 MHz, MeOD) $\delta$ 8.54 (s, 1H), 8.40 (d, *J* = 0.9 Hz, 1H), 7.53 (d, *J* = 1.7 Hz, 1H), 6.87 (d, *J* = 0.8 Hz, 1H), 4.24 (ddd, *J* = 13.0, 9.0, 3.8 Hz, 1H), 3.30 (s, 1H), 3.03 - 2.93 (m, 1H), 2.65 - 2.42 (m, 8H), 2.11 - 2.02 (m, 1H), 1.95 (ddt, *J* = 12.9, 8.6, 4.5 Hz, 1H), 1.88 - 1.74 (m, 1H), 1.59 - 1.47 (m, 1H).

**Embodiment 13**

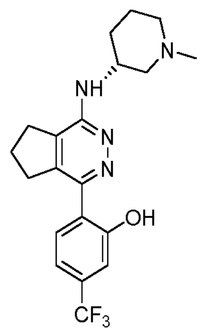

(R)-2-(4-((1-methylpiperidin-3-yl)amino)-6,7-dihydro-5H-cyclopenta*[d]*pyridazin-1-yl)-5-(trifluoromethyl)phenol

The specific reaction Formula is as follows:

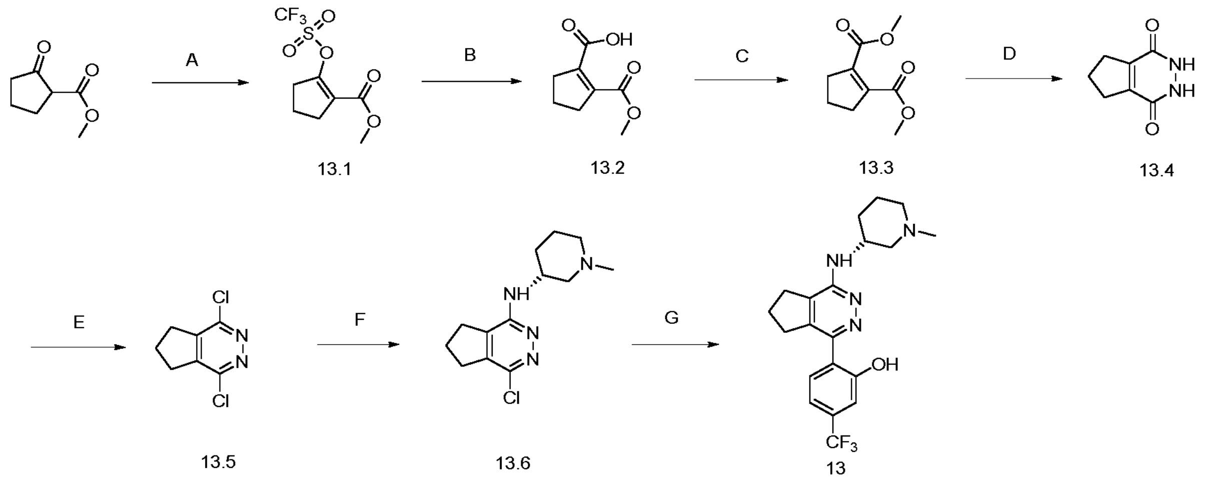


13.1 13.2 13.3 13.4 13.5 13.6 13

Step A: 2-(((trifluoromethyl)sulfonyl)oxy)cyclopenta-1-en-1-carboxylate methyl ester (compound 13.1)

2-oxocyclopentane-1-carboxylic acid methyl ester (5g, 35.17mmol) was dissolved in 70mL of dichloromethane, diisopropylethylamine (6.82g, 52.75mmol) was added at -20 °C, and then trifluoromethanesulfonic anhydride (10.4g, 36.93mmol) was slowly added dropwise, stirred for 1 hour, then heated to room temperature and stirred for 1 hour. The reaction solution was quenched with saturated sodium bicarbonate aqueous solution, extracted with ethyl acetate, washed with brine, dried with anhydrous sodium sulfate, spun dried, and purified by column chromatography to obtain a product of 13.1 (4.99g, yield: 51.4%). LCMS ESI(+)m/z: 275.1 (M+1).

Step B: 2-(Methoxycarbonyl)cyclopenta-1-en-1-carboxylic acid (compound 13.2)

Compound 13.1 (5g, 18.2mmol) was dissolved in 100mL of DMF, and sodium formate (3.7g, 54mmol), lithium chloride (2.3g, 54mmol), acetic anhydride (3.7g, 36mmol), diisopropylamine (3.6g, 36mmol) and palladium acetate (0.4g, 1.8mmol), stirred at room temperature for 3 hours, the reaction solution was quenched with saturated ammonium chloride aqueous solution, extracted with ethyl acetate, washed with brine, drived with anhydrous sodium sulfate, and the product was 13.2 (1.32g, yield: 42.6%) was obtained by spin drying. LCMS ESI(+)m/z: 171.1 (M+1).

Step C: Dimethyl cyclopenta-1-en-1,2-dicarboxylic acid (compound 13.3)

The compound 13.2 (1.32g, 7.75mmol) was dissolved in 50mL of methanol, thionyl chloride (10mL) was slowly added dropwise with an ice bath cooling, stirred at room temperature for 3 hours, the reaction solution was directly dried, and then quenched with saturated sodium bicarbonate aqueous solution, extracted with ethyl acetate, washed with brine, dried with anhydrous sodium sulfate, and spun dried to obtain a product of 13.3 (1.1g, yield: 77.1%). LCMS ESI(+)m/z: 185.1 (M+1).

Step D: 2,3,6,7-tetrahydro-1H-cyclopentano*[d]*pyridazin-1,4(5H)-dione (compound 13.4)

Compound 13.3 (1.1g, 5.98mmol) was dissolved in 50mLethanol, hydrazine hydrate (3g, 60.1 mmol) was added at room temperature, stirred at 90 °C for 3 hours, the reaction solution was directly dried, and then quenched with saturated ammonium chloride aqueous solution, extracted with ethyl acetate, washed with saturated brine, dried with anhydrous sodium sulfate, spin dried, and column purified to obtain a product of 13.4 (230mg, yield: 25%). LCMS ESI(+)m/z: 153.1 (M+1).

Step E: 1,4-dichloro-6,7-dihydro-5H-cyclopenta*[d]*pyridazine (compound 13.5)

The compound 13.4 (230mg, 1.51mmol) was dissolved in 5mL of phosphorus oxychloride, heated to 90 °C and stirred for 1 hour, the reaction solution was directly dried, and then quenched with saturated sodium bicarbonate aqueous solution, extracted with ethyl acetate, washed with brine, drived with anhydrous sodium sulfate, spin dried, and the product was purified by column to obtain 13.5 (280mg, yield: 98.1%). LCMS ESI(+)m/z: 189.1 (M+1).

Step F: (R)-4-chloro-N-(1-methylpiperidin-3-yl)-6,7-dihydro-5H-cyclopenta*[d]*pyridazin-1-amine (compound 13.6)

The compound 13.5 (80mg, 0.422mmol) was dissolved in 3mL n-butanol, diisopropylethylamine (272mg, 2.11mmol) and (R)-1-methylpiperidin-3-amine (100mg, 0.548mmol) were added, microwave heated to 180 °C and stirred for 5 hours, the reaction solution was directly dried, and then extracted with ethyl acetate, washed with brine, dried with anhydrous sodium sulfate, spun dried, and purified by column to obtain the product 13.6 (53mg, yield: 47.1%). LCMS ESI(+)m/z: 267.1 (M+1).

Step G: (R)-2-(4-((1-methylpiperidin-3-yl)amino)-6,7-dihydro-5H-cyclopenta*[d]*pyridazin-1-yl)-5-(trifluoromethyl)phenol (compound 13)

Compounds 13.6 (53 mg, 0.198 mmol) and (2-hydroxy-4-(trifluoromethyl)phenyl) boronic acid (49 mg, 0.238 mmol) were added to a mixture of 3 mL of 1,4-dioxane and 0.5 mL of water, followed by cesium carbonate (161 mg, 0.495 mmol) and Pd $(PPh_3)_4$(45mg, 0.037mmol), under nitrogen, 110°C reaction in microwave environment for 2 hours, quenching reaction with water, extraction with ethyl acetate (40mL×2), combined organic phases, washed with brine, dried with anhydrous sodium sulfate, filtered, concentrated under reduced pressure, and reversed-phase prep-HPLCand purification to obtain compound 13 (13mg, yield: 17.5%). LCMS ESI(+)m/z: 373.2 (M+1).$^1$H NMR (400 MHz, DMSO) δ 11.10 (d, *J* = 120.2 Hz, 1H), 8.39 (d, *J* = 112.9 Hz, 1H), 7.62 (t, *J* = 7.0 Hz, 1H), 7.43 (d, *J* = 1.7 Hz, 1H), 7.33 (d, *J* = 8.1 Hz, 1H), 4.66 - 4.37 (m, 1H), 3.57 (d, *J* = 12.3 Hz, 2H), 3.46- 3.32 (m, 2H), 3.22 (t, *J* = 7.6 Hz, 1H), 2.97 (dt, *J* = 29.4, 7.4 Hz, 4H), 2.79 (dd, *J* = 9.3, 4.6 Hz, 3H), 2.16 (p, *J* = 9.9, 8.8 Hz, 2H), 2.02 - 1.52 (m, 3H).

**Embodiment 14**

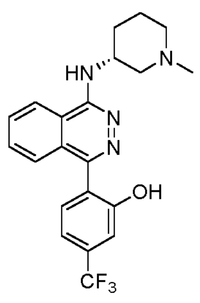

(R)-2-(4-((1-methylpiperidin-3-yl)amino)phthazin-1-yl)-5-(trifluoromethyl)phenol

The specific reaction Formula is as follows:

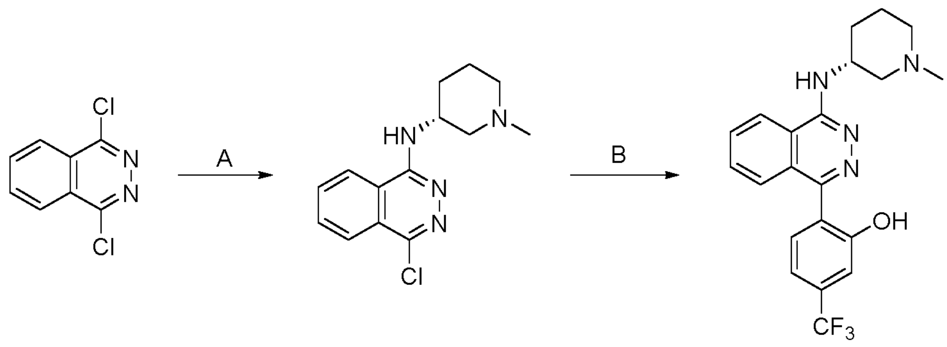


14.1 14

Step A: (R)-4-chloro-N-(1-methylpiperidin-3-yl)phthazine-1-amine (compound 14.1)

1,4-dichlorophthalazine (100mg, 0.5mmol) was dissolved in 2mL of NMP, (R)-1-methylpiperidine-3-amine dihydrochloride (103mg, 0.55mmol) and diisopropylethylamine (323mg, 2.5mmol) were added, and the reaction solution was stirred at 180°C with microwave for 1 hour. Use LCMS to monitor the reaction to completion. The reaction solution was diluted with ethyl acetate (60mL), washed with saturated saline (40mL×3), dried with anhydrous sodium sulfate, and concentrated by vacuum, and the obtained residue was purified by column chromatography to obtain a product of 14.1 (61mg, yield: 46%). LCMS ESI(+)m/z: 277.1 (M+1).

Step B: (R)-2-(4-((1-methylpiperidin-3-yl)atnino)phthazin-1-yl)-5-(trifluoromethyl)phenol (compound 14).

Compound 14.1 (61 mg, 0.22 mmol) was added to 2.2 mL of 1,4-dioxane/H2O (V:V=10:1), followed by (2-hydroxy-4-(trifluoromethyl)phenyl)boronic acid (56 mg, 0.27 mmol), $Pd(PPh_3)_4$ (51 mg, 0.044 mmol), and cesium carbonate (215 mg, 0.66 mmol), Allow the reaction to stir at 120 °C for 2 h under microwave. Use LCMS to monitor the reaction to completion. The reaction solution was quenched with water, extracted with ethyl acetate (30mL×2), combined the organic phases, washed with brine, dried with anhydrous sodium sulfate, filtered, concentrated under reduced pressure, and purified with reversed-phase prep-HPLC to obtain compound 14 (40mg, yield: 45%). LCMS ESI(+)m/z: 403.1 (M+1). $^1$H NMR (400 MHz, DMSO-*d6*) δ 11.28 (s, 1H), 11.11 (s, 2H), 10.77 (s, 1H), 9.76 (s, 2H), 9.44 (d, *J* = 8.0 Hz, 1H), 9.09 (s, 1H), 8.24 - 8.15 (m, 2H), 8.11 (td, *J* = 7.5, 3.4 Hz, 2H), 7.71 *(d, J* = 8.1 Hz, 2H), 7.63 (dd, *J* = 7.6, 3.7 Hz, 2H), 7.48 (s, 2H), 7.40 (d, *J* = 7.9 Hz, 2H), 4.84 (s, 1H), 4.65 (d, *J* = 7.1 Hz, 1H), 3.71 (t, *J* = 13.9 Hz, 3H), 3.28 (s, 2H), 3.09 (d, *J* = 11.6 Hz, 1H), 2.95 (s, 2H), 2.84 (s, 7H), 2.21 (d, *J* = 11.1 Hz, 2H), 2.09 (d, *J* = 9.6 Hz, 1H), 1.99 (s, 2H), 1.84 (dd, *J* = 24.3, 11.2 Hz, 3H).

**Embodiment 15**

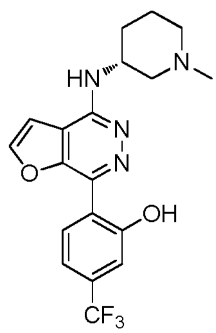

(R)-2-(4-((1-methylpiperidin-3-yl)amino)furo[*2,3-d]*pyridazin-7-yl)-5-(trifluoromethyl)phenol

The specific reaction Formula is as follows:

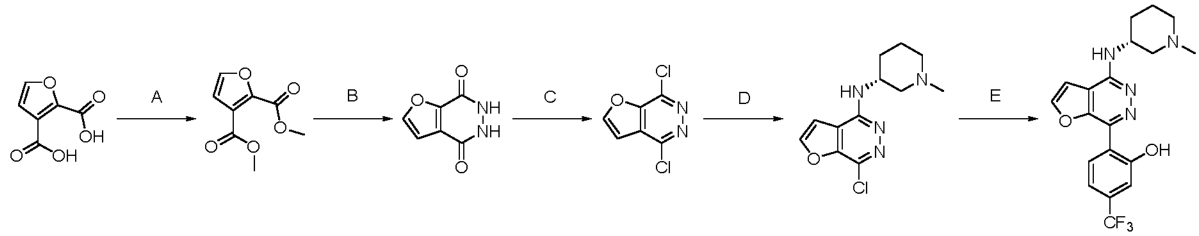


15.1 15.2 15.3 15.4 15

Step A: Dimethyl furan-2,3-dicarboxylic acid (compound 15.1)

Furan-2,3-dicarboxylic acid (4.43 g, 28.38mmol) was dissolved in 80mL of methanol, dichlorothionyl (13.51g, 113.52mmol) was added dropwise in an ice water bath, and the reaction solution was stirred at room temperature for 16 hours. 50mL of water was added to the reaction solution, concentrated under reduced pressure, extracted with ethyl acetate (50mL×2), combined the organic phases, washed with brine, dried with anhydrous sodium sulfate, filtered, and concentrated under reduced pressure to obtain a crude product of 15.1 (5.3g). LCMS ESI(+)m/z: 185 (M+1).

Step B: 5,6-dihydrofuro*[2,3-d]*pyridazin-4,7-dione (compound 15.2)

Compound 15.1 (5.3 g, 28.80 mmol) was dissolved in 100 mL of absolute ethanol, hydrazine hydrate (14.4 g, 288 mmol) was added, and the reaction solution was stirred at 90°C for 16 hours. 50mL of water was added to the reaction solution, concentrated under reduced pressure, 2M HCl (15mL) was added to the residue, stirred at 100°C for 3 hours, filtered at room temperature, and the solid vacuum dried to obtain 15.2 (2.52 g, yield: 57%). LCMS ESI(+)m/z: 153 (M+1).

Step C: 4,7-Dichlorofuran *[2,3-d]*pyridazine (compound 15.3).

Compound 15.2 (820 mg, 5.39 mmol) was dissolved with 15 mL of phosphorus oxychloride, pyridine (0.85 g, 10.79 mmol) was added, and the reaction solution was stirred at 100°C for 1 hour. The reaction solution was spun dry, 50mL of ice water was added to the residue, the pH was adjusted to 7-8 with saturated sodium bicarbonate, extracted with dichloromethane (80mL×2), the organic phase was combined, washed with brine, dried with anhydrous sodium sulfate, filtered, and concentrated under reduced pressure to obtain a product of 15.3 (500mg, yield: 50%). LCMS ESI(+)m/z: 189,191 (M+1).

Step D: (R)-4-chloro-N-(1-methylpiperidin-3-yl)furo[*2,3-d]*pyridazin-7-amine (compound 15.4)

Compound 15.3 (500 mg, 2.67 mmol) was dissolved in 10 mL of n-butanol, (R)-1-methylpiperidin-3-amine (745 mg, 4.01 mmol) and diisopropylethylamine (1.72 g, 13.35 mmol) were added, and the reaction solution was stirred at 180°C for 4 hours. 50mL of water was added to the reaction solution, extracted with ethyl acetate (80mL×2), combined the organic phases, washed with brine, dried with anhydrous sodium sulfate, filtered, concentrated under reduced pressure, and purified by column chromatography to obtain a product of 15.4 (100mg, yield: 14%). LCMS ESI(+)m/z: 267.1 (M+1).

Step E: (R)-2-(4-((1-methylpiperidin-3-yl)amino)furo[*2,3-d]*pyridazin-7-yl)-5-(trifluoromethyl)phenol (compound 15).

The compounds 15.4 (100 mg, 0.38 mmol) and (2-hydroxy-4-(trifluoromethyl)phenyl) boronic acid (78 mg, 0.38 mmol) were dissolved in 4 mL of dioxane and 0.8 mL of water, and cesium carbonate (366 mg, 1.14 mmol) and $Pd(PPh_3)_4$ (46 mg, 0.04 mmol) were added , the reaction solution was stirred at 120 °C and under nitrogen-protected microwave for 3 h. 50mL of water was added to the reaction solution, extracted with ethyl acetate (80mL×2), combined the organic phases, washed with brine solution, dried with anhydrous sodium sulfate, filtered, concentrated under reduced pressure, and purified with reversed-phase prep-HPLC to obtain compound 15 (8.9mg, yield: 6%). LCMS ESI(+)m/z: 393.2 (M+1). $^1$H NMR (400 MHz, MeOD) $\delta$ 8.47 (d, *J* = 2.1 Hz, 1H), 8.46 (d, *J* = 2.0 Hz, 1H), 7.96 (d, *J* = 7.8 Hz, 1H), 7.90 (d, *J* = 2.0 Hz, 1H), 7.60 (d, *J* = 2.1 Hz, 1H), 7.38 (d, *J* = 8.2 Hz, 1H), 7.35 (s, 1H), 4.65 (s, 1H) , 4.57 (tt, *J* = 11.6, 4.0 Hz, 1H), 3.92 - 3.85 (m, 1H), 3.58 (d, *J* = 11.4 Hz, 1H), 3.47 (dd, *J* = 13.4, 3.0 Hz, 1H), 3.21 - 3.03 (m, 3H), 2.98 (s, 3H), 2.93 (s, 1H), 2.33 (d, *J* = 11.4 Hz, 1H), 2.24 - 2.13 (m, 2H), 2.12 - 1.98 (m, 2H), 1.84 (qd, *J* = 12.7, 4.3 Hz, 1H).

**Embodiment 16**

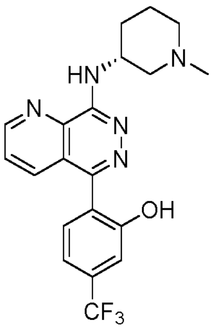

(R)-2-(8-((1-methylpiperidin-3-yl)amino)pyridine*[2,3-d]*pyridazin-5-yl)-5-(trifluoromethyl)phenol

The specific reaction Formula is as follows:

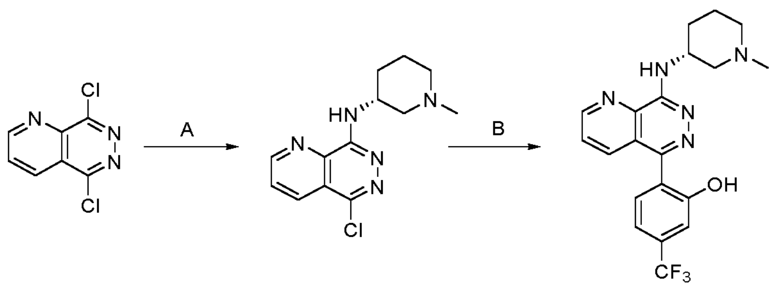


16.1 16

Step A: (R)-5-chloro-N-(1-methylpiperidine-3-yl)pyrido*[2,3-d]*pyridazin-8-amine (compound 16.1)

5,8-dichloropyridine and *[2,3-d]*pyridazine (200mg, 1.0mmol) were dissolved in 2mL of n-butanol, (R)-1-methylpiperidin-3-amine dihydrochloride (206mg, 1.1mmol) and diisopropylethylamine (645mg, 5.0mmol) were added, and the reaction solution was stirred at 150°C with microwave for 1 hour. Use LCMS to monitor the reaction to completion. The reaction solution was concentrated under reduced pressure, and the product was purified by column chromatography to obtain 16.1 (110mg, yield: 40%). LCMS ESI(+)m/z: 278.1 (M+1). $^1$H NMR (400 MHz, DMSO-d6) δ 9.22 (dd, *J* = 4.5, 1.5 Hz, 1H), 8.49 (dd, *J* = 8.3, 1.5 Hz, 1H), 8.06 (dd, *J* = 8.3, 4.5 Hz, 1H), 7.66 (s, 1H), 4.48 (s, 1H), 3.60 (s, 1H), 3.11 (s, 2H), 1.76 (dd, *J* = 27.1, 17.6 Hz, 4H), 1.30 - 1.14 (m, 3H).

Step B: ((R)-2-(8-((1-methylpiperidin-3-yl)amino)pyridin*[2,3-d]*pyridazin-5-yl)-5-(trifluoromethyl)phenol (compound 16).

Compound 16.1 (69 mg, 0.25 mmol) was added to 2.2 mL of 1,4-dioxane/$H_2O$ (V:V=10:1), followed by (2-hydroxy-4-(trifluoromethyl)phenyl)boronic acid (62 mg, 0.30 mmol), $Pd(PPh_3)_4$(58 mg, 0.050 mmol) and cesium carbonate (244 mg, 0.75 mmol) and stir the reaction at 120°C with microwave for 2 hours. Use LCMS to monitor the reaction to completion. The reaction solution was quenched with water, extracted with ethyl acetate (30mL×2), combined the organic phases, washed with brine, dried with anhydrous sodium sulfate, filtered, concentrated under reduced pressure, and purified by reversed-phase prep-HPLCto obtain compound 16 (36mg, yield: 36%). LCMS ESI(+)m/z: 404.1 (M+1). $^1$H NMR (400 MHz, MeOD) δ 9.45 - 9.18 (m, 1H), 8.33 (t, *J* = 9.5 Hz, 1H), 8.20 - 8.00 (m, 1H), 7.69 (d, *J* = 7.9 Hz, 1H), 7.42 (d, *J* = 8.0 Hz, 1H), 7.36 (s, 1H), 4.67 (d, *J* = 32.3 Hz, 1H), 3.89 (dd, *J* = 24.9, 10.4 Hz, 1H), 3.60 (d, *J* = 11.0 Hz, 1H), 3.16 (dd, *J* = 21.3, 7.6 Hz, 1H), 3.06 (t, *J* = 12.0 Hz, 1H), 2.34 (d, *J* = 12.4 Hz, 1H), 2.19 (d, *J* = 14.8 Hz, 1H), 2.14 - 1.98 (m, 1H), 1.89 (dt, *J* = 12.8, 9.7 Hz, 1H).

Embodiment 17

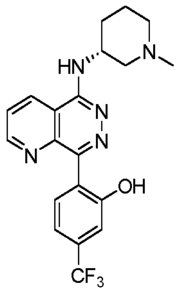

(R)-2-(5-((1-methylpiperidin-3-yl)amino)pyridin*[2,3-d]*pyridazin-8-yl)-5-(trifluoromethyl)phenol

The specific reaction Formula is as follows:

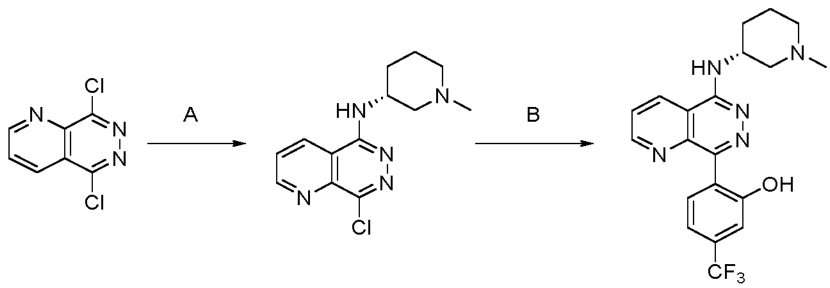


17.1

17

Step A: (R)-8-chloro-N-(1-methylpiperidin-3-yl)pyrido*[2,3-d]*pyridazin-5-amine (compound 17.1)

5,8-dichloropyridine and *[2,3-d]*pyridazine (200mg, 1.0mmol) were dissolved in 2mL of n-butanol, (R)-1-methylpiperidin-3-amine dihydrochloride (206mg, 1.1mmol) and diisopropylethylamine (645mg, 5.0mmol) were added, and the reaction solution was stirred at 150°C with microwave for 1 hour. Use LCMS to monitor the reaction to completion. The reaction solution was concentrated under reduced pressure, and the product was purified by column chromatography to obtain 17.1 (36mg, yield: 13%). LCMS ESI(+)m/z: 278.1 (M+1). $^1$H NMR (400 MHz, DMSO-d6) $\delta$ 9.22 (d, *J* = 4.2 Hz, 1H), 8.90 (dd, *J* = 8.5, 1.4 Hz, 1H), 7.99 (dd, *J* = 8.4, 4.4 Hz, 1H), 7.48 (d, *J* = 7.5 Hz, 1H), 4.39 - 4.16 (m, 1H), 3.04 (d, *J* = 7.6 Hz, 1H), 2.70 (d, *J* = 11.0 Hz, 1H), 2.21 (s, 3H), 1.95 (ddd, *J* = 30.1, 12.5, 6.6 Hz, 3H), 1.80 - 1.69 (m, 1H), 1.59 (td, *J* = 12.2, 3.8 Hz, 1H), 1.42 (ddd, *J* = 23.5, 12.0, 4.1 Hz, 1H), 1.24 (d, *J* = 11.9 Hz, 1H).

Step B: (R)-2-(5-((1-methylpiperidin-3-yl)amino)pyridin*[2,3-d]*pyridazin-8-yl)-5-(trifluoromethyl)phenol (compound 17).

Compound 17.1 (36 mg, 0.13 mmol) was added to 2.2 mL of 1,4-dioxane/$H_2O$ (V:V=10:1), followed by (2-hydroxy-4-(trifluoromethyl)phenyl)boronic acid (32 mg, 0.16 mmol), $Pd(PPh_3)_4$(30 mg, 0.026 mmol), and cesium carbonate (127 mg, 0.39 mmol), Allow the reaction to stir at 120 °C with microwave for 3 h. Use LCMS to monitor the reaction to completion. The reaction solution was quenched with water, extracted with ethyl acetate (30mL$\times$2), combined the organic phases, washed with brine, dried with anhydrous sodium sulfate, filtered, concentrated under reduced pressure, and purified with reversed-phase prep-HPLC to obtain compound 17 (5mg, yield: 10%). LCMS ESI(+)m/z: 404.1 (M+1). $^1$H NMR (400 MHz, MeOD) $\delta$ 9.55 (d, *J* = 8.3 Hz, 1H), 9.35 - 9.24 (m, 4H), 9.14 (d, *J* = 8.1 Hz, 3H), 8.16 (dt, *J* = 8.3, 4.1 Hz, 3H), 7.80 - 7.68 (m, 4H), 7.36 (d, *J* = 8.1 Hz, 3H), 7.31 (s, 3H), 4.68 (t, *J* = 11.5 Hz, 4H), 3.91 (d, *J* = 11.8 Hz, 4H), 3.60 (d, *J* = 12.0 Hz, 4H), 3.48 (dd, *J* = 6.6, 4.9 Hz, 1H), 3.21 - 3.04 (m, 6H), 2.99 (s, 7H), 2.93 (s, 3H), 2.45 - 2.29 (m, 4H), 2.29 - 2.13 (m, 4H), 2.13 - 1.99 (m, 5H), 1.99 - 1.79 (m, 3H).

**Embodiment 18**

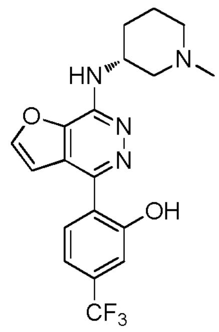

(R)-2-(7-((1-methylpiperidin-3-yl)amino)furo[*2,3-d]*pyridazin-4-yl)-5-(trifluoromethyl)phenol

The specific reaction Formula is as follows:

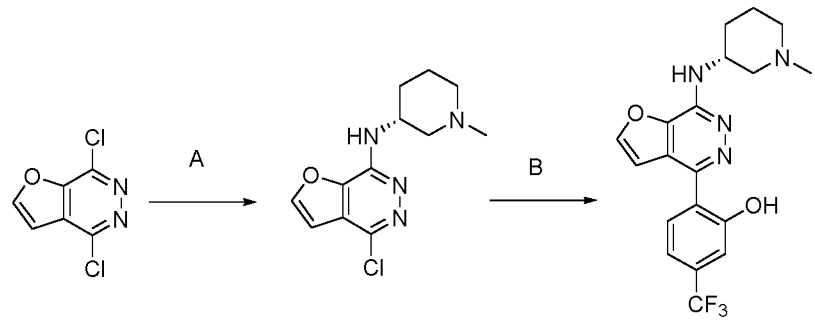


15.3 18.1 18

Step A: (R)-4-chloro-N-(1-methylpiperidin-3-yl)furo[*2,3-d]*pyridazin-7-amine (compound 18.1)

Compound 15.3 (500 mg, 2.67 mmol) was dissolved in 10 mL of n-butanol, (R)-1-methylpiperidin-3-amine (745 mg, 4.01 mmol) and diisopropylethylamine (1.72 g, 13.35 mmol) were added, and the reaction solution was stirred at 180°C with microwave for 4 hours. 50mL of water was added to the reaction solution, extracted with ethyl acetate (80mL×2), combined the organic phases, washed with brine, dried with anhydrous sodium sulfate, filtered, concentrated under reduced pressure, and purified by column chromatography to obtain a product of 18.1 (40mg, yield: 6%). LCMS ESI(+)m/z: 267.1 (M+1).

Step B: (R)-2-(7-((1-methylpiperidin-3-yl)amino)furo[*2,3-d]*pyridazin-4-yl)-5-(trifluoromethyl)phenol (compound 18).

Compounds 18.1 (40 mg, 0.15 mmol) and (2-hydroxy-4-(trifluoromethyl)phenyl) boric acid (46 mg, 0.23 mmol) were dissolved in 4 mL of dioxane and 0.8 mL of water, and cesium carbonate (147 g, 0.45 mmol) and $Pd(PPh_3)_4$(23 mg, 0.02 mmol) were added, the reaction solution was stirred at 120 °C and under nitrogen-protected microwave for 3 h. 50mL of water was added to the reaction solution, extracted with ethyl acetate (80mL×2), combined the organic phases, washed with brine, dried with anhydrous sodium sulfate, filtered, concentrated under reduced pressure, and purified by reversed-phase prep-HPLCto obtain compound 18 (4.0mg, yield: 7%). LCMS ESI(+)m/z: 393.2 (M+1). $^1$H NMR (400 MHz, MeOD) δ 8.23 (d, *J* = 1.6 Hz, 1H), 8.09 (d, *J* = 8.1 Hz, 1H), 7.46 (d, *J* = 1.9 Hz, 1H), 7.29 (d, *J* = 8.2 Hz, 1H), 7.26 (s, 1H), 4.62 (s, 1H), 3.94 (s, 1H), 3.58 (d, *J* = 15.3 Hz, 1H), 2.95 (s, 5H), 2.24 (d, *J* = 24.7 Hz, 2H), 2.02 (s, 1H), 1.81 (s, 1H).

**Embodiment 19**

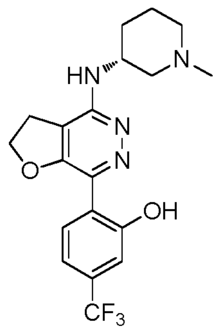

*(R)*-2-(4-((1-methylpiperidin-3-yl)amino)-2,3-dihydrofuro*[2,3-d]*pyridazin-7-yl)-5-(trifluoromethyl)phenol

The specific reaction Formula is as follows:

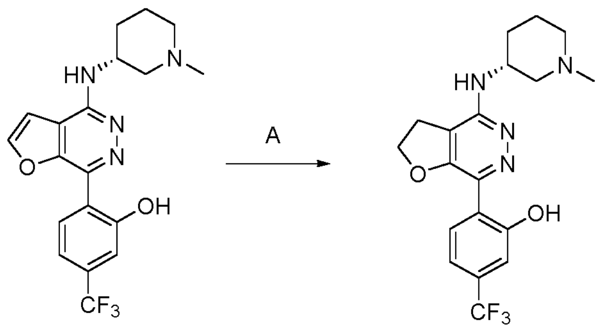


15 19

Step A: (R)-2-(4-((1-methylpiperidin-3-yl)amino)-2,3-dihydrofuro*[2,3-d]*pyridazin-7-yl)-5-(trifluoromethyl)phenol (compound 19).

(R)-2-(4-((1-methylpiperidin-3-yl)amino)furo*[2,3-d]*pyridazin-7-yl)-5-(trifluoromethyl)phenol (55 mg, 0.14mmol) was dissolved in 5mL of methanol, palladium hydroxide carbon (110 mg) was added, and the reaction solution was stirred at room temperature for 24 hours under ambient pressure hydrogen. The reaction solution was filtered, concentrated under reduced pressure, 50mL of water was added to the reaction solution, concentrated under reduced pressure, extracted with ethyl acetate (50mL×2), combined the organic phases, washed with brine, dried with anhydrous sodium sulfate, filtered, concentrated under reduced pressure, and purified by reversed-phase prep-HPLC to obtain compound 19 (17.2mg, yield: 31%). LCMS ESI(+)m/z: 395.2 (M+1). $^1$H NMR (400 MHz, MeOD) δ 7.82 (t, *J* = 7.3 Hz, 1H), 7.26 (d, *J* = 10.0 Hz, 3H), 7.00 - 6.94 (m, 1H), 5.01 (t, *J* = 9.4 Hz, 3H), 4.51 (s, 1H), 4.41 (t, *J* = 11.6 Hz, 1H), 3.79 (t, *J* = 11.5 Hz, 1H), 3.58 (dd, *J* = 18.8, 8.5 Hz, 3H), 3.38 (t, *J* = 9.4 Hz, 3H), 3.17 - 2.98 (m, 3H), 2.96 (s, 3H), 2.90 (s, 1H), 2.28 (t, *J* = 16.5 Hz, 1H), 2.05 (ddd, *J* = 27.9, 23.4, 8.9 Hz, 3H), 1.75 ( qd, *J* = 12.8, 4.1 Hz, 1H).

**Embodiment 20**

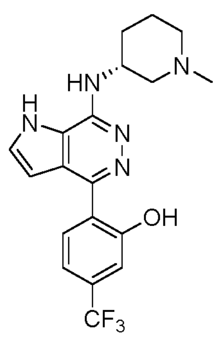

(R)-2-(7-((1-methylpiperidin-3-yl)amino)-1*H*-pyrrolo*[2,3-d]*pyrazin-4-yl)-5-(trifluoromethyl)phenol

The specific reaction Formula is as follows:

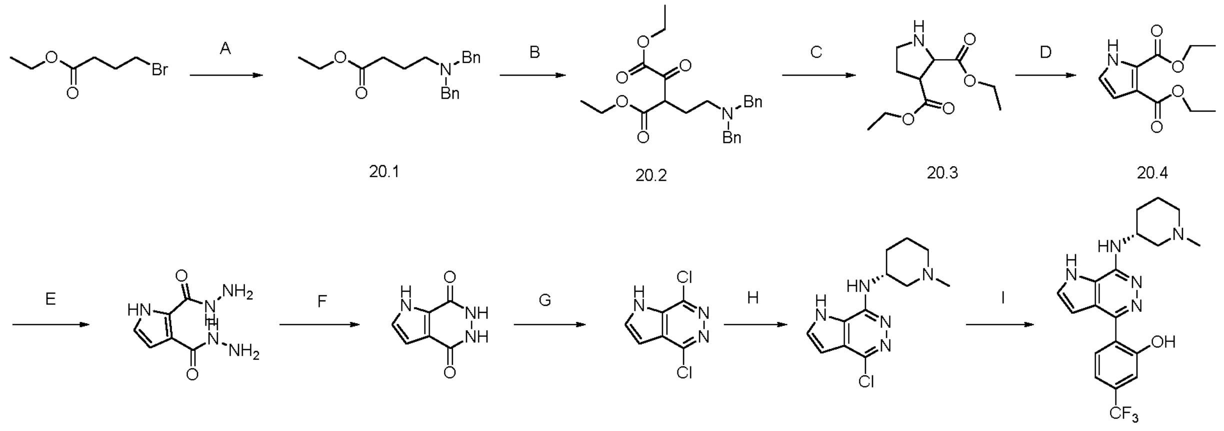


20.1 20.2 20.3 20.4

20.5 20.6 20.7 20.8 20

Step A: Ethyl 4-(dibenzylamino)butyrate (compound 20.1)

Ethyl 4-bromobutyrate (10.0g, 51.3mmol) was dissolved into 100 mL of DMF, and then dibenzylamine (10.1g, 51.3mmol), potassium carbonate (14.2g, 102.6mmol) and potassium iodide (852mg, 5.13mmol) were added sequentially, and the reaction was stirred overnight at 80°C. Use TLC to monitor the reaction to completion. The reaction solution was quenched with water, extracted with ethyl acetate (200mL×3), combined the organic phases, washed with brine (4×100mL), dried with anhydrous sodium sulfate, filtered, concentrated under reduced pressure, and purified by column chromatography to obtain a product of 20.1 (11.4g, yield: 72%).

Step B: 2-(2-(dibenzylamino)ethyl)-3-oxosuccinate diethyl ester (compound 20.2)

Compound 20.1 (11.4g, 36.7mmol) and diethyl oxalate (5.36g, 36.7mmol) were dissolved in 100 mL of toluene, potassium tert-butoxide (4.94g, 44.07 mmol) was added at room temperature, and stirred overnight at room temperature. Use TLC to monitor the reaction to completion. The reaction solution was quenched with saturated ammonium chloride solution, extracted with ethyl acetate (200mL×3), combined the organic phases, washed with brine (200mL), dried with anhydrous sodium sulfate, filtered, concentrated under reduced pressure, and purified by column chromatography to obtain a product of 20.2 (8.6g, yield: 57%).

Step C: Pyrrolidin-2,3-diethyl dicarboxylate (compound 20.3)

Compound 20.2 (4.1g, 10mmol) was dissolved in 50mL of ethanol, Pd/C (820mg, 20%) was added at room temperature, and stirred overnight in a hydrogen atmosphere. Use TLC to monitor the reaction to completion. The reaction solution was passed through a suction funnel coated with diatomaceous earth, concentrated under reduced pressure, and purified by column chromatography to obtain a product of 20.3 (1.5g, yield: 70%).

Step D: 1*H*-pyrrole-2,3-dicarboxylic acid diethyl ester (compound 20.4)

Compound 20.3 (1.5g, 6.97mmol) was dissolved in 60mL of anhydrous methylene chloride, active manganese dioxide (4.5g) was added at room temperature, and stirred overnight at room temperature. Use TLC to monitor the reaction to completion. The reaction solution was passed through a suction funnel coated with diatomaceous earth, concentrated under reduced pressure, and purified by column chromatography to obtain a product of 20.4 (333mg, yield: 23%).

Step E: 1*H*-pyrrole-2,3-dicarboxylhydrazide (compound 20.5)

Compound 20.4 (333mg, 1.58mmol) was dissolved in 20 mL of ethanol, hydrazine hydrate (790 mg, 15.8 mmol) was added at room temperature, and stirred overnight at 80°C. Use LCMS to monitor the reaction to completion. The reaction solution was concentrated under reduced pressure to obtain a crude product of 20.5 (338mg, yield: 100%).

Step F: 5,6-dihydro-1*H*-pyrrolo*[2,3-d]*pyrazine-4,7-dione (compound 20.6)

Dissolve compound 20.5 (338 mg, 1.58 mmol) in 20 mL of 2 M hydrochloric acid solution and stir overnight at 100 °C. Use LCMS to monitor the reaction to completion. The reaction solution was concentrated under reduced pressure to obtain a crude product of 20.6 (358mg, yield: 100%).

Step G: 4,7-dichloro-1*H*-pyrrolo*[2,3-d]*pyridazine (compound 20.7)

Dissolve compound 20.6 (358 mg, 1.58 mmol) in 8 mL of phosphorus oxychloride, add DIPEA (2 mL) at room temperature, and stir overnight at 100 °C. Use LCMS to monitor the reaction to completion. The reaction solution was concentrated under reduced pressure, the residue was dissolved with ethyl acetate (30mL), and the pH was adjusted to 7-8 with saturated sodium bicarbonate solution, the organic phase was washed with brine, filtered, concentrated under reduced pressure, and purified by column chromatography to obtain compound 20.7 (147mg, yield: 49%).

Step H: (R)-4-chloro-N-(1-methylpiperidin-3-yl)-1*H*-pyrrolo*[2,3-d]*pyrazin-7-amine (compound 20.8)

Compound 20.7 (147mg, 0.78mmol) was dissolved in 4mL of NMP, (R)-1-methylpiperidine-3-amine (98mg, 0.86mmol) and diisopropylethylamine (501mg, 3.9mmol) were added, and the reaction solution was stirred at 180°C with microwave for 5 hours. Use LCMS to monitor the reaction to completion. The reaction solution was diluted with ethyl acetate (60mL), washed with saturated saline (40mL×3), dried with anhydrous sodium sulfate, and concentrated in vacuum, and the obtained residue was purified by column chromatography to obtain a product of 20.8 (45mg, yield: 22%). LCMS ESI(+)m/z: 266.1 (M+1).

Step I: (R)-2-(7-((1-methylpiperidin-3-yl)amino)-1*H*-pyrrole*[2,3-d]*pyrazin-4-yl)-5-(trifluoromethyl)phenol (compound 20).

Compound 20.8 (45 mg, 0.17 mmol) was added to 2.2 mL of 1,4-dioxane/$H_2O$ (V:V=10:1), followed by (2-hydroxy-4-(trifluoromethyl)phenyl)boronic acid (42 mg, 0.20 mmol), $Pd(PPh_3)_4$ (39 mg, 0.034 mmol), and cesium carbonate (166 mg, 0.51 mmol) and allow the reaction to stir for 3 h at 120 °C with microwave. Use LCMS to monitor the reaction to completion. The reaction solution was quenched with water, extracted with ethyl acetate (30mL×2), combined the organic phases, washed with brine, dried with anhydrous sodium sulfate, filtered, concentrated under reduced pressure, and purified with reversed-phase prep-HPLC to obtain compound 20 (4mg, yield: 6%). LCMS ESI(+)m/z: 392.1 (M+1).$^{1}$H NMR(400 MHz, MeOD) δ 7.93 -7.84 (m, 1H), 7.80 (d, *J* = 8.0 Hz, 1H), 7.41 (d, *J* = 8.0 Hz, 1H), 7.37 (s, 1H), 6.95 - 6.89 (m, 1H), 4.65 - 4.46 (m, 1H), 4.10 - 3.85 (m, 1H), 3.65 - 3.52 (m, 1H), 3.09 (ddd, *J* = 29.2, 13.8, 11.6 Hz, 2H), 2.97 (s, 2H), 2.89 (d, *J* = 6.3 Hz, 1H), 2.35 (d, *J* = 12.7 Hz, 1H), 2.18 (d, *J* = 14.8 Hz, 1H), 2.10 - 1.93 (m, 2H), 1.87-1.68 (m, 1H).

**Embodiment 21**

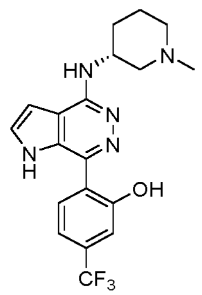

(R)-2-(4-((1-methylpiperidin-3-yl)amino)-1*H*-pyrrolo*[2,3-d]*pyrazin-7-yl)-5-(trifluoromethyl)phenol

The specific reaction Formula is as follows:

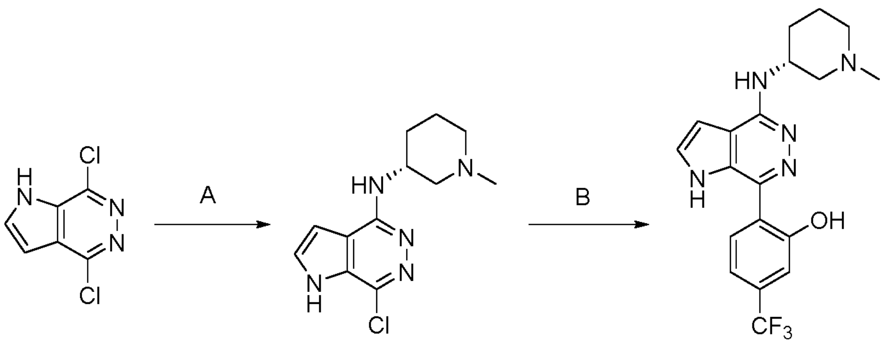


21.1 21

Step A: ((R)-7-chloro-N-(1-methylpiperidin-3-yl)-1*H*-pyrrolo*[2,3-d]*pyrazin-4-amine (compound 21.1)

4,7-dichloro-1*H*-pyrrolo*[2,3-d]*pyrazine (147mg, 0.78mmol) was dissolved in 4mL of NMP, (R)-1-methylpiperidin-3-amine (98mg, 0.86mmol) and diisopropylethylamine (501mg, 3.9mmol) were added, and the reaction solution was stirred at 180°C with microwave for 5 hours. Use LCMS to monitor the reaction to completion. The reaction solution was diluted with ethyl acetate (60mL), washed with saturated saline (40mL×3), dried with anhydrous sodium sulfate, and concentrated in vacuum, and the obtained residue was purified by column chromatography to obtain a product of 21.1 (45mg, yield: 22%). LCMS ESI(+)m/z: 266.1 (M+1).

Step B: (R)-2-(4-((1-methylpiperidin-3-yl)amino)-1*H*-pyrrolo*[2,3-d]*pyrazine-7-yl)-5-(trifluoromethyl)phenol (compound 21).

Compound 21.1 (45 mg, 0.17 mmol) was added to 2.2 mL of 1,4-dioxane/$H_2O$ (V:V=10:1), followed by (2-hydroxy-4-(trifluoromethyl)phenyl)boronic acid (42 mg, 0.20 mmol), $Pd(PPh_3)_4$ (39 mg, 0.034 mmol), and cesium carbonate (166 mg, 0.51 mmol) and allow the reaction to stir for 3 h at 120 °C with microwave. Use LCMS to monitor the reaction to completion. The reaction solution was quenched with water, extracted with ethyl acetate (30mL×2), combined the organic phases, washed with brine, dried with anhydrous sodium sulfate, filtered, concentrated under reduced pressure, and purified by reversed-phase prep-HPLC to obtain compound 21 (7mg, yield: 11%). LCMS ESI(+)m/z: 392.1 (M+1). $^1H$ NMR (400 MHz, MeOD) δ 7.93 (s, 1H), 7.74 (d, *J* = 7.2 Hz, 1H), 7.37 (s, 2H), 7.23 (s, 1H), 4.59 (d, *J* = 39.9 Hz, 1H), 3.89 (d, *J* = 10.9 Hz, 1H), 3.58 (d, *J* = 12.5 Hz, 1H), 3.52 - 3.39 (m, 1H), 3.13 (dd, *J* = 6.0, 4.3 Hz, 1H), 3.06 (t, *J* = 13.0 Hz, 1H), 2.95 (d, *J* = 19.1 Hz, 4H), 2.32 (d, *J* = 9.8 Hz, 1H), 2.17 (d, *J* = 14.3 Hz, 1H), 1.98 (d, *J* = 32.6 Hz, 2H), 1.82 (tt, *J* = 17.2, 8.6 Hz, 1H).

**Embodiment 22**

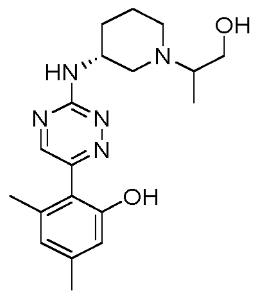

2-(3-(((3R)-1-(1-hydroxypropyl-2-yl)piperidin-3-yl)amino)-1,2,4-triazin-6-yl)-3,5-dimethylphenol

The specific reaction Formula is as follows:

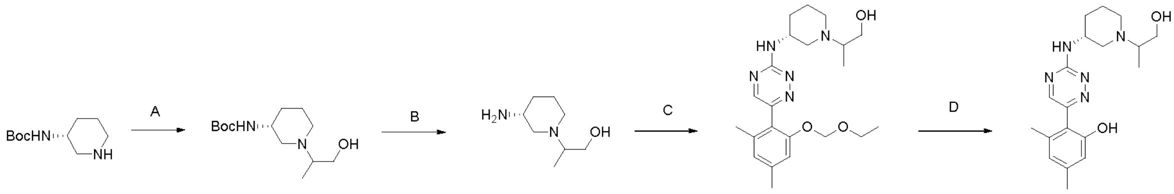


22.1 22.2 22.3 22

Step A: ((3R)-1-(1-hydroxypropyl-2-yl)piperidin-3-yl)tert-butyl carbamate (compound 22.1)

Tert-butyl(R)-piperidin-3-yl carbamate (200 mg, 1 mmol) and 2-bromopropanol (138 mg, 1 mmol) were dissolved in 8 mL of DMF, then potassium carbonate (276 mg, 2 mmol) and potassium iodide (166 mg, 1 mmol) were added, and the reaction solution was stirred at room temperature for 3 hours. Use TLC to monitor the reaction to completion. The reaction solution was diluted with water, extracted with ethyl acetate, washed with brine, dried with anhydrous sodium sulfate, filtered, concentrated under reduced pressure, and the product was obtained by column chromatography 22.1 (230mg, yield: 89.1%). LCMS ESI(+)m/z: 259.1 (M+1).

Step B: 2-((R)-3-atninopiperidin-1-yl)propyl-1-ol (compound 22.2)

Dissolve compound 22.1 (1.4 g, 5.4 mmol) in 15 mL of hydrogen chloride/ethyl acetate (2 M) and stir for 1 h at room temperature. Use TLC to monitor the reaction to completion. The reaction solution was concentrated under reduced pressure to obtain a crude product of 22.2 (1.2g, yield: 100%). LCMS ESI(+)m/z: 159.1 (M+1).

Step C: 2-((R)-3-((6-(2-(ethoxymethoxy)-4,6-dimethylphenyl)-1,2,4-triazin-3-yl)amino)piperidin-1-yl)propane-1-ol (compound 22.3)

The compound 22.2 (30mg, 0.102mmol) was dissolved in 2mL of n-butanol, and 3-chloro-6-(2-(ethoxymethoxy)-4,6-dimethylphenyl)-1,2,4-triazine (62mg, 0.204mmol) and diisopropylethylamine (90mg, 0.70mmol) were added, and the reaction solution was stirred at 150°C with microwave for 1 hour. Use LCMS to monitor the reaction to completion. The reaction solution was concentrated under reduced pressure to obtain a crude product of 22.3 (50mg, yield: 100%). LCMS ESI(+)m/z: 415.2 (M+1).

Step D: 2-(3-(((3R)-1-(1-hydroxypropyl-2-yl)piperidin-3-yl)amino)-1,2,4-triazin-6-yl)-3,5-dimethylphenol (compound 22).

Dissolve compound 22.3 (50 mg, 0.105 mmol) in 3 mL of 2 M hydrochloric acid/ethyl acetate and stir for 1 h at room temperature. Use LCMS to monitor the reaction to completion. The reaction solution was concentrated under reduced pressure and purified with reversed-phase prep-HPLC to obtain compound 22 (1.6 mg). LCMS ESI(+)m/z: 357.1 (M+1). $^1$H NMR (400 MHz, DMSO) δ 9.89 (s, 1H), 9.54 (s, 1H), 9.01 (s, 1H), 8.37 - 8.19 (m, 1H), 7.88 (s, 1H), 6.72 - 6.52 (m, 2H), 4.48 (s, 1H), 4.24 (s, 1H), 3.41 (t, J = 9.8 Hz, 3H), 3.20 (s, 1H), 3.11 - 2.74 (m, 2H), 2.23(s, 3H), 2.07 (s, 4H), 1.92 (d, J = 10.9 Hz, 2H), 1.84 (s, 1H), 1.60 (td, J = 14.3, 11.8, 7.9 Hz, 2H), 1.25 (d, J = 1.5 Hz, 3H).

**Embodiment 23**

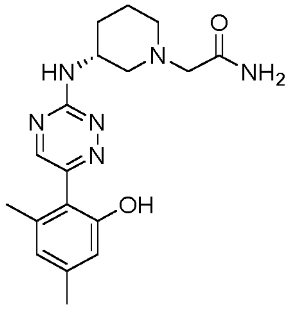

(R)-2-(3-((6-(2-hydroxy-4,6-dimethylphenyl)-1,2,4-triazin-3-yl)amino)piperidin-1-yl)acetatnide

The specific reaction Formula is as follows:

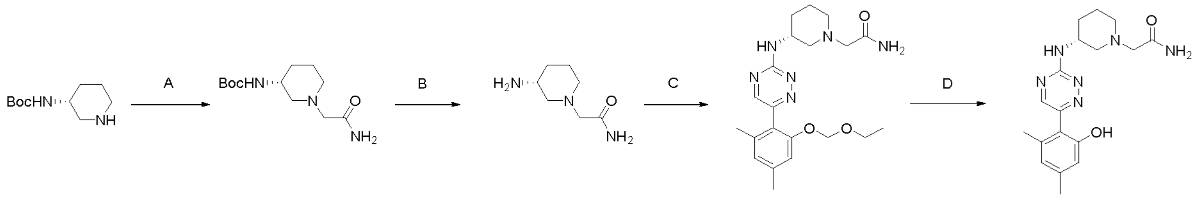


23.1 23.2 23.3 23

Step A: (R)-(1-(2-amino-2-oxoethyl)piperidin-3-yl)tert-butyl carbamate (compound 23.1)

Tert-butyl(R)-piperidin-3-ylcarbamate (200mg, 1mmol) and 2-bromoacetamide (138mg, 1mmol) were dissolved in 8 mL of DMF, then potassium carbonate (276 mg, 2 mmol) and potassium iodide (166 mg, 1 mmol) were added, and the reaction solution was stirred at room temperature for 3 hours. Use TLC to monitor the reaction to completion. The reaction solution was diluted with water, extracted with ethyl acetate, washed with brine, dried with anhydrous sodium sulfate, filtered, concentrated under reduced pressure, and the product was obtained by column chromatography 23.1 (230mg, yield: 89.1%). LCMS ESI(+)m/z: 258.1 (M+1).

Step B: (R)-2-(3-aminopiperidin-1-yl)acetamide (compound 23.2)

Dissolve compound 23.1 (1.4 g, 5.4 mmol) in 15 mL of 2 M hydrochloric acid/ethyl acetate and stir for 1 h at room temperature. Use TLC to monitor the reaction to completion. The reaction solution was concentrated under reduced pressure to obtain a crude product of 23.2 (1.2g, yield: 100%). LCMS ESI(+)m/z: 158.21 (M+1).

Step C: (R)-2-(3-((6-(2-(ethoxymethoxy)-4,6-dimethylphenyl)-1,2,4-triazin-3-yl)amino)piperidin-1-yl)acetamide (compound 23.3)

The compound 23.2 (30 mg, 0.102 mmol) was dissolved in 2 mL of n-butanol, and 3-chloro-6-(2-(ethoxymethoxy)-4,6-dimethylphenyl)-1,2,4-triazine (62 mg, 0.204 mmol) and diisopropylethylamine (90 mg, 0.70 mmol) were added, and the reaction solution was stirred at 150 °C for 1 hour. Use LCMS to monitor the reaction to completion. The reaction solution was concentrated under reduced pressure to obtain a crude product of 23.3 (50mg, yield: 100%). LCMS ESI(+)m/z: 414.1 (M+1).

Step D: (R)-2-(3-((6-(2-hydroxy-4,6-dimethylphenyl)-1,2,4-triazin-3-yl)amino)piperidin-1-yl)acetamide (compound 23).

Compound 23.3 (50 mg, 0.105 mmol) is dissolved in 3 mL of hydrochloric acid/ethyl acetate (2 M) and stirred for 1 h at room temperature. Use LCMS to monitor the reaction to completion. The reaction solution was concentrated under reduced pressure and purified by reversed-phase prep-HPLC to obtain compound 23 (1.6 mg). LCMS ESI(+)m/z: 356.1 (M+1). $^1$H NMR (400 MHz, DMSO) δ 10.30 (s, 1H), 9.62 (d, J = 166.1 Hz, 1H), 8.33 (s, 1H), 8.09 (s, 2H), 7.68 (s, 1H), 6.67 (s, 1H), 6.61 (s, 1H), 4.44 (s, 1H), 3.97 (s, 3H), 3.47 (d, J = 12.1 Hz, 2H), 3.01 (d, J = 36.2 Hz,2H), 2.23 (s, 4H), 2.08 (s, 4H), 1.97 - 1.81 (m, 3H), 1.63 - 1.46 (m, 1H).

**Embodiment 24**

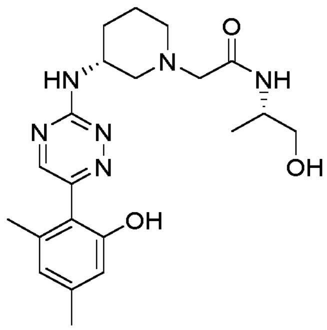

2-((R)-3-((6-(2-hydroxy-4,6-dimethylphenyl)-1,2,4-triazin-3-yl)amino)piperidin-1-yl)-N-((S)-1-hydroxypropyl-2-yl)acetamide

The specific reaction Formula is as follows:

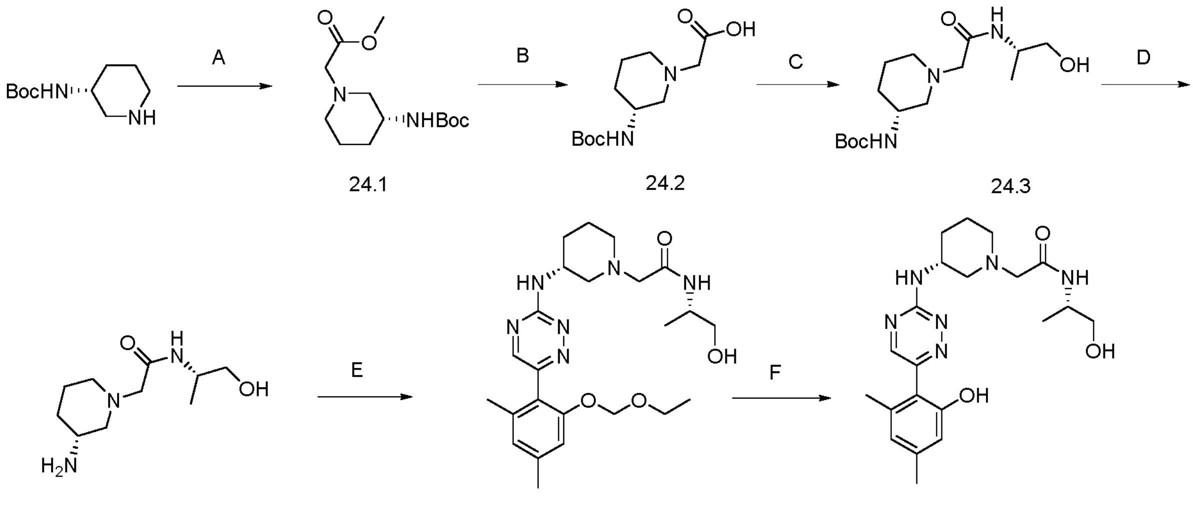


24.1 24.2 24.3

24.4 24.5 24

Step A: (R)-2-(3-((tert-butoxycarbonyl)amino)piperidin-1-yl)methyl acetate (compound 24.1)

Tert-butyl(R)-piperidin-3-ylcarbamate (1g, 5mmol) and methyl 2-bromoacetate (835mg, 5mmol) were dissolved in 10mL of DMF, then potassium carbonate (1.38g, 10mmol) and potassium iodide (830mg, 5mmol) were added, and the reaction solution was stirred at room temperature for 3 hours. Use TLC to monitor the reaction to completion. The reaction solution was diluted with water, extracted with ethyl acetate, washed with brine, dried with anhydrous sodium sulfate, filtered, concentrated under reduced pressure, and column chromatography obtained a product of 24.1 (1.6g, yield: 100%). LCMS ESI(+)m/z: 273.1 (M+1).

Step B: ((R)-2-(3-(tert-butoxycarbonyl)amino)piperidin-1-yl)acetic acid (compound 24.2) dissolve compound 24.1 (1.6 g) in 5 mL of THF solution, add lithium hydroxide aqueous solution (30 mg) and stir for 12 hours at room temperature, and extract with ethyl acetate, combined the organic phases, washed with brine, dried with anhydrous sodium sulfate, filtered, and concentrated under reduced pressure to obtain a crude product of 24.2 (1.5g). LCMS ESI(+)m/z: 259.1 (M+1).

Step C: ((R)-1-(2-(((S)-1-hydroxypropyl-2-yl)amino)-2-oxethyl)piperidin-3-yl)tert-butyl carbamate (compound 24.3)

Compound 24.2 (200 mg, 0.775 mmol) was dissolved in 3 mL of DMF, and (S)-2-atninopropane-1-ol (70 mg, 0.93 mmol), diisopropylethylamine (295 mg, 2.32 mmol) and $T_3P$ solution in DMF (50 wt%, 739 mg, 1.16 mmol) were added with an ice bath cooling, then stir for 2 hours at room temperature. The reaction solution was diluted with water, extracted with ethyl acetate, washed with brine, dried with anhydrous sodium sulfate, and obtained a crude compound of 24.3 (240mg) after spin drying. LCMS ESI(+)m/z: 316.1 (M+1).

Step D: 2-((R)-3-atninopiperidin-1-yl)-N-((S)-1-hydroxypropyl-2-yl)acetatnide (compound 24.4)

Dissolve compound 24.3 (240 mg) in 15 mL of 2 M hydrochloric acid/ethyl acetate and stir for 1 h at room temperature. Use TLC to monitor the reaction to completion. The reaction solution was concentrated under reduced pressure to obtain a crude product of 24.4 (120mg, yield: 100%). LCMS ESI(+)m/z: 216.1 (M+1).

Step E: 2-((R)-3-((6-(2-(ethoxymethoxy)-4,6-dimethylphenyl)-1,2,4-triazin-3-yl)amino)piperidin-1-yl)-N-((S)-1-hydroxypropyl-2-yl)acetamide (compound 24.5)

Compound 24.4 (120 mg, 0.6 mmol) was dissolved in 2 mL of n-butanol, and 3-chloro-6-(2-(ethoxymethoxy)-4,6-dimethylphenyl)-1,2,4-triazine (50mg, 0.17 mmol) and diisopropylethylamine (132 mg, 1.02 mmol) were added, and the reaction solution was stirred at 150 °C with microwave for 1 hour. Use LCMS to monitor the reaction to completion. The reaction solution was concentrated under reduced pressure to obtain a crude product of 24.5 (70mg). LCMS ESI(+)m/z: 472.1 (M+1).

Step F: 2-((R)-3-((6-(2-hydroxy-4,6-dimethylphenyl)-1,2,4-triazin-3-yl)amino)piperidin-1-yl)-N-((S)-1-hydroxypropyl-2-yl)acetamide (compound 24)

Dissolve compound 24.5 (70 mg) in 3 mL of hydrogen chloride/ethyl acetate (2 M) solution and stir for 1 h at room temperature. Use LCMS to monitor the reaction to completion. The reaction solution was concentrated under reduced pressure and purified with reversed-phase prep-HPLC to obtain compound 24 (6 mg). LCMS ESI(+)m/z: 414.1 (M+1). $^1$H NMR (400 MHz, DMSO) $\delta$ 10.24 (s, 1H), 9.67 (d,*J*= 90.1 Hz, 1H), 8.51 (d, *J* = 7.9 Hz, 1H), 8.29 (s, 1H), 7.96 (s, 1H), 6.66 - 6.57 (m, 2H), 4.42 (s, 1H), 3.94 (s, 2H), 3.83 (p, *J* = 6.3 Hz, 1H), 3.67 (d, *J* = 11.3Hz, 1H), 3.46 (d, *J* = 12.2 Hz, 1H), 3.33 (qd, *J* = 10.8, 5.6 Hz, 2H), 3.02 (s, 1H), 2.90 (dd, *J* = 11.0, 7.0 Hz, 1H), 2.23 (s, 3H), 2.07 (s, 4H), 1.88 (d, *J* = 32.1 Hz, 2H), 1.57 (q, *J* = 10.4, 9.3 Hz, 1H), 1.04 (d, *J* = 6.7 Hz,3H).

**Embodiment 25**

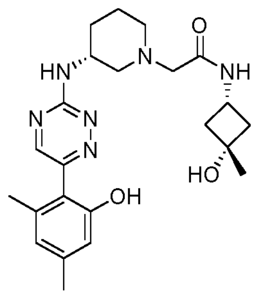

N-((1s,3 S)-3-hydroxy-3-methylcyclobutyl)-2-((R)-3-((6-(2-hydroxy-4,6-dimethylphenyl)-1,2,4-triazin)-3-yl)amino)piperidin-1-yl)acetamide

The specific reaction Formula is as follows:

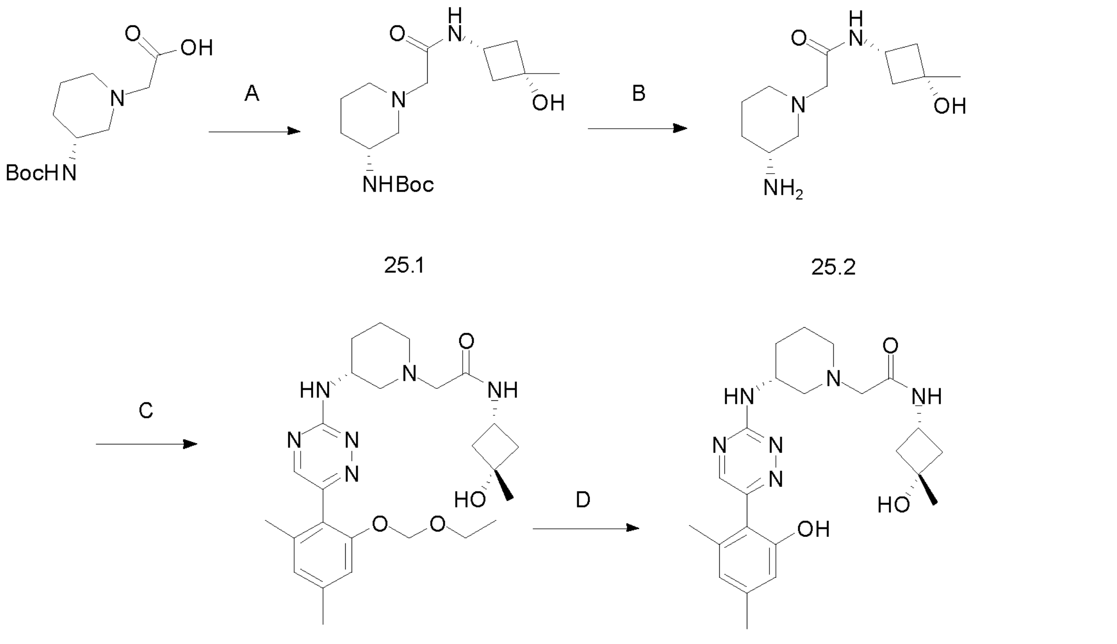


25.1 25.2

25.3 25

Step A: ((R)-1-(2-(((1s,3S)-3-hydroxy-3-methylcyclobutyl)amino)-2-oxoethyl)piperidin-3-yl)tert-butyl carbamate (compound 25.1)

(R)-2-(3-((tert-butoxycarbonyl)amino)piperidin-1-yl)acetic acid (200mg, 0.775mmol) was dissolved in 10mL of DMF, added (1s,3s)-3-amino-1-methylcyclobutane-1-ol (162mg, 0.929 mmol), diisopropylethylamine (500mg, 3.1mmol) and the 50% of DMF solution of T 3P was (739mg, 0.929 mmol), then stir for 2 h at room temperature. The reaction solution was diluted with water, extracted with ethyl acetate, washed with brine, dried with anhydrous sodium sulfate, and obtained a crude compound of 25.1 (250mg) after spin drying. LCMS ESI(+)m/z: 342.1 (M+1).

Step B: 2-((R)-3-aminopiperidin-1-yl)-N-((1s,3S)-3-hydroxy-3-methylcyclobutyl)acetamide (compound 25.2)

Dissolve compound 25.1 (250 mg) in 15 mL of 2 M hydrochloride/ethyl acetate and stir for 1 h at room temperature. Use TLC to monitor the reaction to completion. The reaction solution was concentrated under reduced pressure to obtain a crude product of 25.2 (120mg, yield: 100%). LCMS ESI(+)m/z: 242.1 (M+1).

Step C: 2-((R)-3-((6-(2-(ethoxymethoxy)-4,6-dimethylphenyl)-1,2,4-triazin-3-yl)amino)piperidin-1-yl)-N-((1s,3S)-3-hydroxy-3-methylcyclobutyl)acetamide (compound 25.3)

Compound 25.2 (120mg, 0.25mmol) was dissolved in 2mL of n-butanol, and 3-chloro-6-(2-(ethoxymethoxy)-4,6-dimethylphenyl)-1,2,4-triazine (50mg, 0.17mmol) and diisopropylethylamine (132mg, 1.02mmol) were added, and the reaction solution was stirred at 150°C microwave for 1 hour. Use LCMS to monitor the reaction to completion. The reaction solution was concentrated under reduced pressure to obtain a crude product of 25.3 (85mg). LCMS ESI(+)m/z: 498.1 (M+1).

Step D: N-((1s,3S)-3-hydroxy-3-methylcyclobutyl)-2-((R)-3-((6-(2-hydroxy-4,6-dimethylphenyl)-1,2,4-triazine)-3-yl)amino)piperidin-1-yl)acetatnide (compound 25)

Dissolve compound 25.3 (85 mg, 0.09 mmol) in 3 mL of hydrogen chloride/ethyl acetate (2 M) and stir for 1 h at room temperature. Use LCMS to monitor the reaction to completion. The reaction solution was concentrated under reduced pressure and purified with reversed-phase prep-HPLCt o obtain a product of 25 (36 mg). LCMS ESI(+)m/z: 440.1 (M+1).$^1$H NMR (400 MHz, DMSO) δ 10.36 (s, 1H), 9.71 (d, J = 113.8 Hz, 1H), 8.93 (d, J = 7.0 Hz, 1H), 8.31 (s, 1H), 8.01 (s, 1H), 6.73 - 6.51 (m, 2H), 4.39 (d, J = 24.6 Hz, 1H), 3.95 (d, J = 3.0 Hz, 2H), 3.78 (p, J = 7.9 Hz,1H), 3.73 - 3.55 (m, 1H), 3.44 (d, J = 11.8 Hz, 1H), 3.13 - 2.82 (m, 2H), 2.33 - 2.17 (m, 5H), 2.07 (s, 4H), 2.00 - 1.75 (m, 4H), 1.67 - 1.44 (m, 1H), 1.22 (s, 3H).

**Embodiment 26**

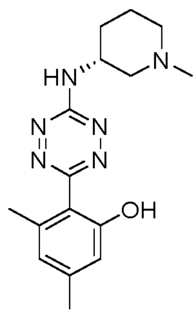

(R)-3,5-Dimethyl-2-(6-((1-methylpiperidin-3)amino)-1,2,4,5-tetraazin-3)phenol

The specific reaction Formula is as follows:

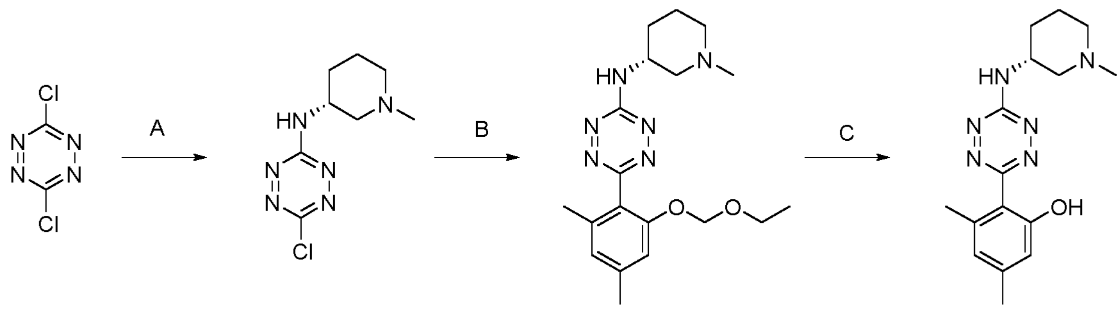


26.1 26.2 26

Step A: (R)-6-chloro-N-(1-methylpiperidin-3-yl)-1,2,4,5-tetraazin-3-amine (compound 26.1)

3,6-dichloro-1,2,4,5-tetrazine (250mg,1.66mmol) was dissolved in 13mL of methyl tert-butyl ether, and (R)-1-methylpiperidin-3-amine hydrochloride (467mg,2.5mmol) and diisopropylethylamine (1.5mL,8.3mmol) were added to the reaction overnight at room temperature. The reaction solution was quenched with sodium bicarbonate aqueous solution, extracted with ethyl acetate (10mL×5), combined the organic phases, dried with anhydrous sodium sulfate, filtered, concentrated under reduced pressure, and purified by column chromatography to obtain a product of 26.1 (22.5mg, yield: 5.9%). LCMS ESI(+)m/z: 229.1 (M+1).

Step B: (R)-6-(2-(ethoxymethoxy)-4,6-dimethylphenyl)-N-(1-methylpiperidin-3-yl)-1,2,4,5-tetraazine-3-amine (compound 26.2)

The compounds 26.1 (12.5 mg, 0.055 mmol) and (2-ethoxymethoxy)-4,6-dimethylphenyl) boronic acid (18.4 mg, 0.08 mmol) were dissolved in 2 mL of 1,4-dioxane and 0.2 mL of water, and cesium carbonate (45 mg, 0.14 mmol) was added BrettPhos-Pd-G4 (5mg) under nitrogen, overnight at room temperature. The reaction solution was concentrated under reduced pressure, and the product was purified by column chromatography to obtain 26.2 (8mg, yield: 39%). LCMS ESI(+)m/z: 373.2 (M+1).

Step C: (R)-3,5-dimethyl-2-(6-((1-methylpiperidin-3yl)amino)-1,2,4,5-tetraazin-3yl)phenol (compound 26)

Compound 26.2 (8 mg, 0.022 mmol) was dissolved in 2 mL of methanol, 2 mL of methanol (4 M) of hydrogen chloride was added, and stirred for 0.5 hours at room temperature. The reaction solution was concentrated under reduced pressure and purified by reversed-phase prep-HPLC to obtain compound 26 (5.9 mg, yield: 76.5%). LCMS ESI(+)m/z: 315.2 (M+1). $^1H$ NMR (400 MHz, MeOD) δ 6.69 (s, 1H), 6.64 (s, 1H), 4.52 - 4.31 (m, 1H), 3.98 - 3.79 (m, 1H), 3.61 - 3.47 (m, 1H), 3.02 (td, J = 12.9, 3.1 Hz, 1H), 2.95 (s, 3H), 2.89 (s, 1H), 2.30 (s, 3H), 2.29 - 2.24 (m, 1H), 2.21 - 2.16 (m, 1H), 2.13 (s, 3H), 2.10 - 1.98 (m, 1H), 1.75 (ddd, J = 25.4, 12.8, 3.9 Hz, 1H).

**Embodiment 27**

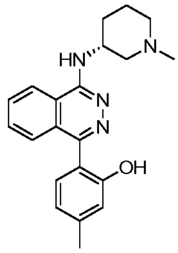

(R)-5-methyl-2-(4-((1-methylpiperidin-3-yl)amino)phthalazin-1-yl)phenol

The specific reaction Formula is as follows:

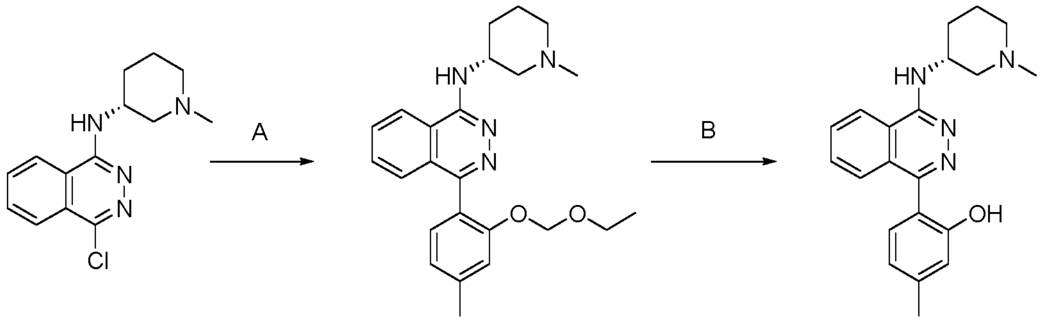


27.1

27

Step A: (R)-4-(2-(ethoxymethoxy)-4-methylphenyl)-N-(1-methylpiperidin-3-yl)phthalazin-1-amine (compound 27.1)

(R)-4-chloro-N-(1-methylpiperidin-3-yl)phthalazin-1-amine (100 mg, 0.36 mmol) and 2-(2-(ethoxymethoxy)-4-methylphenyl)-4,4,5,5-tetramethyl-1,3,2-dioxybenzaldehyde (127 mg, 0.432 mmol) were dissolved in 4 mL of dioxane and 0.4 mL of water, and cesium carbonate (352 mg, 1.08 mmol) was added$PdCl_2$(dppf) (53mg, 0.072mmol), the reaction solution was stirred at 120 °C with microwave for 3 hours. Use LCMS to monitor the reaction to completion. 30mL of water was added to the reaction solution and quenched, extracted with ethyl acetate (50mL×2), combined the organic phases, washed with brine, dried with anhydrous sodium sulfate, filtered, concentrated under reduced pressure, and purified by column chromatography to obtain a product of 27.1 (103mg, yield: 69%). LCMS ESI(+)m/z: 407.2 (M+1).

Step B: (R)-5-methyl-2-(4-((1-methylpiperidin-3-yl)amino)phthalazin-1-yl)phenol (compound 27).

Dissolve compound 27.1 (103 mg, 0.25 mmol) in 2 M hydrochloric acid/ethyl acetate 5 mL and stir for 1 h at room temperature. Use LCMS to monitor the reaction to completion. The reaction solution was concentrated under reduced pressure and purified by reversed-phase prep-HPLC to obtain compound 27 (30 mg, yield: 34%). LCMS ESI(+)m/z: 349.1 (M+1). $^1$H NMR (400 MHz, MeOD) δ 9.03 (s, 1H), 8.68 (d, *J* = 7.0 Hz, 3H), 8.27 (t, *J* = 7.6 Hz, 4H), 8.12 (t, *J* = 7.7 Hz, 4H), 8.04 (d, *J* = 8.0 Hz, 4H), 7.35 (d, *J* = 7.7 Hz, 4H), 7.04 - 6.87 (m, 9H), 4.72 (d, *J* = 23.8 Hz, 5H), 3.91 (d, *J* = 11.8 Hz, 4H), 3.58 (d, *J* = 12.0 Hz, 4H), 3.46 (dd, *J* = 9.5, 7.8 Hz, 1H), 3.20 - 3.00 (m, 8H), 2.95 (d, *J* = 19.2 Hz, 13H), 2.43 (s, 13H), 2.31 (t, *J* = 13.6 Hz, 5H), 2.18 (d, *J* = 14.6 Hz, 3H), 1.99 (dt, *J* = 36.6, 8.6 Hz, 9H).

**Embodiment 28**

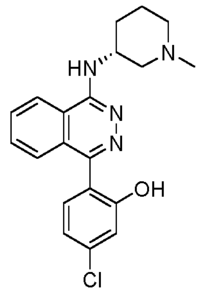

(R)-5-chloro-2-(4-((1-methylpiperidin-3-yl)amino)phthalazin-1-yl)phenol

The specific reaction Formula is as follows:

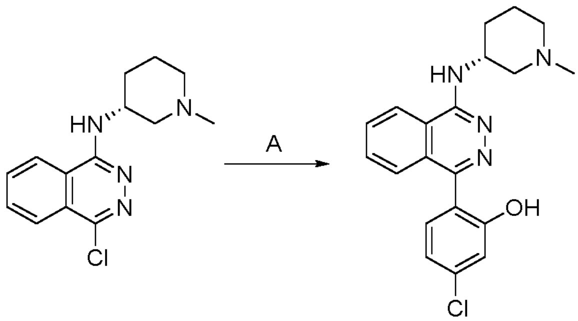


28

Step A: (R)-5-chloro-2-(4-((1-methylpiperidin-3-yl)amino)phthalazin-1-yl)phenol (compound 28)

(R)-4-chloro-N-(1-methylpiperidin-3-yl)phthalazin-1-amine (70 mg, 0.25 mmol) was added to 3.3 mL of 1,4-dioxane/$H_2O$ (V:V=10:1), followed by (4-chloro-2-hydroxyphenyl)boronic acid (52 mg, 0.30 mmol), $Pd(PPh_3)_4$(58 mg, 0.05 mmol) and cesium carbonate (244 mg, 0.75 mmol) and stir the reaction at 120 °C with microwave for 2 hours. Use LCMS to monitor the reaction to completion. The reaction solution was quenched with water, extracted with ethyl acetate (30mL×2), combined the organic phases, washed with brine, dried with anhydrous sodium sulfate, filtered, concentrated under reduced pressure, and purified with reversed-phase prep-HPLC to obtain compound 28 (14mg, yield: 15%). LCMS ESI(+)m/z: 469.1 (M+1). $^1$H NMR (400 MHz, MeOD) δ 9.06 (s, 1H), 8.68 (d, *J* = 4.3 Hz, 3H), 8.28 (t, *J* = 7.8 Hz, 4H), 8.13 (t, *J* = 7.7 Hz, 5H), 7.99 (d, *J* = 8.2 Hz, 4H), 7.48 (d, *J* = 8.1 Hz, 4H), 7.23 - 7.00 (m, 9H), 4.70 (d, *J* = 27.6 Hz, 5H), 3.90 (d, *J* = 11.7 Hz, 4H), 3.59 (d, *J* = 12.1 Hz, 4H), 3.47 (dd, *J* = 8.7, 7.1 Hz, 1H), 3.12 (ddd, *J* = 31.7, 22.9, 12.5 Hz, 8H), 2.95 (d, *J* = 19.0 Hz, 13H), 2.31 (t, *J* = 17.9 Hz, 5H), 2.18 (d, *J* = 14.4 Hz, 3H), 2.10 - 1.76 (m, 9H).

**Embodiment 29**

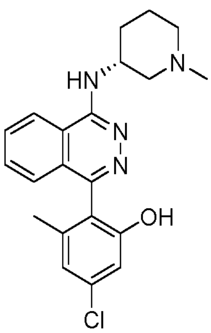

5 -Chloro-3-methyl-2-(4-((R)-1-methylpiperidin-3-yl)amino)phthazine-1-yl)phenol

The specific reaction Formula is as follows:

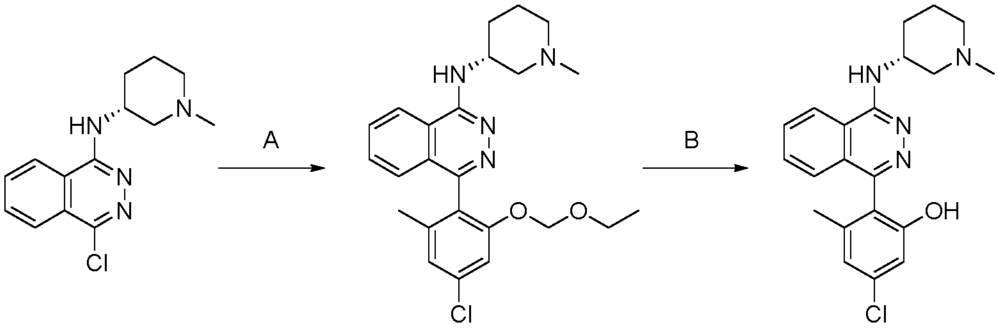


29.1 29

Step A: (4-(4-chloro-2-(ethoxymethoxy)-6-methylphenyl)-N-((R)-1-methylpiperidin-3-yl)phthalazin-1-amine (compound 29.1)

(R)-4-chloro-N-(1-methylpiperidin-3-yl)phthalazin-1-amine (100 mg, 0.36 mmol) and 2-(4-chloro-2-(ethoxymethoxy)-6-methylphenyl)-4,4,5,5-tetramethyl-1,3,2-dioxybenzaldehyde (141 mg, 0.432mmol) was dissolved in 4mL of dioxane and 0.4mL of water, cesium carbonate (352mg, 1.08mmol) and $PdCl_2$(dppf) (53mg, 0.072mmol) were added, and the reaction solution was stirred at 120°C with microwave for 3 hours. Use LCMS to monitor the reaction to completion. 30mL of water was added to the reaction solution and quenched, extracted with ethyl acetate (50mL×2), combined the organic phases, washed with brine, dried with anhydrous sodium sulfate, filtered, concentrated under reduced pressure, and purified by column chromatography to obtain a product of 29.1 (35mg, yield: 22%). LCMS ESI(+)m/z: 441.1 (M+1).

Step B: 5-chloro-3-methyl-2-(4-((R)-1-methylpiperidin-3-yl)amino)phthalazin-1-yl)phenol (compound 29).

Dissolve compound 29.1 (35 mg, 0.08 mmol) in 5 mL of hydrogen chloride/ethyl acetate (2 M) and stir for 1 h at room temperature. Use LCMS to monitor the reaction to completion. The reaction solution was concentrated under reduced pressure and purified by reversed-phase prep-HPLC to obtain compound 29 (12 mg, yield: 39%). LCMS ESI(+)m/z: 383.1 (M+1). $^1$H NMR (400 MHz, MeOD) δ 9.08 (s, 1H), 8.72 (s, 3H), 8.28 (d, *J* = 7.7 Hz, 4H), 8.13 (t, *J* = 7.7 Hz, 5H), 7.83 (d, *J* = 8.0 Hz, 5H), 7.05 (s, 4H), 6.95 (d, *J* = 1.3 Hz, 4H), 4.73 (d, *J* = 25.8 Hz, 7H), 3.90 (d, *J* = 11.5 Hz, 4H), 3.59 (d, *J* = 11.7 Hz, 5H), 3.51- 3.40 (m, 1H), 3.23 - 2.98 (m, 9H), 2.95 (d, *J* = 16.6 Hz, 13H), 2.48 - 2.24 (m, 6H), 2.19 (d, *J* = 15.1 Hz, 4H), 2.06 (s, 18H), 1.93 (d, *J* = 4.9 Hz, 5H).

**Embodiment 30**

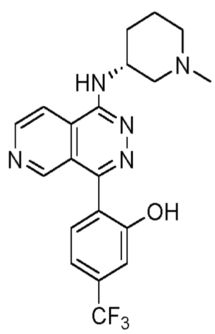

(R)-2-(1-((1-methylpiperidin-3-yl)amino)pyrido*[3,4-d]*pyridazin-4-yl)-5-(trifluoromethyl)phenol

The specific reaction Formula is as follows:

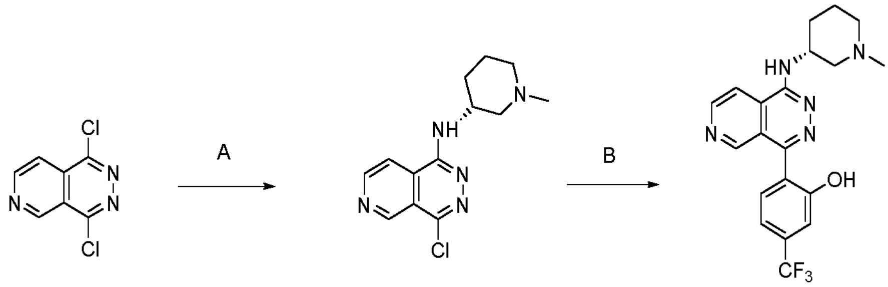


30.1 30

Step A: (R)-4-chloro-N-(1-methylpiperidine-3-yl)pyrido*[3,4-d]*pyridazin-1-amine (compound 30.1)

1,4-Dichloropyridine *[3,4-d]*pyridazine (100mg, 0.5 mmol) was dissolved in 3 mL of n-butanol, and diisopropylethylamine (387 mg, 3 mmol) and (R)-1-methylpiperidine-3-amine (112 mg, 0.6 mmol) were added. After microwave heating to 180 °C and stirring for 2 hours, the reaction solution was directly dried, then extracted with ethyl acetate, washed with brine, dried with anhydrous sodium sulfate, spun dry, and purified by column to obtain 30.1 (22mg). LCMS ESI(+)m/z: 278.1 (M+1).

Step B: (R)-2-(1-((1-methylpiperidin-3-yl)amino)pyrido*[3,4-d]*pyridazin-4-yl)-5-(trifluoromethyl)phenol (compound 30)

Compounds 30.1 (40 mg, 0.144 mmol) and (2-hydroxy-4-(trifluoromethyl)phenyl) boronic acid (36 mg, 0.173 mmol) were added to a mixture of 3 mL of 1,4-dioxane and 0.5 mL of water, followed by cesium carbonate (118 mg, 0.36 mmol) and Pd $(PPh_3)_4$(33mg, 0.028mmol), under nitrogen, the reaction was carried out at 110°C in a microwave environment for 2 hours, the reaction was quenched with water, extracted with ethyl acetate (40mL×2),the organic phase was combined, washed with brine, dried with anhydrous sodium sulfate, filtered, concentrated under reduced pressure, and purified by reversed-phase prep-HPLCto obtain compound 30 (2.8mg). LCMS ESI(+)m/z: 404.25 (M+1). $^1$H NMR (400 MHz, MeOD) $\delta$9.11 (t, J = 3.0 Hz, 2H), 8.70 - 8.29 (m, 1H), 7.68 (d, J = 7.9 Hz, 1H), 7.47 - 7.38 (m, 1H),7.34 (d, J = 1.7 Hz, 1H), 4.66 (t, J = 11.2 Hz, 1H), 3.97 (d, J = 11.1 Hz, 1H), 3.60 (d, J = 12.5 Hz, 1H), 3.18 - 2.85 (m, 5H),2.26 (dd, J = 55.7, 13.7 Hz, 2H), 2.13 - 1.77 (m, 2H).

**Embodiment 31**

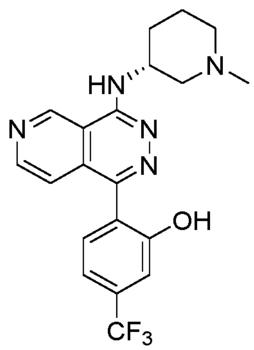

(R)-2-(4-((1-methylpiperidin-3-yl)amino)pyrido*[3,4-d]*pyridazin-1-yl)-5-(trifluoromethyl)phenol

The specific reaction Formula is as follows:

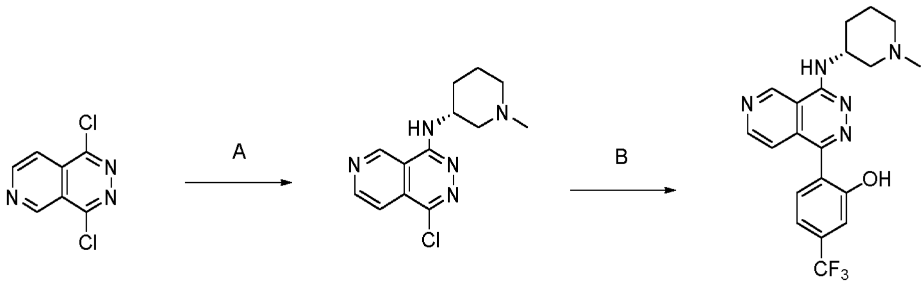


31.1 31

Step A: ((R)-1-chloro-N-(1-methylpiperidine-3-yl)pyrido*[3,4-d]*pyridazin-4-amine (compound 31.1)

1,4-dichloropyridine *[3,4-d]*pyridazine (100mg, 0.5mmol) was dissolved in 3mL n-butanol, diisopropylethylamine (387mg, 3mmol) and (R)-1-methylpiperidin-3-amine (112mg, 0.6mmol) were added, microwave heated to 180 °C and stirred for 2 hours, the reaction solution was directly dried, and then extracted with ethyl acetate, washed with brine, dried with anhydrous sodium sulfate, spun dried, and purified by column to obtain the product 31.1 (44mg,) . LCMS ESI(+)m/z: 278.1 (M+1).

Step B: (R)-2-(4-((1-methylpiperidin-3-yl)amino)pyrido*[3,4-d]*pyridazin-1-yl)-5-(trifluoromethyl)phenol (compound 31)

Compounds 31.1 (40 mg, 0.144 mmol) and (2-hydroxy-4-(trifluoromethyl)phenyl) boronic acid (36 mg, 0.173 mmol) were added to a mixture of 3 mL of 1,4-dioxane and 0.5 mL of water, followed by cesium carbonate (118 mg, 0.36 mmol) and Pd $(PPh_3)_4$(33mg, 0.028mmol), under nitrogen, the reaction was 110°C in a microwave environment for 2 hours, the reaction was quenched with water, extracted with ethyl acetate (40mL×2), the organic phase was combined, washed with brine, dried with anhydrous sodium sulfate, filtered, concentrated under reduced pressure, and purified by reversed-phase prep-HPLCto obtain compound 31 (32mg). LCMS ESI(+)m/z: 404.25 (M+1). $^1$H NMR (400 MHz, MeOD) δ 9.78 (s, 1H), 8.98 (d, J = 5.6 Hz, 1H), 7.67 - 7.51 (m, 2H), 7.41 - 7.33 (m, 1H), 7.30 (d, J = 1.7 Hz, 1H), 4.77 - 4.62 (m, 1H), 4.00 (d, J = 12.1 Hz, 1H), 3.60 (d, J = 12.7 Hz, 1H), 2.95 (d,J = 18.3 Hz, 4H), 2.35 (d, J = 12.6 Hz, 1H), 2.20 (d, J = 15.2 Hz, 1H), 1.93 (dq, J = 51.4, 13.4, 12.7 Hz, 2H).

**Embodiment 32**

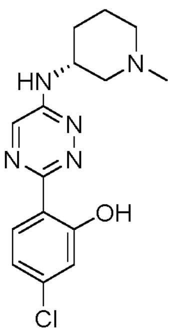

(R)-5-chloro-2-(6-((1-methylpiperidin-3-yl)amino)-1,2,4-triazin-3-yl)phenol

The specific reaction Formula is as follows:

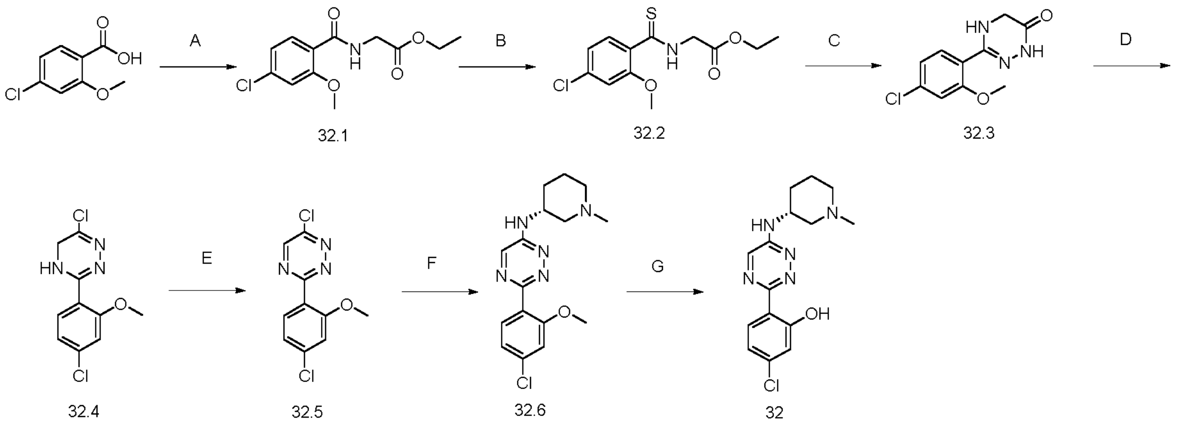


32.1 32.2 32.3 32.4 32.5 32.6 32

Step A: 2-(4-chloro-2-methoxybenzoylamido)ethyl acetate) (compound 32.1)

4-chloro-2-methoxybenzoic acid (10g, 53.6mmol) was dissolved in 200mL of DMF solution, DIPEA (17.3g, 134mmol), HATU (30.5g, 80mmol), ethyl glycinate hydrochloride (9g, 64.3mmol) were added, and stirred for two hours at room temperature. The reaction was quenched with saturated ammonium chloride aqueous solution, extracted in three times with 200mL ethyl acetate, combined the organic phases, and dried with anhydrous sodium sulfate. Compound 32.1 (14g, yield: 96%) was obtained by spinning and purification. LCMS ESI(+)m/z: 272.1(M+1).

Step B: Ethyl acetate (compound 32.2) of 2-(4-chloro-2-methoxyphenylthioamino)acetate

Compound 32.1 (14g, 51.47mmol) was dissolved in 150mL of toluene solution, Lawson's reagent (10.4g, 25.73mmol) was added, and stirred at 100°C for two hours. The reaction solution was cooled to 0 °C, and the solids were collected by filtration, and a compound of 32.2 (10 g, yield: 67.5%) was obtained. LCMS ESI(+)m/z: 288(M+1).

Step C: 3-(4-chloro-2-methoxyphenyl)-4,5-dihydro-1,2,4-triazin-6(1H)-one (compound 32.3)

Compound 32.2 (9.6g, 33.45mmol) was dissolved in 100mL of n-butanol solution, hydrazine hydrate (99%) (8.4g, 167mmol) was added, and stirred overnight at 120°C. The solvent was spun dry and the compound was purified to obtain 32.3 (4 g, yield: 50%). LCMS ESI(+)m/z: 240.1(M+1).

Step D: 6-chloro-3-(4-chloro-2-methoxyphenyl)-4,5-dihydro-1,2,4-triazine (compound 32.4)

Compound 32.3 (1g, 4.18mmol) was added to 20mL of phosphorus oxychloride solution, stirred at 100 °C for two hours, the solvent was spun dry, and the crude product was spun dried again with toluene solution, and the operation was repeated twice to obtain crude compound 32.4 (1g, yield: 93%). LCMS ESI(+)m/z: 258(M+1).

Step E: 6-chloro-3-(4-chloro-2-methoxyphenyl)-1,2,4-triazine (compound 32.5)

Compound 32.4 (1g, 3.88mmol) was dissolved in 20mLDMF solution, manganese dioxide (3.37g, 3 8.8mmol) was added in batches with an ice water bath cooling, and the reaction solution was stirred overnight at 50°C. Diatomaceous earth filtered the reaction solution, spun dry the solvent, and purified to obtain a compound of 32.5 (290mg, yield: 29%). LCMS ESI(+)m/z: 256(M+1).

Step F: (R)-3-(4-chloro-2-methoxyphenyl)-N-(1-methylpiperidin-3-yl)-1,2,4-triazin-6-amine (compound 32.6)

Compound 32.5 (100mg, 0.4mmol) was dissolved in 5mL of n-butanol solution, (R)-1-methylpiperidin-3-amine (63mg, 0.55mmol) and DIPAE (153mg, 1.18mmol) were added, and stirred at 150°C in the microwave for 3 hours. The solvent was spun dry and the compound was purified to obtain 32.6 (50 mg, yield: 38%). LCMS ESI(+)m/z: 334.1(M+1).

Step G: (R)-5-chloro-2-(6-((1-methylpiperidin-3-yl)amino)-1,2,4-triazin-3-yl)phenol (compound 32)

Dissolve compound 32.6 (50mg, 0.15mmol) in 5mL of dichloromethane solution, add 0.2mL of boron tribromide solution dropwise with an ice-water bath cooling, and stir at 0°C for 1 hour. 10mL of aqueous solution was added to

quench the reaction, and 10mL of saturated sodium bicarbonate aqueous solution was added, and 20mL of dichloromethane was used for extraction in three times, combined the organic phases, and dried with anhydrous sodium sulfate. Compound 32 (10 mg, yield: 21%) was obtained by spinning and purification. LCMS ESI(+)m/z: 320.1(M+1). $^1$H NMR (400 MHz, DMSO) δ 13.00 (s, 1H), 8.46 (s, 1H), 8.11 (d, *J* = 8.5 Hz, 1H), 7.80 (d, *J* = 7.6 Hz, 1H), 7.05 (d, *J* = 2.1 Hz, 1H), 7.02 (dd, *J* = 8.5, 2.1 Hz, 1H), 4.08 - 4.02 (m, 1H), 2.80 (d, *J* = 9.4 Hz, 1H), 2.22 (s, 3H), 2.14 (dd, *J* = 29.8, 2.5 Hz, 2H), 1.84 - 1.71 (m, 2H), 1.55 (dd, *J* = 9.2, 3.8 Hz, 1H), 1.41 (d, *J* = 8.8 Hz, 1H), 1.23 (s, 1H).

Referring to embodiment 7, embodiment 8 and embodiment 10, (2-methoxy-4-(trifluoromethyl)phenyl) boronic acid and (2-methoxy-4-chlorophenyl) boronic acid and intermediate 1.3 are used as raw materials, (R)-1-methylpiperidin-3-amine hydrochloride is replaced with (*1R,2R*)-2-aminocyclohexyl-1-ol, (cis)-3-amino-methylcyclobutyl-1-ol, (1R,3R)-3- aminocyclohexanol, and the intermediates 3.2, 4.2, 22.2, 23.2 after a five-step chemical reactions, respectively, to obtain the following embodiments 33~48.

**Embodiment 33**

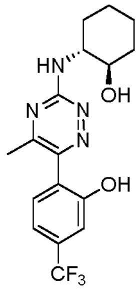

2-(3-((((*1R,2R*)-2-hydroxycyclohexyl)amino)-5-methyl-1,2,4-triazin-6-yl)-5-(trifluoromethyl)phenol

LCMS ESI(+)m/z: 369.2 (M+1). $^1$H NMR(400 MHz, Methanol-*d4*) δ 7.46 (d, *J* = 8.0 Hz, 1H), 7.24 (d, *J* = 8.0 Hz, 1H), 7.18 (s, 1H), 3.95 - 3.80 (m, 1H), 3.60 - 3.48 (m, 1H), 2.29 (s, 3H), 2.13-2.04 (m, 2H), 1.85 - 1.67 (m, 2H), 1.57 - 1.19 (m, 4H).

**Embodiment 34**

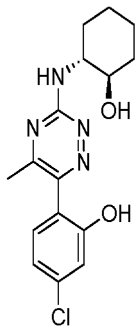

5-Chloro-2-(3-(((*1R,2R*)-2-hydroxycyclohexyl)amino)-5-methyl-1,2,4-triazin-6-yl)phenol

LCMS ESI(+)m/z: 335.1 (M+1).

**Embodiment 35**

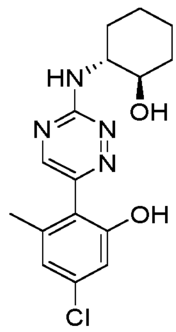

5-Chloro-2-(3-(((*1R,2R*)-2-hydroxycyclohexyl)amino)-1,2,4-triazin-6-yl)-3-methylphenol

LCMS ESI(+)m/z: 335.1 (M+1).

**Embodiment 36**

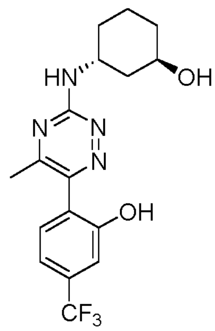

2-(3-(((1R,3R)-3-hydroxycyclohexyl)amino)-5-methyl-1,2,4-triazin-6-yl)-5-(trifluoromethyl)phenol

LCMS ESI(+)m/z: 369.2 (M+1). $^1$H NMR (400 MHz, Methanol-*d4*) δ 7.52 (d, *J* = 8.0 Hz, 1H), 7.30 (d, *J* = 8.0 Hz, 1H), 7.23 (s, 1H), 4.23-4.20 (m, 1H), 4.05-4.01 (m, 1H), 2.52 (s, 3H), 2.16 - 1.99 (m, 2H), 1.90 (q, *J* = 12.5 Hz, 1H), 1.79 - 1.64 (m, 3H), 1.63 - 1.45 (m, 2H).

**Embodiment 37**

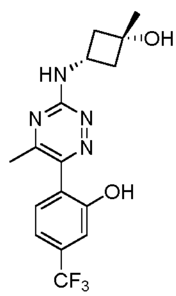

2-(3-(((cis)-3-hydroxy-3-methylcyclobutyl)amino)-5-methyl-1,2,4-triazin-6-yl)-5-(trifluoromethyl)phenol

LCMS ESI(+)m/z: 355.1 (M+1). $^1$H NMR (400 MHz, Methanol-d4) δ 7.51 (d, J= 7.8 Hz, 1H), 7.34 - 7.27 (m, 1H), 7.24 (s, 1H), 4.10 (d, J = 163.7 Hz, 1H), 2.66-2.62 (m, 2H), 2.53 (s, 3H), 2.28 (td, J = 8.7, 2.4 Hz, 2H), 1.42 (s, 3H).

**Embodiment 38**

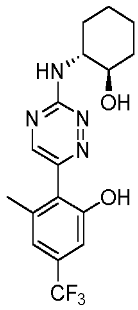

2-(3-(((*1R,2R*)-2-hydroxycyclohexyl)amino)-1,2,4-triazin-6-yl)-3-methyl-5-(trifluoromethyl)phenol

LCMS ESI(+)m/z: 369.2 (M+1).

**Embodiment 39**

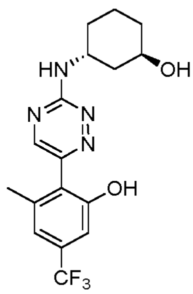

2-(3-(1R,3R)-3-hydroxycyclohexyl)atnino)-1,2,4-triazin-6-yl)-3-methyl-5-(trifluoromethyl)phenol

LCMS ESI(+)m/z: 369.2 (M+1). $^1$H NMR (400 MHz, Methanol-*d4*) δ 7.09 (s, 1H), 6.98 (s, 1H), 5.35 (d, J = 2.8 Hz, 1H), 4.15 (s, 1H), 3.89 (td, J = 10.5, 4.1 Hz, 1H), 2.45 - 2.32 (m, 3H), 2.01 (d, J = 13.9 Hz, 2H), 1.87 (q, J = 11.8 Hz, 1H), 1.77 - 1.61 (m, 3H), 1.61 - 1.42 (m, 2H).

**Embodiment 40**

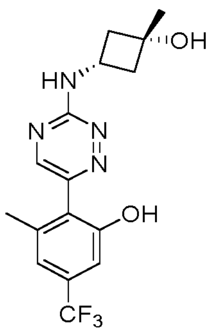

2-(3-(((cis)-3-hydroxy-3-methylcyclobutyl)amino)-1,2,4-triazin-6-yl)-3-methyl-5-(trifluoromethyl)phenol

LCMS ESI(+)m/z: 355.1 (M+1). $^1$H NMR (400 MHz, DMSO) δ 10.46 (s, 1H), 8.41 (d, J = 18.9 Hz, 2H), 7.15 (d, J = 1.8 Hz, 1H), 7.12 (d, J = 1.8 Hz, 1H), 3.97 (s, 1H), 2.46 - 2.31 (m, 3H), 2.19 (s, 3H), 2.11 (td, J = 8.8, 2.7 Hz, 2H), 1.28 (s, 3H).

**Embodiment 41**

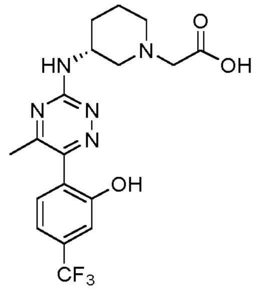

(R)-2-(3-((6-(2-hydroxy-4-(trifluoromethyl)phenyl)-5-methyl-1,2,4-triazin-3-yl)amino)piperidin-1 - yl) acetic acid

LCMS ESI(+)m/z: 412.2 (M+1). $^1$H NMR (400 MHz, MeOD) δ 7.51 (d, *J* = 7.8 Hz, 1H), 7.30 (d, *J* = 7.9 Hz, 1H), 7.23 (s, 1H), 4.58 - 4.48 (m, 1H), 4.18 (s, 2H), 3.96 (dd, *J* = 9.9, 4.1 Hz, 1H), 3.69 (dd, *J* = 17.8, 8.0 Hz, 1H), 3.22 - 2.97 (m, 2H), 2.48 (s, 3H), 2.22 (dd, *J* = 10.0, 4.8 Hz, 2H), 2.04 (dd, *J* = 7.7, 3.8 Hz, 2H).

**Embodiment 42**

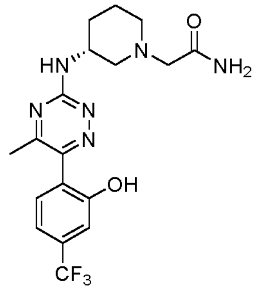

(R)-2-(3-((6-(2-hydroxy-4-(trifluoromethyl)phenyl)-5-methyl-1,2,4-triazin-3-yl)amino)piperidin-1 - yl) acetamide

LCMS ESI(+)m/z: 411.2 (M+1). $^1$H NMR (400 MHz, Methanol-*d4*) δ 7.54 (d, *J* = 7.6 Hz, 1H), 7.32 (d, *J* = 7.6 Hz, 1H), 7.25 (s, 1H), 4.68-4.63 (m, 1H), 4.08 (s, 2H), 3.94-3.90 (m, 1H), 3.68-3.63 (m, 1H), 3.17-3.12 (m, 2H), 2.59 (s, 3H), 2.34 - 1.75 (m, 4H).

**Embodiment 43**

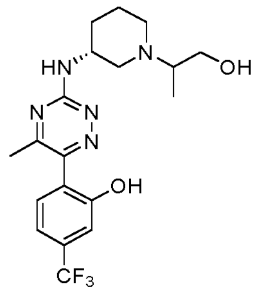

2-(3-(((3R)-1-(1-hydroxypropyl-2-yl)piperidin-3-yl)amino)-5-methyl-1,2,4-triazin-6-yl)-5-(trifluoromethyl)phenol

LCMS ESI(+)m/z: 412.2 (M+1). $^1$H NMR (400 MHz, MeOD) δ 7.52 (d, *J* = 7.9 Hz, 1H), 7.31 (d, *J* = 7.9 Hz, 1H), 7.24 (s, 1H), 4.59 (s, 1H), 3.92 (dd, *J* = 12.6, 3.8 Hz, 1H), 3.87 - 3.69 (m, 2H), 3.65 - 3.48 (m, 2H), 3.25 - 2.99 (m, 2H), 2.67 - 2.41 (m, 3H), 2.27 - 2.01 (m, 3H), 1.82 - 1.74 (m, 1H), 1.39 (d, *J* = 6.8 Hz, 3H).

**Embodiment 44**

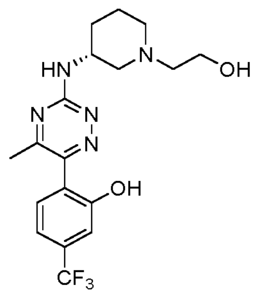

(R)-2-(3-((1-(2-hydroxyethyl)piperidin-3-yl)amino)-5-methyl-1,2,4-triazin-6-yl)-5-(trifluoromethyl)phenol

LCMS ESI(+)m/z: 398.2 (M+1).

**Embodiment 45**

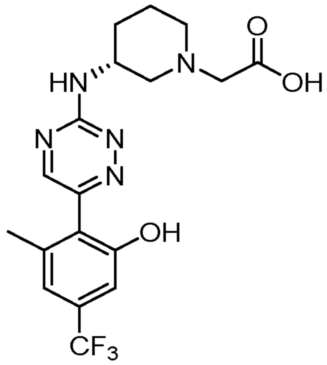

(R)-2-(3-((6-(2-hydroxy-6-methyl-4-(trifluoromethyl)phenyl)-1,2,4-triazin-3-yl)amino)piperidin-1-yl) acetic acid

LCMS ESI(+)m/z: 412.2 (M+1).

**Embodiment 46**

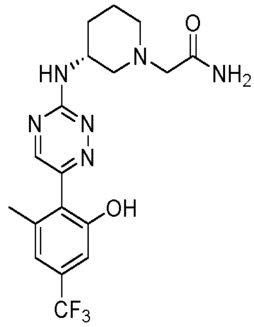

(R)-2-(3-((6-(2-hydroxy-6-methyl-4-(trifluoromethyl)phenyl)-1,2,4-triazin-3-yl)amino)piperidin-1-yl) acetamide

LCMS ESI(+)m/z: 411.2 (M+1).

**Embodiment 47**

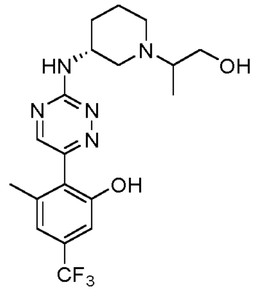

2-(3-(((3R)-1-(1-hydroxypropyl-2-yl)piperidin-3-yl)amino)-1,2,4-triazin-6-yl)-3-methyl-5-(trifluoromethyl)phenol

LCMS ESI(+)m/z: 412.2 (M+1). $^{1}$H NMR (400 MHz, MeOD) δ 7.11 (d, J = 13.5 Hz, 1H), 7.06 - 6.95 (m, 1H), 5.42 (dd, J = 9.0, 1.2 Hz, 1H), 4.20 (d, J = 18.7 Hz, 1H),3.95 (dd, J = 13.3, 4.3 Hz, 1H), 3.85 - 3.68 (m, 2H), 3.62 - 3. 49 (m, 2H), 3.18 (dt, J = 15.0, 7.3 Hz, 2H), 2.39 (s, 3H),2.27 (d, J = 10.3 Hz, 1H), 2.10 (d, J = 16.6 Hz, 2H), 1.69 (dt, J = 17.4, 12.2 Hz, 1H), 1.45 - 1.37 (m, 3H).

**Embodiment 48**

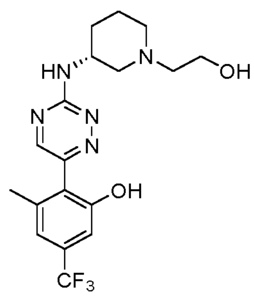

(R)-2-(3-((1-(2-hydroxyethyl)piperidin-3-yl)amino)-1,2,4-triazin-6-yl)-3-methyl-5-(trifluoromethyl)phenol

LCMS ESI(+)m/z: 398.2 (M+1). $^1$H NMR (400 MHz, MeOD) $\delta$ 7.19 - 7.06 (m, 1H), 6.99 (d, J = 1.7 Hz, 1H), 5.42 (d, J = 8.9 Hz, 1H), 4.20 (d, J = 13.2 Hz, 1H),4.05 - 3.82 (m, 3H), 3.68 (d, J = 12.7 Hz, 1H), 3.46 - 3.33 (m, 2H), 3. 08 (td, J = 12.1, 11.7, 3.3 Hz, 2H), 2.39 (s, 3H),2.32 - 2.19 (m, 1H), 2.17 - 2.05 (m, 2H), 1.71 (d, J = 12.7 Hz, 1H).

Referring to embodiment 9, 3-chloro-5-(trifluoromethyl)picolinonitrile, 3-chloro-5-(trifluoromethyl)picolinontrile were used as raw materials, and (R)-1-methylpiperidin-3-amine hydrochloride was replaced with (*1R,2R*)-2-aminocyclohexyl-1-ol, (cis)-3-amino-methylcyclobutyl-1-ol, (1R,3R)-3-Aminocyclohexanol, and the intermediates 3.2, 4.2, 22.2 and 23.2, after six-step chemical reactions, the compounds of the following embodiment 49-63 are synthesized respectively.

**Embodiment 49**

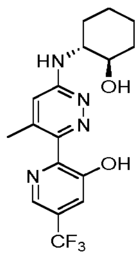

2-(6-(((*1R,2R*)-2-hydroxycyclohexyl)amino)-4-methylpyridazin-3-yl)-5-(trifluoromethyl)pyridin-3-ol

LCMS ESI(+)m/z: 369.2 (M+1). $^1$H NMR (400 MHz, MeOD) $\delta$ 8.53 (d, *J* = 0.9 Hz, 1H), 7.68 (d, *J* = 1.4 Hz, 1H), 7.44 (s, 1H), 3.65 (td, *J* = 10.6, 4.1 Hz, 1H), 3.52 (td, *J* = 9.9, 4.4 Hz, 1H), 2.28 (d, *J* = 0.6 Hz, 3H), 2.08 (dd, *J* = 7.4, 5.1 Hz, 2H), 1.81 (d, *J* = 8.8 Hz, 2H), 1.46 (ddd, *J* = 23.6, 12.6, 6.7 Hz, 4H).

**Embodiment 50**

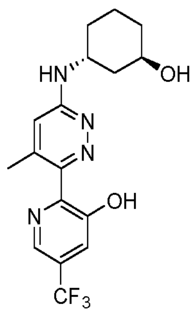

2-(6-(1R,3R)-3-hydroxycyclohexyl)amino)-4-methylpyridazin-3-yl)-5-(trifluoromethyl)pyridin-3-ol

LCMS ESI(+)m/z: 369.2 (M+1).

**Embodiment 51**

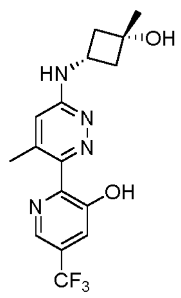

2-(6-(cis)-3-hydroxy-3-methylcyclobutyl)amino)-4-methylpyridazin-3-yl)-5-(trifluoromethyl)pyridin-3-ol

LCMS ESI(+)m/z: 355.1(M+1). $^1$H NMR (400 MHz, MeOD) $\delta$ 8.53 (d, *J* = 0.9 Hz, 1H), 7.68 (d, *J* = 1.8 Hz, 1H), 7.44 (s, 1H), 3.92 (p, *J* = 7.7 Hz, 1H), 2.76 - 2.62 (m, 2H), 2.29 (s, 3H), 2.23 (dd, *J* = 14.7, 5.5 Hz, 2H), 1.42 (s, 3H).

**Embodiment 52**

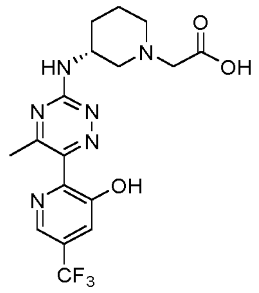

(R)-2-(3-((6-(3-hydroxy-5-(trifluoromethyl)pyridin-2-yl)-5-methyl-1,2,4-triazin-3-yl)amino)piperidine-1-yl)acetic acid

LCMS ESI(+)m/z: 413.2 (M+1).

**Embodiment 53**

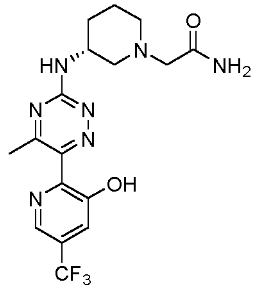

(R)-2-(3-((6-(3-hydroxy-5-(trifluoromethyl)pyridin-2-yl)-5-methyl-1,2,4-triazin-3-yl)amino)piperidin-1-yl)acetamide

LCMS ESI(+)m/z: 412.2 (M+1).

**Embodiment 54**

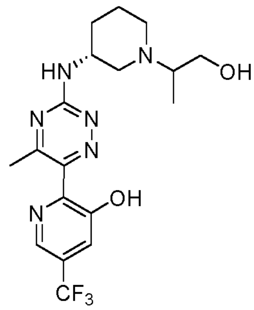

2-(3-(((3R)-1-(1-hydroxypropyl-2-yl)piperidin-3-yl)amino)-5-methyl-1,2,4-triazin-6-yl)-5-(trifluoromethyl)pyridin-3 -ol

LCMS ESI(+)m/z: 413.2 (M+1).

**Embodiment 55**

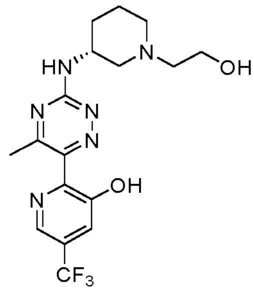

(R)-2-(3-((1-(2-hydroxyethyl)piperidin-3-yl)amino)-5-methyl-1,2,4-triazin-6-yl)-5-(trifluoromethyl)pyridin-3 -ol

LCMS ESI(+)m/z: 399.2 (M+1).

**Embodiment 56**

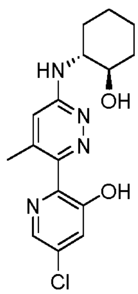

5-Chloro-2-(6-(((*1R,2R*)-2-hydroxycyclohexyl)amino)-4-methylpyridazin-3-yl)pyridin-3 -ol

LCMS ESI(+)m/z: 335.1 (M+1).

**Embodiment 57**

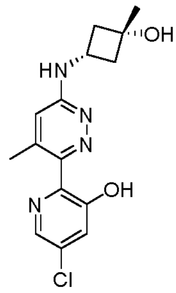

5-Chloro-2-(6-(((cis)-3-hydroxy-3-methylcyclobutyl)atnino)-4-methylpyridazin-3-yl)pyridin-3-ol

LCMS ESI(+)m/z: 321.1 (M+1).

**Embodiment 58**

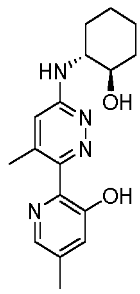

2-(6-(((*1R,2R*)-2-hydroxycyclohexyl)amino)-4-methylpyridazin-3-yl)-5-methylpyridin-3-ol

LCMS ESI(+)m/z: 315.2 (M+1).

**Embodiment 59**

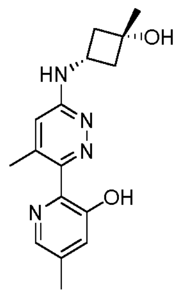

2-(6-(((cis)-3-hydroxy-3-methylcyclobutyl)atnino)-4-methylpyridazin-3-yl)-5-methylpyridin-3-ol

LCMS ESI(+)m/z: 301.2 (M+1).

**Embodiment 60**

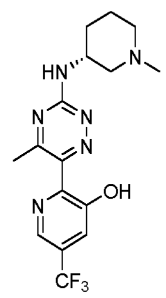

(R)-2-(5-methyl-3-((1-methylpiperidin-3-yl)amino)-1,2,4-triazin-6-yl)-5-(trifluoromethyl)pyridin-3-ol

LCMS ESI(+)m/z: 369.2 (M+1).

**Embodiment 61**

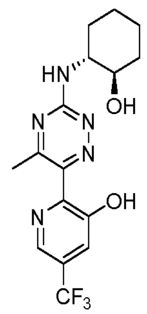

2-(3-(((*1R,2R*)-2-hydroxycyclohexyl)amino)-5-methyl-1,2,4-triazin-6-yl)-5-(trifluoromethyl)pyridin-3 -ol

LCMS ESI(+)m/z: 370.1 (M+1).

**Embodiment 62**

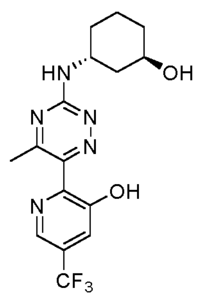

2-(3-(((1R,3R)-3-hydroxycyclohexyl)amino)-5-methyl-1,2,4-triazin-6-yl)-5-(trifluoromethyl)pyridin-3 -ol

LCMS ESI(+)m/z: 370.1 (M+1).

**Embodiment 63**

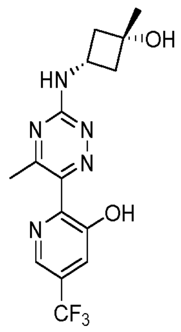

2-(3-(((cis)-3-hydroxy-3-methylcyclobutyl)amino)-5-methyl-1,2,4-triazin-6-yl)-5-(trifluoromethyl)pyridin-3 -ol

LCMS ESI(+)m/z: 356.1 (M+1).

Referring to embodiment 14, with 1,4-dichlorophthalazine and its analogues as raw materials, (R)-1-methylpiperidin-3-amine hydrochloride is replaced with (*1R,2R*)-2-aminocyclohexyl-1-ol, (cis)-3-aminocyclobutyl-1-ol, (1R,3R)-3-atninocyclohexanol, and intermediates 3.2, 4.2, 22.2, 23.2 respectively, and the following embodiments 64 ~ 101 are synthesized after two step chemical reactions, respectively.

**Embodiment 64**

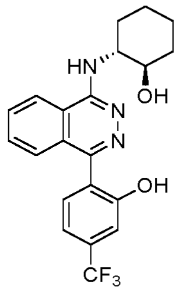

2-(4-((*1R,2R*)-2-hydroxycyclohexyl)amino)phthalazin-1-yl)-5-(trifluoromethyl)phenol

LCMS ESI(+)m/z: 404.1 (M+1). $^1$H NMR (400 MHz, MeOD) δ 8.73 (d, *J* = 8.0 Hz, 1H), 8.09 (pd, *J* = 7.3, 1.4 Hz, 2H), 7.78 - 7.69 (m, 1H), 7.57 (d, *J* = 7.9 Hz, 1H), 7.34 (d, *J* = 7.9 Hz, 1H), 7.28 (s, 1H), 4.03 - 3.84 (m, 1H), 3.75 (td, *J* = 10.3, 4.5 Hz, 1H), 2.15 (dd, *J* = 9.7, 4.8 Hz, 2H), 1.86 (d, *J* = 9.6 Hz, 2H), 1.68 (dt, *J* = 12.6, 7.9 Hz, 1H), 1.62 - 1.37 (m, 3H).

**Embodiment 65**

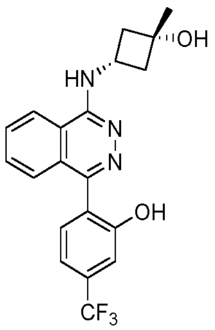

2-(4-((cis)-3-hydroxy-3-methylcyclobutyl)amino)phthalazin-1-yl)-5-(trifluoromethyl)phenol

LCMS ESI(+)m/z: 390.1 (M+1). $^1$H NMR (400 MHz, MeOD) δ 8.85 - 8.58 (m, 1H), 8.24 - 7.87 (m, 2H), 7.81 - 7.67 (m, 1H), 7.58 (d, *J* = 7.9 Hz, 1H), 7.35 (d, *J* = 7.9 Hz, 1H), 7.29 (s, 1H), 4.14 (p, *J* = 7.8 Hz, 1H), 2.82 - 2.70 (m, 2H), 2.44 (t, *J* = 10.1 Hz, 2H), 1.48 (s, 3H).

**Embodiment 66**

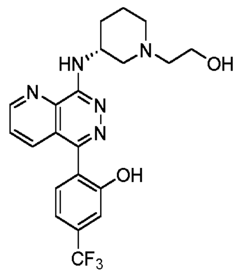

(R)-2-(8-((1-(2-hydroxyethyl)piperidin-3-yl)amino)pyridino*[2,3-d]*pyridazin-5-yl)-5-(trifluoromethyl)phenol

LCMS ESI(+)m/z: 434.2 (M+1).

**Embodiment 67**

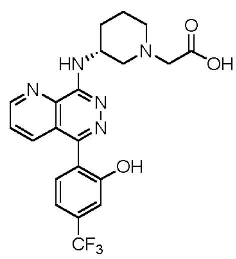

(R)-2-(3-((5-(2-hydroxy-4-(trifluoromethyl)phenyl)pyrido*[2,3-d]*pyridazin-8-yl)amino)piperidin-1-yl)acetic acid

LCMS ESI(+)m/z: 448.2 (M+1).

**Embodiment 68**

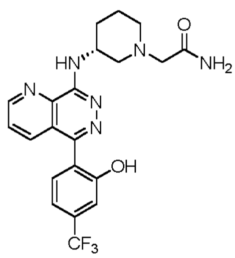

(R)-2-(3-((5-(2-hydroxy-4-(trifluoromethyl)phenyl)pyridino*[2,3-d]*pyridazin-8-yl)amino)piperidin-1-yl)acetamide

LCMS ESI(+)m/z: 447.2 (M+1).

**Embodiment 69**

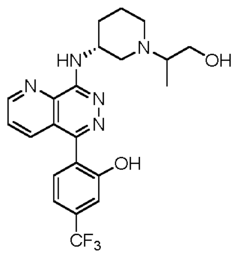

2-(8-(((3R)-1-(1-hydroxypropyl-2-yl)piperidin-3-yl)amino)pyridino*[2,3-d]*pyridazin-5-yl)-5 - (trifluoromethyl)phenol

LCMS ESI(+)m/z: 448.2 (M+1).

**Embodiment 70**

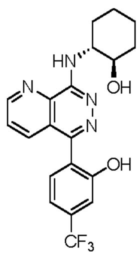

2-(8-(((*1R,2R*)-2-hydroxycyclohexyl)amino)pyrido*[2,3-d]*pyridazin-5-yl)-5-(trifluoromethyl)phenol

LCMS ESI(+)m/z: 405.2 (M+1). $^1$H NMR (400 MHz, CD3OD) $\delta$ 9.25 (d,*J* = 4.2 Hz, 1H), 8.14 (d, *J* = 8.2 Hz, 1H), 8.05 (dd, *J* = 8.2, 4.3 Hz, 1H), 7.62 (d, *J* = 7.9 Hz, 1H), 7.35 (d, *J* = 7.9 Hz, 1H), 7.31 (s, 1H), 4.03 - 3.89 (m, 1H), 3.78 (td, *J* = 10.0, 4.3 Hz, 1H), 2.14 (d, *J* = 12.8 Hz, 2H), 1.93 - 1.72 (m, 3H), 1.49 (m, 3H).

**Embodiment 71**

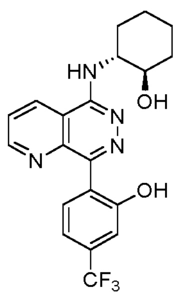

2-(5-(*1R,2R*)-2-hydroxycyclohexyl)amino)pyrido*[2,3-d]*pyridazin-8-yl)-5-(trifluoromethyl)phenol

LCMS ESI(+)m/z: 405.2 (M+1). $^1$H NMR (400 MHz, MeOD) δ 9.23 (dd, J= 4.4, 1.2 Hz, 1H), 9.16 (dd, *J* = 8.5, 1.2 Hz, 1H), 8.04 (dd, *J* = 8.5, 4.5 Hz, 1H), 7.57 (d, *J* = 7.9 Hz, 1H), 7.28 (d, *J* = 7.9 Hz, 1H), 7.22 (s, 1H), 3.97 (td, *J* = 11.8, 4.0 Hz, 1H), 3.76 (td, *J* = 10.4, 4.5 Hz, 1H), 2.24 - 2.06 (m, 2H), 1.96 - 1.82 (m, 2H), 1.70 (ddd, *J* = 24.9, 12.7, 3.1 Hz, 1H), 1.60 - 1.40 (m, 3H).

**Embodiment 72**

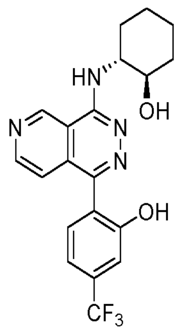

2-(4-(((*1R,2R*)-2-hydroxycyclohexyl)amino)pyrido*[3,4-d]*pyridazin-1-yl)-5-(trifluoromethyl)phenol

LCMS ESI(+)m/z: 405.2 (M+1). $^1$H NMR (400 MHz,MeOD) δ 10.11 (s, 1H), 9.14 (d, *J* = 5.5 Hz, 1H), 7.65 (d, *J* = 5.5 Hz, 1H), 7.61 (d, *J* = 7.9 Hz, 1H), 7.35 (d, *J* = 8.0 Hz, 1H), 7.30 (s, 1H), 4.05 - 3.94 (m, 1H), 3.79 (td, *J* = 10.5, 4.5 Hz, 1H), 2.22 - 2.11 (m, 2H), 1.87 (d, *J* = 2.7 Hz, 2H), 1.81 - 1.66 (m, 1H), 1.63 - 1.37 (m, 3H).

**Embodiment 73**

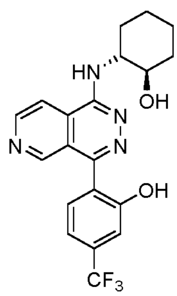

2-(1-(((*1R*,*2R*)-2-hydroxycyclohexyl)amino)pyrido[*3*,*4-d]*pyridazin-4-yl)-5-(trifluoromethyl)phenol

LCMS ESI(+)m/z: 405.2 (M+1).

**Embodiment 74**

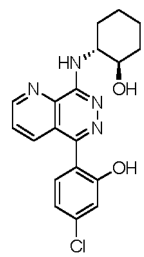

5-Chloro-2-(8-(((*1R*,*2R*)-2-hydroxycyclohexyl)amino)pyrido*[2,3-d]*pyridazin-5-yl)phenol

LCMS ESI(+)m/z: 371.1 (M+1).

**Embodiment 75**

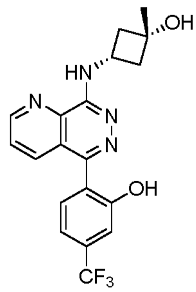

2-(8-(((cis)-3-hydroxy-3-methylcyclobutyl)amino)pyrido*[2,3-d]*pyridazin-5-yl)-5-(trifluoromethyl)phenol

LCMS ESI(+)m/z: 391.1 (M+1). $^1$H NMR (400 MHz,MeOD) δ 9.25 (dd, J = 4.4, 1.6 Hz, 1H), 8.16 (dd, J = 8.3, 1.6 Hz, 1H), 8.04 (dd, J = 8.4, 4.5 Hz, 1H), 7.62 (d, J = 7.9 Hz, 1H), 7.36 (dd, J = 7.9, 0.9 Hz, 1H), 7.30 (s, 1H), 4.17 (p, J = 7.9 Hz, 1H), 2.79 - 2.72 (m, 2H), 2.47 (td, J = 8.7, 2.5 Hz, 2H), 1.48 (s, 3H).

**Embodiment 76**

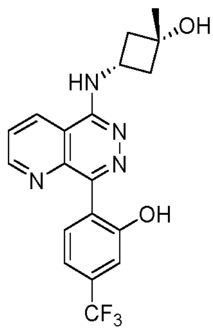

2-(5-(((cis)-3-hydroxy-3-methylcyclobutyl)amino)pyrido*[2,3-d]*pyridazin-8-yl)-5-(trifluoromethyl)phenol

LCMS ESI(+)m/z: 391.1 (M+1). $^1$H NMR (400 MHz, MeOD) δ 9.23 (dd, J = 4.5, 1.5 Hz, 1H), 9.11 (dd, J = 8.5, 1.5 Hz, 1H), 8.04 (dd, J = 8.5, 4.5 Hz, 1H), 7.63 (d, J = 7.9 Hz, 1H), 7.28 (dd, J = 8.0, 0.9 Hz, 1H), 7.22 (s, 1H), 4.15 (p, J = 8.0 Hz, 1H), 2.84 - 2.72 (m, 2H), 2.45 (td, J = 8.8, 2.5 Hz, 2H), 1.48 (s, 3H).

**Embodiment 77**

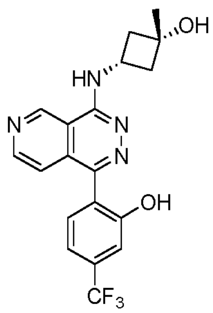

2-(4-(((cis)-3-hydroxy-3-methylcyclobutyl)amino)pyrido*[3,4-d]*pyridazin-1-yl)-5-(trifluoromethyl)phenol

LCMS ESI(+)m/z: 391.1 (M+1).

**Embodiment 78**

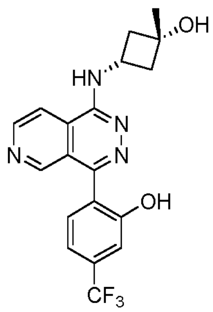

2-(1-((((cis)-3-hydroxy-3-methylcyclobutyl)amino)pyrido*[3,4-d]*pyridazin-4-yl)-5-(trifluoromethyl)phenol

LCMS ESI(+)m/z: 391.1 (M+1). $^1$H NMR (400 MHz, MeOD) δ 10.05 (s, 1H), 9.13 (d, *J* = 5.5 Hz, 1H), 7.63 (d, *J* = 5.5 Hz, 1H), 7.60 (d, *J* = 7.9 Hz, 1H), 7.36 (d, *J* = 8.0 Hz, 1H), 7.31 (s, 1H), 4.16 (t, *J* = 7.7 Hz, 1H), 2.82 - 2.75 (m, 2H), 2.50 (dd, *J* = 11.4, 8.7 Hz, 2H), 1.48 (s, 3H).

**Embodiment 79**

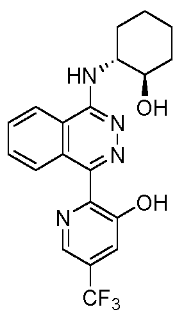

2-(4-(((*1R,2R*)-2-hydroxycyclohexyl)amino)phthalazin-1-yl)-5-(trifluoromethyl)pyridin-3-ol

LCMS ESI(+)m/z: 405.2 (M+1).

**Embodiment 80**

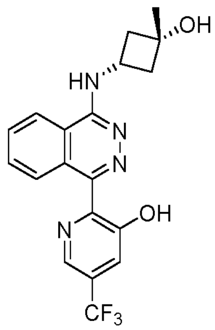

2-(4-(((cis)-3-hydroxy-3-methylcyclobutyl)atnino)phthalazin-1-yl)-5-(trifluoromethyl)pyridin-3-ol

LCMS ESI(+)m/z: 391.1 (M+1).

**Embodiment 81**

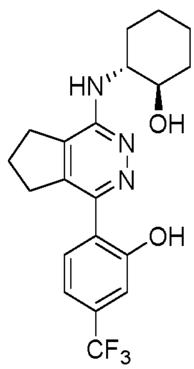

2-(4-(((*1R,2R*)-2-hydroxycyclohexyl)amino)-6,7-dihydro-5H-cyclopenta*[d]*pyridazin-1-yl)-5-(trifluoromethyl)phenol

LCMS ESI(+)m/z: 394.2 (M+1). $^1$H NMR (400 MHz, MeOD) δ 7.52 (d, *J* = 7.9 Hz, 1H), 7.26 (d, *J* = 8.0 Hz, 1H), 7.22 (s, 1H), 3.78 (td, *J* = 11.3, 4.2 Hz, 1H), 3.60 (td, *J* = 10.0, 4.4 Hz, 1H), 3.13 - 2.94 (m, 4H), 2.27 (p, *J* = 7.7 Hz, 2H), 2.15 - 1.95 (m, 2H), 1.89 - 1.76 (m, 2H), 1.62 - 1.21 (m, 4H).

**Embodiment 82**

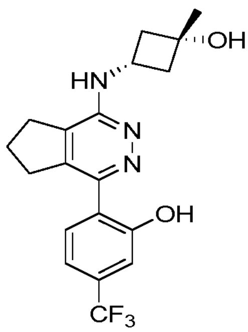

2-(4-(((cis)-3-hydroxy-3-methylcyclobutyl)amino)-6,7-dihydro-5H-cyclopenta*[d]*pyridazin-1-yl)-5-(trifluoromethyl)phenol

LCMS ESI(+)m/z: 380.2 (M+1). $^1$H NMR (400 MHz, Methanol-*d4*) δ 7.54 (d, *J* = 8.0 Hz, 1H), 7.28 (d, *J* = 8.0 Hz, 1H), 7.24 (s, 1H), 3.99 (p, *J* = 8.0 Hz, 1H), 3.05 (q, *J* = 8.2 Hz, 4H), 2.66 (dd, *J* = 12.0, 7.4 Hz, 2H), 2.28 (t, *J* = 7.7 Hz, 4H), 1.43 (s, 3H).

**Embodiment 83**

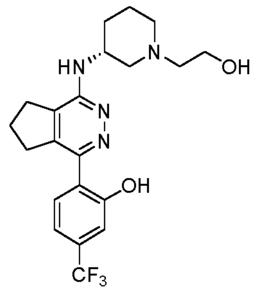

(R)-2-(4-((1-(2-hydroxyethyl)piperidin-3-yl)amino)-6,7-dihydro-5H-cyclopenta*[d]*pyridazin-1-yl)-5 -(trifluoromethyl)phenol

LCMS ESI(+)m/z: 423.2 (M+1).

**Embodiment 84**

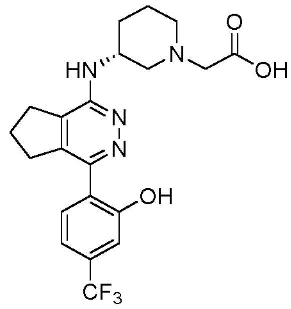

(R)-2-(3-((4-(2-hydroxy-4-(trifluoromethyl)phenyl)-6,7-dihydro-5H-cyclopenta*[d]*pyridazin-1-yl)amino)piperidin-1-yl)acetic acid

LCMS ESI(+)m/z: 437.2 (M+1).

**Embodiment 85**

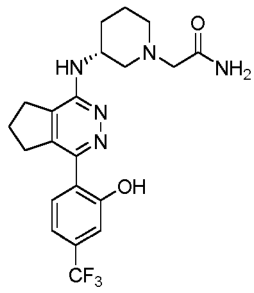

(R)-2-(3-((4-(2-hydroxy-4-(trifluoromethyl)phenyl)-6,7-dihydro-5H-cyclopenta*[d]*pyridazin-1-yl)amino)piperidin-1-yl)acetamide

LCMS ESI(+)m/z: 436.2 (M+1).

**Embodiment 86**

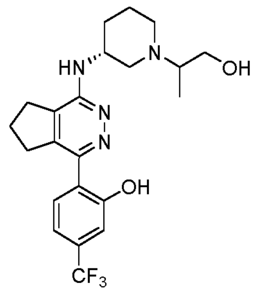

2-(4-(((3R)-1-(1-hydroxypropyl-2-yl)piperidin-3-yl)amino)-6,7-dihydro-5H-cyclopenta*[d]*pyridazin-1-yl)-5-(trifluoromethyl)phenol

LCMS ESI(+)m/z: 437.2 (M+1).

**Embodiment 87**

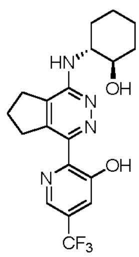

2-(4-(((*1R,2R*)-2-hydroxycyclohexyl)amino)-6,7-dihydro-5H-cyclopenta*[d]*pyridazin-1-yl)-5-(trifluoromethyl)pyridin-3 -ol

LCMS ESI(+)m/z: 395.2 (M+1).

**Embodiment 88**

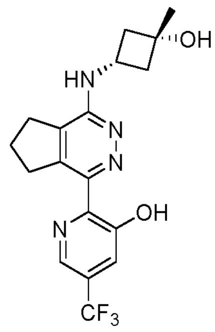

2-(4-(((cis)-3-hydroxy-3-methylcyclobutyl)amino)-6,7-dihydro-5H-cyclopenta*[d]*pyridazin-1-yl)-5-(trifluoromethyl)pyridin-3-ol

LCMS ESI(+)m/z: 381.2 (M+1).

**Embodiment 89**

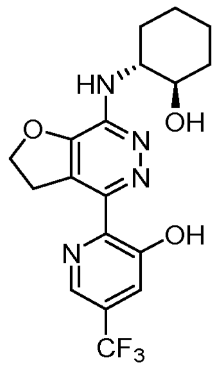

2-(7-(*1R,2R*)-2-hydroxycyclohexyl)amino)-2,3-dihydrofuro*[2,3-d]*pyridazin-4-yl)-5-(trifluoromethyl)pyridin-3 -ol

LCMS ESI(+)m/z: 397.1 (M+1).

**Embodiment 90**

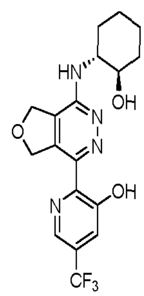

2-(4-(*1R,2R*)-2-hydroxycyclohexyl)amino)-5,7-dihydrofuro*[3,4-d]*pyridazin-1-yl)-5-(trifluoromethyl)pyridin-3 -ol

LCMS ESI(+)m/z: 397.1 (M+1).

**Embodiment 91**

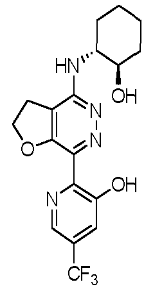

2-(4-(((*1R,2R*)-2-hydroxycyclohexyl)amino)-2,3-dihydrofuro*[2,3-d]*pyridazin-7-yl)-5-(trifluoromethyl)pyridin-3 -ol

LCMS ESI(+)m/z: 397.1 (M+1).

**Embodiment 92**

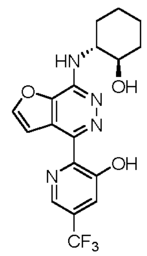

2-(7-(((*1R,2R*)-2-hydroxycyclohexyl)amino)furo*[2,3-d]*pyridazin-4-yl)-5-(trifluoromethyl)pyridin-3-ol

LCMS ESI(+)m/z: 395.1 (M+1).

**Embodiment 93**

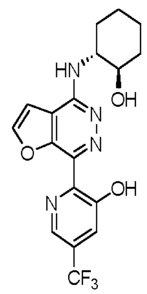

2-(4-(((*1R,2R*)-2-hydroxycyclohexyl)amino)furo[*2,3-d]*pyridazin-7-yl)-5-(trifluoromethyl)pyridin-3-ol

LCMS ESI(+)m/z: 395.1 (M+1).

**Embodiment 94**

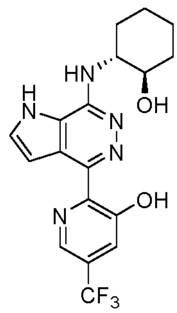

2-(7-(((*1R,2R*)-2-hydroxycyclohexyl)amino)-1H-pyrrolo*[2,3-d]*pyrazine-4-yl)-5-(trifluoromethyl)pyridin-3 -ol

LCMS ESI(+)m/z: 394.1 (M+1).

**Embodiment 95**

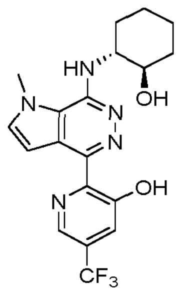

2-(7-(((*1R,2R*)-2-hydroxycyclohexyl)amino)-1-methyl-1H-pyrrolo*[2,3-d]*pyrazine-4-yl)-5-(trifluoromethyl)pyridin-3 -ol

LCMS ESI(+)m/z: 408.2 (M+1).

**Embodiment 96**

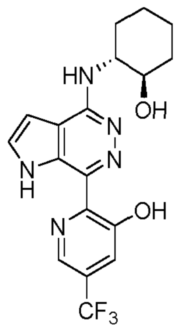

2-(4-(((*1R,2R*)-2-hydroxycyclohexyl)amino)-1H-pyrrolo*[2,3-d]*pyrazine-7-yl)-5-(trifluoromethyl)pyridin-3 -ol

LCMS ESI(+)m/z: 394.1 (M+1).

**Embodiment 97**

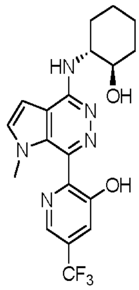

2-(4-(((*1R,2R*)-2-hydroxycyclohexyl)amino)-1-methyl-1H-pyrrolo*[2,3-d]*pyrazine-7-yl)-5-(trifluoromethyl)pyridin-3 -ol

LCMS ESI(+)m/z: 408.2 (M+1).

**Embodiment 98**

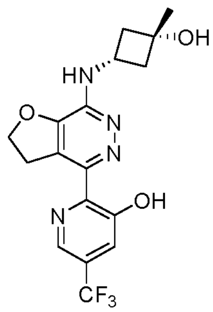

2-(7-(cis)-3-hydroxy-3-methylcyclobutyl)amino)-2,3-dihydrofuro*[2,3-d]*pyridazin-4-yl)-5-(trifluoromethyl)pyridin-3 -ol

LCMS ESI(+)m/z: 383.1 (M+1).

**Embodiment 99**

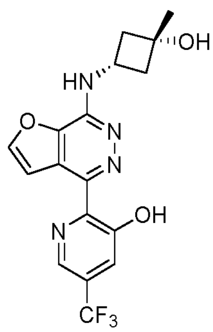

2-(7-(((cis)-3-hydroxy-3-methylcyclobutyl)amino)furo[*2,3-d]*pyridazin-4-yl)-5-(trifluoromethyl)pyridin-3 -ol

LCMS ESI(+)m/z: 381.1 (M+1).

**Embodiment 100**

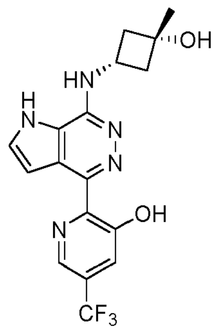

2-(7-(((cis)-3-hydroxy-3-methylcyclobutyl)amino)-1H-pyrrolo*[2,3-d]*pyridazin-4-yl)-5-(trifluoromethyl)pyridin-3 -ol

LCMS ESI(+)m/z: 380.1 (M+1).

**Embodiment 101**

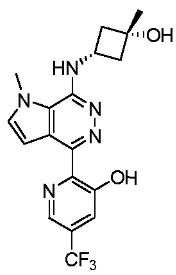

2-(7-(((cis)-3-hydroxy-3-methylcyclobutyl)amino)-1-methyl-1H-pyrrolo*[2,3-d]*pyridazin-4-yl)-5-(trifluoromethyl)pyridin-3 -ol

LCMS ESI(+)m/z: 394.1 (M+1).

Referring to embodiment 32, we obtain the compounds of the following embodiments 102-106.

**Embodiment 102**

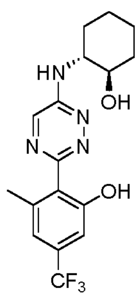

2-(6-(((*1R*,*2R*)-2-hydroxycyclohexyl)amino)-1,2,4-triazin-3-yl)-3-methyl-5-(trifluoromethyl)phenol

LCMS ESI(+)m/z: 369.2 (M+1).

**Embodiment 103**

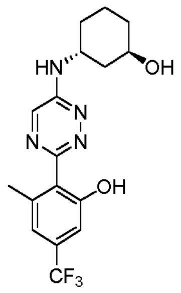

2-(6-(((*1R*,*3R*)-3-hydroxycyclohexyl)amino)-1,2,4-triazin-3-yl)-3-methyl-5-(trifluoromethyl)phenol

LCMS ESI(+)m/z: 369.2 (M+1).

**Embodiment 104**

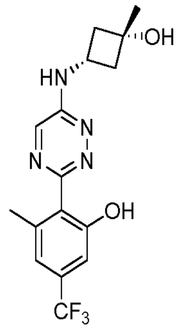

2-(6-(((cis)-3-hydroxy-3-methylcyclobutyl)amino)-1,2,4-triazin-3-yl)-3-methyl-5-(trifluoromethyl)phenol

LCMS ESI(+)m/z: 355.1 (M+1).

**Embodiment 105**

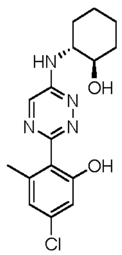

5-Chloro-2-(6-(((*1R,2R*)-2-hydroxycyclohexyl)amino)-1,2,4-triazin-3-yl)-3-methylphenol

LCMS ESI(+)m/z: 335.1 (M+1).

**Embodiment 106**

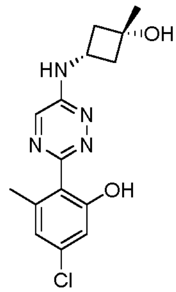

5-Chloro-2-(6-(((cis)-3-hydroxy-3-methylcyclobutyl)amino)-1,2,4-triazin-3-yl)-3-methylphenol

LCMS ESI(+)m/z: 321.1 (M+1).

Embodiment 107~161

| Embodiment | structure | Synthesis methods and characterization data |
|---|---|---|
| 107 | 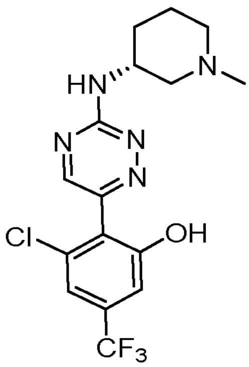 | The synthesis method refers to embodiment 1<br>LCMS ESI(+)m/z: 388(M+1). $^1$HNMR(400 MHz, MeOD) δ 8.34 (s, 1H), 7.34 (s, 1H), 7.22 (s, 1H), 5.45 (d, *J* = 8.9 Hz, 1H), 4.07 (dd, *J* = 16.1, 6.7 Hz, 1H), 3.64 - 3.47 (m, 2H), 2.94 (s, 3H), 2.21 (d, *J* = 7.5 Hz, 2H), 2.03 (d, *J* = 4.5 Hz, 2H), 1.69 (d, *J* = 12.4 Hz, 2H). |
| 108 | 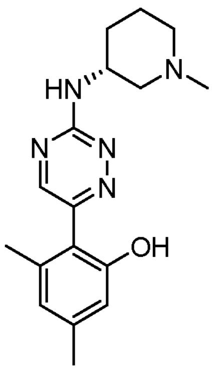 | The synthesis method refers to embodiment 1<br>LCMS ESI(+)m/z: 314 (M+1). $^1$H NMR (400 MHz, DMSO-d6) δ 10.89 (s, 1H), 8.28 (s, 1H), 7.94-7.64 (m, 1H), 6.67 (s, 1H), 6.62 (s, 1H), 4.39-4.37 (m, 1H), 3.62 (d, *J* = 12.6 Hz, 1H), 3.39 (d, *J* = 9.6 Hz, 1H), 3.09 - 2.81 (m, 2H), 2.80 (s, 3H), 2.26 (s, 3H), 2.10 (s, 4H), 1.90 (dd, *J* = 41.2, 8.6 Hz, 2H), 1.69 - 1.50 (m, 1H). |
| 109 | 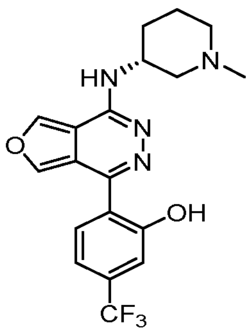 | The synthesis method refers to Embodiment 14<br>LCMS ESI(+)m/z: 393(M+1). $^1$H NMR (400 MHz, DMSO) δ 10.46 (s, 1H), 9.45 (s, 1H), 8.90 (s, 1H), 7.79 (s, 1H), 7.41 - 7.28 (m, 2H), 4.65 - 4.47 (m, 1H), 3.72 (d, *J* = 11.3 Hz, 2H), 2.83 (s, 3H), 2.20 (s, 1H), 1.99 (d, *J* = 7.5 Hz, 2H), 1.87 (d, *J* = 11.0 Hz, 1H), 1.68 (d, *J* = 10.7 Hz, 1H), 1.54 - 1.42 (m, 1H), 1.36 - 1.28 (m, 1H). |

(continued)

| Embodiment | structure | Synthesis methods and characterization data |
|---|---|---|
| 110 | 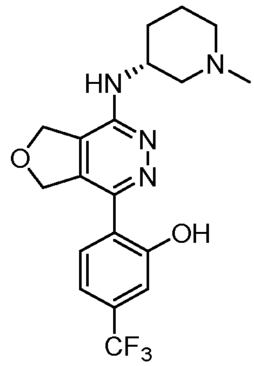 | The synthesis method refers to Embodiment 14<br>LCMS ESI(+)m/z: 395(M+1). |
| 111 | 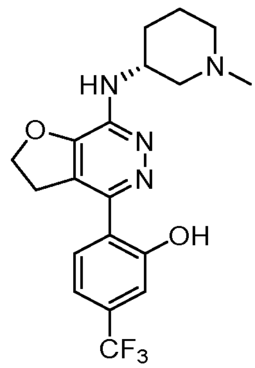 | The synthesis method refers to Embodiment 14<br>LCMS ESI(+)m/z: 395 ( M+1). $^1$H NMR (400 MHz, MeOD) $\delta$ 7.70 (d, *J* = 7.2 Hz, 1H), 7.37 (d, *J* = 6.6 Hz, 1H), 7.33 (s, 1H), 5.14 (t, *J* = 8.1 Hz, 2H), 4.46 (s, 1H), 3.82 (dd, *J* = 31.8, 11.8 Hz, 1H), 3.59 - 3.43 (m, 3H), 3.20 - 2.88 (m, 5H), 2.22 (d, *J* = 11.6 Hz, 1H), 2.12 (d, *J* = 13.9 Hz, 1H), 2.00 (t,*J* = 13.2 Hz, 1H), 1.73 (dd, *J* = 23.1, 10.8 Hz, 1H). |
| 112 | 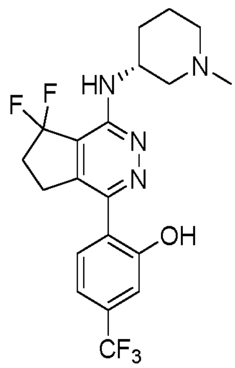 | The synthesis method refers to Embodiment 14<br>LCMS ESI(+)m/z: 429 (M+1). $^1$H NMR (400 MHz, DMSO-*d6*) $\delta$ 10.75 (s, 1H), 7.59 (d, *J* = 8.0 Hz, 1H), 7.39 (s, 1H), 7.29 (d, *J* = 8.0 Hz, 1H), 7.07 (d, *J* = 8.4 Hz, 1H), 4.85 - 4.69 (m, 1H), 3.58 (d, *J* = 11.4 Hz, 1H), 3.42 (d, *J* = 12.0 Hz, 1H), 3.02 (s, 3H), 2.88 - 2.76 (m, 4H), 2.72 - 2.56 (m, 2H), 2.03 (d, *J* = 10.3 Hz, 1H), 1.97 - 1.80 (m, 2H), 1.71 (q, *J* = 16.8, 16.4 Hz, 1H). |
| 113 | 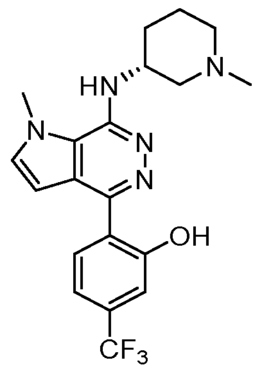 | The synthesis method refers to Embodiment 14<br>LCMS ESI(+)m/z: 406.1(M+1). $^1$H NMR (400 MHz, MeOD) $\delta$ 7.83 - 7.73 (m, 1H), 7.40 (d, *J* = 8.2 Hz, 1H), 7.37 (s, 1H), 6.87 (d, *J* = 3.0 Hz, 1H), 4.60 (dd, *J* = 15.4, 3.5 Hz, 1H), 4.45 (d, *J* = 61.5 Hz, 2H), 3.94 (dd, *J* = 29.3, 12.2 Hz, 1H), 3.58 (d, *J* = 12.3 Hz, 1H), 3.09 (ddd, *J* = 29.0, 20.0, 12.8 Hz, 1H), 2.92 (d, *J* = 34.9 Hz, 2H), 2.31 (t, *J* = 13.8 Hz, 1H), 2.16 (d, *J* = 14.8 Hz, 1H), 2.04 (dd, *J* = 24.4, 14.9 Hz, 1H), 1.96 - 1.77 (m, 1H). |

(continued)

| Embodiment | structure | Synthesis methods and characterization data |
|---|---|---|
| 114 | 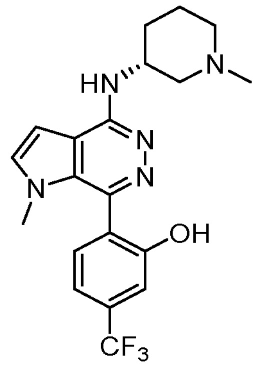 | The synthesis method refers to Embodiment 14 LCMS ESI(+)m/z: 406.1(M+1). $^1$H NMR (400 MHz, MeOD) δ 7.91 (s, 1H), 7.71 (s, 2H), 7.42 (d, *J* = 6.0 Hz, 2H), 7.34 (s, 1H), 7.22 (s, 1H), 4.57 (d, *J* = 37.3 Hz, 1H), 3.87 (d, *J* = 10.2 Hz, 1H), 3.65 (s, 4H), 3.57 (d, *J* = 12.0 Hz, 1H), 3.19 - 3.01 (m, 2H), 2.96 (s, 3H), 2.91 (s, 1H), 2.30 (d, *J* = 9.6 Hz, 1H), 2.16 (d, *J* = 14.4 Hz, 1H), 2.02 (d, *J* = 10.7 Hz, 2H), 1.82 (t, *J* = 12.5 Hz, 1H). |
| 115 | 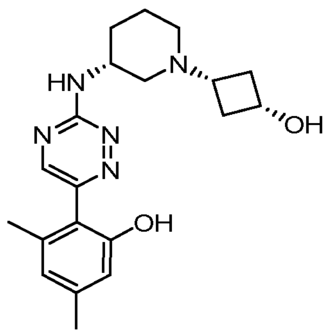 | The synthesis method refers to embodiment 1 LCMS ESI(+)m/z: 370.21 (M+1). $^1$H NMR (400 MHz, MeOD) δ 6.65 - 6.50 (m, 2H), 5.42 (d, J = 9.1 Hz, 1H), 4.03 (hept, J = 11.7 Hz, 2H), 3.82 - 3.63 (m, 1H), 3.53 (d, J = 12.4 Hz, 1H), 2.90 - 2.65 (m, 4H), 2.27 (d, J = 9.7 Hz, 8H), 2.16 - 1.88 (m, 3H), 1.67 (ddt, J = 21.1, 13.4, 6.2 Hz, 1H). |
| 116 | 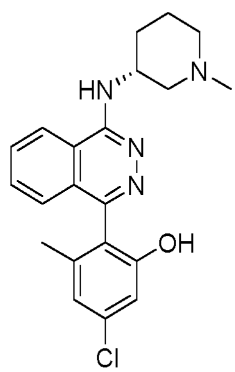 | The synthesis method refers to Embodiment 14 LCMS ESI(+)m/z: 383.1 (M+1). $^1$H NMR (400 MHz, MeOD) δ 9.08 (s, 1H), 8.72 (s, 3H), 8.28 (d, *J* = 7.7 Hz, 4H), 8.13 (t, *J* = 7.7 Hz, 5H), 7.83 (d, *J* = 8.0 Hz, 5H), 7.05 (s, 4H), 6.95 (d, *J* = 1.3 Hz, 4H), 4.73 (d, *J* = 25.8 Hz, 7H), 3.90 (d, *J* = 11.5 Hz, 4H), 3.59 (d, *J* = 11.7 Hz, 5H), 3.51 - 3.40 (m, 1H), 3.23 - 2.98 (m, 9H), 2.95 (d, *J* = 16.6 Hz, 13H), 2.48 - 2.24 (m, 6H), 2.19 (d, *J* = 15.1 Hz, 4H), 2.06 (s, 18H), 1.93 (d, *J* = 4.9 Hz, 5H). |
| 117 | 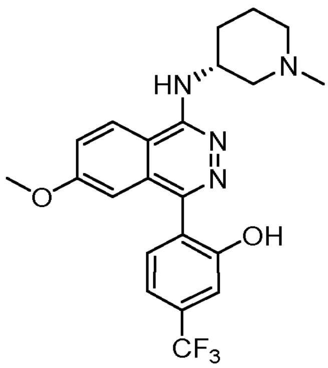 | The synthesis method refers to Embodiment 14 LCMS ESI(+)m/z: 433.2 (M+1). $^1$H NMR (400 MHz, MeOD) *δ* 8.68 (d, *J* = 8.6 Hz, 1H), 7.77 (d, *J* = 7.8 Hz, 1H), 7.63 (d, *J* = 7.7 Hz, 1H), 7.43 - 7.27 (m, 2H), 7.09 (s, 1H), 4.64 (d, *J* = 30.4 Hz, 1H), 3.88 (s, 4H), 3.70 - 3.51 (m, 2H), 3.11 (d, *J* = 35.5 Hz, 2H), 2.95 (s, 3H), 2.30 (d, *J* = 10.3 Hz, 1H), 2.21 - 1.82 (m, 3H). |

(continued)

| Embodiment | structure | Synthesis methods and characterization data |
|---|---|---|
| 118 | 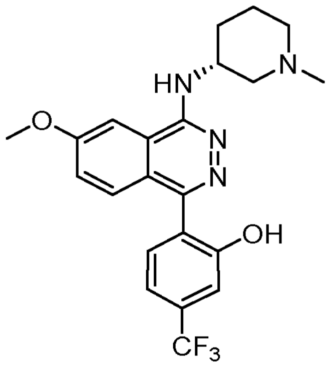 | The synthesis method refers to Embodiment 14<br>LCMS ESI(+)m/z: 433.2 (M+1). $^1$H NMR (400 MHz, MeOD) $\delta$ 8.48 (s, 1H), 8.13 (s, 1H), 7.87 (d, *J* = 9.0 Hz, 1H), 7.67 (d, *J* = 8.0 Hz, 2H), 7.42 (d, *J* = 8.0 Hz, 1H), 7.37 (s, 1H), 4.69 (s, 1H), 4.18 (d, *J* = 21.1 Hz, 3H), 3.90 (d, *J* = 6.7 Hz, 1H), 3.53 (d, *J* = 34.1 Hz, 1H), 3.21 - 3.04 (m, 2H), 2.95 (d, *J* = 19.5 Hz, 3H), 2.33 (d, *J* = 10.0 Hz, 1H), 2.18 (d, *J* = 12.9 Hz, 1H), 2.00 (dd, *J* = 26.1, 13.2 Hz, 2H). |
| 119 | 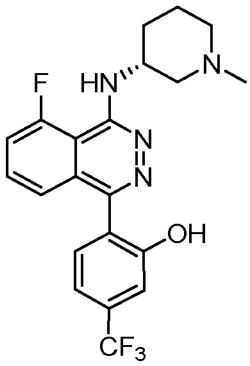 | The synthesis method refers to Embodiment 14<br>LCMS ESI(+)m/z: 421.1 (M+1). $^1$H NMR (400 MHz, MeOD) δ 8.18 - 8.07 (m, 1H), 8.02 (dd, J = 12.5, 7.7 Hz, 1H), 7.77 (d, J = 8.1 Hz, 1H), 7.69 (d, J = 7.8 Hz, 1H), 7.43 (d, J = 8.0 Hz, 1H), 7.37 (s, 1H), 4.73 (s, 1H), 4.07 -3.78 (m, 1H), 3.59 (d, J = 11.5 Hz, 1H), 3.25 - 3.01 (m, 2H), 2.95 (d, J = 21.0 Hz, 3H), 2.53 - 1.75 (m, 4H). |
| 120 | 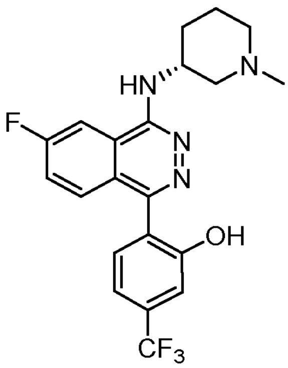 | The synthesis method refers to Embodiment 14<br>LCMS ESI(+)m/z: 421(M+1). $^1$H NMR (400 MHz, MeOD) δ 8.46 (d, *J* = 8.7 Hz, 1H), 7.99 (dd, *J* = 9.1, 5.3 Hz, 1H), 7.89 (dd, *J* = 8.3, 2.2 Hz, 1H), 7.67 (d, *J* = 7.9 Hz, 1H), 7.41 (d, *J* = 7.9 Hz, 1H), 7.36 (s, 1H), 4.69 (d, *J* = 11.3 Hz, 1H), 3.92 (d, *J* = 9.2 Hz, 1H), 3.58 ( d, *J* = 10.3 Hz, 1H), 3.07 (d, *J* = 10.0 Hz, 2H), 2.97 (s, 3H), 2.33 (d, *J* = 11.6 Hz, 1H), 2.18 (d, *J* = 12.6 Hz, 1H), 2.09 - 1.89 (m, 2H). |
| 121 | 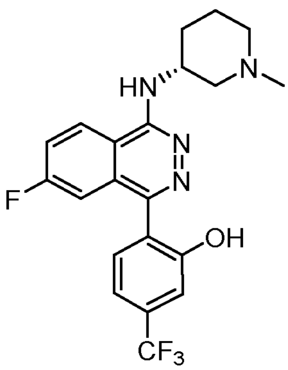 | The synthesis method refers to Embodiment 14<br>LCMS ESI(+)m/z: 421(M+1). $^1$H NMR (400 MHz,) δ 8.84 (d, *J* = 4.4 Hz, 1H), 8.01 (t, *J* = 7.5 Hz, 1H), 7.66 (d, *J* = 7.9 Hz, 1H), 7.51 (dd, *J* = 8.7, 2.3 Hz, 1H), 7.41 (d, *J* = 7.7 Hz, 1H), 7.35 (s, 1H), 4.70 (d, *J* = 25.3 Hz, 1H), 3.90 (d, *J* = 9.5 Hz, 1H), 3.59 (d, *J* = 10.8 Hz, 1H), 3.23 - 3.03 (m, 2H), 2.98 (s, 3H), 2.34 (d, *J* = 11.3 Hz, 1H), 2.18 (d, *J* = 12.7 Hz, 1H), 2.02 (dd, *J* = 32.2, 17.8 Hz, 2H). |

(continued)

| Embodiment | structure | Synthesis methods and characterization data |
|---|---|---|
| 122 | 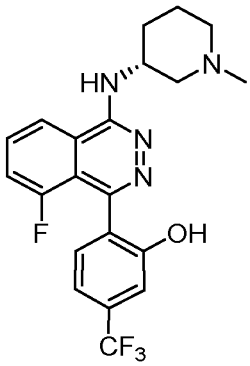 | The synthesis method refers to Embodiment 14<br>LCMS ESI(+)m/z: 421.1 (M+1). $^1$H NMR (400 MHz, MeOD) δ 9.05 - 8.41 (m, 1H), 8.27 (td, J = 8.2, 5.3 Hz, 1H), 7.86 (dd, J = 11.1, 8.2 Hz, 1H), 7.66 (d, J = 7.9 Hz, 1H), 7.35 (d, J = 7.9 Hz, 1H), 7.26 (s, 1H), 4.77 - 4.52 (m, 1H), 3.90 (d, J = 11.9 Hz, 1H), 3.59 (d, J = 12.3 Hz, 1H), 3.53 - 3.03 (m, 2H), 2.95 (d, J = 18.6 Hz, 3H), 2.42 - 2.11 (m, 2H), 2.11 - 1.74 (m, 2H). |
| 123 | 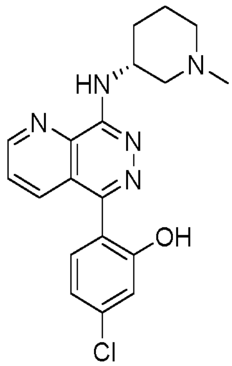 | The synthesis method refers to Embodiment 14<br>LCMS ESI(+)m/z: 370.1 (M+1). $^1$H NMR (400 MHz, MeOD) δ 9.43 - 9.33 (m, 1H), 8.37 (t, *J* = 9.9 Hz, 1H), 8.18 - 8.03 (m, 1H), 7.50 (t, *J* = 8.3 Hz, 1H), 7.21 - 7.06 (m, 2H), 4.66 (dd, *J* = 23.0, 11.3 Hz, 1H), 3.89 (dd, *J* = 30.6, 10.4 Hz, 1H), 3.71 - 3.43 (m, 1H), 3.26 - 3.11 (m, 1H), 3.07 (td, *J* = 12.7, 3.1 Hz, 1H), 2.97 (d, *J* = 6.3 Hz, 3H), 2.32 (d, *J* = 12.3 Hz, 1H), 2.28 - 2.13 (m, 1H), 2.06 (dd, *J* = 27.1, 13.6 Hz, 1H), 1.90 (qd, *J* = 12.6, 3.9 Hz, 1H). |
| 124 | 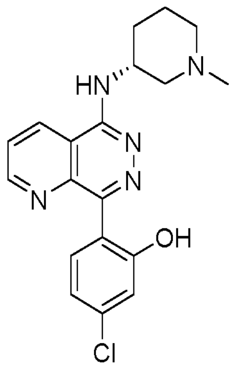 | The synthesis method refers to Embodiment 14<br>LCMS ESI(+)m/z: 370.1 (M+1). $^1$H NMR (400 MHz, DMSO) δ 11.00 (d, J = 129.0 Hz, 1H), 10.32 - 9.37 (m, 2H), 9.29 (td, J = 4.2, 1.3 Hz, 1H), 8.36 - 7.92 (m, 1H), 7.49 (t, J = 9.1 Hz, 1H), 7.12 (s, 1H), 7.05 (dd, J = 8.2, 2.0 Hz, 1H), 4.92 - 4.46 (m, 1H), 3.76 - 3.60 (m, 1H), 3.53 - 3.39 (m, 1H), 3.26 (dd, J = 22.7, 12.8 Hz, 1H), 3.17 - 2.87 (m, 1H), 2.83 (d, J = 4.5 Hz, 3H), 2.20 (d, J = 11.6 Hz, 1H), 2.13 - 1.66 (m, 3H). |
| 125 | 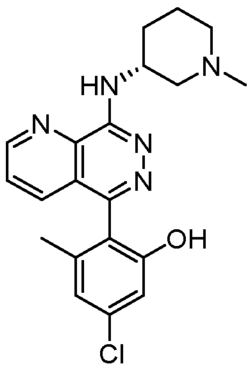 | The synthesis method refers to Embodiment 14<br>LCMS ESI(+)m/z: 384.1 (M+1). $^1$H NMR (400 MHz, MeOD) δ 9.41 (dd, *J* = 13.6, 4.0 Hz, 1H), 8.19 (d, *J* = 8.4 Hz, 1H), 8.09 (dd, *J* = 8.3, 4.4 Hz, 1H), 7.05 (s, 1H), 6.94 (d, *J* = 1.7 Hz, 1H), 4.68 (d, *J* = 25.6 Hz, 1H), 3.86 (d, *J* = 10.7 Hz, 1H), 3.68 - 3.42 (m, 1H), 3.22 - 3.01 (m, 2H), 2.98 (d, *J* = 2.5 Hz, 3H), 2.33 (d, *J* = 12.7 Hz, 1H), 2.24 - 1.99 (m, 5H), 1.98 - 1.78 (m, 1H). |

(continued)

| Embodiment | structure | Synthesis methods and characterization data |
|---|---|---|
| 126 | 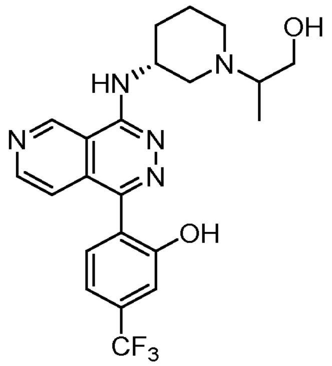 | The synthesis method refers to Embodiment 14 LCMS ESI(+)m/z: 448.1 (M+1). $^1$H NMR (400 MHz, MeOD) δ 10.07 (d, J = 3.2 Hz, 1H), 9.17 (d, J = 5.5 Hz, 1H), 7.76 (d, J = 5.5 Hz, 1H), 7.68 (d, J = 7.9 Hz, 1H), 7.45 - 7.37 (m, 1H), 7.35 (d, J = 1.6 Hz, 1H), 4.76 (d, J = 14.0 Hz, 1H), 4.04 - 3.86(m, 2H), 3.77 (dt, J = 12.7, 7.1 Hz, 1H), 3.70 - 3.52 (m, 2H), 3.37 (dq, J = 11.5, 6.5, 4.7 Hz, 1H), 3.25 (dd, J = 13.8, 10.8 Hz, 1H), 2.39 (d, J = 12.8 Hz, 1H), 2.27 - 2.05 (m, 2H), 2.02 - 1.87 (m, 1H), 1.50 - 1.38 (m, 3H). |
| 127 | 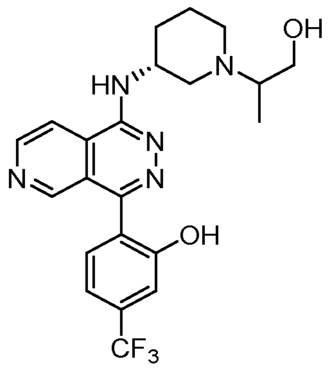 | The synthesis method refers to Embodiment 14 LCMS ESI(+)m/z: 448.1 (M+1). $^1$H NMR (400 MHz, MeOD) δ 9.39 - 9.11 (m, 2H), 8.56 (dd, J = 5.8, 3.6 Hz, 1H), 7.75 (d, J = 8.0 Hz, 1H), 7.46 (dd, J = 8.0, 1.7 Hz, 1H), 7.39 (d, J = 1.7 Hz, 1H), 4.80 (s, 1H), 3.94 (dd, J = 12.6, 3.8 Hz, 2H), 3.75 (ddd, J = 12.6, 6.7, 4.2 Hz, 1H), 3.68 - 3.50 (m, 2H), 3.30 - 3.15 (m, 2H), 2.36 (d, J = 12.6 Hz, 1H), 2.25 - 2.01 (m, 2H), 1.94 (qd, J = 12.4, 4.1 Hz, 1H), 1.49 - 1.37 (m, 3H). |
| 128 | 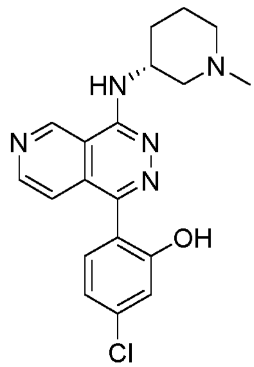 | The synthesis method refers to Embodiment 14 LCMS ESI(+)m/z: 370.1 (M+1). $^1$H NMR (400 MHz, DMSO) δ 9.79 (s, 1H), 8.85 (d, J = 5.6 Hz, 1H), 8.24 (d, J = 2.4 Hz, 1H), 7.66 (s, 1H), 7.40 - 7.24 (m, 2H), 7.11 - 6.97 (m, 2H), 4.50 (s, 1H), 3.18 (dd, J = 11.1, 3.9 Hz, 1H), 2.82 (d, J = 10.3 Hz,1H), 2.32 (d, J = 1.4 Hz, 3H), 2.20 - 1.96 (m, 3H), 1.82 (dt, J = 13.2, 3.6 Hz, 1H), 1.75 - 1.42 (m, 2H). |
| 129 | 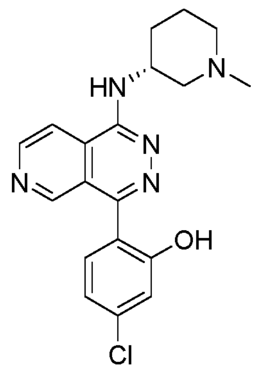 | The synthesis method refers to Embodiment 14 LCMS ESI(+)m/z: 370.1 (M+1). $^1$H NMR (400 MHz, DMSO) δ 10.36 (s, 1H), 8.93 (d, J = 5.7 Hz, 1H), 8.84 (d, J = 0.8 Hz, 1H), 8.30 (dd, J = 5.8, 1.0 Hz, 1H), 8.17 (d, J = 4.1 Hz, 1H), 7.41 (dd, J = 13.2, 8.1 Hz, 2H), 7.05 (dq, J = 3.7, 2.1 Hz, 2H), 4.43(d, J = 9.6 Hz, 1H), 3.14 (d, J = 10.5 Hz, 2H), 2.79 (d, J = 11.0 Hz, 1H), 2.29 (d, J = 3.4 Hz, 3H), 2.12 -1.96 (m, 3H), 1.80 (d, J = 13.1 Hz, 1H), 1.63 (d, J = 12.5 Hz, 1H), 1.57 - 1.41 (m, 1H). |

(continued)

| Embodiment | structure | Synthesis methods and characterization data |
|---|---|---|
| 130 | 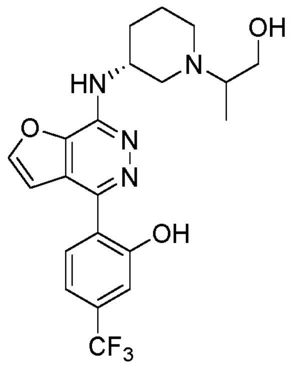 | The synthesis method refers to Embodiment 14 LCMS ESI(+)m/z: 437.2 (M+1). $^1$H NMR (400 MHz, MeOD) δ 8.43 (s, 1H), 7.86 (d, *J* = 7.9 Hz, 1H), 7.46 - 7.36 (m, 2H), 7.32 (s, 1H), 4.75 - 4.71 (m, 1H), 4.00 - 3.83 (m, 2H), 3.82 - 3.72 (m, 1H), 3.64 - 3.57 (m, 2H), 3.24 - 3.10 (m, 2H), 2.31 (d, *J* = 11.3 Hz, 1H), 2.21 - 2.02 (m, 2H), 1.96 - 1.77 (m, 1H), 1.41 (d, *J* = 6.7 Hz, 3H). |
| 131 | 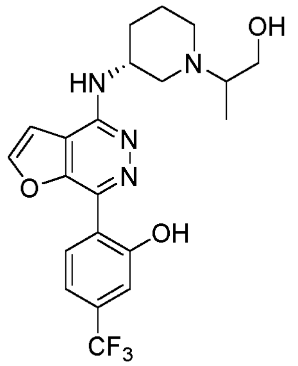 | The synthesis method refers to Embodiment 14 LCMS ESI(+)m/z: 437.1 (M+1). $^1$H NMR (400 MHz, MeOD) δ 8.47 (d, *J* = 2.0 Hz, 1H), 7.99 (d, *J* = 8.0 Hz, 1H), 7.58 (s, 1H), 7.38 (d, *J* = 8.2 Hz, 1H), 7.35 (s, 1H), 4.71-4.60 (m, 1H), 4.02 - 3.84 (m, 2H), 3.81 - 3.72 (m, 1H), 3.64 - 3.53 (m, 2H), 3.26 - 3.15 (m, 2H), 2.34 (d, *J* = 11.1 Hz, 1H), 2.22 - 2.05 (m, 2H), 1.86 (qd, *J* = 12.4, 4.3 Hz, 1H), 1.43 (dd, *J* = 13.9, 6.3 Hz, 3H). |
| 132 | 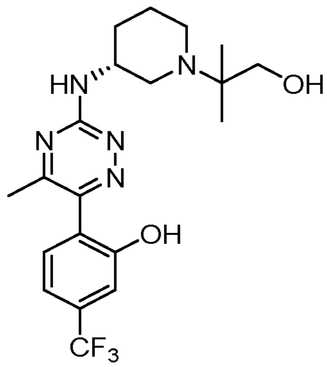 | The synthesis method refers to Example 8 LCMS ESI(+)m/z: 426.2 (M+1). $^1$H NMR (400 MHz, MeOD) *δ* 7.56 (d, *J* = 7.1 Hz, 1H), 7.36 - 7.23 (m, 2H), 4.78 (s, 1H), 3.89 (s, 1H), 3.77 - 3.63 (m, 3H), 3.10 (dd, *J* = 20.6, 9.9 Hz, 2H), 2.59 (s, 3H), 2.28 - 2.03 (m, 3H), 1.85 (s, 1H), 1.44 (s, 6H). |
| 133 | 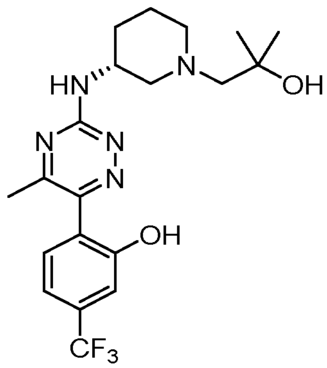 | The synthesis method refers to Example 8 LCMS ESI(+)m/z: 426.2 (M+1). $^1$H NMR (400 MHz, MeOD) δ 7.52 (d, *J* = 7.9 Hz, 1H), 7.30 (d, *J* = 7.9 Hz, 1H), 7.23 (s, 1H), 4.61 (s, 1H), 4.05 (d, *J* = 8.2 Hz, 1H), 3.81 - 3.71 (m, 1H), 3.45 - 3.34 (m, 1H), 3.25 - 3.20 (m, 2H), 3.11 - 2.98 (m, 1H), 2.51 (s, 3H), 2.26 - 2.07 (m, 3H), 1.93 - 1.69 (m, 1H), 1.38 - 1.36 (m, 6H). |

(continued)

| Embodiment | structure | Synthesis methods and characterization data |
|---|---|---|
| 134 | 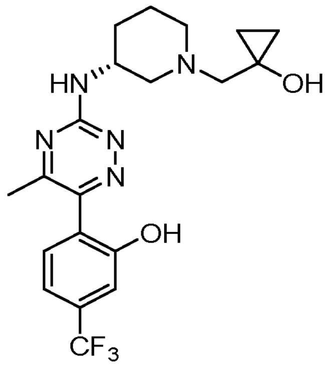 | The synthesis method refers to Example 8<br>LCMS ESI(+)m/z: 424.2 (M+1). $^1$H NMR (400 MHz, MeOD) $\delta$ 7.51 (d, *J* = 7.9 Hz, 1H), 7.30 (d, *J* = 7.9 Hz, 1H), 7.23 (s, 1H), 4.58 (s, 1H), 4.02 (d, *J* = 8.9 Hz, 1H), 3.79 (d, *J* = 12.2 Hz, 1H), 3.34 (d, *J* = 4.1 Hz, 2H), 3.05 (ddd, *J* = 28.2, 18.6, 7.3 Hz, 2H), 2.47 (s, 3H), 2.26 (d, *J* = 12.4 Hz, 1H), 2.15 (d, *J* = 14.6 Hz, 1H), 2.06 - 1.99 (m, 1H), 1.76 (d, *J* = 9.9 Hz, 1H), 0.95 - 0.90 (m, 2H), 0.83 - 0.74 (m, 2H). |
| 135 | 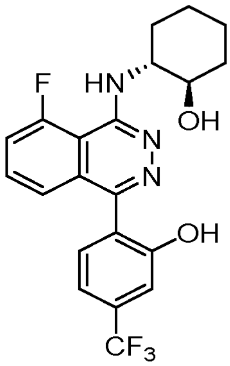 | The synthesis method refers to Embodiment 14<br>LCMS ESI(+)m/z: 422.1 (M+1). $^1$H NMR (400 MHz, MeOD) δ 8.09 (td, J = 8.2, 5.2 Hz, 1H), 7.89 (ddd, J = 12.9, 8.2, 0.7 Hz, 1H), 7.61 - 7.55 (m, 2H), 7.35 (dd, J = 7.9, 0.9 Hz, 1H), 7.28 (s, 1H), 3.95 (t, J = 10.1 Hz, 1H), 3.78 (td, J = 10.0, 4.5 Hz, 1H), 2.26 - 2.06 (m, 2H), 1.92 - 1. 81 (m, 2H), 1.67 (qd, J = 12.5, 3.1 Hz, 1H), 1.61 - 1.33 (m, 3H). |
| 136 | 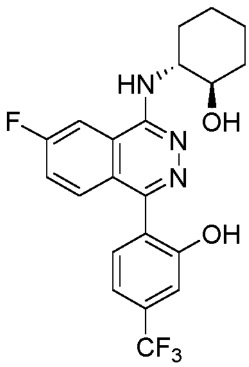 | The synthesis method refers to Embodiment 14<br>LCMS ESI(+)m/z: 422.2(M+1). $^1$H NMR (400 MHz, MeOD) δ 8.56 (dd, *J* = 9.5, 2.1 Hz, 1H), 7.86 (qd, *J* = 9.0, 3.9 Hz, 2H), 7.58 (d, *J* = 7.9 Hz, 1H), 7.35 (d, *J* = 8.0 Hz, 1H), 7.28 (s, 1H), 3.97 (td, *J* = 11.6, 4.2 Hz, 1H), 3.74 (td, *J* = 10.3, 4.5 Hz, 1H), 2.19 - 2.10 (m, 2H), 1.86 (d, *J* = 9.5 Hz, 2H), 1.66 (dt, *J* = 12.6, 7.7 Hz, 1H), 1.54 (d, *J* = 11.3 Hz, 1H), 1.50 - 1.39 (m, 2H). |
| 137 | 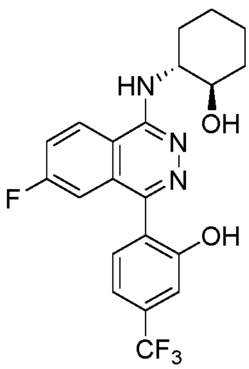 | The synthesis method refers to Embodiment 14<br>LCMS ESI(+)m/z: 422.2(M+1). $^1$H NMR (400 MHz, MeOD) δ 8.86 (dd, *J* = 9.2, 4.8 Hz, 1H), 7.92 (ddd, *J* = 9.1, 8.1, 2.6 Hz, 1H), 7.58 (d, *J* = 7.9 Hz, 1H), 7.38 - 7.33 (m, 2H), 7.29 (s, 1H), 3.94 (ddd, *J* = 11.6, 9.5, 4.2 Hz, 1H), 3.80 - 3.72 (m, 1H), 2.18 - 2.09 (m, 2H), 1.90-1.81 (m, 2H), 1.70 (qd, *J* = 12.6, 3.3 Hz, 1H), 1.59 - 1.51 (m, 1H), 1.44 (dd, *J* = 16.9, 7.0 Hz, 2H). |
| | | |

(continued)

| Embodiment | structure | Synthesis methods and characterization data |
|---|---|---|
| 138 | 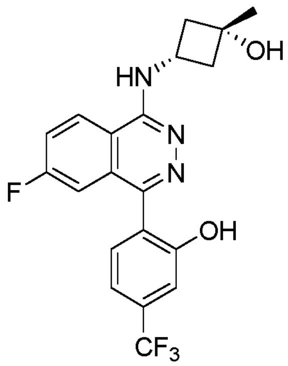 | The synthesis method refers to Embodiment 14<br>LCMS ESI(+)m/z: 408.1(M+1). $^1$H NMR (400 MHz, MeOD) δ 8.87 (dd, *J* = 9.1, 4.7 Hz, 1H), 7.96 - 7.89 (m, 1H), 7.59 (d, J= 7.8 Hz, 1H), 7.38 (dd, *J* = 9.1, 2.4 Hz, 1H), 7.36 (d, *J* = 9.1 Hz, 1H), 7.31 (s, 1H), 4.13 (p, *J* = 7.7 Hz, 1H), 2.84 - 2.73 (m, 2H), 2.46 (t, *J* = 9.8 Hz, 2H), 1.48 (s, 3H). |
| 139 | 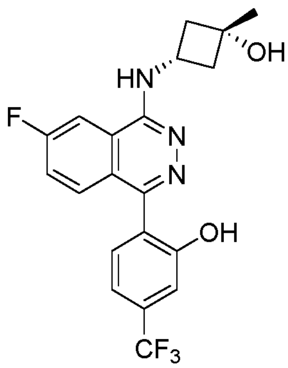 | The synthesis method refers to Embodiment 14<br>LCMS ESI(+)m/z: 408.1(M+1). $^1$H NMR (400 MHz, MeOD) δ 8.57 - 8.52 (m, 1H), 7.88 (d, *J* = 2.3 Hz, 1H), 7.86 (s, 1H), 7.59 (d, *J* = 7.9 Hz, 1H), 7.36 (d, *J* = 8.0 Hz, 1H), 7.30 (s, 1H), 4.13 (p, *J* = 8.0 Hz, 1H), 2.76 (ddd, *J* = 7.4, 6.3, 2.8 Hz, 2H), 2.42 (td, *J* = 8.8, 2.5 Hz, 2H), 1.47 (s, 3H). |
| 140 | 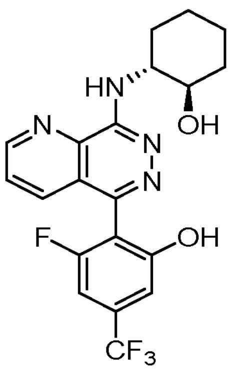 | The synthesis method refers to Embodiment 14<br>LCMS ESI(+)m/z: 423.1 (M+1). $^1$H NMR (400 MHz, MeOD) δ 9.25 (dd, J = 4.4, 1.7 Hz, 1H), 8.08 (dd, J = 8.3, 1.1 Hz, 1H), 8.03 (dd, J = 8.3, 4.4 Hz, 1H), 7.17 (d, J = 9.1 Hz, 1H), 7.14 (s, 1H), 3.96 (ddd, J = 11.6, 9.5, 4.2 Hz, 1H), 3.76 (td, J = 9.9, 4.4 Hz, 1H), 2.26 - 2.08 (m, 2H), 1.85 (dd, J = 6.9, 2.1 Hz, 2H), 1.72 (ddd, J = 24.7, 12.5, 3.2 Hz, 1H), 1.60 - 1.37 (m, 3H). |
| 141 | 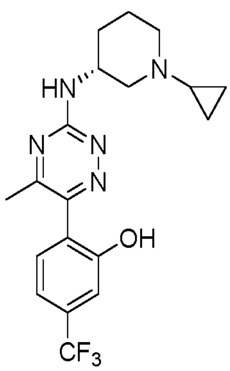 | The synthesis method refers to Example 8<br>LCMS ESI(+)m/z: 394.2 (M+1). $^1$H NMR (400 MHz, MeOD) δ 7.53 (d, *J* = 7.9 Hz, 1H), 7.30 (d, *J* = 7.9 Hz, 1H), 7.24 (s, 1H), 4.74 - 4.44 (m, 1H), 3.90 (d, *J* = 9.3 Hz, 1H), 3.69 (d, *J* = 12.1 Hz, 1H), 3.24 - 3.14 (m, 1H), 2.93 (d, *J* = 4.5 Hz, 1H), 2.52 (s, 3H), 2.26 - 1.97 (m, 4H), 1.91 - 1.70 (m, 1H), 1.16 (s, 2H), 0.99 (d, *J* = 3.8 Hz, 2H). |

(continued)

| Embodiment | structure | Synthesis methods and characterization data |
|---|---|---|
| 142 | 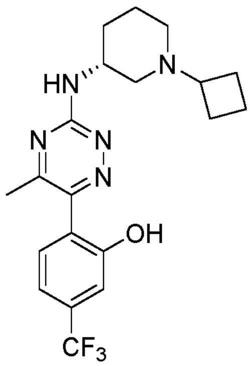 | The synthesis method refers to Example 8<br>LCMS ESI(+)m/z: 408(M+1). $^1$H NMR (400 MHz, MeOD) δ 7.49 (d, *J* = 7.8 Hz, 1H), 7.28 (d, *J* = 7.8 Hz, 1H), 7.21 (s, 1H), 4.38 (s, 1H), 3.81 - 3.65 (m, 2H), 3.51 (d, *J* = 11.5 Hz, 1H), 2.78 (t, *J* = 11.4 Hz, 1H), 2.67 (t, *J* = 11.6 Hz, 1H), 2.41 (s, 1H), 2.40 (d, *J* = 5.6 Hz, 3H), 2.36 - 2.32 (m, 2H), 2.28 (s, 1H), 2.24 (d, *J* = 10.6 Hz, 1H), 2.13 (s, 1H), 2.00 (dd, *J* = 28.4, 11.0 Hz, 1H), 1.88 (dd, *J* = 18.2, 10.5 Hz, 2H), 1.73 (dd, *J* = 12.6, 3.1 Hz, 1H). |
| 143 | 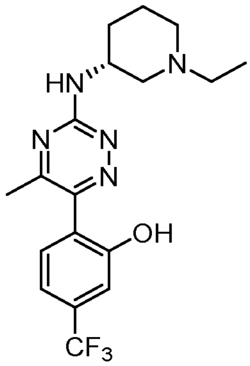 | The synthesis method refers to Example 8<br>LCMS ESI(+)m/z: 382(M+1). $^1$H NMR (400 MHz, MeOD) δ 7.55 (d, *J* = 7.8 Hz, 1H), 7.32 (d, *J* = 7.8 Hz, 1H), 7.25 (s, 1H), 4.66 (d, *J* = 11.1 Hz, 1H), 4.01 - 3.67 (m, 2H), 3.63 (d, *J* = 11.5 Hz, 1H), 2.99 (t, *J* = 10.9 Hz, 2H), 2.60 (s, 3H), 2.27 (d, *J* = 8.7 Hz, 1H), 2.16 ( d, *J* = 14.0 Hz, 1H), 2.05 (dd, *J* = 23.0, 9.3 Hz, 2H), 1.83 (s, 1H), 1.41 (t, *J* = 7.2 Hz, 3H). |
| 144 | 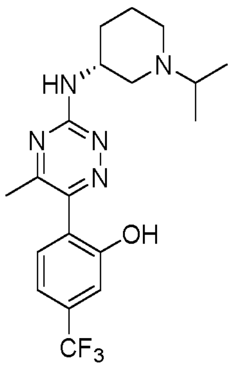 | The synthesis method refers to Example 8<br>LCMS ESI(+)m/z: 396(M+1). $^1$H NMR (400 MHz, MeOD) δ 7.53 (d, *J* = 7.9 Hz, 1H), 7.31 (d, *J* = 7.7 Hz, 1H), 7.24 (s, 1H), 4.67 (s, 1H), 3.72 (d, *J* = 9.6 Hz, 1H), 3.62 (dd, *J* = 12.8, 6.3 Hz, 1H), 3.51 (d, *J* = 12.2 Hz, 1H), 3.05 (dd, *J* = 28.0, 12.5 Hz, 2H), 2.55 (s, 3H), 2.26 (d, *J* = 10.8 Hz, 1H), 2.17 (d, *J* = 14.6 Hz, 1H), 2.04 (d, *J* = 9.1 Hz, 1H), 1.81 (s, 1H), 1.41 (t, *J* = 8.9 Hz, 6H). |
| 145 | 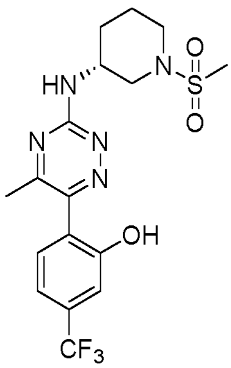 | The synthesis method refers to Example 8<br>LCMS ESI(+)m/z: 432.1 (M+1). $^1$H NMR (400 MHz, MeOD) δ 7.51 (d, *J* = 7.9 Hz, 1H), 7.30 (dd, *J* = 7.9, 0.9 Hz, 1H), 7.23 (s, 1H), 4.24 (s, 1H), 3.76 (d, *J* = 10.3 Hz, 1H), 3.48 (s, 1H), 3.05 (d, *J* = 6.8 Hz, 2H), 2.89 (s, 3H), 2.52 (s, 3H), 2.14 - 1.93 (m, 2H), 1.83 - 1.69 (m, 2H). |

(continued)

| Embodiment | structure | Synthesis methods and characterization data |
|---|---|---|
| 146 | 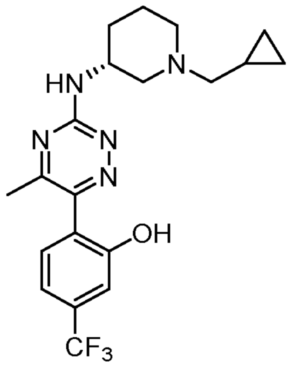 | The synthesis method refers to Example 8<br>LCMS ESI(+)m/z: 408.2 (M+1). $^1$H NMR (400 MHz, MeOD) δ 7.53 (d, *J* = 7.9 Hz, 1H), 7.31 (d, *J* = 7.9 Hz, 1H), 7.24 (s, 1H), 4.63 (s, 1H), 3.89 (dd, *J* = 30.0, 11.6 Hz, 1H), 3.69 (dd, *J* = 26.1, 12.6 Hz, 1H), 3.22 - 3.11 (m, 2H), 3.08 - 2.93 (m, 2H), 2.54 (s, 3H), 2.31 - 2.00 (m, 3H), 1.94 - 1.63 (m, 1H), 1.27 - 1.12 (m, 1H), 0.93 - 0.67 (m, 2H), 0.49 (q, *J* = 4.9 Hz, 2H). |
| 147 | 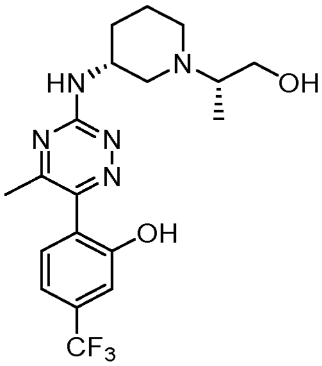 | The synthesis method refers to Example 8<br>LCMS ESI(+)m/z: 412.1 (M+1). $^1$H NMR (400 MHz, MeOD) δ 7.54 (d, *J* = 7.9 Hz, 1H), 7.37 - 7.28 (m, 1H), 7.25 (s, 1H), 4.63 (s, 1H), 4.37 - 4.18 (m, 1H), 3.92 (d, *J* = 9.0 Hz, 1H), 3.72 (d, *J* = 11.5 Hz, 1H), 3.58 - 3.39 (m, 1H), 3.24 (dd, *J* = 13.1, 2.7 Hz, 1H), 3.05 (ddd, *J* = 24.4, 12.8, 6.9 Hz, 2H), 2.56 (d, *J* = 5.0 Hz, 3H), 2.25 (d, *J* = 10.8 Hz, 1H), 2.16 (d, *J* = 14.9 Hz, 1H), 2.07 - 1.89 (m, 1H), 1.81 (s, 1H), 1.25 (t, *J* = 7.3 Hz, 3H). |
| 148 | 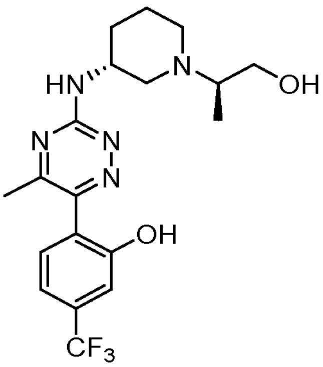 | The synthesis method refers to Example 8<br>LCMS ESI(+)m/z: 412.1 (M+1). $^1$H NMR (400 MHz, MeOD) δ 7.55 (d, *J* = 7.9 Hz, 1H), 7.32 (d, *J* = 7.9 Hz, 1H), 7.25 (s, 1H), 4.68 (s, 1H), 4.25 (s, 1H), 3.92 (d, *J* = 9.2 Hz, 1H), 3.69 (dt, *J* = 40.5, 17.3 Hz, 1H), 3.25 (d, *J* = 12.7 Hz, 1H), 3.18 - 2.96 (m, 3H), 2.59 (s, 3H), 2.25 (d, *J* = 10.1 Hz, 1H), 2.08 (d, *J* = 19.1 Hz, 2H), 1.83 (s, 1H), 1.25 (t, *J* = 6.5 Hz, 3H). |
| 149 | 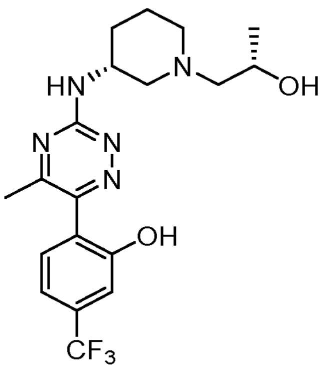 | The synthesis method refers to Example 8<br>LCMS ESI(+)m/z: 368 ( M+1). $^1$H NMR (400 MHz, MeOD) δ 7.55 (d, *J* = 7.9 Hz, 1H), 7.31 (d, *J* = 8.0 Hz, 1H), 7.26 (s, 1H), 4.65 (s, 1H), 4.31 - 4.24 (m, 1H), 3.92 (d, *J* = 9.9 Hz, 1H), 3.72 (d, *J* = 12.1 Hz, 1H), 3.26 (dd, J = 13.0, 2.6 Hz, 1H), 3.16 - 3.01 (m, 3H), 2.56 (s, 3H), 2.26 (d, *J* = 11.4 Hz, 1H), 2.16 (d, *J* = 15.0 Hz, 1H), 2.02 (d, *J* = 14.6 Hz, 1H), 1.83 (s, 1H), 1.24 (d, *J* = 6.2 Hz, 3H). |

(continued)

| Embodiment | structure | Synthesis methods and characterization data |
|---|---|---|
| 150 | 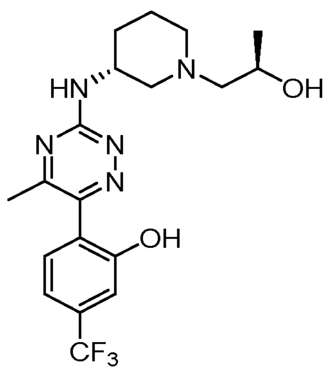 | The synthesis method refers to Example 8<br>LCMS ESI(+)m/z: 412.2 (M+1). $^1$H NMR (400 MHz, MeOD) δ 7.54 (d, *J* = 7.9 Hz, 1H), 7.31 (d, *J* = 7.9 Hz, 1H), 7.25 (s, 1H), 4.63 (s, 1H), 4.24 (d, *J* = 6.3 Hz, 1H), 3.93 (d, *J* = 10.2 Hz, 1H), 3.77 - 3.63 (m, 1H), 3.27 - 3.20 (m, 1H), 3.20 - 2.92 (m, 3H), 2.55 (s, 3H), 2.28 - 2.04 (m, 3H), 1.81 (d, *J* = 9.9 Hz, 1H), 1.24 (d, *J* = 6.2 Hz, 3H). |
| 151 | 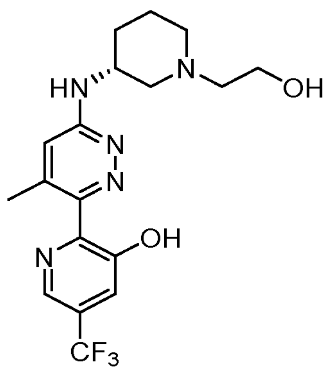 | The synthesis method refers to embodiment 12<br>LCMS ESI(+)m/z: 398.2 (M+1). $^1$H NMR (400 MHz, MeOD) *δ* 8.58 (d, *J* = 0.8 Hz, 1H), 7.70 (d, *J* = 1.4 Hz, 1H), 7.57 (s, 1H), 4.46 - 4.34 (m, 1H), 3.94 (dd, *J* = 9.5, 4.5 Hz, 3H), 3.68 (d, *J* = 12.2 Hz, 1H), 3.40 - 3.33 (m, 2H), 3.13 - 3.04 (m, 1H), 2.98 (t, *J* = 11.7 Hz, 1H), 2.38 (s, 3H), 2.28 (d, *J* = 9.2 Hz, 1H), 2.12 (t, *J* = 13.5 Hz, 1H), 2.07 - 2.01 (m, 1H), 1.72 (qd, *J* = 12.6, 4.3 Hz, 1H). |
| 152 | 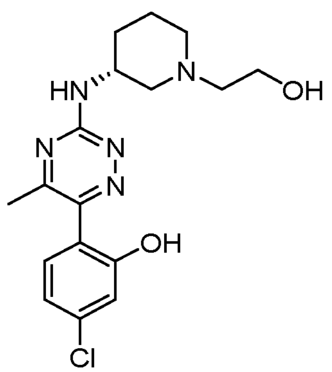 | The synthesis method refers to Example 8<br>LCMS ESI(+)m/z: 364.2 (M+1). $^1$H NMR (400 MHz, MeOD) δ 7.33 (d, *J* = 8.1 Hz, 1H), 7.13 - 6.95 (m, 2H), 4.64 (s, 1H), 4.04 - 3.86 (m, 3H), 3.69 (d, *J* = 12.4 Hz, 1H), 3.41 - 3.33 (m, 2H), 3.19 - 2.96 (m, 2H), 2.58 (s, 3H), 2.28 - 2.02 (m, 3H), 1.81 (d, *J* = 10.4 Hz, 1H). |
| 153 | 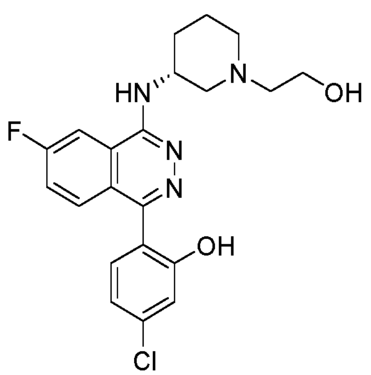 | The synthesis method refers to Embodiment 14<br>LCMS ESI(+)m/z: 417(M+1). $^1$H NMR (400 MHz, MeOD) δ 8.51 (dd, *J* = 9.4, 2.3 Hz, 1H), 8.11 (dd, *J* =9.0, 5.3 Hz, 1H), 7.93 (td, *J* = 9.0, 2.3 Hz, 1H), 7.49 (d, *J* = 8.1 Hz, 1H), 7.17 (dt, *J* = 5.3, 1.8 Hz, 2H), 4.77 - 4.68 (m, 1H), 4.07 - 4.00 (m, 1H), 3.95 (dd, *J* = 12.9, 7.9 Hz, 2H), 3.71 (d, *J* = 12.4 Hz, 1H), 3.43 - 3.34 (m, 2H), 3.12 (dt, *J* = 19.2, 7.1 Hz, 2H), 2.33 (d, *J* = 9.6 Hz, 1H), 2.18 (d, *J* = 14.6 Hz, 1H), 2.13 - 2.01 (m, 1H), 2.00 - 1.88 (m, 1H). |

(continued)

| Embodiment | structure | Synthesis methods and characterization data |
|---|---|---|
| 154 | 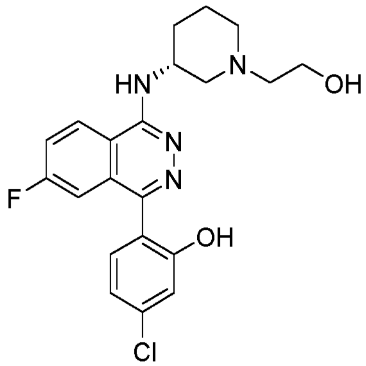 | The synthesis method refers to Embodiment 14<br>LCMS ESI(+)m/z: 417(M+1). $^1$H NMR (400 MHz, MeOD) δ 8.85 (dd, *J* = 9.0, 4.6 Hz, 1H), 8.06 (t, *J* = 7.5 Hz, 1H), 7.60 (d, *J* = 6.8 Hz, 1H), 7.48 (d, *J* = 7.9 Hz, 1H), 7.16 (d, *J* = 7.4 Hz, 2H), 4.75 (d, *J* = 11.8 Hz, 1H), 4.02 (d, *J* = 11.3 Hz, 1H), 3.96 (t, *J* = 4.5 Hz, 2H), 3.72 (d, *J* = 12.1 Hz, 1H), 3.38 (s, 2H), 3.16 (dd, *J* = 16.3, 6.5 Hz, 2H), 2.34 (d, *J* = 10.2 Hz, 1H), 2.18 (d, *J* = 14.4 Hz, 1H), 2.12 - 1.88 (m, 2H). |
| 155 | 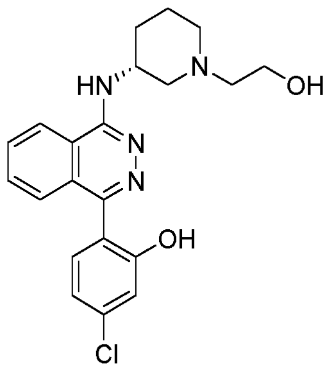 | The synthesis method refers to Embodiment 14<br>LCMS ESI(+)m/z: 399.2 (M+1). $^1$H NMR (400 MHz, MeOD) δ 8.68 (d, *J* = 8.2 Hz, 1H), 8.28 (t, *J* = 7.7 Hz, 1H), 8.13 (t, *J* = 7.4 Hz, 1H), 8.00 (d, *J* = 7.9 Hz, 1H), 7.48 (d, *J* = 8.1 Hz, 1H), 7.20 - 7.11 (m, 2H), 4.78 - 4.69 (m, 1H), 4.09 - 3.90 (m, 3H), 3.67 (dd, *J* = 37.9, 10.7 Hz, 1H), 3.45 - 3.34 (m, 2H), 3.19 - 2.98 (m, 2H), 2.34 (d, *J* = 10.8 Hz, 1H), 2.21 - 1.89 (m, 3H). |

**Effect example 1: Measuring IL-1β (interleukin-1β) secretion on the products of Embodiments 1 to 155.**

**1. Reagent preparation**

1.1 Medium preparation

Cell culture medium

| reagent | concentration |
|---|---|
| RPMI 1640 medium | 89% |
| FBS | 10% |
| Pen/Strep | 1% |
| 2-mercaptoethanol | 0.05mM |

Cryopreservation medium

| reagent | concentration |
|---|---|
| DMSO | 10% |
| FBS | 90% |

1.2 Cell preparation

1.2.1 Cells thawing

Remove THP-1 cells from liquid nitrogen and place in a 37 °C water bath until ice melt. Add the cells to 5 mL of warm cell culture medium and centrifuge at 1,000 rpm for 5 min. Discard the supernatant and resuspend and culture in a new cell culture medium.

1.2.2 Cell culturing and passaging

THP-1 cells are cultured in the culture medium and passaged every 2-3 days. Cell is passaged every two days at concentration 5×105 cells/mL, is passaged every three days at concentration 3×105 cells/mL, and the cell density is maintained between 5 × 105-1.5 × 106 viable cells/mL (it is best to use cells that have been passaged less than 30 times to maintain high transfection efficiency).

1.2.3 Cell cryopreservation

Resuspend the cells with fresh cell cryopreservation medium, adjust the cell density to 3×106~6×106/mL, add 1mL of adjusted cell density medium to each cryovial, put it in a freezer containing methanol to freeze the cells at -80 °C, and transfer to liquid nitrogen one day later.

1.3 Preparation of IFN-y solution

Dissolve IFN-y in 1 mL of water to yield 100 μg/mL mother liquor, store at -20°C. The mother liquor was further diluted to 25 ng/mL with cell culture medium.

1.4 LPS solution preparation

Dissolve LPS in 1 mL of PBS to obtain a 1 mg/mL stock solution, split into aliquots and store at 4°C. The stock solution was further diluted to 50 ng/mL with serum-free medium.

**2 Compound activity assay**

2.1 THP-1 cells are differentiated under the action of IFN-y

2.1.1 Cells are diluted to 100K cells/well with cell culture medium and IFN-y (final concentration 25 ng/mL) is added to the cell suspension.

2.1.1 Add 100 μL of cell suspension per well to the cell plate and incubate the cell plate in a 37°C, 5%$CO_2$ incubator for 24 hours.

2.2 LPS induction

2.2.1 Discard the supernatant of IFN-γ differentiation after differentiation treatment. Add 100 μL of serum-free medium containing LPS (final concentration of 50 ng/mL) to the cell plate. The cell plates were incubated in a 37 °C, 5% $CO_2$ incubator for 4 h.

2.2.2 According to the plate map, use Tecan to add 1% of the products of embodiments 1 to 155 into the cell plate as the sample treatment group and DMSO as the blank control group. Place the cell plate in an incubator at 37°C and 5% CO2 for 1 hour.

2.2.3 Add 20 μL of 6× ATP per well according to the plate map (final concentration 5 mM) and incubate the cell plate in a 37°C, 5% $CO_2$ incubator for 1h. Collect the supernatant (60 μL) and store at -20 °C.

2.3 Cell plate coating

The capture antibody mAb Mt175 is diluted to 2 μg/mL with PBS (1:250) and added to the cell plate at 15 μL per well. Keep the plate overnight at 4°C.

2.4 IL-1β detection

2.4.1 Discard the coated antibody and wash 4 times with PBST.

2.4.2 Add 25 μL of blocking buffer (Licor-927-40010) containing 0.1% Tween 20 per well to the cell plate and incubate for 1 h at room temperature.

2.4.3 Discard the blocking buffer and wash 4 times with PBST.

2.4.4 Add 25 μL of 2.2.3 collected supernatant sample per well and incubate for 2 h at room temperature.

2.4.5 Discard the samples and wash 4 times with PBST.

2.4.6 Add mAb7P10-biotin (concentration 0.5 ug/mL) to blocking buffer, add 15 μL per well to the cell plate, and incubate for 1 hour at room temperature.

2.4.7 Discard the antibody and wash 4 times with PBST.

2.4.8 Streptavidin-HRP was diluted 1:1000 in blocking buffer, 15 μL per well was added to the cell plate, and incubated for 1 hour at room temperature.

2.4.9 Discard streptavidin-HRP and wash 4 times with PBST.

2.4.10 Add 25 μL of HRP substrate (Surmodics-TMBW-0100-01) per well and incubate the cell plate until a blue product appears.

2.4.11 Add 25 μL of stop solution (Surmodics-LSTP-0100-01) per well, and the blue product turns yellow.

2.4.12 Read the cell plate at 450nm with a microplate reader.

## 3 Data Processing

The half inhibition rate IC50 value is obtained from the readings of the sample treatment group and the blank control group through a microplate reader, as shown in Table 1.

Among them, in addition to measuring the products of embodiment 1-155, the IC50 values of control 1,

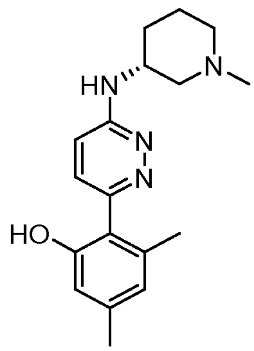

control 2

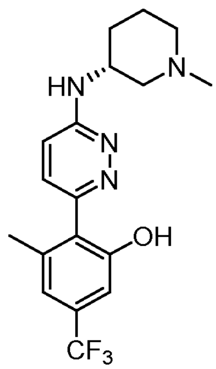

,

control 3

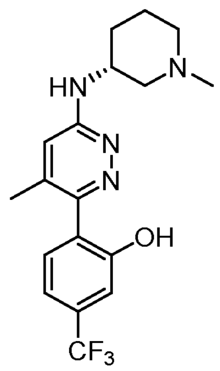

and control 4

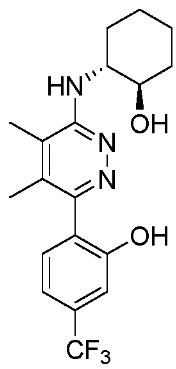

were also measured.

Table 1 Half inhibition rate of NLRP3 of the product and positive control 1-4 (IC50)

| Embodiment | IC50 (μM) | Embodiment | IC50 (μM) |
|---|---|---|---|
| 1 | 0.0109 | 2 | 0.0402 |
| 3 | 0.0535 | 4 | 0.0321 |
| 6 | 0.0074 | 7 | 0.0012 |
| 8 | 0.0015 | 9 | 0.067 |
| 10 | 0.0155 | 11 | 0.004 |
| 12 | 0.0311 | 13 | 0.0024 |
| 14 | 0.001 | 15 | 0.028 |
| 16 | 0.0043 | 17 | 0.0155 |
| 18 | 0.0057 | 19 | 0.0852 |
| 20 | 0.0213 | 21 | 0.0494 |
| 22 | 0.024 | 23 | 0.376 |
| 24 | 0.193 | 25 | 0.317 |
| 26 | 0.446 | 27 | 0.0014 |
| 28 | 0.0011 | 29 | 0.0051 |
| 30 | 0.0029 | 31 | 0.0022 |
| 33 | 0.0058 | 35 | 0.0124 |
| 36 | 0.0542 | 37 | 0.0273 |

(continued)

| Embodiment | IC50 (μM) | Embodiment | IC50 (μM) |
|---|---|---|---|
| 40 | 0.0124 | 42 | 0.0627 |
| 43 | 0.0065 | 44 | 0.0004 |
| 47 | 0.0046 | 48 | 0.0091 |
| 54<br>65 | 0.0464<br>0.0104 | 64<br>70 | 0.0047<br>0.0053 |
| 71 | 0.0928 | 72 | 0.343 |
| 76 | 0.0113 | 77 | 0.0194 |
| 78 | 0.0028 | 81 | 0.0348 |
| 82 | 0.0474 | 107 | 0.0292 |
| 108 | 0.0109 | 109 | 0.0406 |
| 110 | 0.003 | 111 | 0.0197 |
| 112 | 0.002 | 113 | 0.0161 |
| 114 | 0.031 | 116 | 0.0106 |
| 117 | 0.0047 | 118 | 0.0029 |
| 119 | 0.005 | 120 | 0.0041 |
| 121 | 0.0012 | 122 | 0.0029 |
| 123 | 0.00547 | 124 | 0.0206 |
| 125 | 0.0099 | 126 | 0.0039 |
| 127 | 0.0035 | 128 | 0.003 |
| 129 | 0.0038 | 130 | 0.0019 |
| 131 | 0.0073 | 132 | 0.0041 |
| 133 | 0.0066 | 134 | 0.014 |
| 135 | 0.0051 | 136 | 0.0044 |
| 137 | 0.002 | 138 | 0.0047 |
| 139 | 0.0019 | 140 | 0.0095 |
| 141 | 0.0015 | 142 | 0.0113 |
| 143 | 0.0009 | 144 | 0.0018 |
| 146 | 0.0074 | 147 | 0.009 |
| 148 | 0.0144 | 149 | 0.0248 |
| 150 | 0.0099 | 152 | 0.0104 |
| 153 | 0.0024 | 154 | 0.0032 |
| 155 | 0.0025 | | |
| Reference 1 | 0.00916 | Control 2 | 0.0014 |
| Comparison 3 | 0.0014 | Comparison 4 | 0.0224 |

The results show that the compound of the present invention can significantly inhibit pyroptosis of human cells THP-1.

**Effect example 2: Effect of the compounds on the hERG pathway current**

The fast-activating potassium channel encoded by the human ether-a-go-go-related gene (hERG) is an important ion channel involved in the formation of phase 3 repolarization of myocardial action potential. Drug blockade of the hERG channel can lead to prolonged cardiac repolarization, which is called long QT syndrome on ECG. Drug-induced delayed ventricular repolarization may cause a fatal arrhythmia-torsade de pointes in some cases.

In this study, a whole-cell patch-clamp technique was used to study the inhibitory effect of compounds on hERG potassium channels and to evaluate the risk of ventricular repolarization toxicity.

**cell culture**

The HEK293 cell line stably expressing the hERG potassium channel cells was purchased from Creacell, Inc. (Cat. No. A-0320).

The HEK293 cell line that can stably express the hERG potassium channel, was cultured in DMEM medium containing 10% fetal bovine serum and 0.8 mg/mL G418 at 37°C and 5% carbon dioxide.

Cell passaging: Remove the old medium and wash once with PBS, then add 2 mL of TrypLE™ Express solution and incubate at 37°C for about 1 min. When the cells are detached from the bottom of the dish, add approximately 5 mL of complete medium pre-warmed at 37°C. Gently pipette the cell suspension with a pipette to detach the clustered cells. Transfer the cell suspension to a sterile centrifuge tube and centrifuge at 1000 rpm for 5 minutes to collect the cells. To expand or maintain the culture, cells are seeded in 10 cm cell culture dishes with a cell volume of $6\times10^5$ cells per dish (final volume: 10 mL).

To maintain the electrophysiological activity of cells, the cell density must not exceed 80%.

Before the test, the cells were separated with TrypLE™ Express, centrifuged after adding medium to terminate digestion, resuspended the cell count, adjusted the cell density to $2\text{-}3\times10^6$ cells/mL, and then lightly mixed the cells on a room temperature equilibration shaker for 15-20 min.

**Patch-clamp testing**

Electrophysiological assays were performed using a fully automated patch-clamp QPatch 48 X (Sophion) device. The prepared cells were placed on a centrifuge on the Qpatch bench, the cells were washed using multiple centrifugation/suspension methods, and the cell culture medium was replaced with extracellular fluid. Remove an MTP-96 plate and place it in the location of the MTP source. Remove the QPlate chip and subsequently place the QPlate at the Qplate source location. The robotic arm scans the MTP-96 board and the QPlate chip barcode and grabs it to the measuring station. Draw the intracellular and external fluid from the liquid pool and add them to the intracellular fluid pool, cell and test cell pool of the QPlate chip, respectively. At the measuring station, all measuring sites on the QPlate undergo initial quality control. The quality control process includes aspirating the cell suspension from the cell vessel of the centrifuge and positioning the cells to the chip wells via a pressure controller to establish a high-resistance seal and create a whole-cell recording pattern. Once a stable baseline of control currents has been obtained, the test substance can be aspirated from the TEST MTP-96 plate and applied to the cells in a concentration gradient.

The voltage stimulation protocol for whole-cell patch-clamp recording of whole-cell hERG potassium currents is as follows: the cell membrane voltage clamps at -80 mV when a whole-cell closure is formed. The clamping voltage is depolarized from -80 mV to -50 mV for 0.5 s (as a leakage current detection), then steps to 30 mV for 2.5 s, and then quickly recovers to -50 mV for 4 s to excite the tail current of the hERG channel. The data were collected repeatedly every 10 s to observe the effect of the drug on the hERG tail current. -50 mV stimulation at 0.5 s was used as the leakage current detection. The test data is collected by Qpatch and stored in the connected service station.

**data analysis**

First, the current after the effect of each drug concentration and the blank control current are normalized (), and then the inhibition rate corresponding to each drug concentration is calculated. $\frac{Peak\ tail\ current\ compound}{Peak\ tail\ current\ vehicle}(1-\frac{Peak\ tail\ current\ compound}{Peak\ tail\ current\ vehicle})$ Calculate the mean and standard error for each concentration and calculate the semi-inhibitory concentration for each compound using the following Formula:

Y=Bottom + (Top-Bottom)/(1+10^((LogIC50-X)*HillSlope)) The above Formula was used to fit the dose-dependent effect nonlinearly, where C represents the test substance concentration, IC50 is the semi-inhibitory concentration, and h represents the Hill coefficient. Curve fitting and IC50 calculations were performed using Graphpad software.

The test results of some compounds are shown in Table 2

**Table 2 Semi-inhibitory concentrations (IC50) of some embodiment compounds and control substances 1-4 on hERG current**

| Embodiment | IC50 (μM) | Embodiment | IC50 (μM) |
|---|---|---|---|
| 2 | >30 | 6 | >30 |
| 33 | >30 | 35 | >30 |
| 37 | >30 | 39 | >30 |
| 40 | >30 | 43 | >30 |
| 44 | >30 | 47 | >30 |
| 48 | >30 | 64 | >30 |
| 70 | >30 | 78 | >30 |
| 126 | >30 | 127 | >30 |
| 130 | >30 | 131 | >30 |
| 132 | >30 | 133 | >30 |
| 135 | >30 | 136 | >30 |
| 137 | >30 | 139 | >30 |
| 147 | >30 | 148 | >30 |
| 150 | >30 | 152 | >30 |
| 153 | >30 | 155 | >30 |
| Reference 1 | <10 | Control 2 | <10 |
| Comparison 3 | <10 | Comparison 4 | 23 |

The results show that the IC50 result of the compound of the present invention acting on the hERG currentis >30μM, and the compound of the present invention has no inhibitory effect on the hERG channel according to the general standard judgment of hERG. The experimental results of the control compounds showed that there was a certain weak inhibitory effect, which had certain cardiac safety risks.

**Effect example 3: in vivo pharmacokinetic evaluation of some compounds in mice**

CD-1 mice, after fasting overnight (free drinking), are divided into tail vein (IV) and gavage administration (PO) groups. The tail vein administration group collected 0 .1mL of blood from the orbital venous plexus before administration and 5 minutes, 15 minutes, 0.5 hours, 1 hour, 2 hours, 4 hours, 8 hours, 12 hours, and 24 hours after administration, and sample centrifugation at 4°C for 5 minutes, and stored at - 70 °C for testing. In the gavage administration group, 0.1mL of blood was collected from the orbital venous plexus before administration and at 15 minutes 0.5 h, 1 h, 2 h, 4 h, 8 h, 12 h and 24 h after administration, and the treatment method was the same as that of the intravenous administration group. The plasma parent drug concentration was determined by LC-MS/MS. The results are shown in Table 3.

**Table 3 Pharmacokinetic test results of some compounds in vivo**

| Embodiment | route of administration | Dosage (mg/kg) | Cmax (ng/mL) | AUC (ng.h/mL) | t1/2 (h) | Cl (mL/h/kg) | Vss (L/kg) | F% |
|---|---|---|---|---|---|---|---|---|
| 33 | IV | 3 | - | 4595 | 4.89 | 0.653 | 2.60 | - |
| | PO | 20 | 8517 | 30015 | 3.60 | - | - | 98.0 |
| 44 | IV | 3 | - | 2947 | 4.02 | 1.01 | 4.35 | - |
| | PO | 20 | 4530 | 18777 | 3.62 | - | - | 95.3 |

(continued)

| Embodiment | route of administration | Dosage (mg/kg) | Cmax (ng/mL) | AUC (ng.h/mL) | t1/2 (h) | Cl (mL/h/kg) | Vss (L/kg) | F% |
|---|---|---|---|---|---|---|---|---|
| 132 | IV | 3 | - | 3159 | 2.81 | 0.948 | 3.56 | - |
| | PO | 20 | 3067 | 18644 | 2.53 | - | - | 88.5 |
| 136 | IV | 3 | - | 3249 | 6.07 | 0.906 | 2.35 | - |
| | PO | 20 | 6516 | 20312 | 5.67 | - | - | 93.8 |
| 147 | IV | 3 | - | 3286 | 3.28 | 0.913 | 3.39 | - |
| | PO | 20 | 5710 | 17232 | 2.76 | - | - | 78.7 |
| 148 | IV | 3 | - | 4748 | 4.17 | 0.632 | 3.00 | - |
| | PO | 20 | 7727 | 30128 | 3.68 | - | - | 95.2 |
| Reference 1 | IV | 3 | - | 10174 | 4.00 | 0.29 | 1.62 | - |
| | PO | 20 | 3957 | 36893 | 4.23 | - | - | 54.6 |
| Comparison 4 | IV | 3 | - | 1590 | 4.76 | 2.43 | 5.89 | - |
| | PO | 20 | 1709 | 5208 | 2.95 | - | - | 49.1 |

The results show that the pharmacokinetic parameters of the compound of the present invention in animals are significantly better than those of the reference substance.

**Effect Example 4: Inhibitory effect of Embodiment compound 33 on plasma IL-1β and IL-18 cytokines in LPS-induced sepsis mouse model**

The purpose of this study was to investigate and evaluate the inhibitory effect of the test compound on IL-1β and IL-18 cytokine production in a mouse model of lipopolysaccharide (LPS)-induced sepsis.

**Animal Study:**

6-8 weeks female C57BL/6J mice, randomized, 5 animals in the Naïve group, and the rest of the groups (vehicle group, 15mg/kg group with embodiment 33 compound, 50mg/kg group with embodiment 33 compound and 150mg/kg group with embodiment 33 compound, wherein 15mg/kg, 50mg/kg and 150mg/ kg is the dosage of embodiment 33, mg/kg is referred to as mpk) 10 animals per group. Among them, the Naive group was not stimulated with LPS, and the 10 mice in each group were intraperitoneally injected with LPS (10 mg/kg) to induce sepsis model in the mouse. The vehicle (corresponding to the vehicle group) and the test compound (corresponding to the 15mpk group of Example 33, the 50mpk group of Example 33 and the 150mpk group of Example 33 respectively) were administered 30 minutes before LPS stimulation. 2 hours after LPS stimulation, the mice were euthanized, and whole blood was collected from the heart and placed in EP tubes, left to stand at room temperature for 1 hour, and centrifuged at 8000 rpm for 10 minutes to collect serum for measurement of cytokines IL-1β and IL-18.

**IL-1β cytokine assay:**

For the standard pellet in the IL-1β cytokine CBA kit, put the IL-1β standard pellet into a 15 mL centrifuge tube, add 4 mL of Assay Diluent, and let it stand at room temperature for more than 15 minutes;

After the supernatant sample is melted and mixed evenly, the supernatant is diluted 2 times;

Preparation of standard reagent: Add the diluent Assay Diluent in advance to the 96-well V-shaped bottom plate, 100 μL/well. Add 200 μL of the standard that has been left to stand for 15 minutes into the well plate, and add 100 μL each time to the diluent in sequence. Gradually dilute the standard to 11 gradients (double dilution each time). The 12th hole is used as Blank, 200 μL/well;

Preparation of Capture beads reagent: Take the capture beads in the IL-1β cytokine CBA kit and vortex for 30 seconds. Prepare the Beads as follows: 60 μL IL-1β beads + 2440 μL capture beads diluent (60 wells); mix evenly, 50 μL/ well, add it to a 96-well V-bottom plate; shake at 200 g for 5 minutes, and incubate at room temperature for

1 hour;
Preparation of Detection reagent: Take the PE Detection from the IL-1β cytokine CBA kit and vortex for 30 seconds. Prepare the PE as follows: 60 μL IL-1β Detection + 2440 μL PE detection diluent; mix evenly. Add 50 μL/well of the standard and sample to be tested; shake at 200 g for 1 minute, and incubate at room temperature for 1 hour;
Washing: Add 200 μL of wash buffer/well, 400g, and centrifuge for 5 minutes at 4°C;
Discard the supernatant, add wash buffer 150 μL/well, and wait for detection on the machine;
Fortessa testing.

**IL-18 cytokine assay:**

After the kit is taken out of the refrigerator, it should be equilibrated at room temperature (25-28°C) for 20 minutes; the remaining reagents should be stored at 4°C immediately after each test.
Prepare an appropriate amount of standard diluent: dilute the standard diluent (5X) to 1X with double-distilled or deionized water. For example, add 10 mL of standard diluent (5X) to 40 mL of water and mix well to obtain a 1X standard. Diluent.
Prepare an appropriate amount of washing liquid: Dilute the washing liquid (20X) with double-distilled or deionized water to 1X. For example, add 10 mL of washing liquid (20X) to 190 mL of water and mix well to obtain a 1X washing liquid.
Standard preparation: Add the standard diluent to one bottle of standard according to the volume (0.5 mL) marked on the standard label, incubate at room temperature for 15 minutes, then mix gently and pipet several times with a pipette to completely dissolve the standard. The final concentration of the standard reaches 1500pg/mL. Take 5 clean 1.5 mL centrifuge tubes, add 250 μL of standard diluent to each tube in advance, and perform dilutions of the standard to obtain a total of 1500, 750, 375, 187.5, 93.75, 46.875, and 23.4375pg/mL. standard concentration, and finally add the diluted standard to the pre-coated plate wells in sequence, and add the standard diluent directly as a concentration of 0pg/mL, for a total of 8 standard concentrations.

**Procedure:**

Calculate and determine the number of pre-coated strips required for an experiment, take out the required strips and place them in a 96-well frame.
Add samples or standards of different concentrations into the corresponding wells at 100 μL/well, seal the reaction wells with sealing film (transparent), and incubate at room temperature for 120 minutes.
Wash the plate 3 times and pat dry on thick absorbent paper for the last time.
Add 100 μL/well of horseradish peroxidase-labeled antibody, seal the reaction well with sealing film (transparent), and incubate at room temperature for 60 minutes.
Wash the plate 3 times and pat dry on thick absorbent paper for the last time.
Add 100 μL/well of chromogen TMB solution, seal the reaction well with sealing film (white), and incubate at room temperature in the dark for 15-20 minutes.
Add 50 μL of stop solution/well, mix well and measure the A450 value immediately.
All data were first tested for homogeneity of variances. After passing the homogeneity of variances test, one-way analysis of variance (ANOVA) was used for intra-group significance analysis, and the Dunnet method was used to compare differences between groups. If the variances are not equal, the rank sum test in the non-parametric test is used for analysis. Among them, *P<0.05, **P<0.01, ***P<0.001.
The experimental results are shown in Table 4: Compared with Naive, the levels of IL-1β and IL-18 in the Vehicle group increased significantly; compared with the Vehicle group, the three dose groups of Embodiment 33 had a significant inhibitory effect on IL-1β. Embodiment 33 has a significant inhibitory effect on IL-18 at two doses of 50 mg/kg and 150 mg/kg.
In summary, Embodiment 33 can dose-dependently inhibit LPS-induced levels of IL-1β and IL-18 in plasma.

**Table 4 Experimental test results of PD in vivo in Embodiment 33**

| | Naïve | Vehicle | Embodiment 33 15mpk | Embodiment 33 50 mpk | Embodiment 33 150 mpk |
|---|---|---|---|---|---|
| **Number of animals** | 5 | 10 | 10 | 10 | 10 |
| **IL-1β** | 0.2684±1.278 | 769.7±128.4 | 310.1±32.88 | 213.4±25.66 | 19.36±5.166 |
| **IL-18** | 142.5±14.1 | 685.3±65.88 | 560.5±46.39 | 320.1±29.69 | 216.2±9.238 |

**Effect embodiment 5: Embodiment 33 effect on DSS-induced acute enteritis mouse model**

**Grouping and modeling:** One day before the start of the experiment, all C57BL/6 mice were earlabeled and recorded, and the animals with appropriate weight and normal status were selected for grouping (Sham group, vehicle group and embodiment 33 group). 10 animals per group; 3.5% dextran sulfate sodium salt (DSS, MP, molecular weight 36000-50000) prepared with sterile water is loaded into the drinking bottle in the vehicle and the 33 testing groups of rearing cages in the embodiment (100 mL per cage, replace the 3.5% DSS solution after 2-3 days with freshly prepared solution), and the animals in the vehicle and the 33 testing groups of the embodiment are free to drink for 10 consecutive days. The Sham group drank the normal water not containing DSS. Embodiment 33 compound is administered orally BID.

**Evaluation Indicators:**

1) Body Weight Change (%) = BWi /BW0 × 100%, BWi is the average body weight of mice in the group on a given day, BW0 is the average body weight of mice before the start of treatment. The results of the assessment are shown in Table 5.

**Table 5 The average rate of change in body weight of each treatment group at day** 11 (n=10).

| Group | Day 11 weight change (%) | Rate of body weight loss (%) | P Value |
|---|---|---|---|
| Sham | 102.27% | / | / |
| Vehicle | 78.73% | 21.27% | |
| Embodiment 33 50mpk P.O Bid | 88.09% | 11.91%** | 0.004 |

**[0969]** Note: Compared with the G2 Vehicle group, the one-way ANOVA method test for each treatment group was: * P<0.05, ** P<0.01, ***P<0.001, ****P<0.0001

2) DAI score: The Disease Activity Index (DAI) scoring criteria are as follows:

| Score | % of Body weight loss | Stool characteristics | Blood in the stool |
|---|---|---|---|
| **0** | 0 | Normal | Negative |
| **1** | 1-5% | soft stool | \ |
| **2** | 6-10% | Semi-loose stool | Occult blood |
| **3** | 11-15% | Loose stool with residue | \ |
| **4** | >15% | Watery stool | Visible bloody stool |

**[0507]** The results of the assessment are shown in Table 6:

**Table 6 DAI score data during treatment (n=10).**

| Group | Day 3 | Day 5 | Day 7 | Day 9 | Day 11 |
|---|---|---|---|---|---|
| **Sham** | 0.20±0.13 | 0.20±0.13 | 0.00 | 0.00 | 0.00 |
| **Vehicle** | 0.30±0.15 | 3.60±0.37 | 6.50±0.31 | 9.60±0.62 | 11.00±0.37 |
| **Embodiment 33 50mpk P.O Bid** | 0.00 | 2.60±0.45 | 4.40±0.6* | 6.20±0.68** | 7.80±0.63** |

[0973] Note: DAI scores compared with the G2 Vehicle group, the one-way ANOVA method test: * P<0.05, ** P<0.01, ***P<0.001, ****P<0.0001.

3) Colon length: After the last dose, all animals were euthanized with CO2 and dissected. The entire colon (anus-cecum section) was isolated, moistened with ice-cold saline, smoothed and laid flat. On the dissecting board, without applying external force to stretch, use a digital vernier caliper to measure the length of the colon and record it, and take photos for retention. The evaluation results are shown in Table 7.

**Table 7 Colon length statistics (n=10).**

| Group | Colon length(mm) | P Value |
|---|---|---|
| Sham | 74.29±0.71 | |
| Vehicle | 49.99±1.55 | |
| Embodiment 33 50mpk P.O Bid | 58.27±1.48 *** | 0.000316 |

Note: *P<0.05, **P<0.01, ***P<0.001, ****P<0.0001, and the one-way ANOVA method test were all compared with the G2 Vehicle group.

4) Detection of inflammatory factors: Blood was collected from all animals under their jaws, and serum was separated; serum and colon tissue IL-1β and IL-18 cytokines were detected according to the detection method in the Effect Embodiment 4.

The experimental results are shown in Table 8. Embodiment compound 33 (50mpk P.O Bid) has significant curative effect on animals with DSS-induced acute enteritis (IBD). It can significantly improve animal weight, reduce intestinal inflammation and bleeding, and improve intestinal inflammation. Reduce colon shortening, and inhibition of serum and colon tissue IL-1β and IL-18 cytokines, and there were statistically significant differences.

**Table 8 Inflammatory factor test results of IBD experiment in vivo in example 33 animals**

| | Sham | Vehicle | Embodiment 33 50mpk,BID |
|---|---|---|---|
| Number of animals | 10 | 10 | 10 |
| IL-1β in serum | 32.07±9.439 | 1227±25.21 | 290.3±30.65 |
| IL-1β in the colon | 0.2737±0.113 | 12.22±1.658 | 6.018±0.9675 |

**Effect Embodiment 6: Safety investigation of the compound of Embodiment 33 administered orally to SD rats for 14 consecutive days**

The purpose of this experiment was to study the reactions of SD rats after oral administration of different doses of the compound of Embodiment 33 and to examine the tolerance of the compound.

In this trial, low-dose and high-dose groups were administered orally with 50 and 100 mg/mL solutions respectively. The administration volume was 20 mL/kg, and the administration doses were 1000 and 2000 mg/kg respectively. Administer continuously for 14 days. Drink water freely during the experiment. The experimental results are shown in Table 9.

**Table 9 Test dose and experimental design**

| Groups | Dosage administered (mg/kg) | concentration (mg /mL) | Frequency of administration | Dosing volume (mL/kg) | Number of animals/animal | |
|---|---|---|---|---|---|---|
| | | | | | ♀ | ♂ |
| Low-dose group | 1000 | 50 | 1 time/day for 14 consecutive days | 20 | 5 | 5 |
| High-dose group | 2000 | 100 | | 20 | 5 | 5 |

After administration, no obvious toxicity reactions were seen in the animals in each dose group. And there was no significant impact on the animal body weight. Gross anatomy showed no obvious pathological changes in the surface color, shape, size, texture, etc. of the heart, liver, spleen, lungs, kidneys, brain, and gastrointestinal tract of the animals in each dose group. Based on the above analysis, the compound of Embodiment 33 may not have any toxic effects at a dose of ≤ 2000 mg/kg, indicating that it is well tolerated.

Although the specific embodiments of the present invention are described above, those skilled in the art should understand that these are only illustrations, and that various changes or modifications may be made to these embodiments without deviating from the principle and substance of the present invention. Accordingly, the scope of the present invention is defined by the appended claims.

## Claims

1. A compound represented by formula I, a solvate thereof, a pharmaceutically acceptable salt thereof or a solvate of the pharmaceutically acceptable salt thereof,

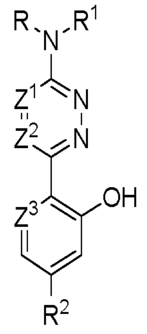

I

among them, $Z^1$, $Z^2$ and $Z^3$ are independently N or $CR^Z$;
each $R^Z$ is independently H, halogen, or $C_1$-$C_6$ alkyl;
or, $Z^1$ and $Z^2$ are $CR^Z$, and the $R^Z$ on $Z^1$ and $Z^2$ together form a ring Y with the carbon connected to it; the ring Y is a $C_5$~$C_6$ cycloalkenyl, a 5-6-membered heteroalkyneyl, a benzene, a 5-6-membered heteroaromatic ring, a $C_5$~$C_6$ cycloalkenyl substituted by one or more $R^{Z-1}$, a 5- to 6-membered heterocycloalkenyl substituted by one or more $R^{Z-2}$, a one or more $R^{Z-3}$-substituted benzene ring, or a one or more $R^{Z-4}$-substituted 5- to 6-membered heteroaromatic ring;
$R^{Z-1}$, $R^{Z-2}$, $R^{Z-3}$ and $R^{Z-4}$ are independent $C_1$~$C_6$ alkyls, $C_1$~$C_6$ alkoxy or halogens;
R is H;
R1 is $C_1$~$C_6$ alkyl, $C_3$~$C_6$ cycloalkyl, 3-10-membered heterocycloalkyl, $C_1$~$C_6$ alkyl group substituted by one or more $R^{1-1}$, $C_3$~$C_6$ cycloalkyl group substituted by one or more $R^{1-2}$ or 3-10-memberedheterocycloalkyl group substituted by one or more $R^{1-3}$;
$R^{1-1,}$ $R^{1-2}$ and $R^{1-3}$ are independently $-NH_2$, -OH, $C_1$~$C_6$ alkyl, $C_3$~$C_6$ cycloalkyl, 3-6-memberedheterocycloalkyl, $-(S{=}O)_2$-$C_1$~$C_6$ alkyl, $C_1$~$C_6$ alkyl group substituted by one or more $R^a$, $C_3$~$C_6$ cycloaklyl group substituted by one or more $R^b$, or 3~6-memberedmembered heterocycloalkyl group substituted by one or more $R^g$;
$R^a$, $R^b$, and $R^g$ are independently -OH, -COOH, $-(C{=}O)NH_2$, $-(C{=}O)NHR^c$, $C_3$~$C_6$ cycloalkyl, or $C_3$~$C_6$ cycloalkyl group substituted by one or more $R^d$s;
$R^c$ and R $^d$are independently -OH, $C_1$~$C_6$ alkyl, $C_1$~$C_6$ alkyl group substituted by one or more $R^e$, $C_3$~$C_6$ cycloalkyl group, or $C_3$~$C_6$ cycloalkyl group substituted by one or more $R^f$s;
$R^e$ and $R^f$ are independently -OH or $C_1$~$C_6$ alkyls;
$R^2$ is a halogen, $C_1$~$C_6$ alkyl group or $C_1$~$C_6$ alkyl group substituted by one or more halogens;
as described in the above, the 5-6-membered heterocycloalkenyl group, 5-6-membered heteroaromatic ring, and 3-10-membered heterocycloalkyl group and 3~6-membered heterocycloalkyl group, the heteroatom is independently one or more of N, O and S, and the number of heteroatoms is 1, 2 or 3 independently;
the compound shown in general formula i satisfies at least one of the following conditions:

(1) at least one of $Z^1$, $Z^2$ and $Z^3$ is N;
(2) $Z^1$ and $Z^2$ are $CR^Z$s, and the $R^Z$s on $Z^1$ and $Z^2$ form a ring Y together with the carbon connected to them.

2. The compound represented by formula I, the solvate thereof, the pharmaceutically acceptable salt thereof or the solvate of the pharmaceutically acceptable salt thereof according to claim 1, **characterized in that** each satisfies one or more of the following conditions:

(1) in $R^Z$, the $C_1$-$C_6$ alkyl group is a $C_1$-$C_3$ alkyl group;
(2) in $R^Z$, the halogen is F, Cl, Br or I;
(3) in ring Y, the $C_5$~$C_6$ alkenyl ring is independently cyclopentenyl;
(4) in ring Y, the 5- to 6-membered heterocycloalkenyl ring is independently a dihydrofuran ring;
(5) in ring Y, the 5- to 6-membered heteroaromatic ring is independently a furan ring, a pyridine ring or a pyrrole ring;
(6) in $R^{Z-1}$, $R^{Z-2}$, $R^{Z-3}$ and $R^{Z-4}$, the $C_1$~$C_6$ alkyl groups are independently $C_1$~$C_3$ alkyl groups;

(7) in $R^{Z-1}$, $R^{Z-2}$, $R^{Z-3}$ and $R^{Z-4}$, the $C_1$~$C_6$ alkoxy groups are independently $C_1$~$C_3$ alkoxy groups;
(8) in $R^{Z-1}$, $R^{Z-2}$, $R^{Z-3}$ and $R^{Z-4}$, the halogen is independently F, Cl, Br or I;
(9) the carbon atom in $R^1$ connected to N in the general formula I has chirality;
(10) in $R^1$, the $C_1$~$C_6$ alkyl groups are independently $C_1$~$C_3$ alkyl groups;
(11) in $R^1$, the $C_3$~$C_6$ cycloalkyl group is independently cyclopropyl, cyclobutyl, cyclopentyl or cyclohexyl;
(12) in $R^1$, the 3 to 10-membered heterocycloalkyl group is independently a 3 to 10-membered monocyclic or bicyclic heterocycloalkyl group;
(13) in $R^{1-1}$, $R^{1-2}$ and $R^{1-3}$, the $C_1$~$C_6$ alkyl group is independently methyl, ethyl, n-propyl, isopropyl, tert-butyl or isobutyl;
(14) among $R^{1-1}$, $R^{1-2}$ and $R^{1-3}$, the $C_3$~$C_6$ cycloalkyl group is independently cyclopropyl or cyclobutyl;
(15) among $R^{1-1}$, $R^{1-2}$ and $R^{1-3}$, the 3 to 6-membered heterocycloalkyl group is independently a 5 to 6-membered heterocycloalkyl group;
(16) in $R^a$ and $R^b$, the $C_3$~$C_6$ cycloalkyl group is independently a cyclopropyl group;
(17) in $R^c$ and $R^d$, the $C_1$~$C_6$ alkyl groups are independently $C_1$~$C_3$ alkyl groups;
(18) in Rc and Rd, the $C_3$~$C_6$ cycloalkyl group is independently a cyclobutyl group;
(19) in Re and Rf, the $C_1$~$C_6$ alkyl groups are independently $C_1$~$C_3$ alkyl groups;
(20) in $R^2$, the halogen is independently F, Cl, Br or I;
(21) in $R^2$, the $C_1$~$C_6$ alkyl group is independently a $C_1$~$C_3$ alkyl group;
(22) the numbers of $R^{Z-1}$, $R^{Z-2}$, $R^{Z-3}$ and $R^{Z-4}$ are denoted as a, b, c and d respectively, where a, b, c and d are independently 1, 2 or 3.

**3.** The compound represented by formula I, the solvate thereof, the pharmaceutically acceptable salt thereof or the solvate of the pharmaceutically acceptable salt thereof according to claim 2, **characterized in that** each satisfies one or more of the following conditions:

(1) in $R^Z$, the $C_1$-$C_6$ alkyl group is a methyl group;
(2) in $R^Z$, the halogen is F or Cl;
(3) in the ring Y, the $C_5$~$C_6$ alkenyl ring is independently cyclopentene, and in this case, the structure in formula I

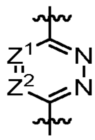

can be

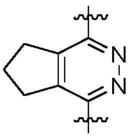

or

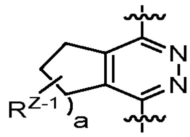

;

(4) in the ring Y, the 5-6-membered heterocycloalkenyl ring is a dihydrofuran ring independently, and in this case, the structure in formula I

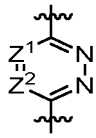

is

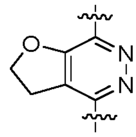, 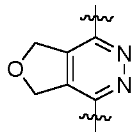 or 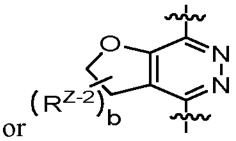;

or in General formula I, the

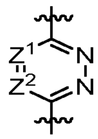

structure is

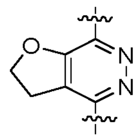, 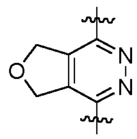, 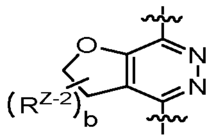

or

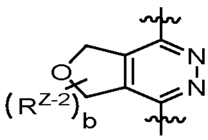;

(5) in ring Y, the 5~6 -membered heteroaromatic ring is independently furan ring, pyridine ring or pyrrole ring, and in these cases, the structure in formula I

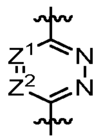

can be

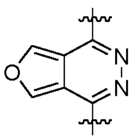, 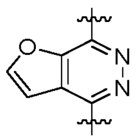, 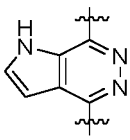

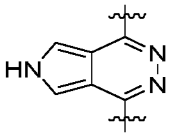 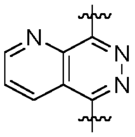 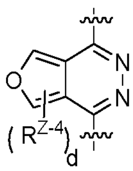 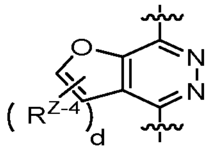 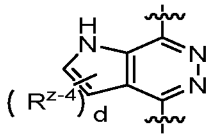 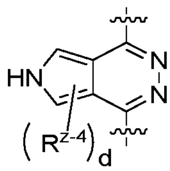

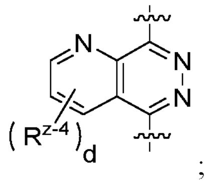

;

(6) in $R^{Z-1}$, $R^{Z-2,}$ $R^{Z-3}$ and $R^{Z-4}$, the $C_1$~$C_6$ alkyl group is a methyl group;
(7) in $R^{Z-1}$, $R^{Z-2}$, $R^{Z-3}$ and $R^{Z-4}$ , the alkoxy group of described $C_1$~$C_6$ is a methoxy group;
(8) in $R^{Z-1}$, $R^{Z-2}$, $R^{Z-3}$ and $R^{Z-4}$, the halogen is F;
(9) the carbon atom in $R^1$ connected to N in General formula I is chiral, and the configuration of the carbon atom is R configuration;
(10) in $R^1$, the $C_1$~$C_6$ alkyl group is a methyl group;
(11) in $R^1$, the C3~C6 cycloalkyl group is cyclobutyl or cyclohexyl;
(12) in $R^1$, when the 3-10-membered heterocycloalkyl group is a bicyclic heterocycloalkyl group, it is a 6-membered heterocyclic-ring fusing a 5-membered heterocycloalkyl group;
(13) in $R^1$, when the 3-10-memberedheterocycloalkyl group is a monocyclic heterocycloalkyl group, it is a 3~6-member heterocycloalkyl group;
(14) in $R^{1-1}$, $R^{1-2}$ and $R^{1-3}$, the 3~6-membered heterocycloalkyl group is a tetrahydropyrrole group;
(15) in $R^c$ and $R^d$, the $C_1$~$C_6$ alkyl group is isopropyl;
(16) in $R^e$ and $R^f$, the $C_1$~$C_6$ alkyl group is a methyl group;
(17) $R^2$, the halogen is F or Cl;
(18) $R^2$, the $C_1$~$C_6$ alkyl group is a methyl group.

4. The compound represented by formula I, the solvate thereof, the pharmaceutically acceptable salt thereof or the solvate of the pharmaceutically acceptable salt thereof according to claim 3, **characterized in that** each satisfies one or more of the following conditions:

(1) $R^Z$ is independently H, F, Cl or $-CH_3$;
(2)

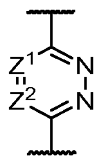

can be

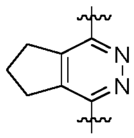 , 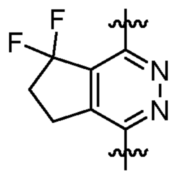 , 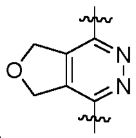 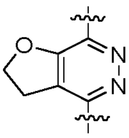 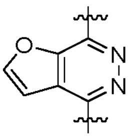 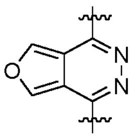 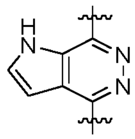

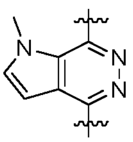 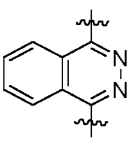 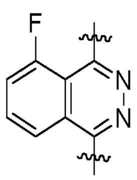 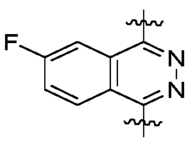 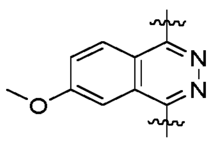 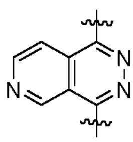 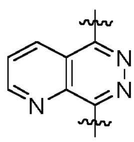 ;

(3) $R^{Z-1}$ is F;
(4) $R^{Z-3}$ is methoxy or F;
(5) $R^{Z-4}$ is methyl;
(6) in $R^1$, when the 3-10-membered heterocycloalkyl group is a bicyclic heterocycloalkyl group, it is a piperidine ring and tetrahydropyrrole ring, for example

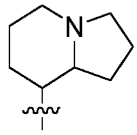;

(7) in $R^1$, when the 3-10-membered heterocycloalkyl group is a monocyclic heterocycloalkyl group, it is a piperidine group, for example

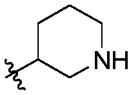;

(8) $R^{1-1}$, $R^{1-2}$ and $R^{1-3}$, the 3~6-membered heterocycloalkyl group is

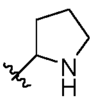;

(9) $R^1$ is any one of the following structures:

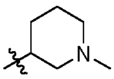 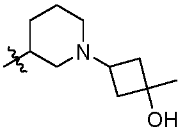 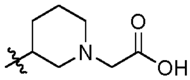 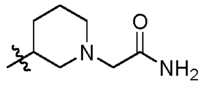 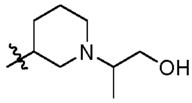

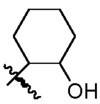 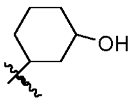 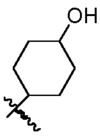 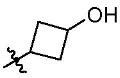 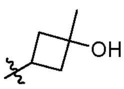 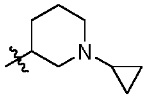 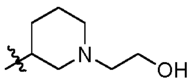

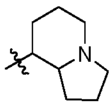 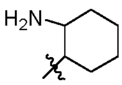 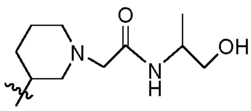 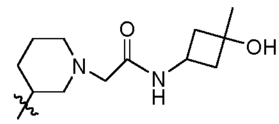

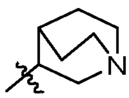 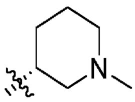 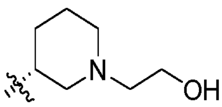 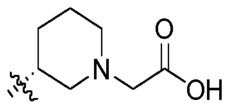 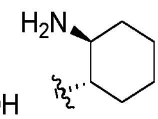 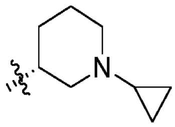

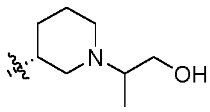 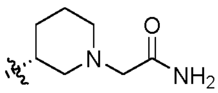 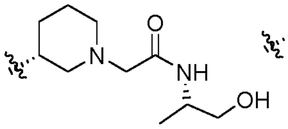 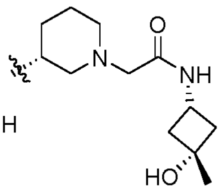 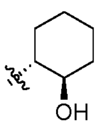

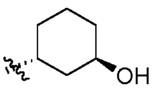 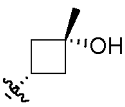 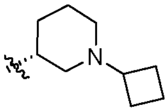 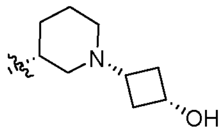 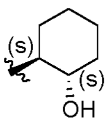 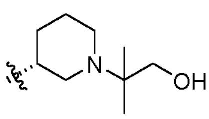

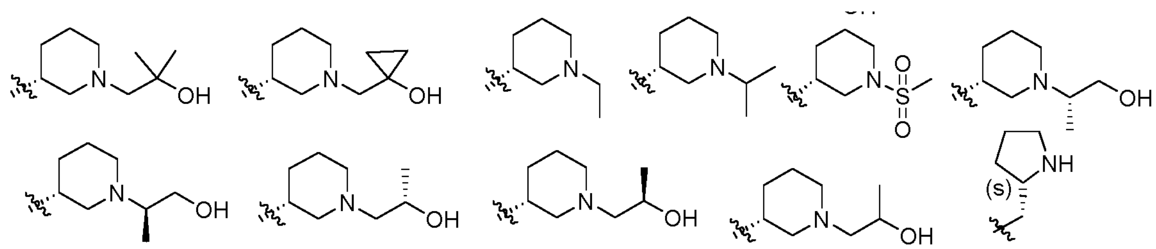

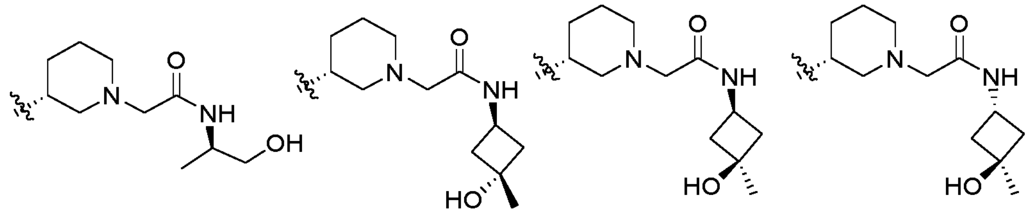

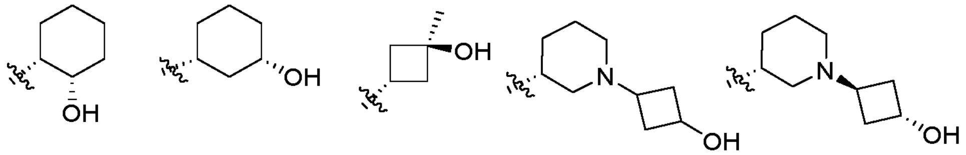

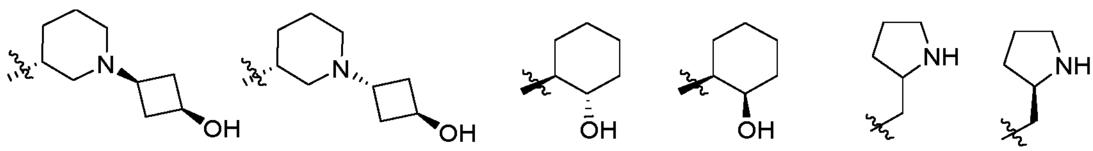

;

(10) $R^2$ is $-CH_3$, Cl or $-CF_3$.

**5.** The compound represented by formula I, the solvate thereof, the pharmaceutically acceptable salt thereof or the solvate of the pharmaceutically acceptable salt thereof according to claim 1, **characterized in that** when $R^1$ is a one or more $R^{1-1}$s substituted $C_1$~$C_6$ alkyl group, a one or more $R^{1-2}$s substituted $C_3$~$C_6$ cycloalkyl group or a one or more $R^{1-3}$s substituted 3-10-membered heterocycloalkyl group, the compound shown in formula 1 is:

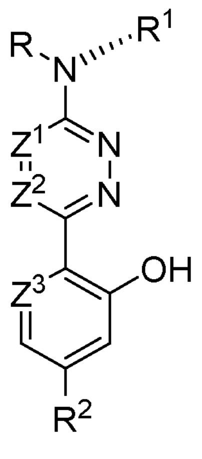

**I**

.

**6.** The compound represented by formula I, the solvate thereof, the pharmaceutically acceptable salt thereof or the solvate of the pharmaceutically acceptable salt thereof according to claim 1, **characterized in that** each satisfies one or more of the following conditions:

(1) $Z^1$ is N;
(2) $Z^2$ and $Z^3$ are independent $CR^Z$;
(3) each $R^Z$ is independently H or C1-C6 alkyl group;
(4) when $Z^1$ and $Z^2$ are $CR^Z$, and the $R^Z$ on $Z^1$ and $Z^2$ forms a ring Y together with the carbon connected to it, the ring Y is a benzene ring, a 5-6-membered heteroaromatic ring, a benzene ring substituted by one or more $R^{Z-3}$ or a 5-6-membered heteroaromatic ring substituted by one or more $R^{Z-4}$s;
(5) when $Z^1$ and $Z^2$ are $CR^Z$, and the $R^Z$ on $Z^1$ and $Z^2$ together form a ring Y with the carbon connected thereto, the ring Y is a benzene ring, a 5-6-member heteroaromatic ring, a benzene ring substituted by one or more R $^{Z-3}$ or a 5-6-member heteroaromatic ring substituted by one or more $R^{Z-4}$, and the $R^{Z-3}$ and $R^{Z-4}$ are halogens independently;
(6) $R^1$ is $C_3$~$C_6$ cycloalkyl, 3-10-membered heterocycloalkyl, $C_3$~$C_6$ cycloalkyl group substituted by one or more $R^{1-2}$ or 3-10-membered heterocycloalkyl group substituted by one or more $R^{1-3}$;
(7) $R^{1-2}$ and $R^{1-3}$ are independently -OH, $C^1$~$C^6$ alkyl, $C^3$~$C^6$ cycloalkyl or $C^1$~$C^6$ alkyl group substituted by one or more $R^a$;
(8) $R^a$ is -OH independently.

**7.** The compound represented by formula I, the solvate thereof, the pharmaceutically acceptable salt thereof or the solvate of the pharmaceutically acceptable salt thereof according to claim 1, **characterized in that** it is defined as described in any one of the following scenarios:

scenario 1:

$Z^1$ is N;
$Z^2$ and $Z^3$ are independently for $CR^Z$;
each $R^Z$ is independently of H or $C_1$-$C_6$ alkyl group;
alternatively, $Z^1$ and $Z^2$ are $CR^Z$, and the $R^Z$ on $Z^1$ and $Z^2$ forms a ring Y together with the carbon connected to it, and the ring Y is a benzene ring, a 5-6-membered heteroaromatic ring, a benzene ring substituted by one or
more R $^{Z-3}$ or a 5-6-membered heteroaromatic ring substituted by one or more $R^{Z-4}$s;
$R^{Z-3}$ and $R^{Z-4}$ are halogens independently;
R is H;
$R^1$ is C3~C6 cycloalkyl, 3~10-memberedmembered heterocycloalkyl, $C_3$~$C_6$ cycloalkyl group substituted by one or more $R^{1-2}$ or 3-10-memberedmembered heterocycloalkyl group substituted by one or more R1-3;
$R^{1-2}$ and $R^{1-3}$ are independently -OH, $C_1$~$C_6$ alkyl, $C_3$~$C_6$ cycloalkyl or $C_1$~$C_6$ alkyl group substituted by one or more $R^a$;
$R^a$ is -OH independently;

scenario 2:

the compound shown in general formula I is a compound as shown in general formula I-1

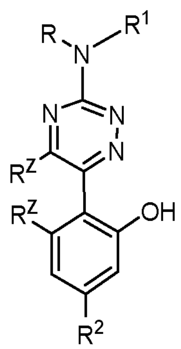

**I-1** ;

wherein, $R^1$ is a $C_3$~$C_6$ cycloalkyl group substituted by one or more $R^{1-2}$s, $R^{1-2}$ is $-NH_2$ or -OH independently, and preferably, the $R^{1-2}$ is located at the ortho position of the linker group in R1

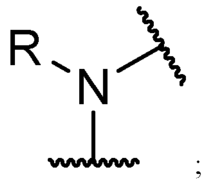

R is H;
each $R^Z$ is independently of H or $C_1$-$C_6$ alkyl group;
$R^2$ is a $C_1$~$C_6$ alkyl group substituted by one or more halogens

scenario 3:

the compound shown in general formula I is a compound as shown in general formula I-1

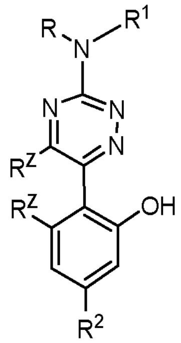

**I-1**

;

wherein $R^1$ is a cyclohexyl group substituted by one or more $R^{1-2}$s, $R^{1-2}$ is $-NH_2$ or -OH independently, and preferably, the $R^{1-2}$ is located at the ortho position of the linker group in $R^1$

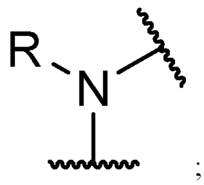

R is H;
each $R^Z$ is independently of H or $C_1$-$C_6$ alkyl group;
$R^2$ is a $C_1$~$C_6$ alkyl group substituted by one or more halogens.

8. The compound represented by formula I, the solvate thereof, the pharmaceutically acceptable salt thereof or the solvate of the pharmaceutically acceptable salt thereof according to claim 1, **characterized in that** each satisfies one or more of the following conditions:

(1) $Z^1$ is N;
(2) $Z^2$ is N;
(3) $Z^3$ is N.

9. The compound represented by formula I, the solvate thereof, the pharmaceutically acceptable salt thereof or the solvate of the pharmaceutically acceptable salt thereof according to claim 1, **characterized in that** compounds shown in formula I can be any one of the following structures:

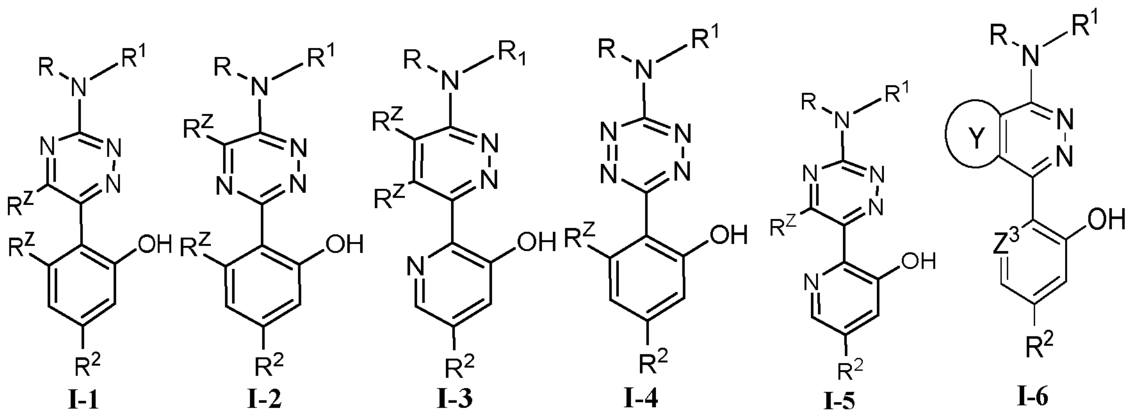

I-1 I-2 I-3 I-4 I-5 I-6

.

**10.** The compound represented by formula I, the solvate thereof, the pharmaceutically acceptable salt thereof or the solvate of the pharmaceutically acceptable salt thereof according to claim 1, **characterized in that** the compound shown in formula I can be any one of the following structures:

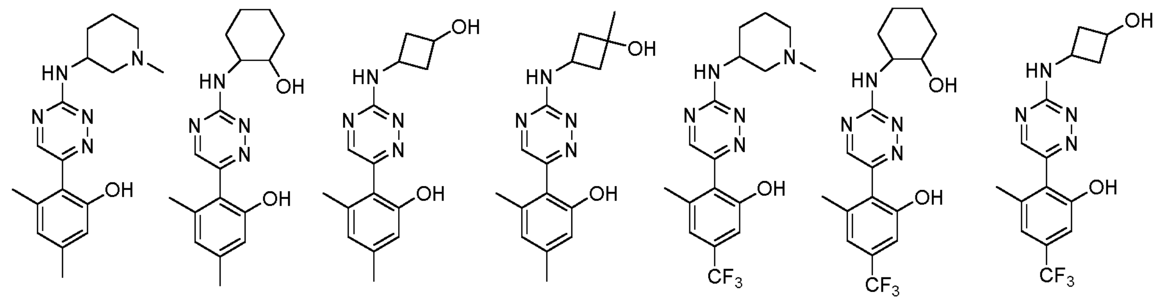

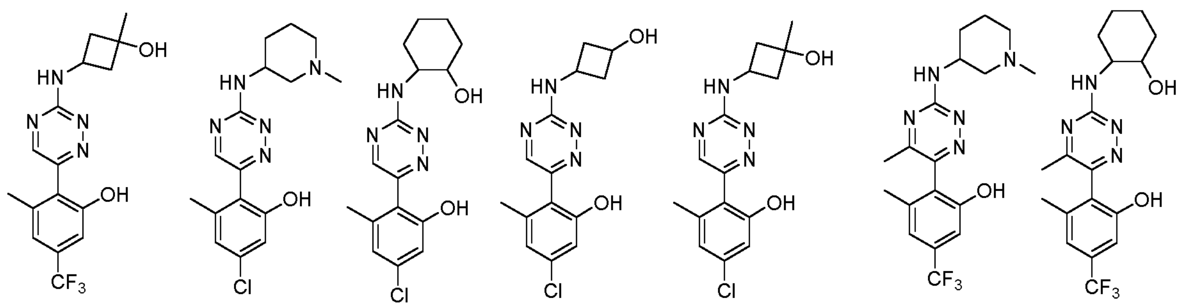

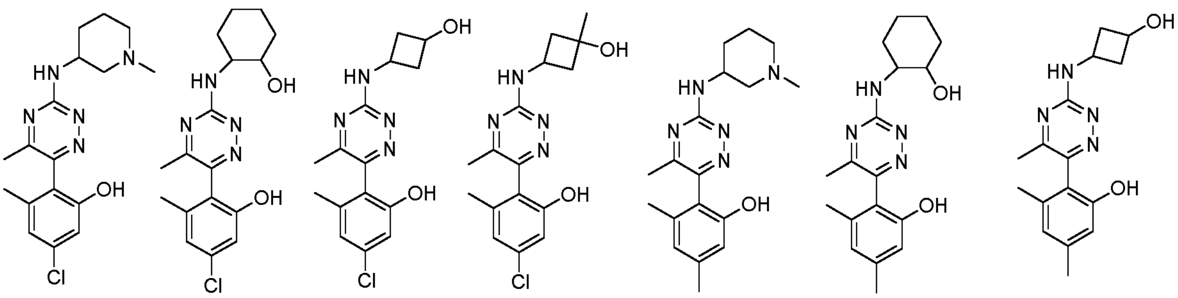

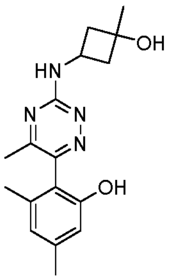 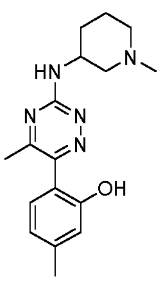 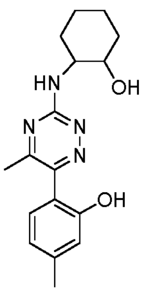 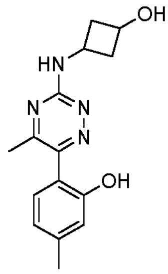 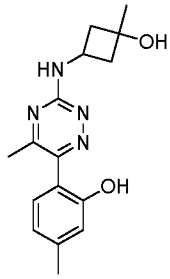 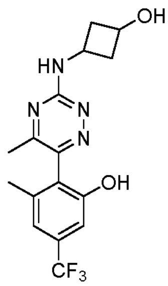 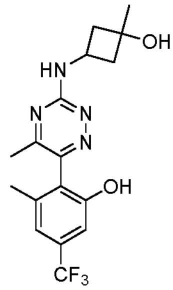

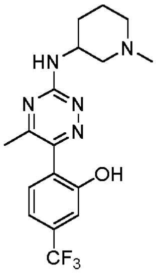 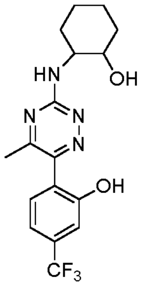 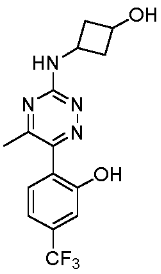 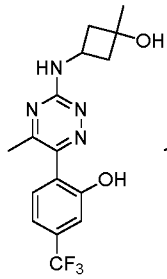 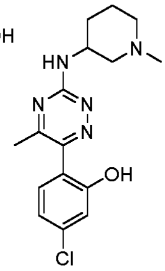 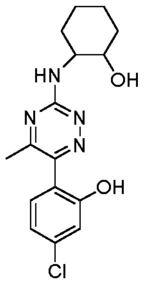 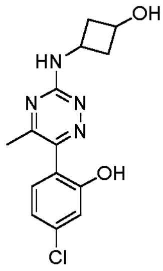

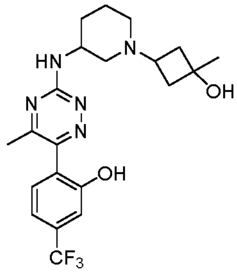 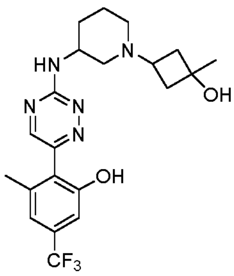 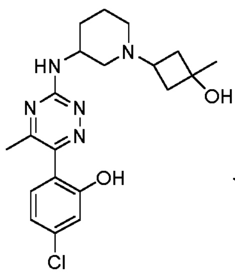 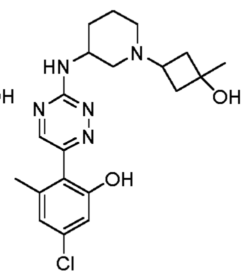 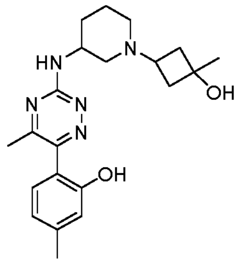

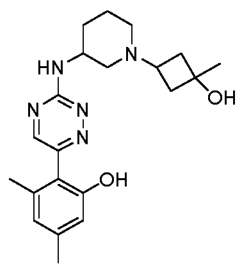 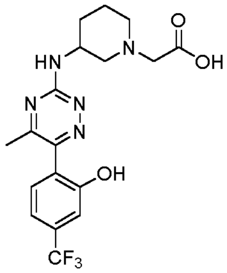 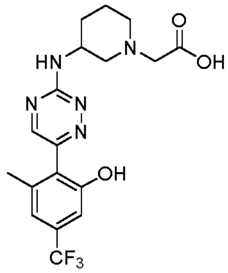 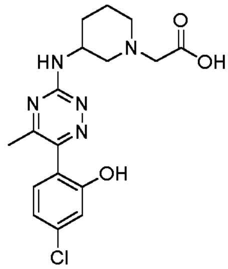 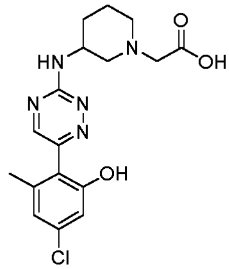

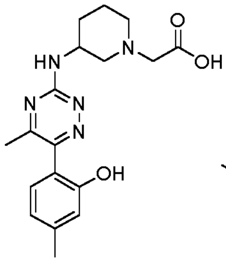 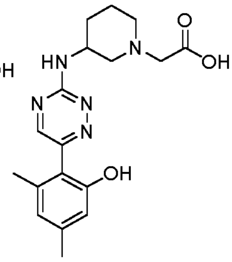 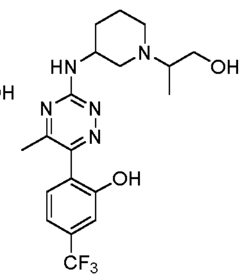 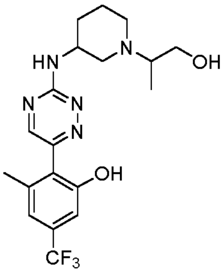 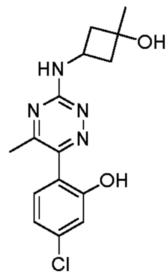

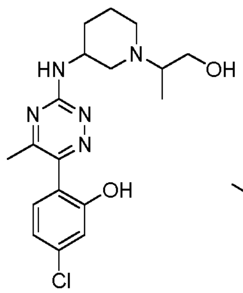 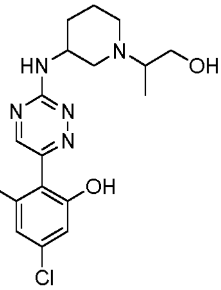 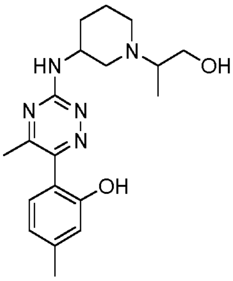 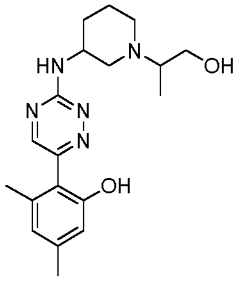 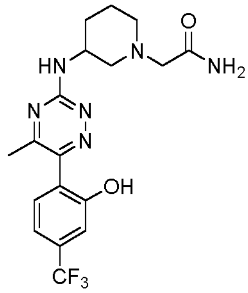

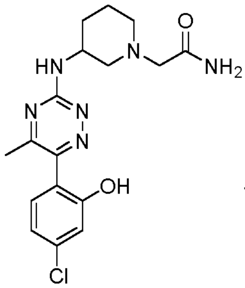 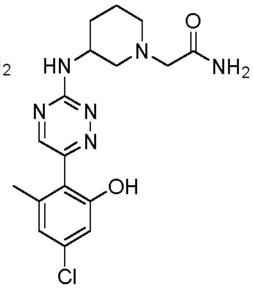 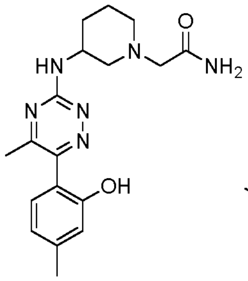 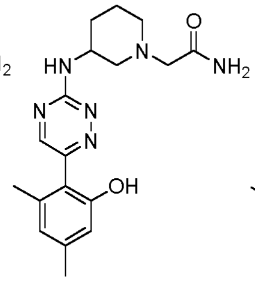 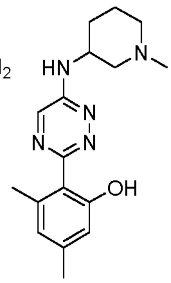

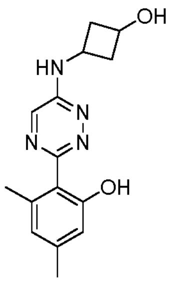 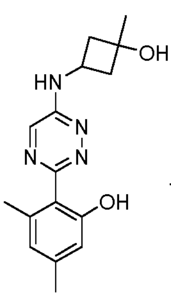 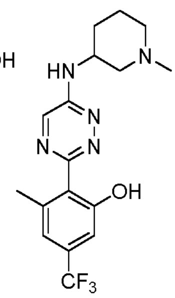 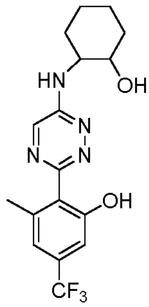 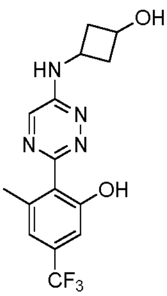 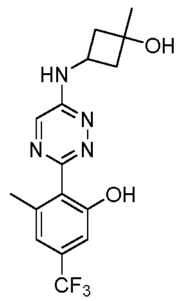

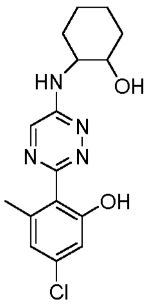 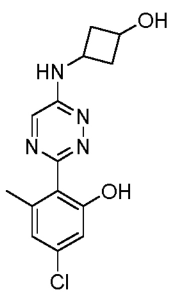 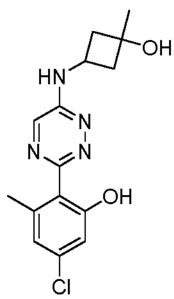 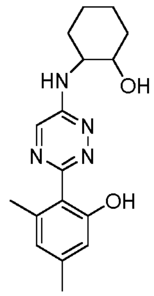 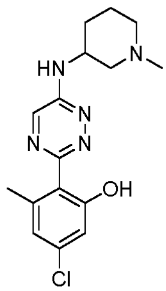 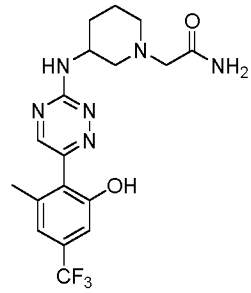

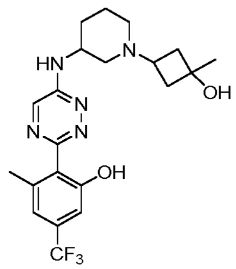 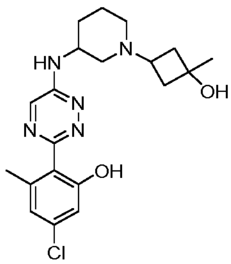 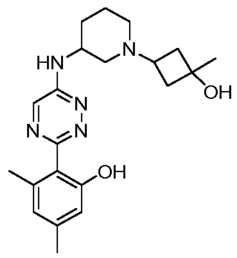 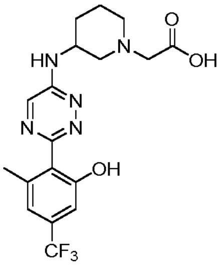 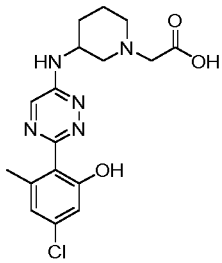

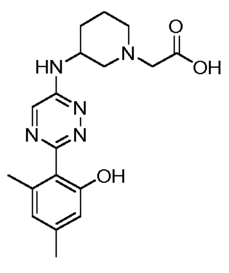
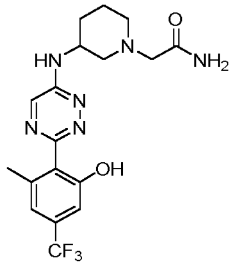
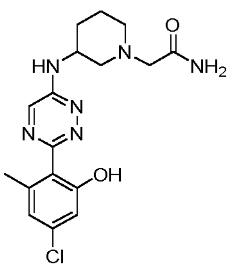
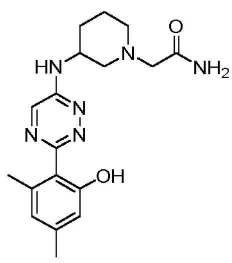
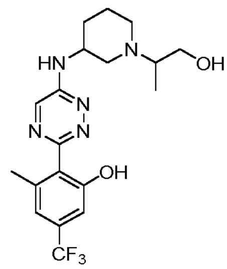

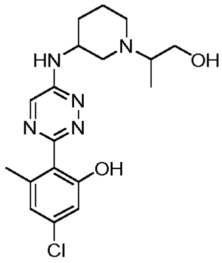
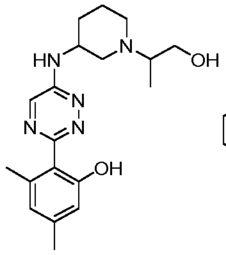
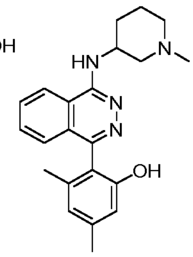
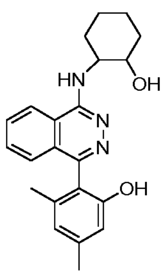
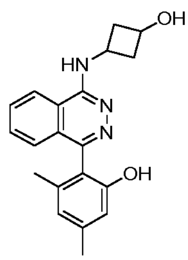

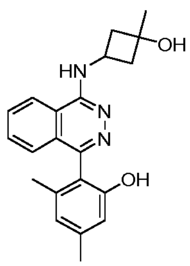
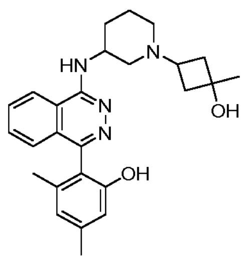
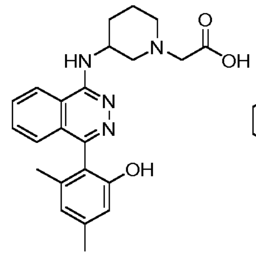
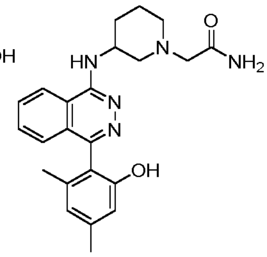
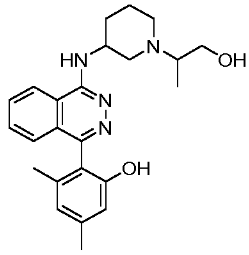

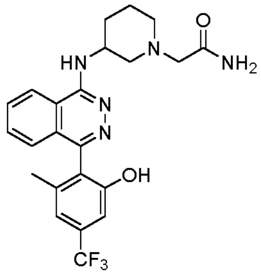
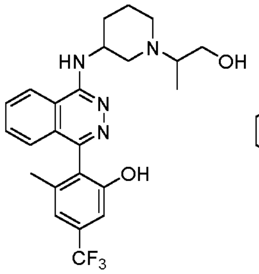
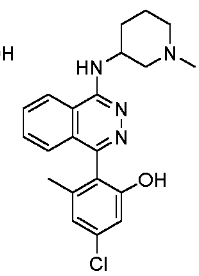
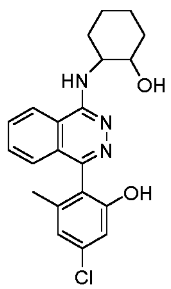
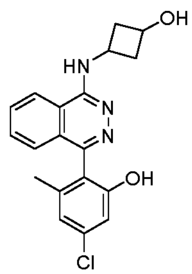

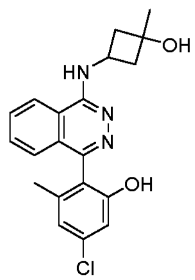
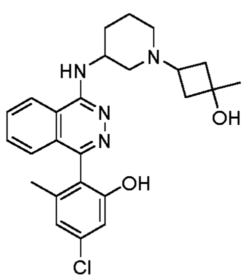
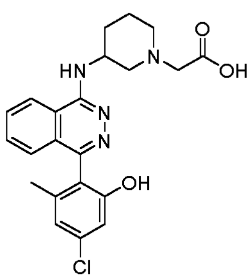
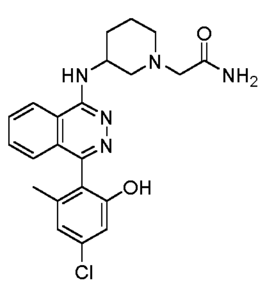
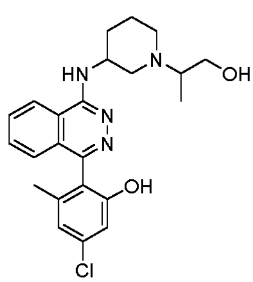

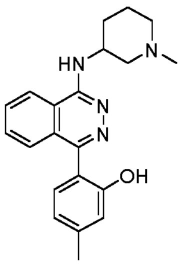
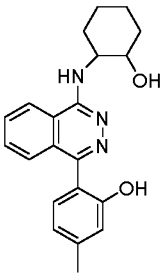
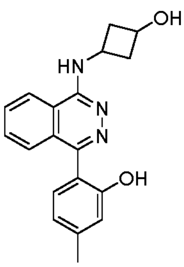
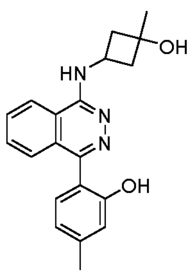
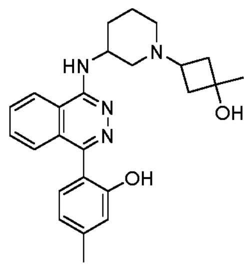

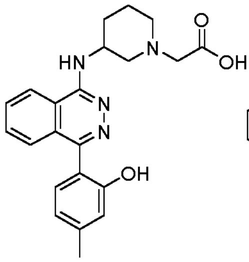
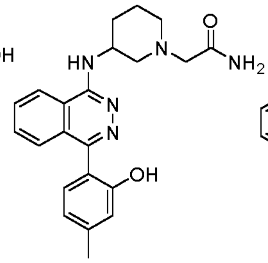
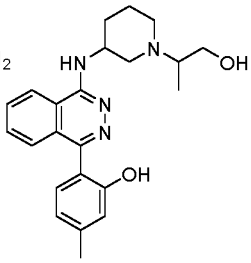
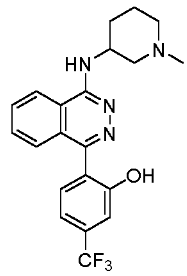
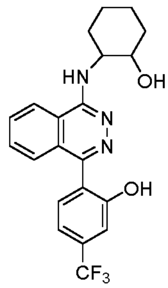

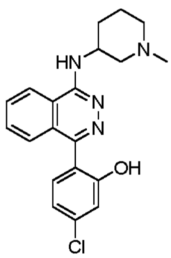
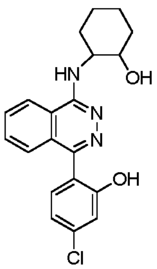
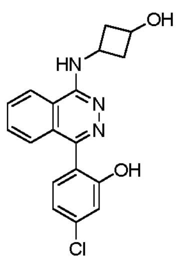
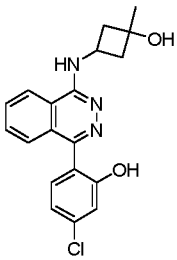
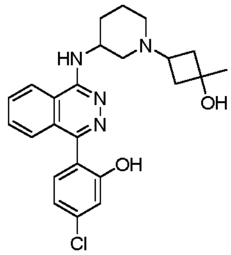
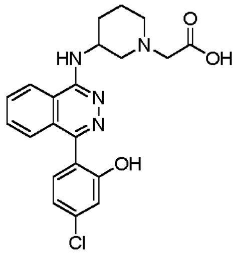

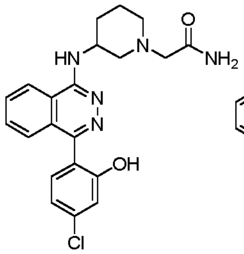
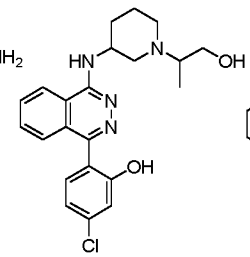
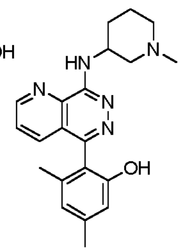
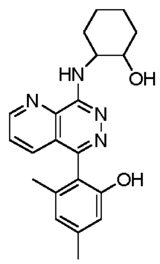
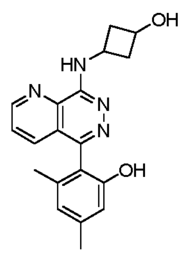

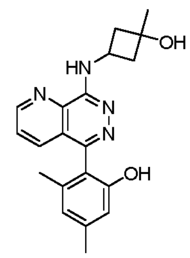
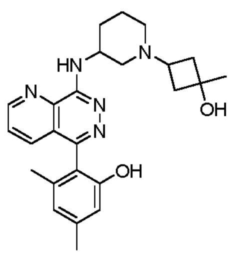
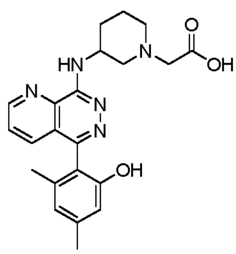
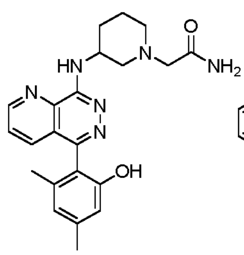
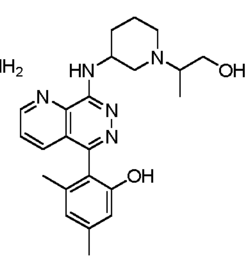

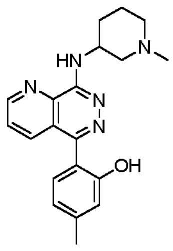
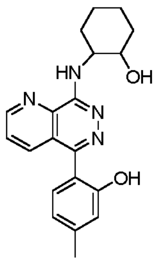
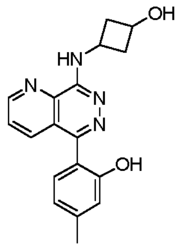
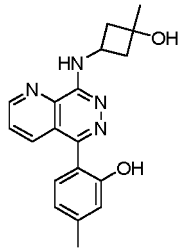
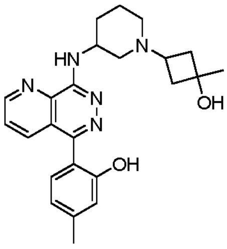
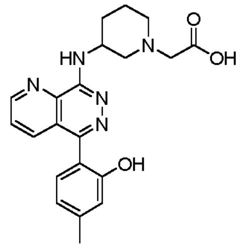

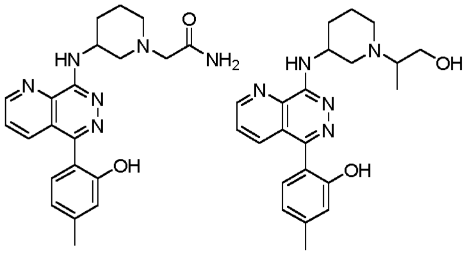
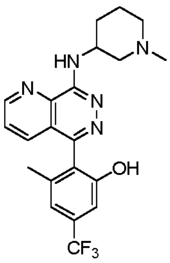
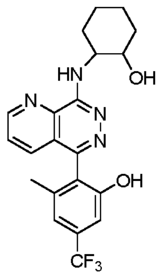
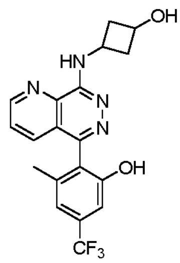
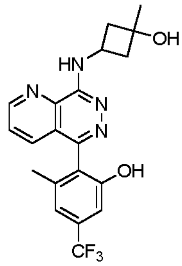

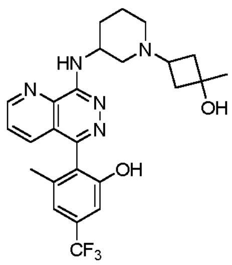
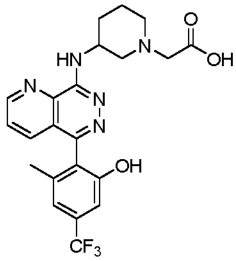
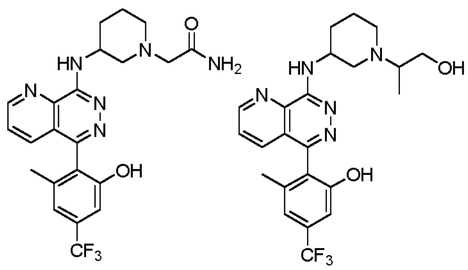
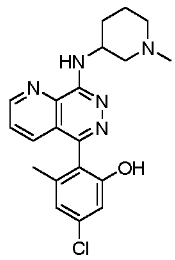

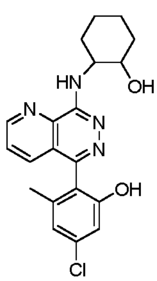
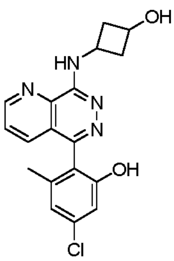
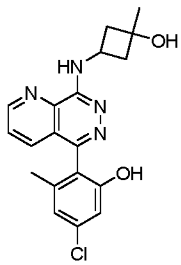
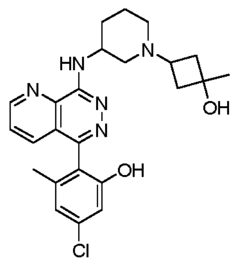
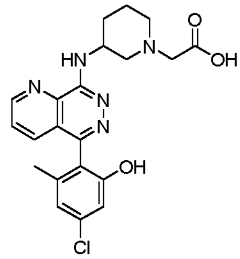

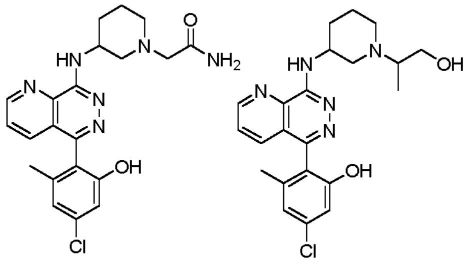
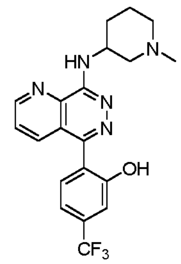
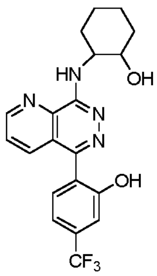
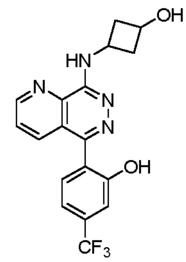

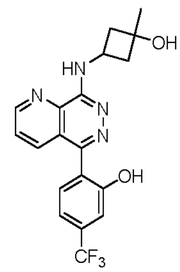
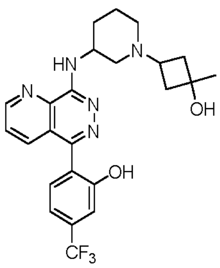
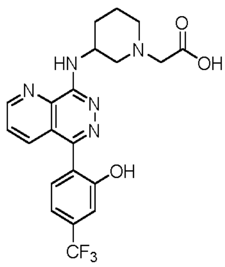
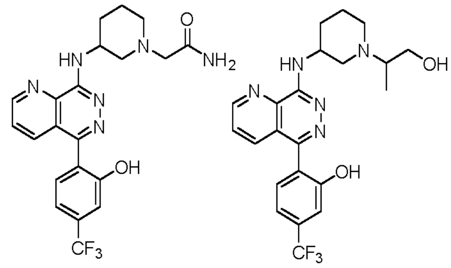

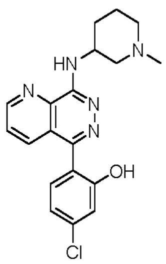
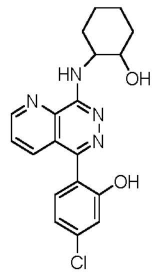
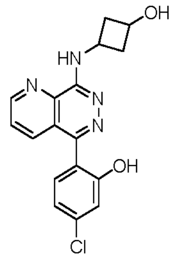
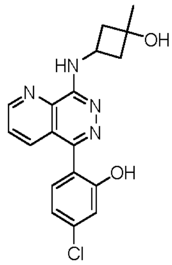
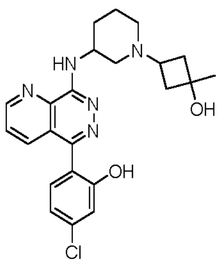
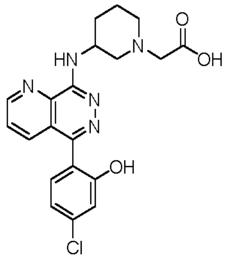

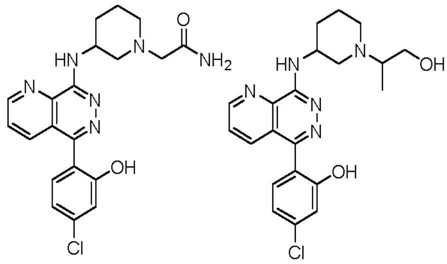
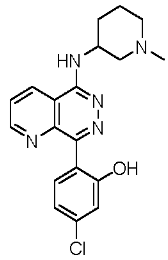
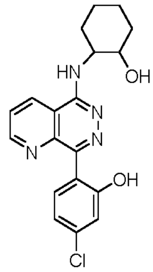
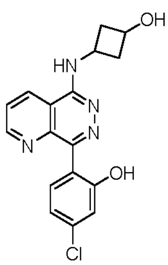
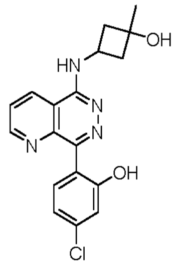

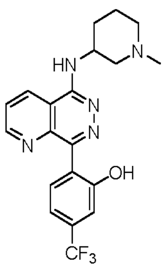
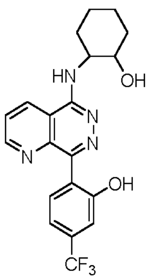
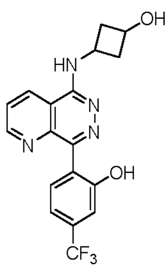
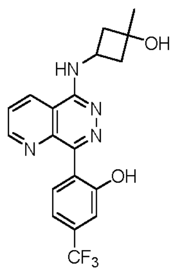
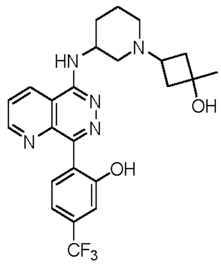
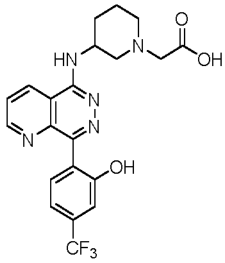

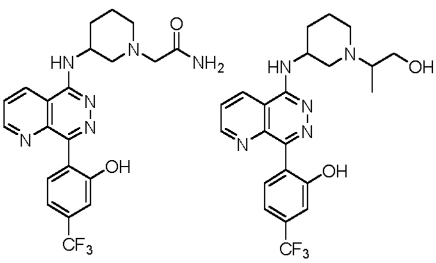
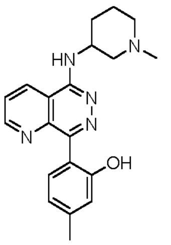
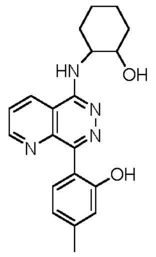
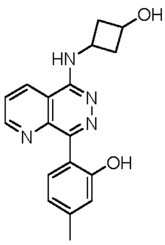
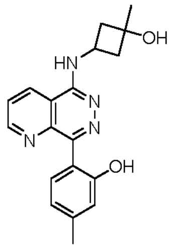

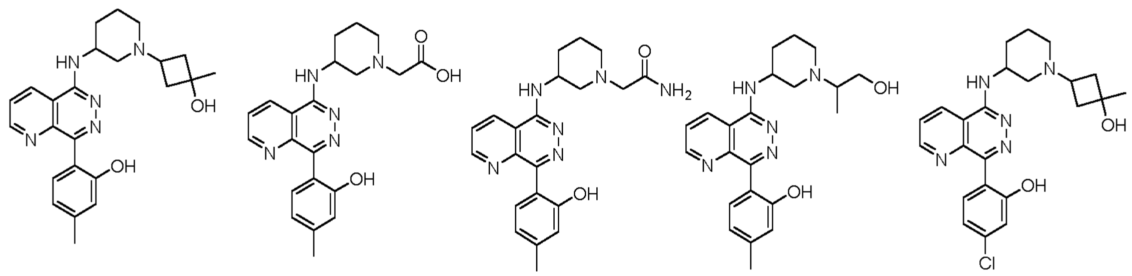

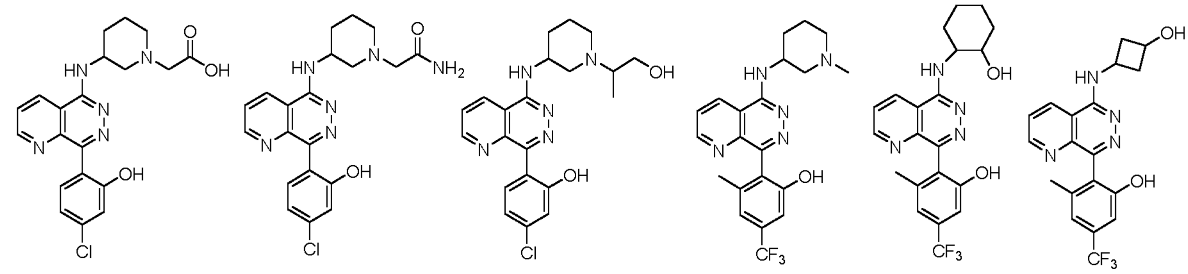

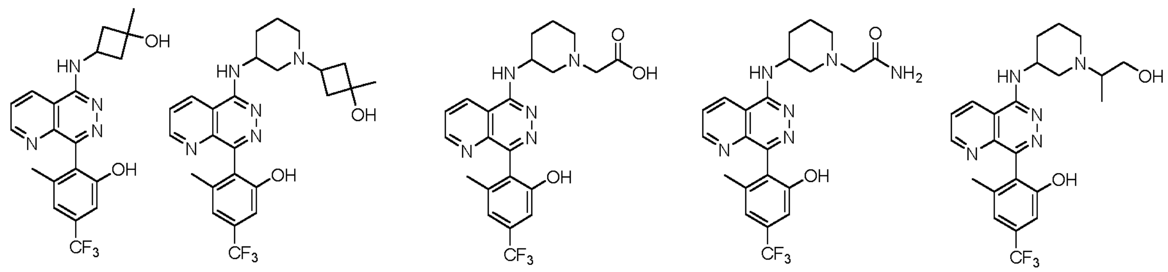

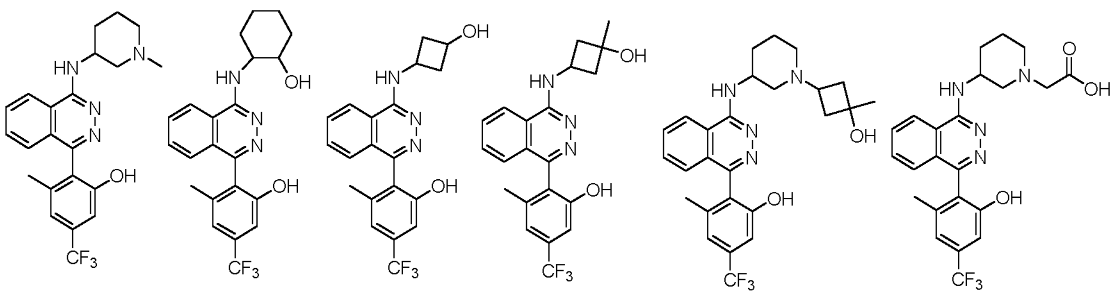

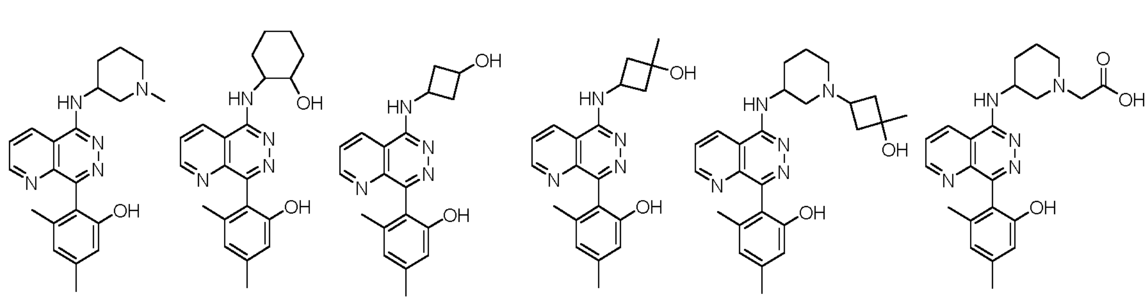

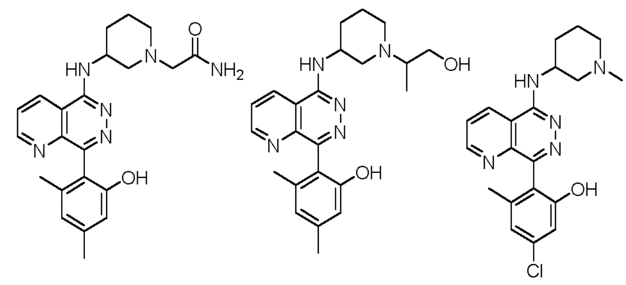
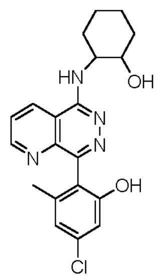
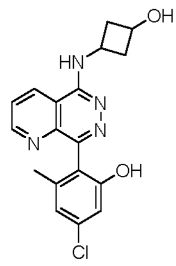

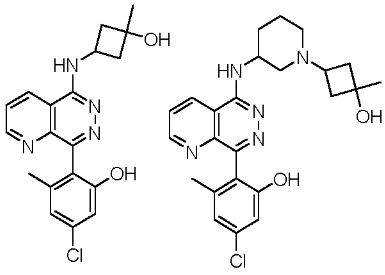
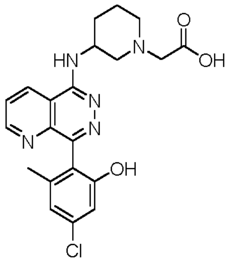
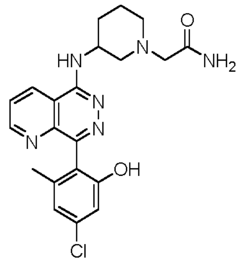
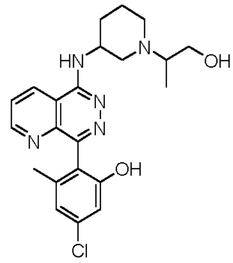

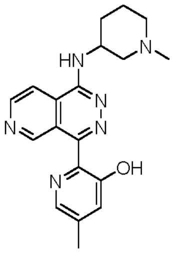
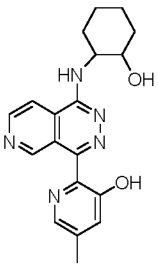
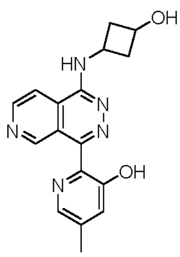
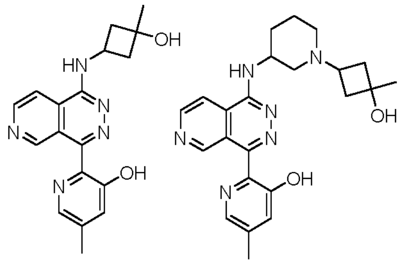
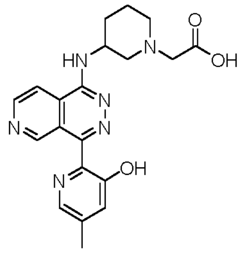

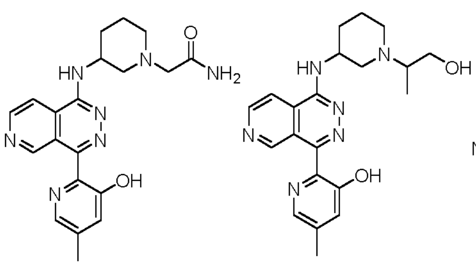
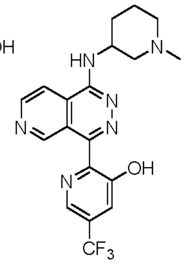
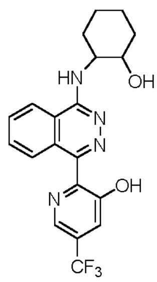
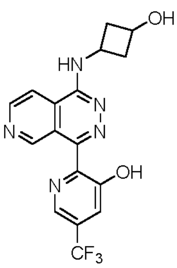

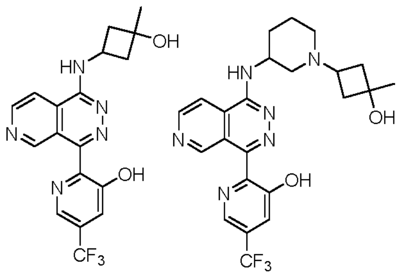
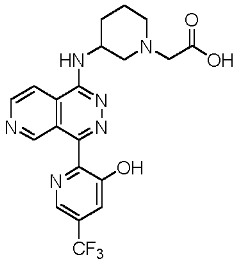
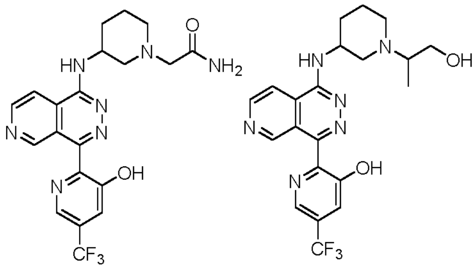

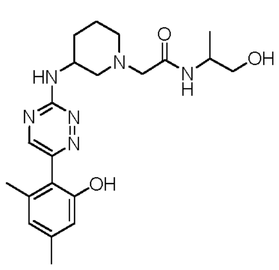 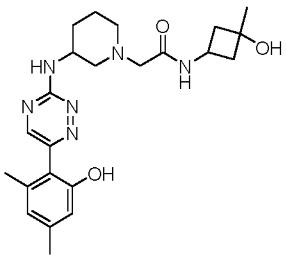 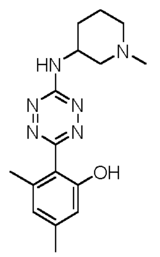 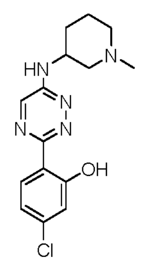

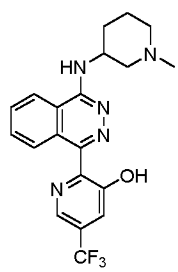 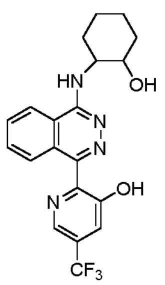 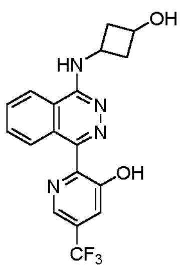 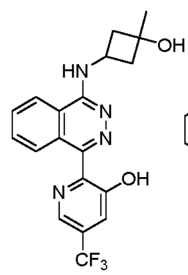 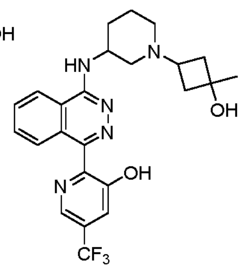 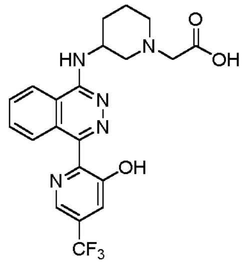

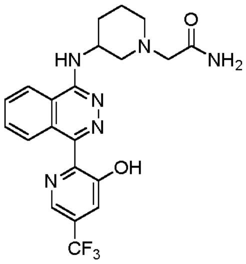 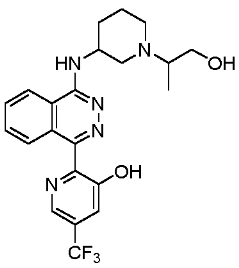 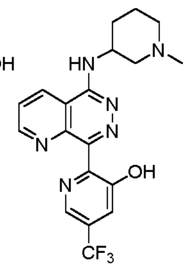 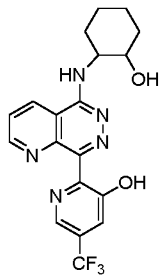 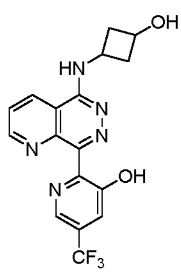

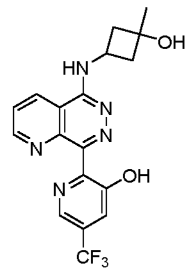 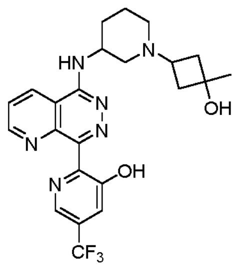 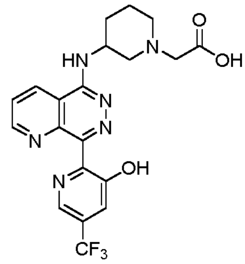 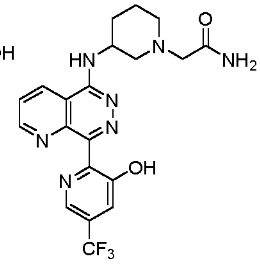 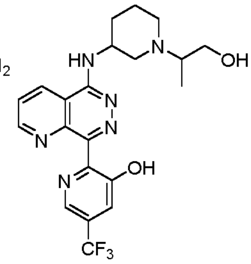

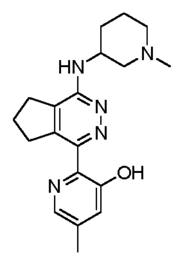 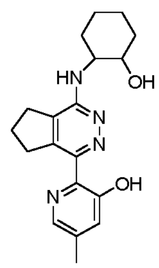 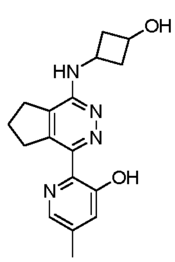 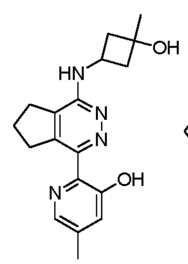 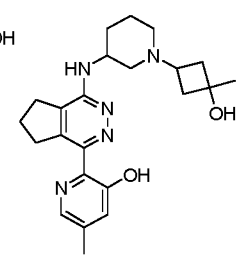 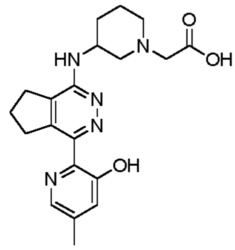

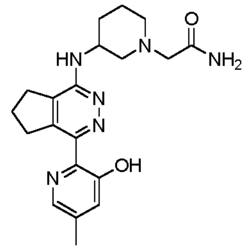
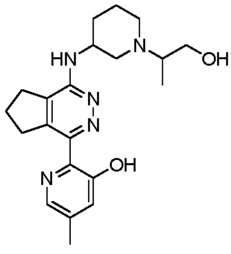
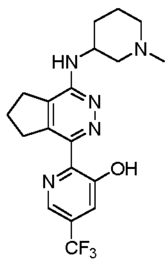
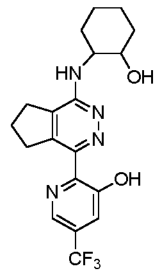
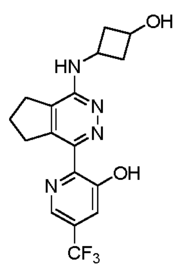

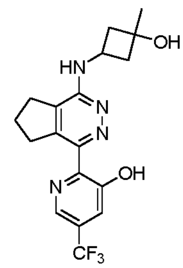
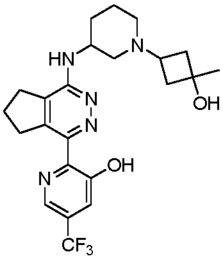
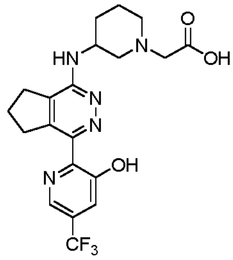
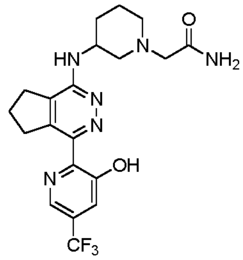
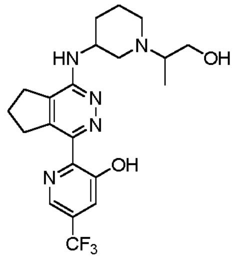

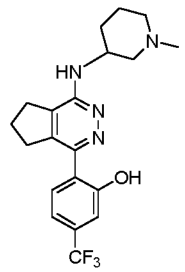
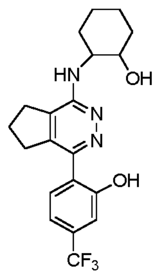
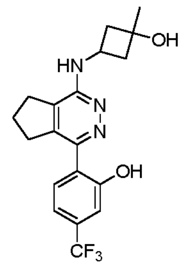

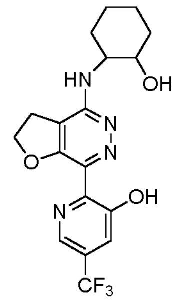
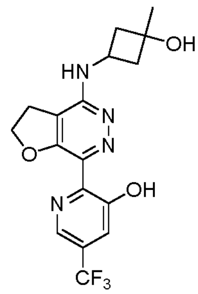
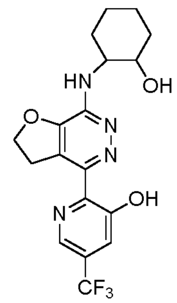
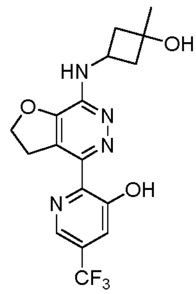
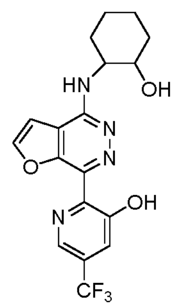
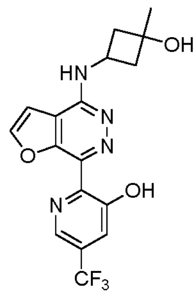

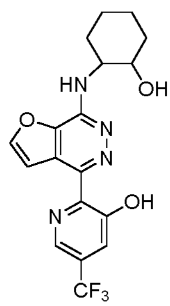
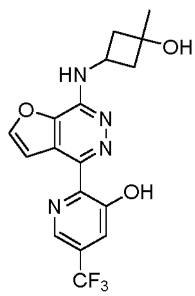
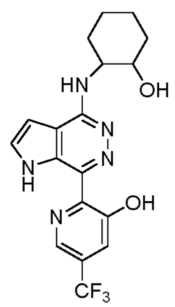
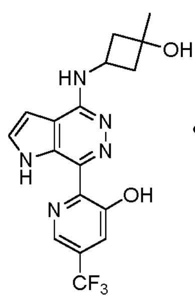
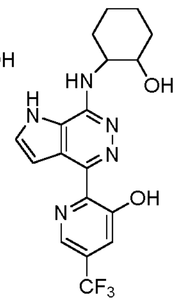
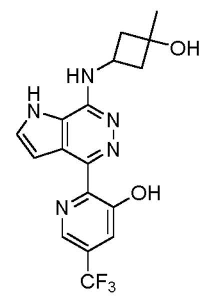

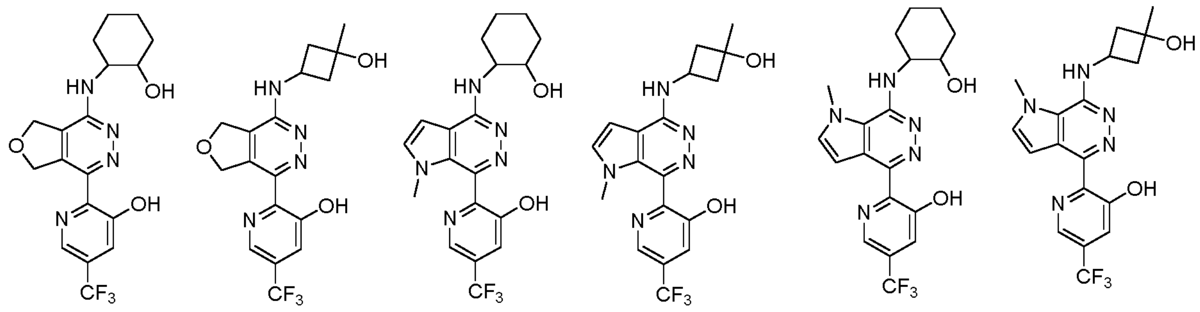

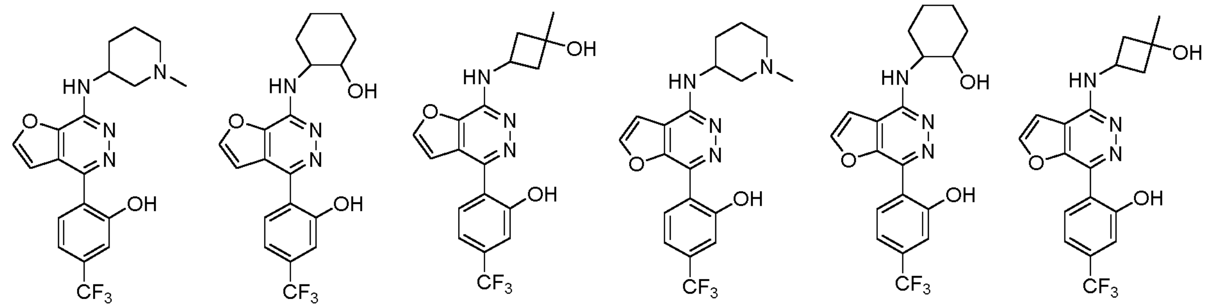

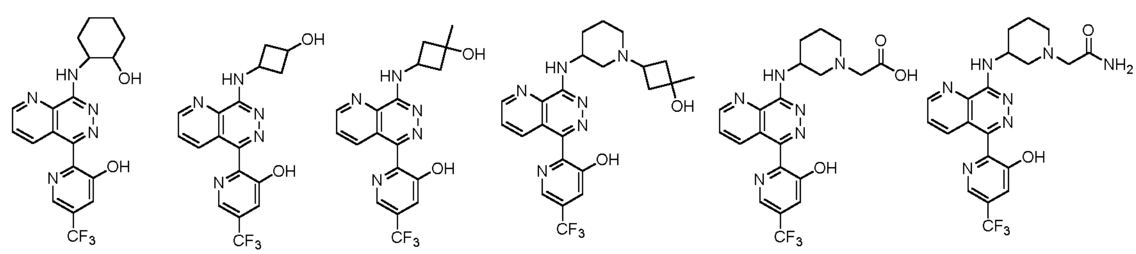

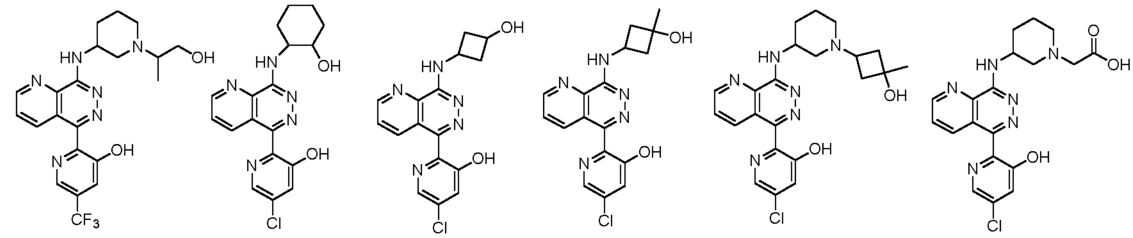

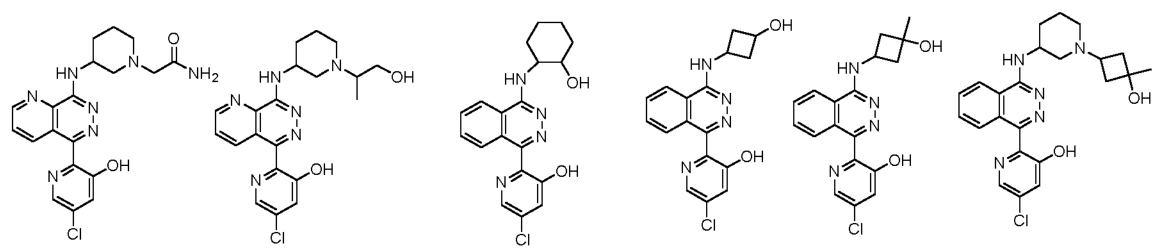

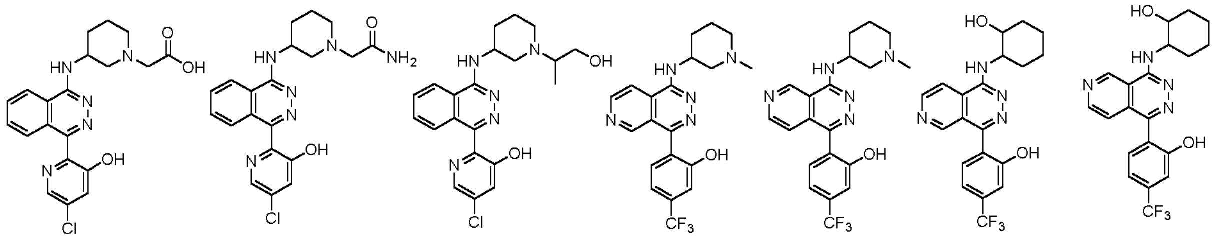

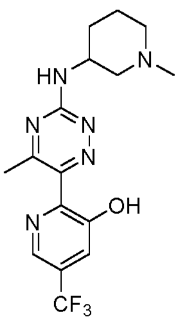
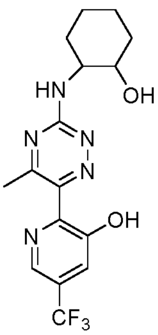
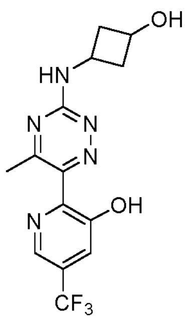
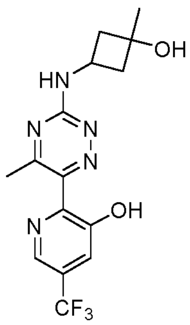
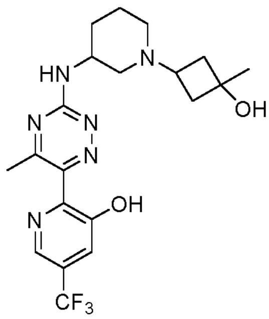

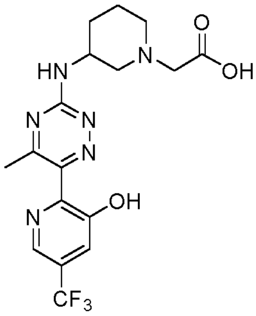
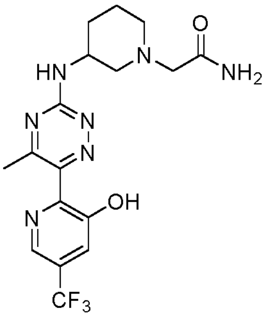
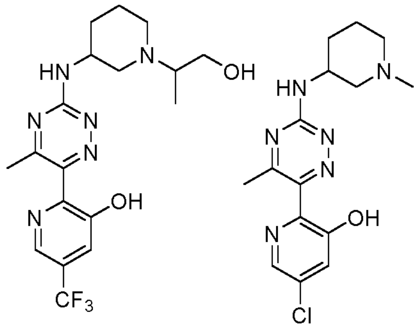
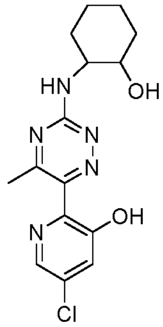

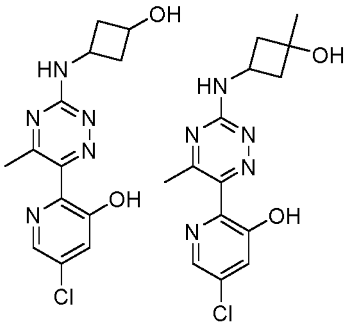
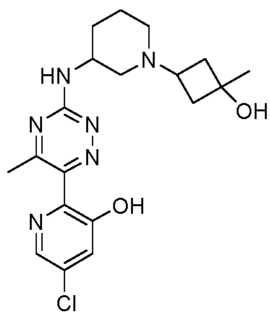
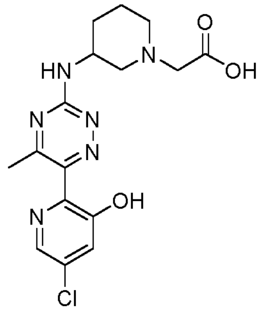
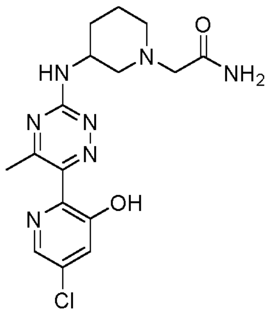

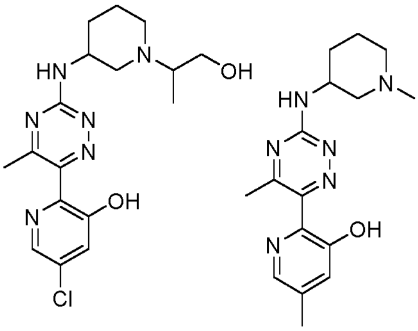
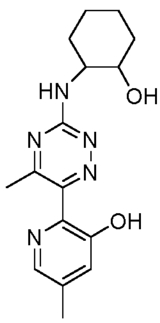
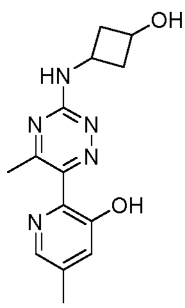
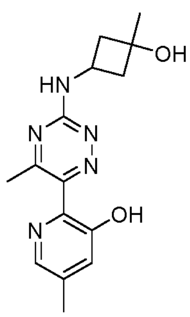

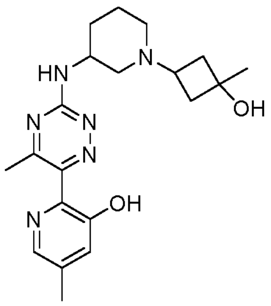
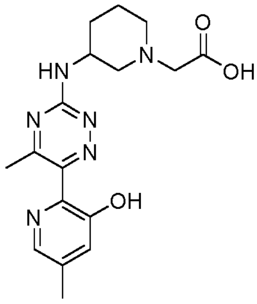
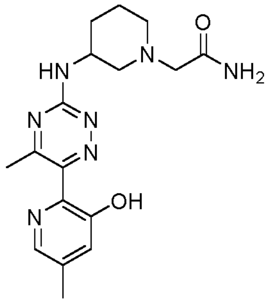
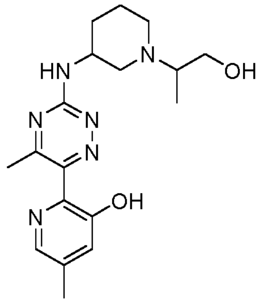

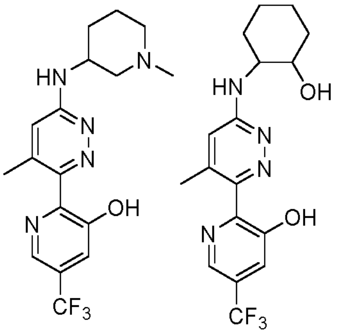
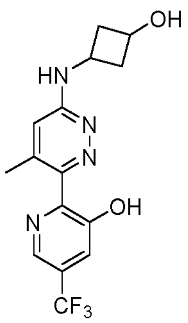
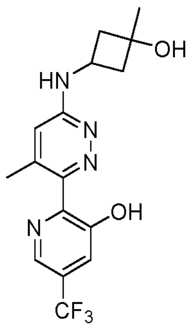
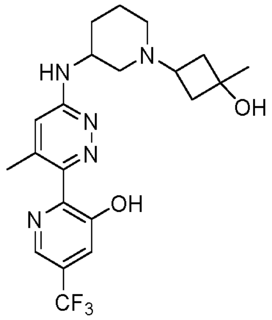

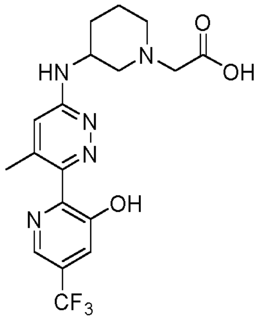
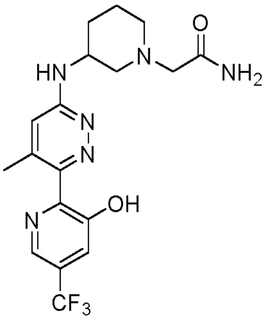
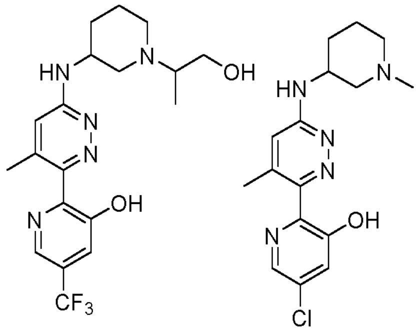
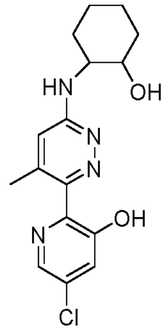

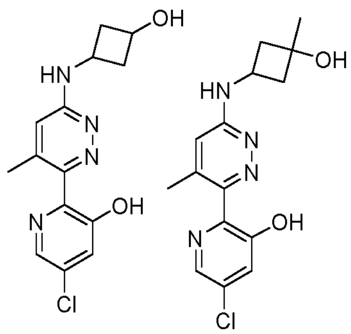
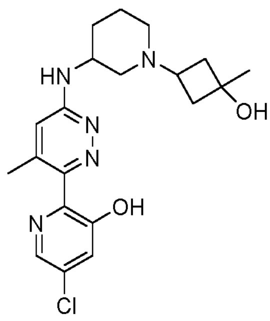
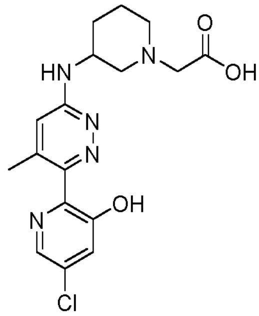
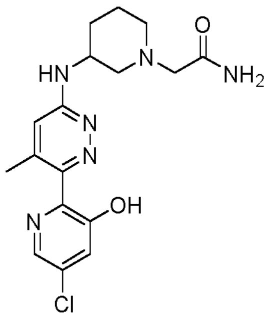

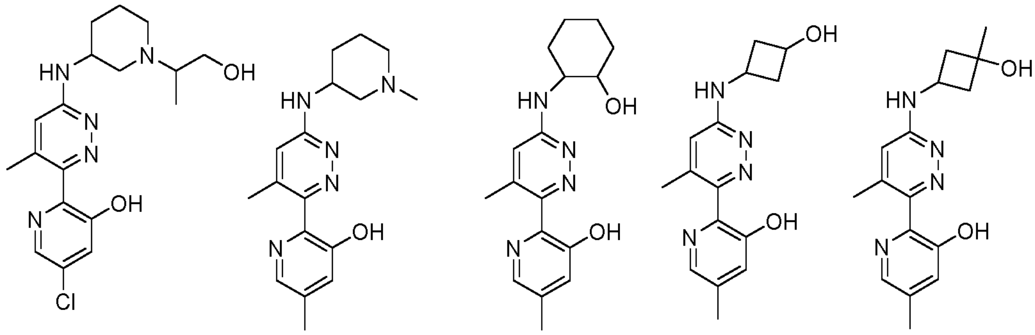

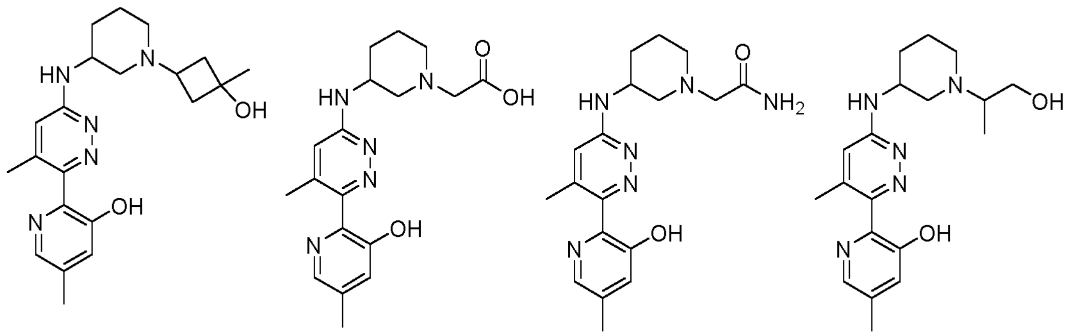

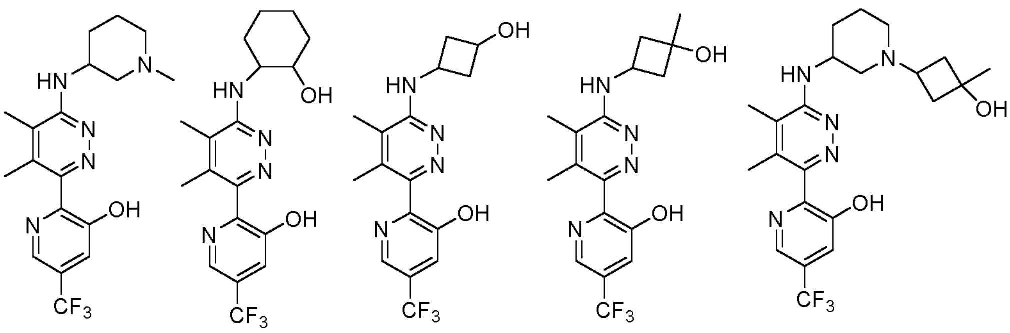

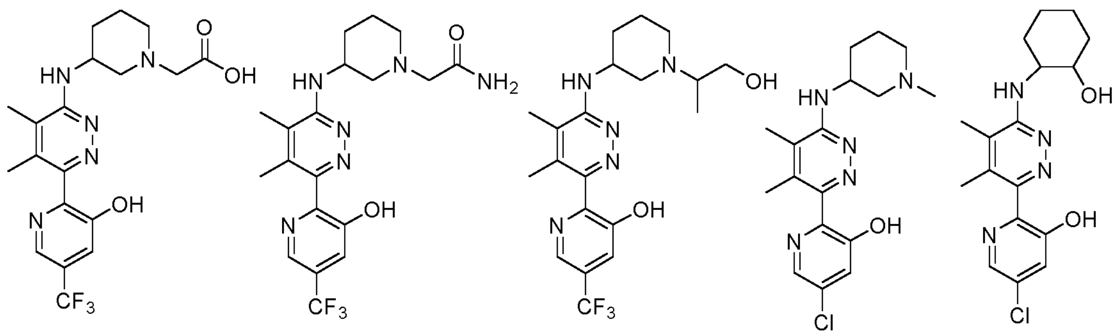

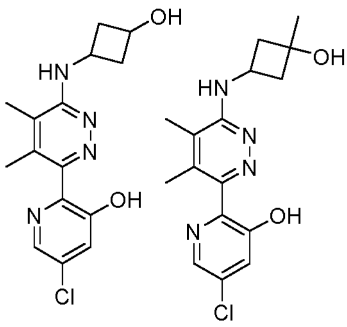

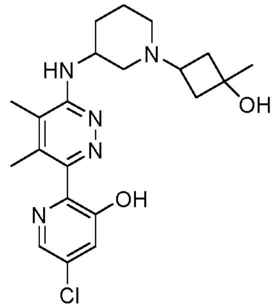

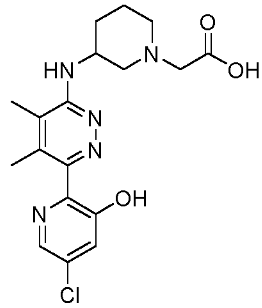

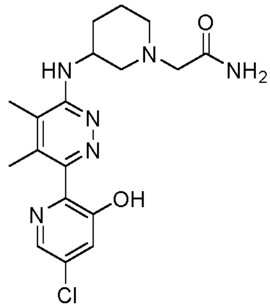

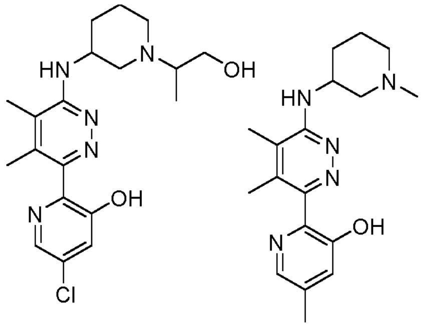

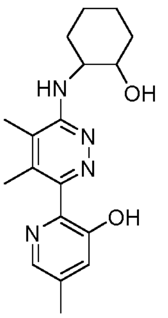

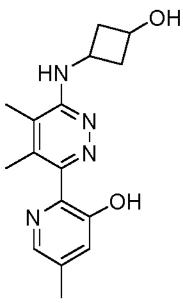

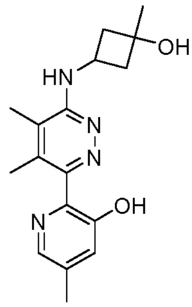

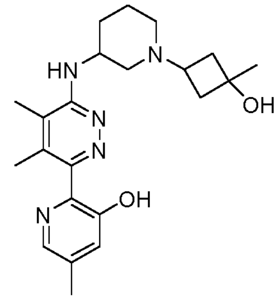

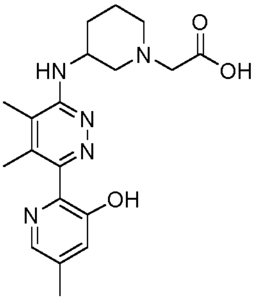

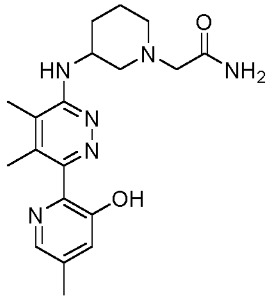

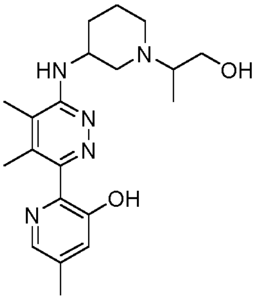

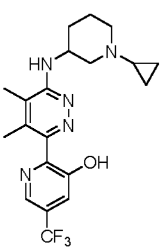

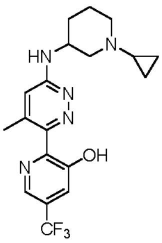

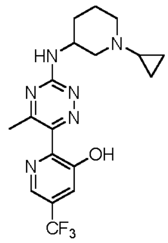

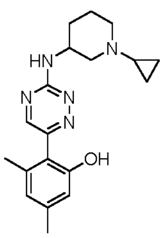

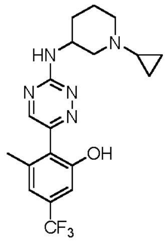

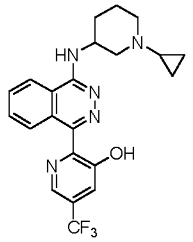

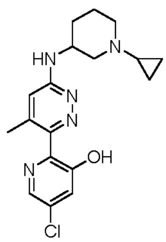

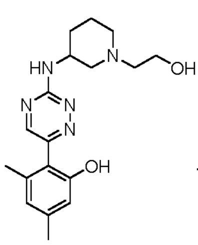

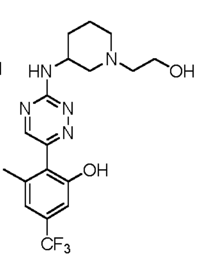

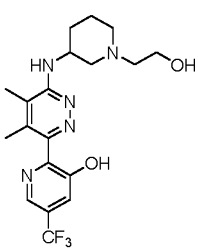

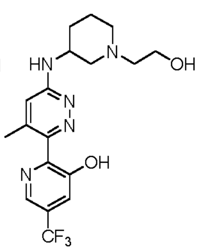

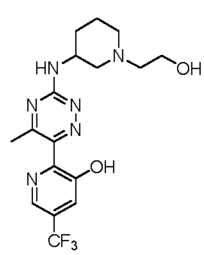

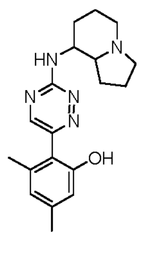
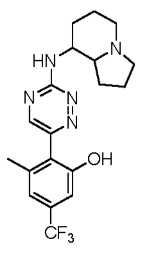
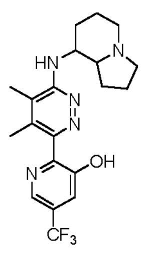
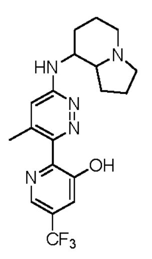
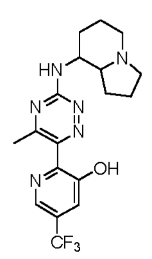
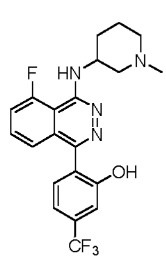
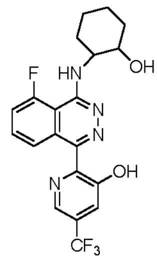

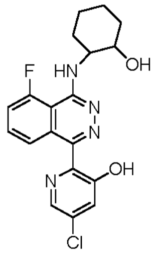
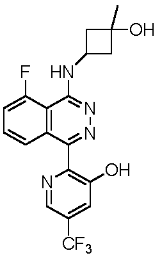
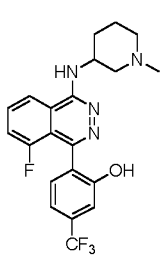
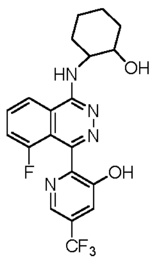
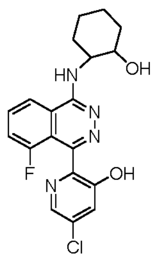
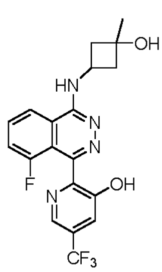
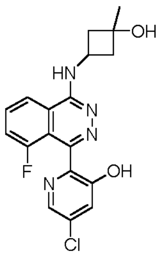

.

11. The compound represented by formula I, the solvate thereof, the pharmaceutically acceptable salt thereof or the solvate of the pharmaceutically acceptable salt thereof according to claim 1, **characterized in that** the compound shown in formula I can be any one of the following structures:

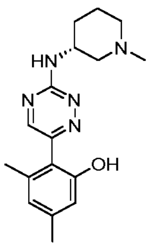
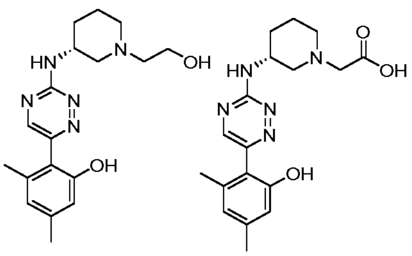
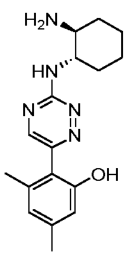
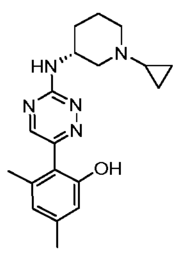
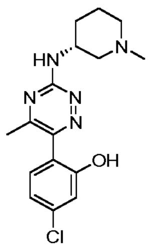
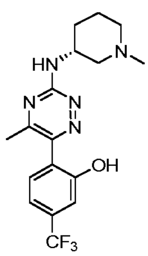

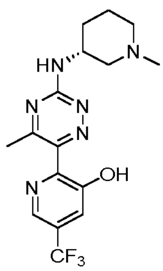
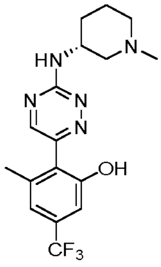
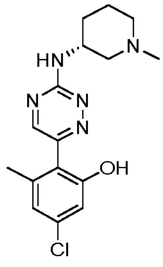
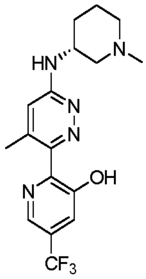
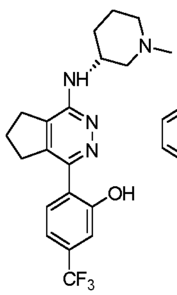
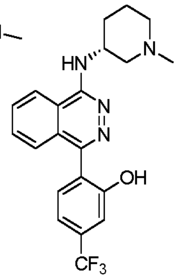
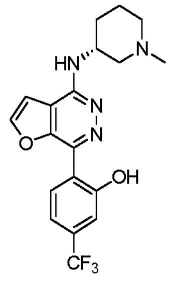

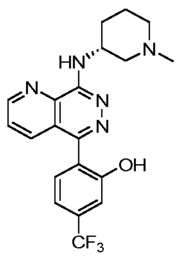
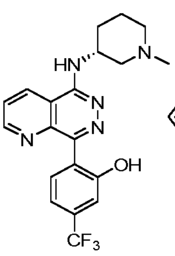
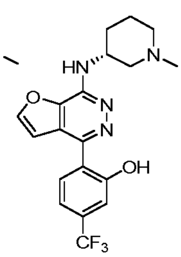
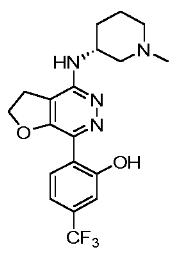
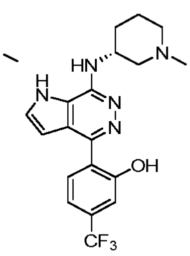
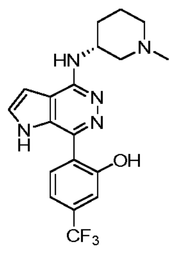
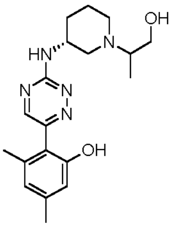

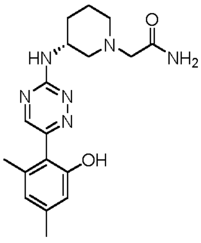 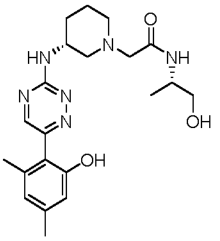 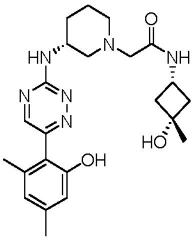 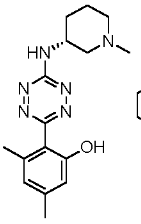 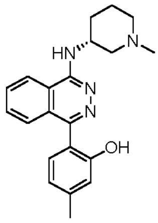 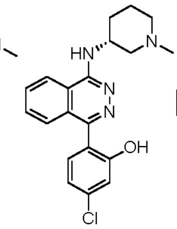 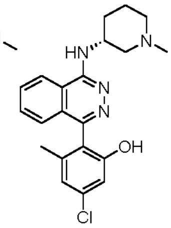

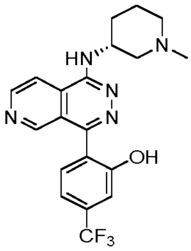 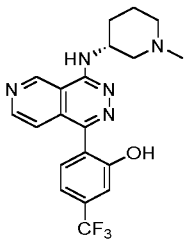 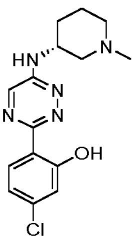 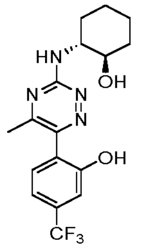 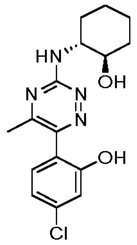 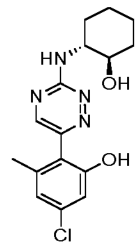 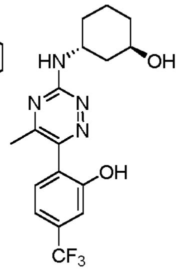

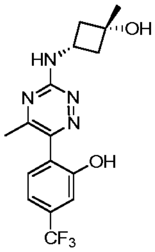 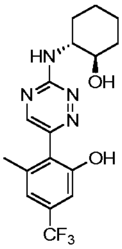 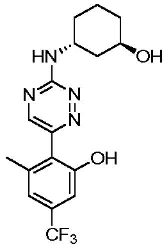 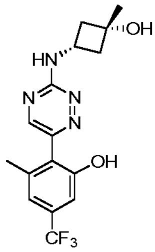 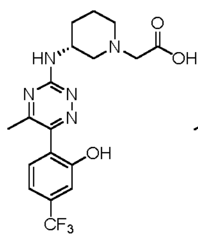 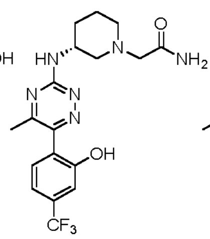 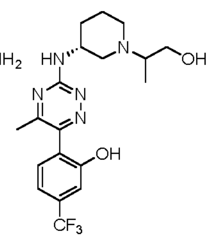

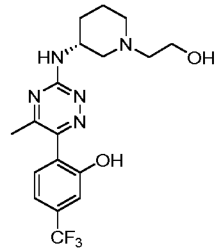 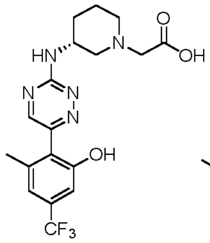 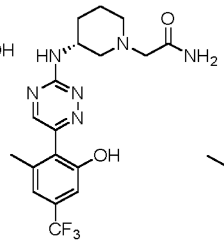 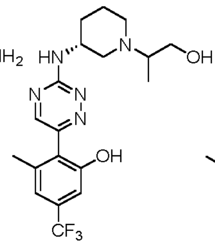 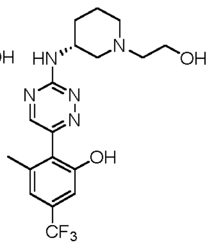 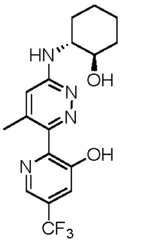

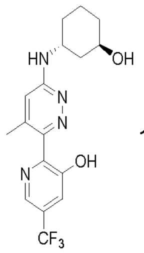 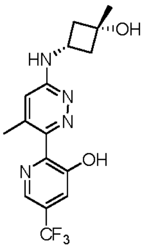 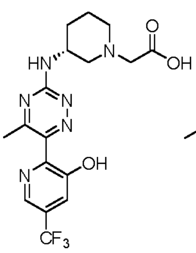 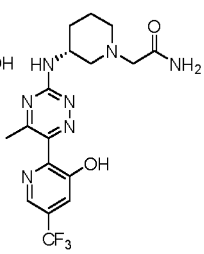 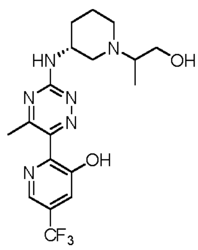 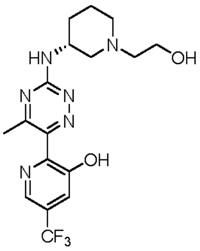 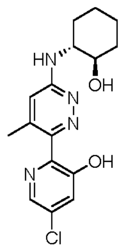

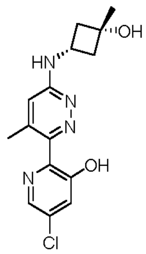
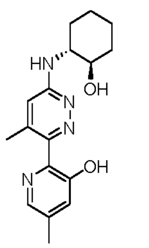
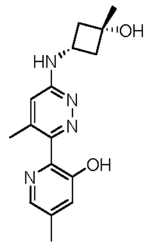
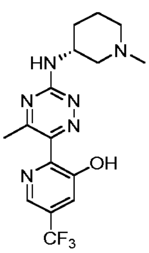
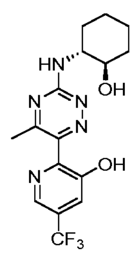
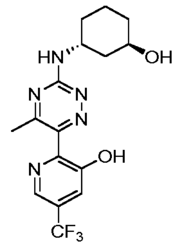
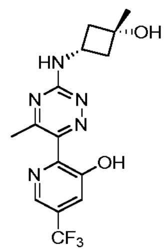

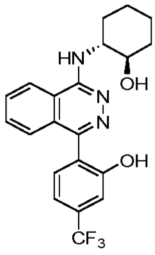
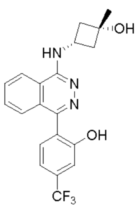
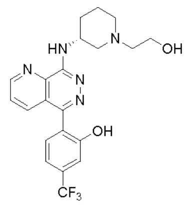
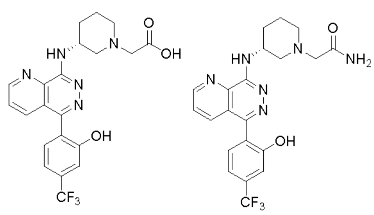
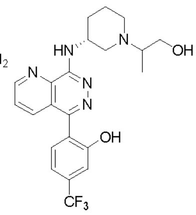
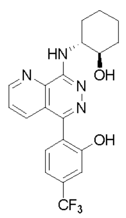

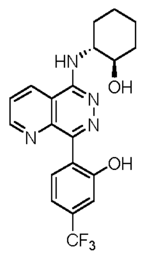
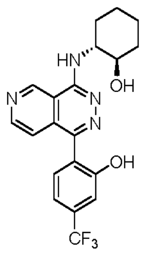
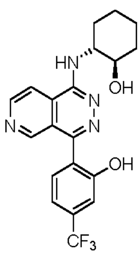
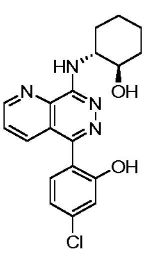
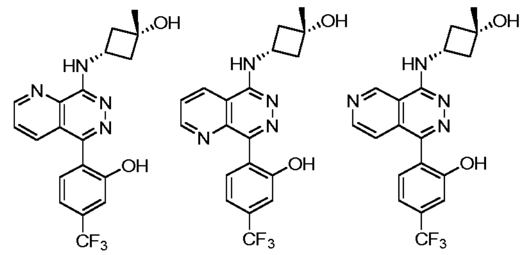

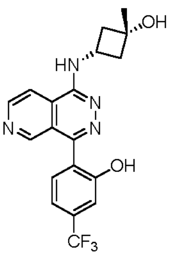
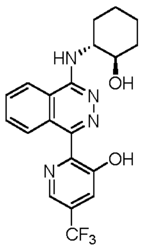
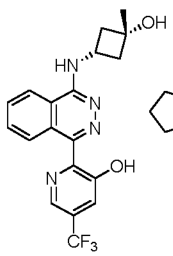
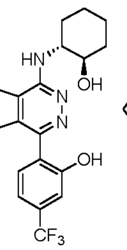
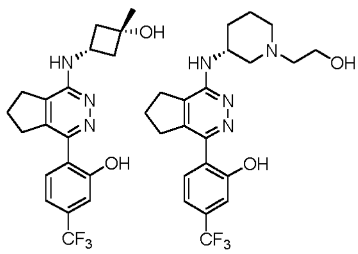
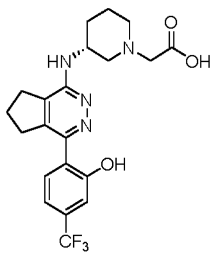

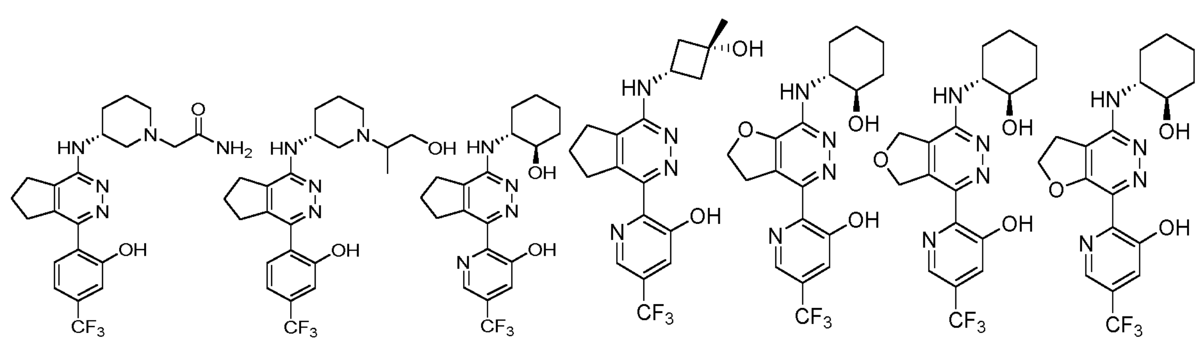

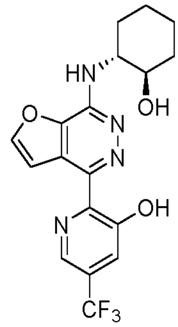
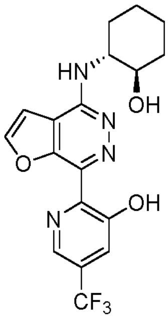
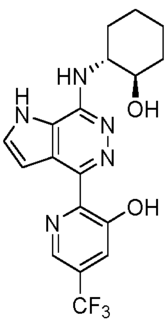
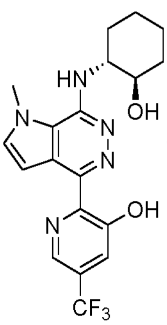
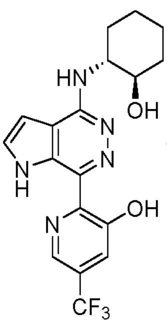
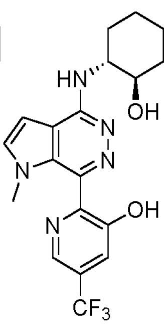
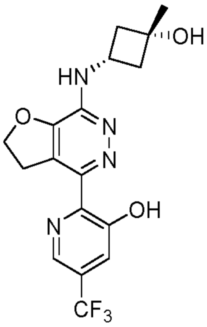

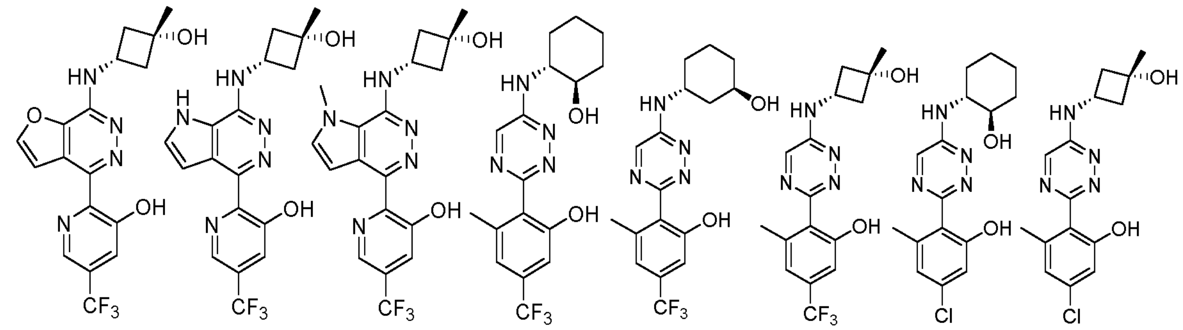

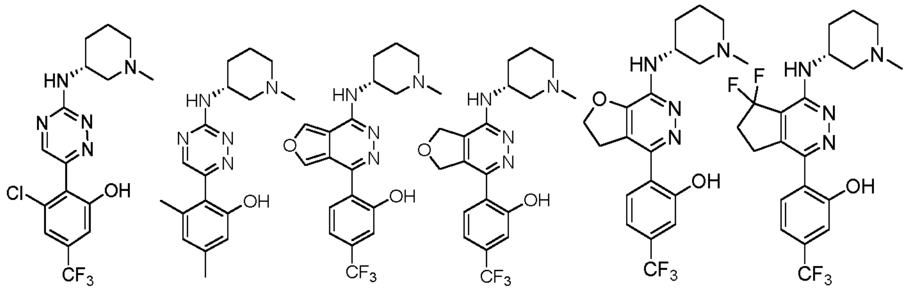

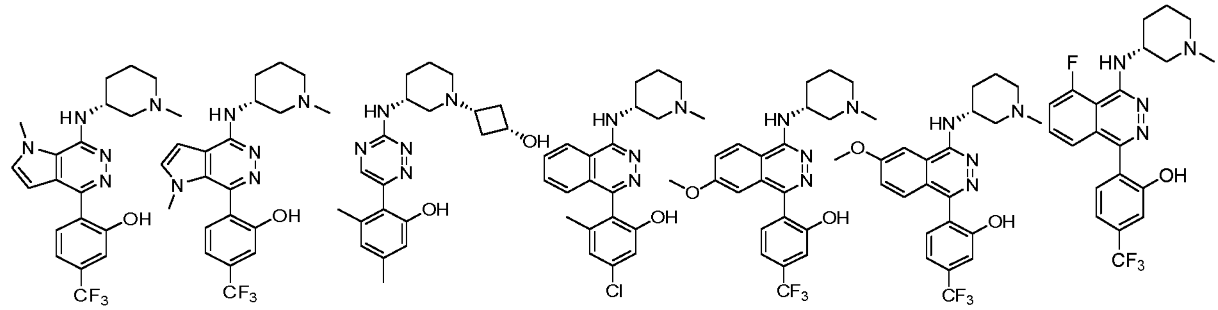

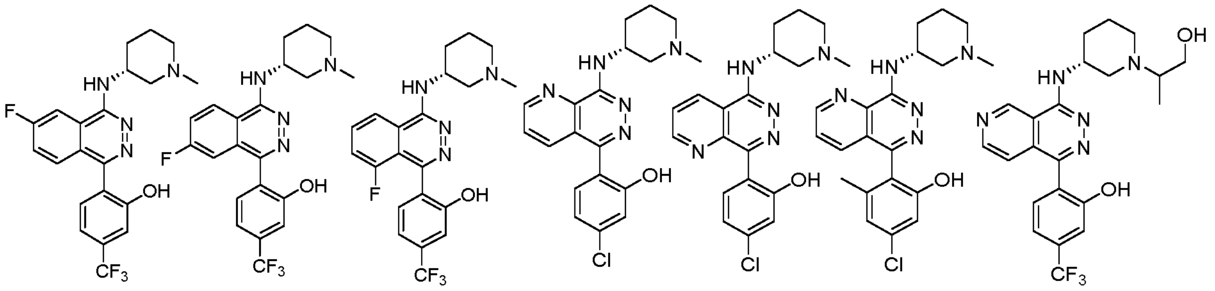

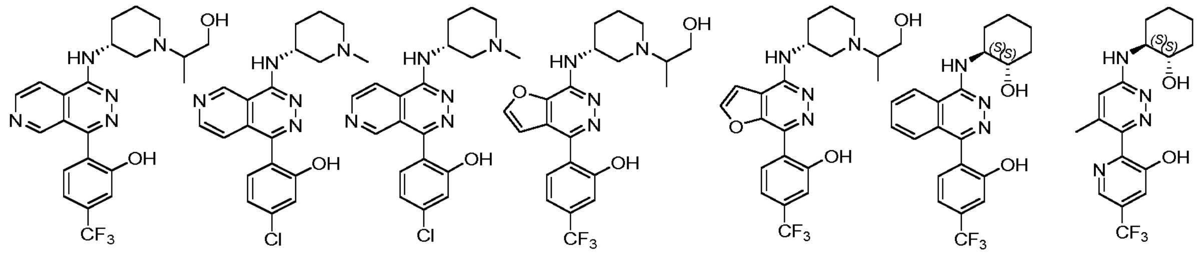

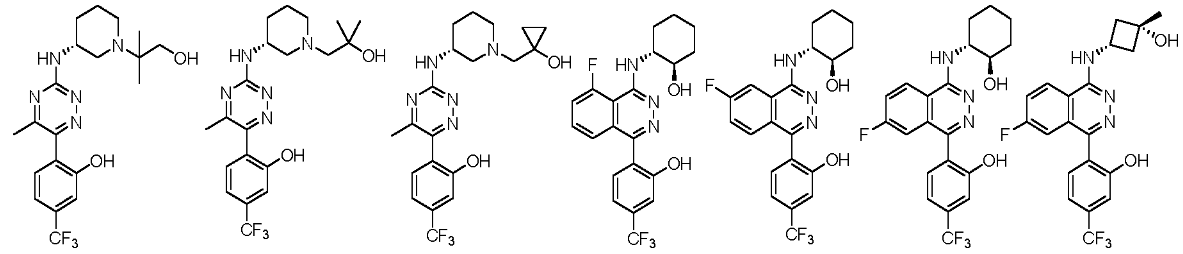

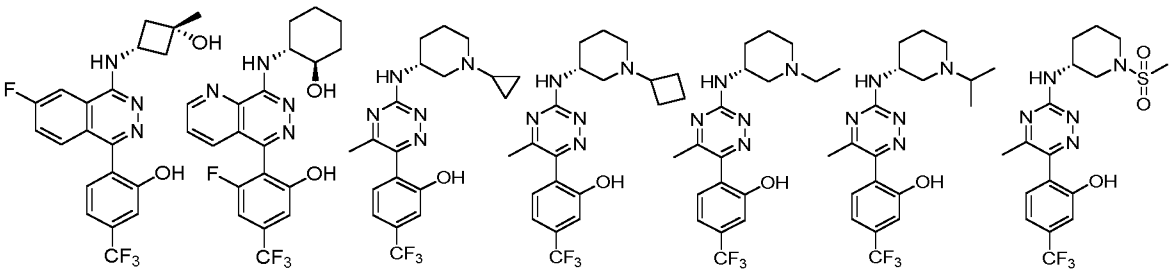

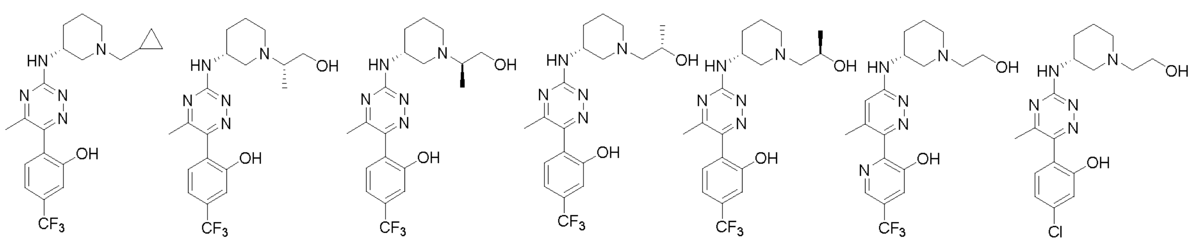

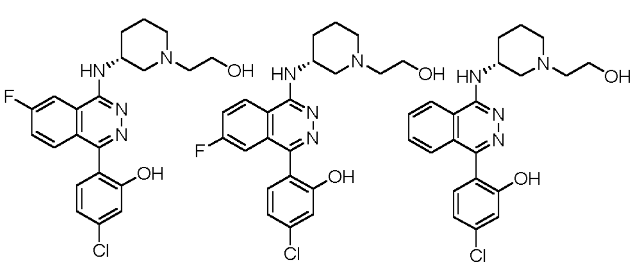

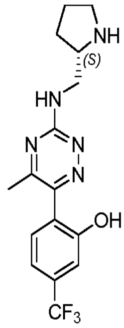

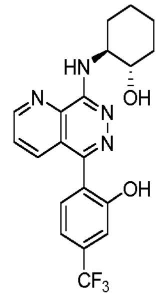

.

**12.** A preparation method for a compound as shown in formula I as at least one of claims 1~11, wherein it is any one of the following methods:

method 1:

it consists of the following steps: in a solvent, under the action of an acid, compound 1 undergoes a reaction as shown below;

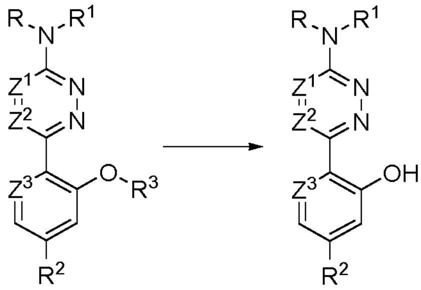

1 I ;

wherein, in the above formulas except for $R^3$, the definitions of the other substituents are as described in at least one of claims 1 to 8; $R^3$ is a conventional hydroxyl protecting group in the art, such as $C_1$~$C_6$ alkyl or $C_1$~$C_6$ alkoxy substituted $C_1$~$C_6$ alkyl;
the $C_1$~$C_6$ alkyl group is preferably $-CH_3$; The $C_1$~$C_6$ alkoxy substituted $C_1$~$C_6$ alkyl is preferably $-CH_2OCH_2CH_3$;
the solvent can be commonly used solvent in the art for such reactions, such as one or more of ester, alcohol and halogenated hydrocarbon; the ester solvent is preferably ethyl acetate; the alcohol solvent is preferably methanol; the halogenated hydrocarbon solvent is preferably dichloromethane;
the acid may be an acid commonly used in such reactions in the art, such as inorganic acid and/or lewis acid;
preferably, when $R^3$ is $C_1$~$C_6$ alkyl group, the solvent is a halogenated hydrocarbon solvent, and the acid is lewis acid;
preferably, when $R^3$ is $C_1$~$C_6$ alkoxy substituted $C_1$~$C_6$ alkyl, the solvent is an ester solvent and/or an alcohol solvent, and the acid is an inorganic acid;
the method 1 may further include the following reaction: in a solvent, under the action of base, compound 2 reacts with compound 3 to obtained the the compound 1;

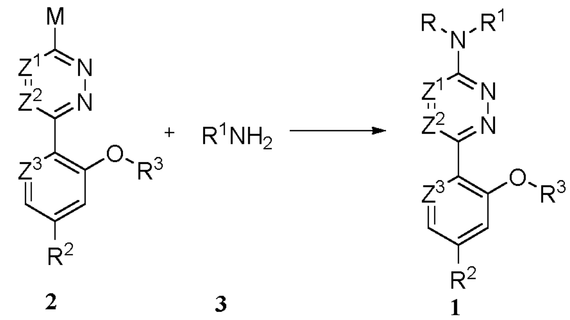

2 3 1 ;

where M is a halogen, such as F, Cl, Br or I, preferably Cl;
the solvent can be a commonly used solvent for such a reaction in the art, such as one or more of nitrogen-containing organic solvents, ether solvents or alcohol solvents, and more options include for example, one or more of N-methylpyrrolidone, n-butanol and dioxane;
the base may be a commonly used base for such reactions in the art, such as diisopropyl ethylamine;

method 2:

it consists of the following step: in a solvent, under the action of a base, compound 9 reacts with compound 3 to yield the compound 1 as shown below;

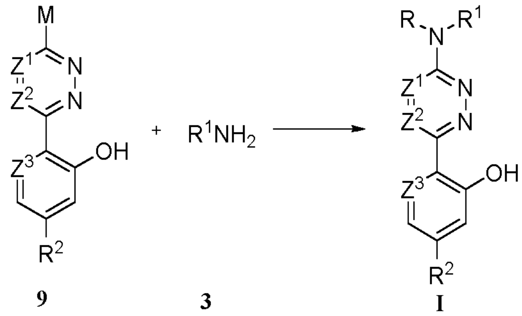

9 3 I ;

wherein, except for M, the definition of each of the above substituents is as described in at least one of claims 1-8; M is a halogen, such as F, Cl, Br or I, preferably Cl;
the solvent can be a commonly used solvent for such reactions in the art, such as N-methylpyrrolidone;
the base may be a commonly used base for such reactions in the art, such as diisopropyl ethylamine;

method 3:

it consists of the following step: in a solvent, under the action of a palladium catalyst and a base, compound 4 and compound 5 undergo the reaction as shown below;

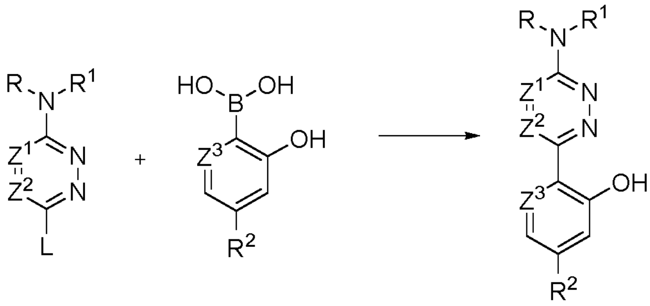

4 5 I ;

wherein, except for L, the definition of each of the above substituents is as described in at least one of claims 1-8; L is a halogen, such as F, Cl, Br or I, preferably Cl;
the solvent can be a commonly used solvent for such reaction in this art, such as ether solvent and/or water; preferably, the solvent is 1,4-dioxane and $H_2O$;
the palladium catalyst can be a palladium catalyst commonly used for such reactions in this art, such as $Pd(PPh_3)_4$;
the base can be a base commonly used in such reactions in the art, such as the carbonate of an alkali metal, preferably cesium carbonate.

**13.** A pharmaceutical composition, wherein it comprises substance X and a pharmaceutically acceptable excipient; the substance X is as described in at least one item of claims 1~11 as shown in formula I, a solvate thereof, a pharmaceutically acceptable salt or a solvate of a pharmaceutically acceptable salt.

**14.** An application of a substance X or a pharmaceutical composition as described in claim 13 in the preparation of NLRP3 inhibitors or drugs for the prevention and/or treatment of a disease related to NLRP3; the substance X is as described in at least one item of claims 1~11 as shown in formula I, a solvate thereof, a pharmaceutically acceptable salt or a solvate of a pharmaceutically acceptable salt.

**15.** The application according to claim 14, wherein the disease related to NLRP3 refers to a disease that responds to NLRP3 inhibition, and the disease is selected from the group consisting of cryopyrin-associated periodic syndrome (CAPS), Muckle-Wells syndrome (MWS), familial cold autoinflammatory syndrome (FCAS), neonatal-onset multisystemic inflammatory diseases (NOMID), multiple sclerosis (MS), amyotrophic lateral sclerosis, rheumatoid arthritis, psoriasis, Alzheimer's disease, Parkinson's disease, Non-alcoholic fatty liver disease, atherosclerosis, asthma,

COPD, pulmonary idiopathic fibrosis, chronic kidney disease, inflammatory bowel diseases, tumors, type 1 diabetes, type 2 diabetes, and gout.

**16.** A compound as shown in formula 1, 2, 3, 4, 5 or 9

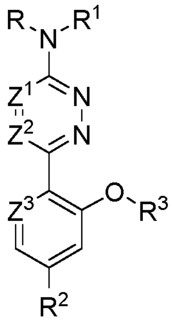
**1**

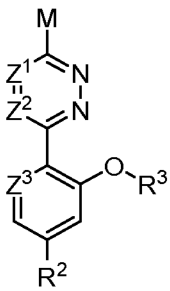
**2**

$R^1NH_2$
**3**

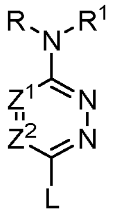
**4**

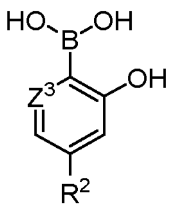
**5**

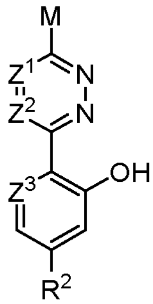
**9**

;

wherein, $Z^1$, $Z^2$, $Z^3$, R, $R^1$ and $R^2$ are defined as at least one of claims 1~8, and M, $R^3$ and L are defined as claim 12; preferably, the

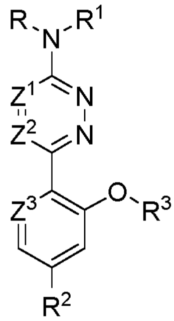
**1**

may be

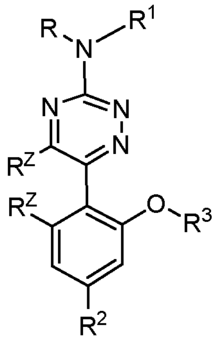
**6**

,

or may be

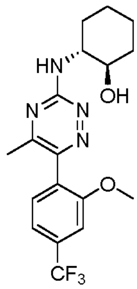 or 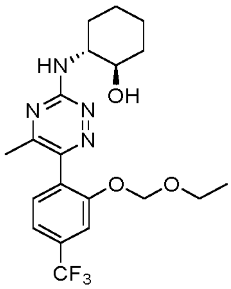 ;

preferably, the

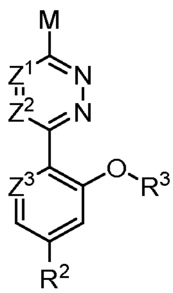

**2**

may be

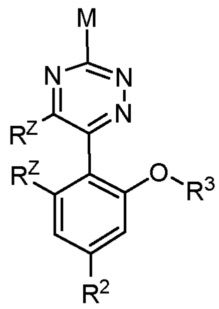

**7** ,

or may be

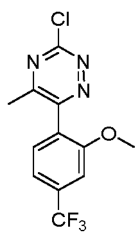 or 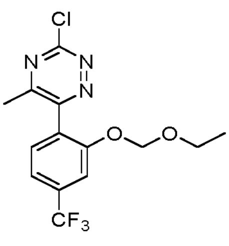 ;

preferably, the

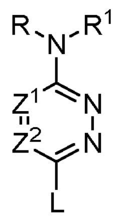

4

can be

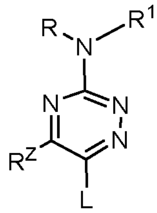

8

;

preferably, the

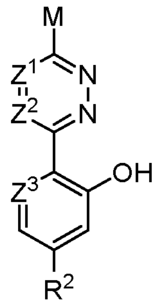

9

can be

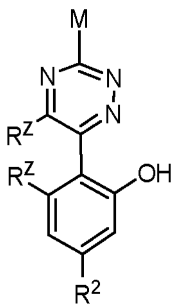

10

,

or can be

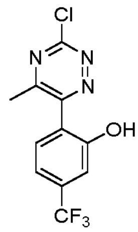

.

**17.** An application of a substance X or a pharmaceutical composition as described in claim 13 in the preparation of NLRP3 inhibitors or drugs for the prevention and/or treatment of inflammatory bowl diseases (IBD); the substance X is as described in at least one item of claims 1~11 as shown in formula I, a solvate thereof, a pharmaceutically acceptable salt or a solvate of a pharmaceutically acceptable salt.

## INTERNATIONAL SEARCH REPORT

International application No. **PCT/CN2022/126702**

### A. CLASSIFICATION OF SUBJECT MATTER

C07D 237/22(2006.01)i; C07D 237/34(2006.01)i; C07D 253/07(2006.01)i; C07D 401/12(2006.01)i; C07D 401/14(2006.01)i; C07D 403/12(2006.01)i; C07D 405/12(2006.01)i; C07D 471/04(2006.01)i; C07D 487/04(2006.01)i; C07D 491/048(2006.01)i; A61K 31/501(2006.01)i; A61K 31/502(2006.01)i; A61K 31/5025(2006.01)i; A61K 31/53(2006.01)i; A61P 29/00(2006.01)i; A61P 19/02(2006.01)i; A61P 17/00(2006.01)i

According to International Patent Classification (IPC) or to both national classification and IPC

### B. FIELDS SEARCHED

Minimum documentation searched (classification system followed by classification symbols)

C07D237/-; C07D253/-; C07D401/-; C07D403/-; C07D405/-; C07D471/-; C07D487/-; C07D491/-; A61K31/-; A61P29/-; A61P19/-; A61P17/-

Documentation searched other than minimum documentation to the extent that such documents are included in the fields searched

Electronic data base consulted during the international search (name of data base and, where practicable, search terms used)

WPI, CNPAT, CNKI, STN(REGISTRY): 核苷酸结合寡聚化结构域样受体蛋白3, 抑制剂, 氨基, 三嗪, 邻苯二甲嗪, 哒嗪, 酞嗪, 苯酚, 炎症, NLRP3, NOD-like receptor protein 3, amino, triazine, pyridazine, phthalazine, phenol, inflammasome, inflammatory, inhibitor, structural formula search

### C. DOCUMENTS CONSIDERED TO BE RELEVANT

| Category* | Citation of document, with indication, where appropriate, of the relevant passages | Relevant to claim No. |
|---|---|---|
| X | WO 2020234715 A1 (NOVARTIS AG.) 26 November 2020 (2020-11-26)<br>description, page 3, line 12 to page 140, line 1 | 1-17 |
| X | WO 2021193897 A1 (ASTELLAS PHARMA INC.) 30 September 2021 (2021-09-30)<br>description, paragraphs [0009]-[0133] | 1-17 |
| PX | WO 2022135567 A1 (TUOJIE BIOTECH (SHANGHAI) CO., LTD.) 30 June 2022 (2022-06-30)<br>description, page 1, line 18 to page 75, line 23 | 1-17 |
| PX | WO 2022166890 A1 (MEDSHINE DISCOVERY INC.) 11 August 2022 (2022-08-11)<br>description, page 1, line 24 to page 121, line 2 | 1-17 |
| PX | WO 2022216971 A1 (VENTUS THERAPEUTICS U.S., INC. et al.) 13 October 2022 (2022-10-13)<br>description, paragraphs [0006]-[0577] | 1-17 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 December 2022** | **28 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/126702**

**C. DOCUMENTS CONSIDERED TO BE RELEVANT**

| Category* | Citation of document, with indication, where appropriate, of the relevant passages | Relevant to claim No. |
|---|---|---|
| E | WO 2022230912 A1 (ASTELLAS PHARMA INC. et al.) 03 November 2022 (2022-11-03) description, paragraphs [0014]-[0192] | 1-17 |

Form PCT/ISA/210 (second sheet) (January 2015)

**INTERNATIONAL SEARCH REPORT**
**Information on patent family members**

International application No.
**PCT/CN2022/126702**

| Patent document cited in search report | Publication date (day/month/year) | Patent family member(s) | Publication date (day/month/year) |
|---|---|---|---|
| WO 2020234715 A1 | 26 November 2020 | DO P2021000228 A | 15 December 2021 |
| | | US 2020361899 A1 | 19 November 2020 |
| | | EC SP21080740 A | 30 December 2021 |
| | | CN 113784957 A | 10 December 2021 |
| | | US 2022251067 A1 | 11 August 2022 |
| | | BR 112021022796 A2 | 12 April 2022 |
| | | PE 20220133 A1 | 27 January 2022 |
| | | IL 287042 A | 01 December 2021 |
| | | JP 2022532354 A | 14 July 2022 |
| | | CA 3138226 A1 | 26 November 2020 |
| | | AU 2020277738 A1 | 18 November 2021 |
| | | KR 20220008887 A | 21 January 2022 |
| | | US 2020361898 A1 | 19 November 2020 |
| | | SG 11202111029 P A | 30 December 2021 |
| | | CO 2021015030 A2 | 30 November 2021 |
| | | EP 3969441 A1 | 23 March 2022 |
| | | TW 202110809 A | 16 March 2021 |
| | | AR 119731 A1 | 05 January 2022 |
| | | CR 20210552 A | 26 November 2021 |
| | | CU 20210094 A7 | 06 June 2022 |
| | | CL 2021003012 A1 | 09 September 2022 |
| WO 2021193897 A1 | 30 September 2021 | TW 202204323 A | 01 February 2022 |
| | | AR 121669 A1 | 29 June 2022 |
| WO 2022135567 A1 | 30 June 2022 | TW 202231281 A | 16 August 2022 |
| WO 2022166890 A1 | 11 August 2022 | None | |
| WO 2022216971 A1 | 13 October 2022 | None | |
| WO 2022230912 A1 | 03 November 2022 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111233736X
- CN 2022102735083
- WO 2021193897 A
- WO 2020234715 A
- WO 2018015445 A
- WO 2018215818 A


### Non-patent literature cited in the description


- **P. HEINRICH STAHL ; CAMILLE G. WERMUTH.** Handbook of Pharmaceutical Salts: Properties, Selection, and Use. 2011
- Pharmacopoeia of the People's Republic of China. 2020
- **PAUL J SHESKEY ; BRUNO C HANCOCK ; GARY P MOSS ; DAVID J GOLDFARB.** Handbook of Pharmaceutical Excipients. 2020